# EUROPEAN PATENT APPLICATION

(11) **EP 2 112 223 A2**
(43) Date of publication of application: **28.10.2009**
(21) Application number: 09009553.0
(22) Date of filing: 07.11.2006
(51) Int. Cl.: C12N 15/82, C07K 14/415

(54) **DOF (DNA binding with one finger) sequences and method of use**

(30) Priority: 10.11.2005 US 735645 P
(62) Divisional of application: 06837094.9
(71) Applicant: Pioneer Hi-Bred International Inc., Johnston, IA 50131-1014 (US)
(72) Inventor: Gupta, Rajeev, Johnston, IA 50131 (US); Liu, Juan, Johnston, IA 50131 (US); Dhugga, Kanwarpal S., Johnson, IA 50131 (US); Simmons, Carl R., Des Moines, IA 50310 (US)
(74) Representative: Bentham, Andrew

(57) **Abstract**

Methods and compositions are provided to improve nitrogen use efficiency in plants or plant parts, increase carbon fixation in a plant or plant part, increase grain yield or biomass production of the plant, and/or increase the stress tolerance of the plant. The compositions and methods of the invention modulate these various phenotypes by modulating the level of at least one Dof (for DNA binding with one finger) polypeptide having a Dof domain or a biologically active variant or fragment of a Dof domain.

## Description

### FIELD OF THE INVENTION

The present invention is drawn to the field of genetics and molecular biology. More particularly, the compositions and methods are directed to modulation of carbon fixation, improving nitrogen use, improving yield and improving stress tolerance in plants.

### BACKGROUND OF THE INVENTION

Grain yield improvements by conventional breeding have nearly reached a plateau in maize. It is natural then to explore some alternative, non-conventional approaches that could be employed to obtain further yield increases. Since the harvest index in maize has remained essentially unchanged during selection for grain yield over the last hundred or so years, the yield improvements have been realized from the increased total biomass production per unit land area (Sinclair, et al., (1998) Crop Science 38:638-643; Duvick, et al., (1999) Crop Science 39:1622-1630; and, Tollenaar, et al., (1999) Crop Science 39:1597-1604). This increased total biomass has been achieved by increasing planting density, which has led to adaptive phenotypic alterations, such as a reduction in leaf angle and tassel size, the former to reduce shading of lower leaves and the latter perhaps to increase harvest index (Duvick, et al., (1999) Crop Science 39:1622-1630).

Carbon fixation and nitrogen assimilation are two of the key processes that limit biomass production (Sinclair, et al., (1975) Science 189:565-567; Bhatia, et a/,. (1976) Science 194:1418-1421; Dhugga, et al,. (1989) Crop Sci. 29:1232-1239; and Sinclair, et al., (1998) Crop Science 38:638-643). The energetic cost of making protein using nitrate nitrogen, which is the main form of nitrogen acquired from the soil in maize, from a unit of photosynthate is approximately twice of that needed to make carbohydrates (Penning, et al., (1974) J. Theor. Biol. 45:339-377 and Sinclair, et al., (1975) Science 189:565-567). In agreement with this, protein concentration in the maize grain has gone down as a result of selection for grain yield at higher planting densities (Duvick, et al., (1999) Crop Science 39:1622-1630). Ideally, grain yield is maximized with minimal amount of applied nitrogen. Aside from increasing fertilizer prices, run-off and leached nitrate cause undesirable environmental effects (Frink, et al., (1999) PNAS 96:1175-1180). Whereas one of the open avenues is to select for reduced grain protein content that might be reflected in increased grain yield because of accumulation of a greater amount of carbohydrates, the other is to increase the rate of photosynthesis (Leakey, et al., (2004) Global Change Biology 10:951-962).

Given the complexity of the metabolic pathways, it is unlikely that single gene alterations will prove fruitful in improving biomass production (Morandini, et al., (2003) Trends in Plant Science 8:70-75). However, synchronous improvement in different components of a whole pathway might allow overcoming, at least to some extent, the complexity of the metabolic pathways. Upstream regulators of gene expression could help accomplish this goal (Morandini, et al., (2003) Trends in Plant Science 8:70-75). A single upstream 'master-regulatory' gene, for example, may be utilized to alter the expression of multiple metabolic genes in a pathway (Rabinowicz, et al., (1999) Genetics 153:427-444; DellaPenna (2001) Plant Physiol 125:160-153; Morandini, et al., (2003) Trends in Plant Science 8:70-75). These types of genes are referred to as transcription factors (TF). TF operate at a higher level of molecular hierarchy and play key roles in various biological processes. This is obvious from the fact that approximately 5% of *Arabidopsis* genome encodes TF (∼1500), which are classified into approximately 50 different groups based on the DNA-binding domains that are specific to each group (Riechmann, et al., (2000) Science 290:2105-2110).

Methods and compositions are needed in the art which can employ such master regulatory sequence to modulate carbon fixation and nitrogen assimilation in plants.

### BRIEF SUMMARY OF THE INVENTION

Compositions of the invention comprise isolated polypeptides comprising an amino acid sequence selected from the group consisting of the amino acid sequence comprising SEQ ID NO: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127, 130, 133, 135, 138, 141, 144, 154, 155, 156, 157, 158, 159 or 160 or a variant or fragment thereof.

Compositions also comprise isolated polynucleotides comprising a nucleotide sequence selected from the group consisting of the nucleotide sequence comprising SEQ ID NO: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152 or 153 or a variant or fragment thereof.

Expression cassettes, plants, plant cells, plant parts and seeds comprising these sequences are further provided. In specific embodiment, the polynucleotide is operably linked to a tissue-preferred promoter including, but not limited to, a leaf-preferred promoter, a mesophyll-preferred promoter, a bundle sheath-preferred promoter, a vascular-preferred promoter, a seed-preferred promoter, an endosperm-preferred promoter, or an embryo-preferred promoter.

Methods for modulating the level of a Dof polypeptide in a plant or a plant part are provided. The methods comprise introducing into a plant or plant part a heterologous polynucleotide comprising a Dof sequence of the invention. The level of the Dof polypeptide can be increased or decreased. Such method can be used to increase the yield in plants, increase the nitrogen use efficiency of a plant, and/or improve the stress response of the plant.

Further compositions of the invention comprise isolated expression cassettes comprising a polynucleotide operably linked to a leaf-preferred promoter or a vascular-preferred promoter, wherein the polynucleotide is selected from the group consisting of (a) a polynucleotide encoding a Dof polypeptide comprising an amino acid sequence set forth in SEQ ID NO: 145; (b) a polynucleotide encoding a Dof polypeptide comprising an amino acid sequence having at least 80% sequence identity to SEQ ID NO: 145, wherein the Dof polypeptide is capable of modulating transcription; (c) a polynucleotide which when expressed in a plant decrease the expression level of a Dof polypeptide comprising an amino acid sequence set forth in SEQ ID NO: 145; and, (d) a polynucleotide which when expressed in a plant decrease the expression level of a Dof polypeptide comprising an amino acid sequence having at least 80% sequence identity to SEQ ID NO: 145, wherein the Dof polypeptide is capable of modulating transcription. Plants, plant parts, cells, and seeds having this expression cassette are also provided.

Further provide are methods for increasing nitrogen efficiency in a plant, increasing yield in a plant, and improving the stress response of a plant. Such methods comprise introducing into the plant a heterologous polynucleotide; and, expressing the polynucleotide in the plant from an operably linked leaf-preferred promoter or a vascular preferred promoter. In such methods, the expression of the heterologous polynucleotide modulates the level of at least one Dof polypeptide comprising an amino acid sequence set forth in SEQ ID NO: 145 or a biologically active variant or fragment thereof.

### BRIEF DESCRIPTION OF THE DRAWINGS

Figure 1 provides an alignment of characterized Dof domains from various Dof polypeptides from rice.
Figure 2A and 2B provide an alignment of the Dof domain from the various members of the maize Dof family. Conserved regions are highlighted. The consensus Dof domain (SEQ ID NO: 145) is set forth above the alignment.

### DETAILED DESCRIPTION OF THE INVENTION

The present invention will be described more fully hereinafter with reference to the accompanying drawings, in which some, but not all embodiments of the inventions are shown. Indeed, this invention may be embodied in many different forms and should not be construed as limited to the embodiments set forth herein; rather, these embodiments are provided so that this disclosure will satisfy applicable legal requirements. Like numbers refer to like elements throughout.

Many modifications and other embodiments of the invention set forth herein will come to mind to one skilled in the art to which this invention pertains having the benefit of the teachings presented in the foregoing descriptions. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### I. Overview

Methods and compositions are provided to improve nitrogen use efficiency in plants or plant parts, increase carbon fixation in a plant or plant part, increase yield or biomass production of the plant, and/or increase the stress tolerance of the plant. The compositions and methods of the invention modulate these various phenotypes by modulating in a plant the level of at least one Dof polypeptide having a Dof domain or a polypeptide having a biologically active variant or fragment of a Dof domain.

### II. Compositions

### A. Dof Polynucleotides and Polypeptides

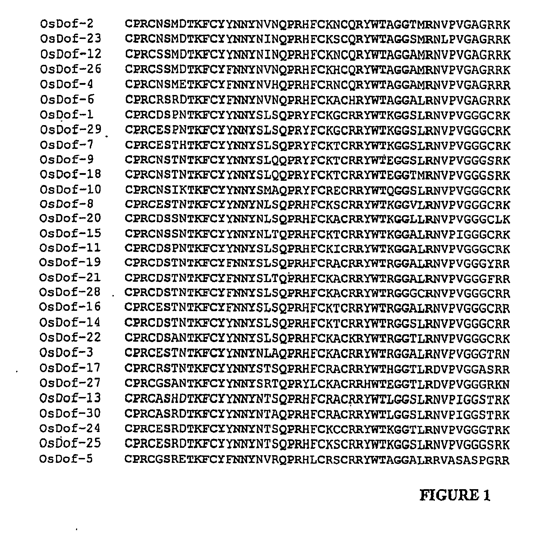

Compositions of the invention include Dof polynucleotides and polypeptides and variants and fragments thereof that are involved in regulating transcription. Dof (for DNA binding with one finger) is a family of DNA binding proteins that have been found in diverse plant species. Members of the Dof family comprise a Dof domain or an active variant or fragment thereof, which is a highly conserved amino acid sequence involved in DNA binding. The Dof domain is characterized by a conserved region of about 50 amino acids with a C2-C2 finger structure, associated with a basic region. The basic region of specific members of the Dof family can bind to DNA sequences with a 5'-T/AAAAG-3' core. See, for example, Lijavetzky, et al., (2003) BMC Evolutionary Biology 3:17 and Yanagisawa, et al., (1999) Plant J. 17:209. Figure 1 provides a sequence alignment of Dof domains from several characterized Dof polypeptides. The consensus sequence for the Dof domain is set forth in SEQ ID NO: 145.

As used herein, a "Dof" sequence comprises a polynucleotide encoding or a polypeptide having the conserved Dof domain or a biologically active variant or fragment of the Dof domain. The consensus Dof domain is as follows: C-P-R-C-X-S-X-[DHN]-T-K-F-C-Y-[FY]-N-N-Y-[NS]-X-X-Q-P-R-[HY]-[FL]-C-[KR]-X-C-[RKQH]-R-[YH]-W-T-X-G-G-[TASV]-[LMI]-R (shaded residues are highly conserved among Dof members, X represents any amino acid, and [] surronds the recited amino acids that can be found in that position). SEQ ID NO: 145 sets forth this conserved domain. It is recognized, however, that the conserved sequences set forth in the Dof domain consensus sequence can be altered and still retain Dof activity (i.e., the ability to modulate transcription). See, for example, Yanagisawa, et al., (2001) Plant Cell Physiol. 42:813-22, and Lijavetzky, et al., (2003) BMC Evolutionary Biology 3:17 and Figure 1. Table 2 also provides representative Dof domains from various maize Dof polypeptides. Biologically active fragments and variants of a Dof domain will continue to retain the ability to modulate transcription when the domain is placed within the context of an appropriate polypeptide.

In one embodiment, the present invention provides isolated Dof polypeptides comprising amino acid sequences as shown in SEQ ID NOS: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127, 130, 133, 135, 138, 141, 144, 154, 155, 156, 157, 158, 159 or 160. Further provided are polynucleotides comprising the nucleotide sequence set forth in SEQ ID NO: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44; 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152 or 153. The conserved Dof domains in SEQ ID NOS: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127, 130, 133, 135, 138, 141, 144, 154, 155, 156, 157, 158, 159 or 160 are outlined in Table 1.

The invention encompasses isolated or substantially purified polynucleotide or protein compositions. An "isolated" or "purified" polynucleotide or protein, or biologically active portion thereof, is substantially or essentially free from components that normally accompany or interact with the polynucleotide or protein as found in its naturally occurring environment. Thus, an isolated or purified polynucleotide or protein is substantially free of other cellular material, or culture medium when produced by recombinant techniques, or substantially free of chemical precursors or other chemicals when chemically synthesized. Optimally, an "isolated" polynucleotide is free of sequences (optimally protein encoding sequences) that naturally flank the polynucleotide (i.e., sequences located at the 5' and 3' ends of the polynucleotide) in the genomic DNA of the organism from which the polynucleotide is derived. For example, in various embodiments, the isolated polynucleotide can contain less than about 5 kb, 4 kb, 3 kb, 2 kb, 1 kb, 0.5 kb or 0.1 kb of nucleotide sequence that naturally flank the polynucleotide in genomic DNA of the cell from which the polynucleotide is derived. A protein that is substantially free of cellular material includes preparations of protein having less than about 30%, 20%, 10%, 5% or 1% (by dry weight) of contaminating protein. When the protein of the invention or biologically active portion thereof is recombinantly produced, optimally culture medium represents less than about 30%, 20%, 10%, 5% or 1% (by dry weight) of chemical precursors or non-protein-of-interest chemicals.

Fragments and variants of the Dof domain or Dof polynucleotides and' proteins encoded thereby are also encompassed by the methods and compositions of the present invention. By "fragment" is intended a portion of the polynucleotide or a portion of the amino acid sequence. Fragments of a polynucleotide may encode protein fragments that retain the biological activity of the native protein and hence regulate transcription. Alternatively, fragments that are used for suppressing or silencing (i.e., decreasing the level of expression) of a Dof sequence need not encode a protein fragment, but will retain the ability to suppress expression of the target Dof sequence. In addition, fragments that are useful as hybridization probes generally do not encode fragment proteins retaining biological activity. Thus, fragments of a nucleotide sequence may range from at least about 18 nucleotides, about 20 nucleotides, about 50 nucleotides, about 100 nucleotides and up to the full-length polynucleotide encoding the proteins of the invention.

A fragment of a polynucleotide encoding a Dof domain or a Dof polypeptide will encode at least 15, 25, 30, 50, 100, 150, 200, 250, 275, 300, 352, 350, 375, 400, 425, 450, 475, 480 contiguous amino acids or up to the total number of amino acids present in a full-length Dof domain or Dof protein (i.e., SEQ ID NO: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127, 130, 133, 135, 138, 141, 144, 154, 155, 156, 157, 158, 159 or 160). Fragments of a Dof domain or a Dof polynucleotide that are useful as hybridization probes, PCR primers, or as suppression constructs generally need not encode a biologically active portion of a Dof protein or a Dof domain.

A biologically active portion of a Dof domain or a Dof protein can be prepared by isolating a portion of a Dof polynucleotide, expressing the encoded portion of the Dof protein (e.g., by recombinant expression *in vitro*), and assessing the activity of the encoded portion of the Dof protein. Polynucleotides that are fragments of a Dof domain or a Dof nucleotide sequence comprise at least 16, 20, 50, 75, 100, 150, 200, 250, 300, 350, 400, 450, 500, 550, 600, 650, 700, 800, 900, 1,000, 1,100, 1,200, 1,300, 1,400, 1,500, 1,600, 1,700, 1,800, 1,900, 2,000, 2,050, 2,080 contiguous nucleotides or up to the number of nucleotides present in a full-length Dof domain or in a Dof polynucleotide (i.e., SEQ ID NOS: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152 or 153).

"Variants" is intended to mean substantially similar sequences. For polynucleotides, a variant comprises a deletion and/or addition of one or more nucleotides at one or more internal sites within the native polynucleotide and/or a substitution of one or more nucleotides at one or more sites in the native polynucleotide. As used herein, a "native" polynucleotide or polypeptide comprises a naturally occurring nucleotide sequence or amino acid sequence, respectively. For polynucleotides, conservative variants include those sequences that, because of the degeneracy of the genetic code, encode the amino acid sequence of one of the Dof polypeptides or of a Dof domain. Naturally occurring allelic variants such as these can be identified with the use of well-known molecular biology techniques, as, for example, with polymerase chain reaction (PCR) and hybridization techniques as outlined below. Variant polynucleotides also include synthetically derived polynucleotide, such as those generated, for example, by using site-directed mutagenesis but which still encode a Dof domain or a Dof polypeptide that is capable of regulating transcription or that is capable of reducing the level of expression (i.e., suppressing or silencing) of a Dof polynucleotide. Generally, variants of a particular polynucleotide of the invention will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular polynucleotide as determined by sequence alignment programs and parameters described elsewhere herein.

Variants of a particular polynucleotide of the invention (i.e., the reference polynucleotide) can also be evaluated by comparison of the percent sequence identity between the polypeptide encoded by a variant polynucleotide and the polypeptide encoded by the reference polynucleotide. Thus, for example, an isolated polynucleotide that encodes a polypeptide with a given percent sequence identity to the polypeptide of SEQ ID NO: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127, 130, 133, 135, 138, 141, 144, 154, 155, 156, 157, 158, 159 or 160 are disclosed. Percent sequence identity between any two polypeptides can be calculated using sequence alignment programs and parameters described elsewhere herein. Where any given pair of polynucleotides of the invention is evaluated by comparison of the percent sequence identity shared by the two polypeptides they encode, the percent sequence identity between the two encoded polypeptides is at least about 40%, 45%, 50%, 55%, 60%, 65%, 70%, 75%, 80%, 85%, 90%, 91%, 92%, 93%, 94%, 95%, 96%, 97%, 98%, 99% or more sequence identity.

"Variant" protein is intended to mean a protein derived from the native protein by deletion or addition of one or more amino acids at one or more internal sites in the native protein and/or substitution of one or more amino acids at one or more sites in the native protein. Variant proteins encompassed by the present invention are biologically active, that is they continue to possess the desired biological activity of the native protein, that is, regulate transcription as described herein. Such variants may result from, for example, genetic polymorphism or from human manipulation. Biologically active variants of a Dof protein of the invention or of a Dof domain will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to the amino acid sequence for the Dof protein or the consensus Dof domain as determined by sequence alignment programs and parameters described elsewhere herein. A biologically active variant of a Dof protein of the invention or of a Dof domain may differ from that protein by as few as 1-15 amino acid residues, as few as 1-10, such as 6-10, as few as 5, as few as 4, 3, 2 or even 1 amino acid residue.

The polynucleotides of the invention may be altered in various ways including amino acid substitutions, deletions, truncations, and insertions. Methods for such manipulations are generally known in the art. For example, amino acid sequence variants and fragments of the Dof proteins or Dof domains can be prepared by mutations in the DNA. Methods for mutagenesis and polynucleotide alterations are well known in the art. See, for example, Kunkel (1985) Proc. Natl. Acad. Sci. USA 82:488-492; Kunkel, et al., (1987) Methods in Enzymol. 154:367-382; U.S. Patent No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest may be found in the model of Dayhoff, et al., (1978) Atlas of Protein Sequence and Structure (Natl. Biomed. Res. Found., Washington, D.C.), herein incorporated by reference. Conservative substitutions, such as exchanging one amino acid with another having similar properties, may be optimal.

Thus, the genes and polynucleotides of the invention include both the naturally occurring sequences as well as mutant forms. Likewise, the proteins of the invention encompass both naturally occurring proteins as well as variations and modified forms thereof. Such variants will continue to possess the desired activity (i.e., the ability to regulate transcription or decrease the level of expression of a target Dof sequence). In specific embodiments, the mutations that will be made in the DNA encoding the variant does not place the sequence out of reading frame and does not create complementary regions that could produce secondary mRNA structure. See, EP Patent Application Publication No. 75,444.

The deletions, insertions, and substitutions of the protein sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. For example, the activity of a Dof polypeptide can be evaluated by assaying for the ability of the polypeptide to regulate transcription. Various methods can be used to assay for this activity, including, directly monitoring the level of expression of a target gene at the nucleotide or polypeptide level. Methods for such an analysis are known and include, for example, Northern blots, S1 protection assays, Western blots, enzymatic or colorimetric assays. In specific embodiments, determining if a sequence has Dof activity can be assayed by monitoring for an increase or decrease in the level or activity of target genes, including various enzymes in the carbon fixation and nitrogen assimilation pathways. For example, in specific embodiments, a Dof sequence can modulate transcription of target genes such as the phophoenolpyruvate carboxylase gene, the cytoplasmic pyruvate ortho-phosphate dikinase gene, nitrate reductase, glutamine synthase, glutamate synthase, glutamate dehydrogenase, isocitrate dehydrogenase, and asparagines synthase. See, for example, Yanagisawa, et al., (2002) Trends in Plant Science 7:555-560 and Yanagisawa, et al., (2000) Plant J. 21:281-288, both of which are herein incorporated by reference. Alternatively, methods to assay for a modulation of transcriptional activity can include monitoring for an alteration in the phenotype of the plant. For example, as discussed in further detail elsewhere herein, modulating the level of a Dof polypeptide can result in increased carbon fixation, improved nitrogen use efficiency and grain yield, and improved tolerance of the plant to environmental stress, including abiotic stresses such as drought, heat, and nitrogen stress. Methods to assay for these changes are discussed in further detail elsewhere herein.

Variant polynucleotides and proteins also encompass sequences and proteins derived from a mutagenic and recombinogenic procedure such as DNA shuffling. With such a procedure, one or more different Dof coding sequences can be manipulated to create a new Dof sequence or Dof domain possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between the Dof gene of the invention and other known Dof genes to obtain a new gene coding for a protein with an improved property of interest, such as an increased Kₘ in the case of an enzyme. Strategies for such DNA shuffling are known in the art. See, for example, Stemmer (1994) Proc. Natl. Acad. Sci. USA 91:10747-10751; Stemmer (1994) Nature 370:389-391; Crameri, et al., (1997) Nature Biotech. 15:436-438; Moore, et al., (1997) J. Mol. Biol. 272:336-347; Zhang, et al., (1997) Proc. Natl. Acad. Sci. USA 94:4504-4509; Crameri, et al., (1998) Nature 391:288-291; and U.S. Patent Nos. 5,605,793 and 5,837,458.

The polynucleotides of the invention can be used to isolate corresponding sequences from other organisms, particularly other plants, more particularly other monocots. In this manner, methods such as PCR, hybridization, and the like can be used to identify such sequences based on their sequence homology to the sequences set forth herein. Sequences isolated based on their sequence identity to the entire DOF sequences set forth herein or to variants and fragments thereof are encompassed by the present invention. Such sequences include sequences that are orthologs of the disclosed sequences. "Orthologs" is intended to mean genes derived from a common ancestral gene and which are found in different species as a result of speciation. Genes found in different species are considered orthologs when their nucleotide sequences and/or their encoded protein sequences share at least 60%, 70%, 75%, 80%, 85%, 90%, 91%, 92%, 93%, 94%, 95%, 96%, 97%, 98%, 99% or greater sequence identity. Functions of orthologs are often highly conserved among species. Thus, isolated polynucleotides that can silence or suppress the expression of a Dof sequence or a polynucleotide that encodes for a Dof protein and which hybridize under stringent conditions to the Dof sequences disclosed herein, or to variants or fragments thereof, are encompassed by the present invention.

In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art and are disclosed in Sambrook, et al., (1989) Molecular Cloning: A Laboratory Manual (2d ed., Cold Spring Harbor Laboratory Press, Plainview, New York). See also, Innis, et al., eds. (1990) PCR Protocols: A Guide to Methods and Applications (Academic Press, New York); Innis and Gelfand, eds. (1995) PCR Strategies (Academic Press, New York); and Innis and Gelfand, eds. (1999) PCR Methods Manual (Academic Press, New York). Known methods of PCR include, but are not limited to, methods using paired primers, nested primers, single specific primers, degenerate primers, gene-specific primers, vector-specific primers, partially-mismatched primers, and the like.

In hybridization techniques, all or part of a known polynucleotide is used as a probe that selectively hybridizes to other corresponding polynucleotides present in a population of cloned genomic DNA fragments or cDNA fragments (i.e., genomic or cDNA libraries) from a chosen organism. The hybridization probes may be genomic DNA fragments, cDNA fragments, RNA fragments, or other oligonucleotides, and may be labeled with a detectable group such as ³²P, or any other detectable marker. Thus, for example, probes for hybridization can be made by labeling synthetic oligonucleotides based on the DOF polynucleotides of the invention. Methods for preparation of probes for hybridization and for construction of cDNA and genomic libraries are generally known in the art and are disclosed in Sambrook, et al., (1989) Molecular Cloning: A Laboratory Manual (2d ed., Cold Spring Harbor Laboratory Press, Plainview, New York).

For example, the entire Dof polynucleotide or a polynucleotide encoding a Dof domain disclosed herein, or one or more portions thereof, may be used as a probe capable of specifically hybridizing to corresponding Dof polynucleotide and messenger RNAs. To achieve specific hybridization under a variety of conditions, such probes include sequences that are unique among Dof polynucleotide sequences and are optimally at least about 10 nucleotides in length, and most optimally at least about 20 nucleotides in length. Such probes may be used to amplify corresponding Dof polynucleotide from a chosen plant by PCR. This technique may be used to isolate additional coding sequences from a desired plant or as a diagnostic assay to determine the presence of coding sequences in a plant. Hybridization techniques include hybridization screening of plated DNA libraries (either plaques or colonies; see, for example, Sambrook, et al., (1989) Molecular Cloning: A Laboratory Manual (2d ed., Cold Spring Harbor Laboratory Press, Plainview, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, optimally less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours. The duration of the wash time will be at least a length of time sufficient to reach equilibrium.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3 or 4°C lower than the thermal melting point (Tₘ); moderately stringent conditions can utilize a hybridization and/or wash at 6, 7, 8, 9 or 10°C lower than the thermal melting point (Tₘ); low stringency conditions can utilize a hybridization and/or wash at 11, 12, 13, 14, 15 or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is optimal to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel, et al., eds. (1995) Current Protocols in Molecular Biology, Chapter 2 (Greene Publishing and Wiley-Interscience, New York). See, Sambrook, et al., (1989) Molecular Cloning: A Laboratory Manual (2d ed., Cold Spring Harbor Laboratory Press, Plainview, New York).

The following terms are used to describe the sequence relationships between two or more polynucleotides or polypeptides: (a) "reference sequence", (b) "comparison window", (c) "sequence identity", and, (d) "percentage of sequence identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two polynucleotides. Generally, the comparison window is at least 20 contiguous nucleotides in length, and optionally can be 30, 40, 50, 100 or longer. Those of skill in the art understand that to avoid a high similarity to a reference sequence due to inclusion of gaps in the polynucleotide sequence a gap penalty is typically introduced and is subtracted from the number of matches.
   Methods of alignment of sequences for comparison are well known in the art. Thus, the determination of percent sequence identity between any two sequences can be accomplished using a mathematical algorithm. Non-limiting examples of such mathematical algorithms are the algorithm of Myers and Miller (1988) CABIOS 4:11-17; the local alignment algorithm of Smith, et al., (1981) Adv. Appl. Math. 2:482; the global alignment algorithm of Needleman and Wunsch (1970) J. Mol. Biol. 48:443-453; the search-for-local alignment method of Pearson and Lipman (1988) Proc. Natl. Acad. Sci. 85:2444-2448; the algorithm of Karlin and Altschul (1990) Proc. Natl. Acad. Sci. USA 872264, modified as in Karlin and Altschul (1993) Proc. Natl. Acad. Sci. USA 90:5873-5877.
   Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, California, USA). Alignments using these programs can be performed using the default parameters. The CLUSTAL program is well described by Higgins, et al., (1988) Gene 73:237-244 (1988); Higgins, et al., (1989) CABIOS 5:151-153; Corpet, et al., (1988) Nucleic Acids Res. 16:10881-90; Huang, et al., (1992) CABIOS 8:155-65; and Pearson, et al., (1994) Meth. Mol. Biol. 24:307-331. The ALIGN program is based on the algorithm of Myers and Miller (1988) *supra.* A PAM120 weight residue table, a gap length penalty of 12, and a gap penalty of 4 can be used with the ALIGN program when comparing amino acid sequences. The BLAST programs of Altschul, et al., (1990) J. Mol. Biol. 215:403 are based on the algorithm of Karlin and Altschul (1990) *supra.* BLAST nucleotide searches can be performed with the BLASTN program, score = 100, wordlength = 12, to obtain nucleotide sequences homologous to a nucleotide sequence encoding a protein of the invention. BLAST protein searches can be performed with the BLASTX program, score = 50, wordlength = 3, to obtain amino acid sequences homologous to a protein or polypeptide of the invention. To obtain gapped alignments for comparison purposes, Gapped BLAST (in BLAST 2.0) can be utilized as described in Altschul, et al., (1997) Nucleic Acids Res. 25:3389. Alternatively, PSI-BLAST (in BLAST 2.0) can be used to perform an iterated search that detects distant relationships between molecules. See, Altschul, *et al*., (1997) *supra.* When utilizing BLAST, Gapped BLAST, PSI-BLAST, the default parameters of the respective programs (e.g., BLASTN for nucleotide sequences, BLASTX for proteins) can be used. See, www.ncbi.nlm.nih.gov. Alignment may also be performed manually by inspection.
   Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by GAP Version 10.
   GAP uses the algorithm of Needleman and Wunsch (1970) J. Mol. Biol. 48:443-453, to find the alignment of two complete sequences that maximizes the number of matches and minimizes the number of gaps. GAP considers all possible alignments and gap positions and creates the alignment with the largest number of matched bases and the fewest gaps. It allows for the provision of a gap creation penalty and a gap extension penalty in units of matched bases. GAP must make a profit of gap creation penalty number of matches for each gap it inserts. If a gap extension penalty greater than zero is chosen, GAP must, in addition, make a profit for each gap inserted of the length of the gap times the gap extension penalty. Default gap creation penalty values and gap extension penalty values in Version 10 of the GCG Wisconsin Genetics Software Package for protein sequences are 8 and 2, respectively. For nucleotide sequences the default gap creation penalty is 50 while the default gap extension penalty is 3. The gap creation and gap extension penalties can be expressed as an integer selected from the group of integers consisting of from 0 to 200. Thus, for example, the gap creation and gap extension penalties can be 0, 1, 2, 3, 4, 5, 6, 7, 8, 9, 10, 15, 20, 25, 30, 35, 40, 45, 50, 55, 60, 65 or greater.
   GAP presents one member of the family of best alignments. There may be many members of this family, but no other member has a better quality. GAP displays four figures of merit for alignments: Quality, Ratio, Identity, and Similarity. The Quality is the metric maximized in order to align the sequences. Ratio is the quality divided by the number of bases in the shorter segment. Percent Identity is the percent of the symbols that actually match. Percent Similarity is the percent of the symbols that are similar. Symbols that are across from gaps are ignored. A similarity is scored when the scoring matrix value for a pair of symbols is greater than or equal to 0.50, the similarity threshold. The scoring matrix used in Version 10 of the GCG Wisconsin Genetics Software Package is BLOSUM62 (see, Henikoff and Henikoff (1989) Proc. Natl. Acad. Sci. USA 89:10915).
(c) As used herein, "sequence identity" or "identity" in the context of two polynucleotides or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

### B. Plants

In specific embodiments, the invention provides plants, plant cells, and plant parts having altered levels (i.e., an increase or decrease) of a Dof sequence. In some embodiments, the plants and plant parts have stably incorporated into their genome at least one heterologous polynucleotide encoding a Dof polypeptide comprising the Dof domain as set forth in SEQ ID NO: 145, or a biologically active variant or fragment thereof. In one embodiment, the polynucleotide encoding the Dof polypeptide is set forth in any one of SEQ ID NOS: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152, 153 or a biologically active variant or fragment thereof.

In yet other embodiments, plants and plant parts are provided in which the heterolgous polynucleotide stably integrated into the genome of the plant or plant part comprises a polynucleotide which when expressed in a plant decreases the level of a Dof polypeptide comprising a Dof domain as set forth in SEQ ID NO: 145 or an active variant or fragment thereof. Sequences that can be used to suppress expression of a Dof polypeptide include, but are not limited to, any of the sequence set forth in SEQ ID NOS: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134; 136, 137, 139, 140, 142, 143, 144, 146, 147, 148, 149, 150, 151, 152 or 153 or variants or fragments thereof.

In specific embodiments, the heterologous polynucleotide in the plant or plant part is operably linked to a tissue-preferred promoter, such as a seed-preferred promoter (i.e., an endosperm-preferred promoter or an embryo-preferred promoter), a vascular-preferred promoter, or a leaf-preferred promoter (i.e., a bundle sheath-preferred promoter or a mesophyll-preferred promoter).

As discussed in further detail elsewhere herein, such plants, plant cells, and plant parts can have an altered phenotype including, for example, a modulation in carbon fixation, improved nitrogen use efficiency, improved yield, or an improved stress tolerance.

As used herein, the term plant includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like. Grain is intended to mean the mature seed produced by commercial growers for purposes other than growing or reproducing the species. Progeny, variants, and mutants of the regenerated plants are also included within the scope of the invention, provided that these parts comprise the introduced or heterologous polynucleotides disclosed herein.

The present invention may be used for transformation of any plant species, including, but not limited to, monocots and dicots. Examples of plant species of interest include, but are not limited to, corn (*Zea mays*), *Brassica* sp. (e.g., *B*. *napus, B. rapa, B. juncea*), particularly those *Brassica* species useful as sources of seed oil, alfalfa (*Medicago sativa*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanuts (*Arachis hypogaea*), cotton (*Gossypium barbadense*, *Gossypium hirsutum*), sweet potato (*Ipomoea batatus*), cassava (*Manihot esculenta*), coffee (*Coffea* spp.), coconut (Cocos *nucifera*), pineapple *(Ananas comosus*), citrus trees (*Citrus* spp.), cocoa (*Theobroma cacao*), tea (*Camellia sinensis*), banana *(Musa* spp.), avocado (*Persea americana*), fig (*Ficus casica*), guava (*Psidium guajava*), mango (*Mangifera indica*), olive (Olea *europaea*), papaya (*Carica papaya*), cashew (*Anacardium occidentale*), macadamia (*Macadamia integrifolia*), almond (*Prunus amygdalus*), sugar beets (*Beta vulgaris*), sugarcane (*Saccharum* spp.), oats, barley, vegetables, ornamentals, and conifers.

Vegetables include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (*C*. *sativus*), cantaloupe (*C. cantalupensis*), and musk melon (*C*. *melo*). Ornamentals include azalea (*Rhododendron* spp.), hydrangea (*Macrophylla hydrangea*), hibiscus (*Hibiscus rosasanensis*), roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*), and chrysanthemum.

Conifers that may be employed in practicing the present invention include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*); Douglas-fir (*Pseudotsuga menziesii*); Western hemlock (*Tsuga canadensis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*). In specific embodiments, plants of the present invention are crop plants (for example, corn, alfalfa, sunflower, *Brassica,* soybean, cotton, safflower, peanut, sorghum, wheat, millet, tobacco, etc.). In other embodiments, corn and soybean plants are optimal, and in yet other embodiments corn plants are optimal.

Other plants of interest include grain plants that provide seeds of interest, oil-seed plants, and leguminous plants. Seeds of interest include grain seeds, such as corn, wheat, barley, rice, sorghum, rye, etc. Oil-seed plants include cotton, soybean, safflower, sunflower, *Brassica*, maize, alfalfa, palm, coconut, etc. Leguminous plants include beans and peas. Beans include guar, locust bean, fenugreek, soybean, garden beans, cowpea, mungbean, lima bean, fava bean, lentils, chickpea, etc.

A "subject plant or plant cell" is one in which an alteration, such as transformation or introduction of a polypeptide, has occurred, or is a plant or plant cell which is descended from a plant or cell so altered and which comprises the alteration. A "control" or "control plant" or "control plant cell" provides a reference point for measuring changes in phenotype of the subject plant or plant cell.

A control plant or plant cell may comprise, for example: (a) a wild-type plant or cell, i.e., of the same genotype as the starting material for the alteration which resulted in the subject plant or cell; (b) a plant or plant cell of the same genotype as the starting material but which has been transformed with a null construct (i.e., with a construct which has no known effect on the trait of interest, such as a construct comprising a marker gene); (c) a plant or plant cell which is a non-transformed segregant among progeny of a subject plant or plant cell; (d) a plant or plant cell genetically identical to the subject plant or plant cell but which is not exposed to conditions or stimuli that would induce expression of the gene of interest; or (e) the subject plant or plant cell itself, under conditions in which the gene of interest is not expressed.

### C. Polynucleotide Constructs

The use of the term "polynucleotide" is not intended to limit the present invention to polynucleotides comprising DNA. Those of ordinary skill in the art will recognize that polynucleotides, can comprise ribonucleotides and combinations of ribonucleotides and deoxyribonucleotides. Such deoxyribonucleotides and ribonucleotides include both naturally occurring molecules and synthetic analogues. The polynucleotides of the invention also encompass all forms of sequences including, but not limited to, single-stranded forms, double-stranded forms, hairpins, stem-and-loop structures; and the like.

The various polynucleotides employed in the methods and compositions of the invention can be provided in expression cassettes for expression in the plant of interest. The cassette will include 5' and 3' regulatory sequences operably linked to a polynucleotide of the invention. "Operably linked" is intended to mean a functional linkage between two or more elements. For example, an operable linkage between a polynucleotide of interest and a regulatory sequence (i.e., a promoter) is functional link that allows for expression of the polynucleotide of interest. Operably linked elements may be contiguous or non-contiguous. When used to refer to the joining of two protein coding regions, by operably linked is intended that the coding regions are in the same reading frame. The cassette may additionally contain at least one additional gene to be cotransformed into the organism. Alternatively, the additional gene(s) can be provided on multiple expression cassettes. Such an expression cassette is provided with a plurality of restriction sites and/or recombination sites for insertion of the DOF polynucleotide to be under the transcriptional regulation of the regulatory regions. The expression cassette may additionally contain selectable marker genes.

The expression cassette can include in the 5'-3' direction of transcription, a transcriptional and translational initiation region (i.e., a promoter), a Dof polynucleotide, and a transcriptional and translational termination region (i.e., termination region) functional in plants. The regulatory regions (i.e., promoters, transcriptional regulatory regions, and translational termination regions) and/or the Dof polynucleotide may be native/analogous to the host cell or to each other. Alternatively, the regulatory regions and/or the Dof polynucleotides may be heterologous to the host cell or to each other. As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide. As used herein, a chimeric gene comprises a coding sequence operably linked to a transcription initiation region that is heterologous to the coding sequence.

While it may be optimal to express the sequences using heterologous promoters, the native promoter sequences may be used. Such constructs can change expression levels of Dof in the plant or plant cell. Thus, the phenotype of the plant or plant cell can be altered.

The termination region may be native with the transcriptional initiation region, may be native with the operably linked Dof polynucleotide of interest, may be native with the plant host, or may be derived from another source (i.e., foreign or heterologous) to the promoter, the Dof polynucleotide of interest, the plant host, or any combination thereof. Convenient termination regions are available from the Ti-plasmid of *A*. *tumefaciens*, such as the octopine synthase and nopaline synthase termination regions. See also, Guerineau, et al., (1991) Mol. Gen. Genet. 262:141-144; Proudfoot (1991) Cell 64:671-674; Sanfacon, et al., (1991) Genes Dev. 5:141-149; Mogen, et al., (1990) Plant Cell 2:1261-1272; Munroe, et al., (1990) Gene 91:151-158; Ballas, et al., (1989) Nucleic Acids Res. 17:7891-7903; and Joshi, et al., (1987) Nucleic Acids Res. 15:9627-9639.

Where appropriate, the polynucleotides may be optimized for increased expression in the transformed plant. That is, the polynucleotides can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray, et al., (1989) Nucleic Acids Res. 17:477-498, herein incorporated by reference.

Additional sequence modifications are known to enhance gene expression in a cellular host. These include elimination of sequences encoding spurious polyadenylation signals, exon-intron splice site signals, transposon repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

The expression cassettes may additionally contain 5' leader sequences. Such leader sequences can act to enhance translation. Translation leaders are known in the art and include: picornavirus leaders, for example, EMCV leader (Encephalomyocarditis 5' noncoding region) (Elroy-Stein, et al., (1989) Proc. Natl. Acad. Sci. USA 86:6126-6130); potyvirus leaders, for example, TEV leader (Tobacco Etch Virus) (Gallie, et al., (1995) Gene 165(2):233-238), MDMV leader (Maize Dwarf Mosaic Virus) (Virology 154:9-20), and human immunoglobulin heavy-chain binding protein (BiP) (Macejak, et al., (1991) Nature 353:90-94); untranslated leader from the coat protein mRNA of alfalfa mosaic virus (AMV RNA 4) (Jobling, et al., (1987) Nature 325:622-625); tobacco mosaic virus leader (TMV) (Gallie, et al., (1989) in Molecular Biology of RNA, ed. Cech (Liss, New York), pp. 237-256); and maize chlorotic mottle virus leader (MCMV) (Lommel, et al., (1991) Virology 81:382-385). See also, Della-Cioppa, et al., (1987) Plant Physiol. 84:965-968.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

A number of promoters can be used in the practice of the invention, including the native promoter of the polynucleotide sequence of interest. The promoters can be selected based on the desired outcome. The nucleic acids can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell, et al., (1985) Nature 313:810-812); rice actin (McElroy, et al., (1990) Plant Cell 2:163-171); ubiquitin (Christensen, et al., (1989) Plant Mol. Biol. 12:619-632 and Christensen, et al., (1992) Plant Mol. Biol. 18:675-689); pEMU (Last, et al., (1991) Theor. Appl. Genet. 81:581-588); MAS (Velten, et al., (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to target enhanced expression within a particular plant tissue. Tissue-preferred promoters include Yamamoto, et al., (1997) Plant J. 12(2):255-265; Kawamata, et al., (1997) Plant Cell Physiol. 38(7):792-803; Hansen, et al., (1997) Mol. Gen Genet. 254(3):337-343; Russell, et al., (1997) Transgenic Res. 6(2):157-168; Rinehart, et al., (1996) Plant Physiol. 112(3):1331-1341; Van Camp, et al., (1996) Plant Physiol. 112(2):525-535; Canevascini, et al., (1996) Plant Physiol. 112(2):513-524; Yamamoto, et al., (1994) Plant Cell Physiol. 35(5):773-778; Lam (1994) Results Probl. Cell Differ. 20:181-196; Orozco, et al., (1993) Plant Mol Biol. 23(6):1129-1138; Matsuoka, et al., (1993) Proc Natl. Acad. Sci. USA 90(20):9586-9590; and Guevara-Garcia, et al., (1993) Plant J. 4(3):495-505. Such promoters can be modified, if necessary, for weak expression.

Leaf-preferred promoters are known in the art. See, for example, Yamamoto, et al., (1997) Plant J. 12(2):255-265; Kwon, et al., (1994) Plant Physiol. 105:357-67; Yamamoto, et al., (1994) Plant Cell Physiol. 35(5):773-778; Gotor, et al., (1993) Plant J. 3:509-18; Orozco, et al., (1993) Plant Mol. Biol. 23(6):1129-1138; and Matsuoka, et al., (1993) Proc. Natl. Acad. Sci. USA 90(20):9586-9590.

Promoters that direct expression in various types of leaf cells can also be employed. For example, mesophyll-preferred promoters are known in the art and include, but are not limited to, the promoter for the C₄ phosphoenolpyruvate carboxylase gene (Gowik, et al., (2004) The Plant Cell 16:1077-1090); the promoter of the chlorophyll a/b binding protein gene (cab) (Hudspeth, et al., (1992) Plant Physiology 98:458-464); the *Arabidopsis* promoter pRbcS2b (Moon, *et al.,* A novel screening approach for selective non-cell autonomous proteins. American Society of Plant Biologists Abs #864); the promoters of the cytosolic fructose-1,6-bisphosphatase genes (cy-FBPase genes) (U.S. Application Publication US2002120955); and, the ribulose-1,5-bisphosphate carboxylase small subunit (rbcS) gene promoters from rice and maize (Schaffner, et al., (1991) The Plant Cell 9:997-1012); each of these references is herein incorporated by reference.

Other leaf-preferred promoters of interest include bundle sheath-preferred promoters. Bundle sheath-preferred promoters are known in the art and include, but are not limited to, a modified form of the promoter from ppcA (Stockhaus (1997) Plant Cell 9:479); and the *ZjPck* promoter which directs expression in bundle sheath cells and in vascular cells (Nomura (2005) Plant and Cell Physiology 46(5):754-761; each of these references is herein incorporated by reference.

Vascular-preferred promoters are also known in the art including, but not limited to, promoters of U.S. Application Publication No. 20040163146.

Various promoters that are induced by light can be used in the methods and compositions of the invention. Such promoter as known in the art and include, but are not limited to, the promoters from cab or rubisco (Simpson, et al., (1985) EMBO J 4:2723-2729 and Timko, et al., (1985) Nature 318:579-582).

Seed-preferred promoters include both seed-specific promoters (those promoters active during seed development such as promoters of seed storage proteins), as well as, seed-germinating promoters (those promoters active during seed germination). See, Thompson, et al., (1989) BioEssays 10:108, herein incorporated by reference. Such seed-preferred promoters include, but are not limited to, Cim1 (cytokinin-induced message); cZ19B1 (maize 19 kDa zein); milps (myo-inositol-1-phosphate synthase) (see WO 00/11177 and U.S. Patent No. 6,225,529; herein incorporated by reference), PCNA2 (U.S. Patent Application No. 10/388,359, filed March 13, 2003) and, CKX1-2 (U.S. Application Publication 20020152500). For dicots, seed-specific promoters include, but are not limited to, bean β-phaseolin, napin, β-conglycinin, soybean lectin, cruciferin, and the like. For monocots, seed-specific promoters include, but are not limited to, maize 15 kDa zein, 22 kDa zein, 27 kDa zein, gamma-zein, waxy, shrunken 1, shrunken 2, Globulin 1, etc. See also, WO 00/12733, where seed-preferred promoters from *end1* and *end2* genes are disclosed and WO 01/21783 and 6,403,862, where the Zm40 promoter is disclosed; both herein incorporated by reference.

Embryo-specific promoters include Globulin 1 (Glb-1), ESR (U.S. Application Publication 20040210960) and lecl (U.S. Patent Application No. 09/718,754, filed November 22, 2000). Additional embryo specific promoters are disclosed in Sato, et al., (1996) Proc. Natl. Acad. Sci. 93:8117-8122; Nakase, et al., (1997) Plant J 12:235-56; and Postma-Haarsma, et al., (1999) Plant Mol. Biol. 39:257-71. Endosperm-preferred promoters include the Gamma-zein, promoter, eppl and eep2 as disclosed in U.S. Patent Application Publication 20040237147. Additional endosperm-specific promoters are disclosed in Albani, et al., (1985) EMBO 3:1505-15; Albani, et al., (1999) Theor. Appl. Gen. 98:1253-62; Albani, et al., (1993) Plant J. 5:353-55; Mena, et al., (1998) The Plant Journal 116:53-62, and Wu, et al., (1998) Plant Cell Physiology 39:885-889. Immature ear tissue-preferred promoters can also be employed.

The expression cassette can also comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). Additional selectable markers include phenotypic markers such as β-galactosidase and fluorescent proteins such as green fluorescent protein (GFP) (Su, et al., (2004) Biotechnol Bioeng 85:610-9 and Fetter, et al., (2004) Plant Cell 16:215-28), cyan florescent protein (CYP) (Bolte, et al., (2004) J. Cell Science 117:943-54 and Kato, et al., (2002) Plant Physiol 129:913-42), and yellow florescent protein (PhiYFP™ from Evrogen, see, Bolte, et al., (2004) J. Cell Science 117:943-54). For additional selectable markers, see generally, Yarranton (1992) Curr. Opin. Biotech. 3:506-511; Christopherson, et al., (1992) Proc. Natl. Acad. Sci. USA 89:6314-6318; Yao, et al., (1992) Cell 71:63-72; Reznikoff (1992) Mol. Microbiol. 6:2419-2422; Barkley, et al., (1980) in The Operon, pp. 177-220; Hu, et al., (1987) Cell 48:555-566; Brown, et al., (1987) Cell 49:603-612; Figge, et al., (1988) Cell 52:713-722; Deuschle, et al., (1989) Proc. Natl. Acad. Aci. USA 86:5400-5404; Fuerst, et al., (1989) Proc. Natl. Acad. Sci. USA 86:2549-2553; Deuschle, et al., (1990) Science 248:480-483; Gossen (1993) Ph.D. Thesis, University of Heidelberg; Reines, et al., (1993) Proc. Natl. Acad. Sci. USA 90:1917-1921; Labow, et al., (1990) Mol. Cell. Biol. 10:3343-3356; Zambretti, et al., (1992) Proc. Natl. Acad. Sci. USA 89:3952-3956; Baim, et al., (1991) Proc. Natl. Acad. Sci. USA 88:5072-5076; Wyborski, et al., (1991) Nucleic Acids Res. 19:4647-4653; Hillenand-Wissman (1989) Topics Mol. Struc. Biol. 10:143-162; Degenkolb, et al., (1991) Antimicrob. Agents Chemother. 35:1591-1595; Kleinschnidt, et al., (1988) Biochemistry 27:1094-1104; Bonin (1993) Ph.D. Thesis, University of Heidelberg; Gossen, et al., (1992) Proc. Natl. Acad. Sci. USA 89:5547-5551; Oliva, et al., (1992) Antimicrob. Agents Chemother. 36:913-919; Hlavka, et al., (1985) Handbook of Experimental Pharmacology, Vol. 78 (Springer-Verlag, Berlin); Gill, et al., (1988) Nature 334:721-724. Such disclosures are herein incorporated by reference. The above list of selectable marker genes is not meant to be limiting. Any selectable marker gene can be used in the present invention.

In certain embodiments the polynucleotides of the present invention can be stacked with any combination of polynucleotide sequences of interest in order to create plants with a desired trait. A trait, as used herein, refers to the phenotype derived from a particular sequence or groups of sequences. The combinations generated can also include multiple copies of any one of the polynucleotides of interest. The polynucleotides of the present invention can also be stacked with traits desirable for disease or herbicide resistance (e.g., fumonisin detoxification genes (U.S. Patent No. 5,792,931); avirulence and disease resistance genes (Jones, et al., (1994) Science 266:789; Martin, et al., (1993) Science 262:1432; Mindrinos, et al., (1994) Cell 78:1089); acetolactate synthase (ALS) mutants that lead to herbicide resistance such as the S4 and/or Hra mutations; inhibitors of glutamine synthase such as phosphinothricin or basta (e.g., bar gene); and glyphosate resistance (EPSPS gene)); and traits desirable for processing or process products such as high oil (e.g., U.S. Patent No. 6,232,529 ); modified oils (e.g., fatty acid desaturase genes (U.S. Patent No. 5,952,544; WO 94/11516)); modified starches (e.g., ADPG pyrophosphorylases (AGPase), starch synthases (SS), starch branching enzymes (SBE), and starch debranching enzymes (SDBE)); and polymers or bioplastics (e.g., U.S. Patent No. 5.602,321; beta-ketothiolase, polyhydroxybutyrate synthase, and acetoacetyl-CoA reductase (Schubert, et al., (1988) J. Bacteriol. 170:5837-5847) facilitate expression of polyhydroxyalkanoates (PHAs)); the disclosures of which are herein incorporated by reference. One could also combine the polynucleotides of the present invention with polynucleotides providing agronomic traits such as male sterility (e.g., see U.S. Patent No. 5,583,210), stalk strength, flowering time, or transformation technology traits such as cell cycle regulation or gene targeting (e.g., WO 99/61619, WO 00/17364, and WO 99/25821); the disclosures of which are herein incorporated by reference.

These stacked combinations can be created by any method including, but not limited to, cross-breeding plants by any conventional or TopCross methodology, or genetic transformation. If the sequences are stacked by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system. See, for example, WO99/25821, WO99/25854, WO99/25840, WO99/25855, and WO99/25853, all of which are herein incorporated by reference.

### D. Method of Introducing

The methods of the invention involve introducing a polypeptide or polynucleotide into a plant. "Introducing" is intended to mean presenting to the plant the polynucleotide or polypeptide in such a manner that the sequence gains access to the interior of a cell of the plant. The methods of the invention do not depend on a particular method for introducing a sequence into a plant, only that the polynucleotide or polypeptides gains access to the interior of at least one cell of the plant. Methods for introducing polynucleotide or polypeptides into plants are' known in the art including, but not limited to, stable transformation methods, transient transformation methods, and virus-mediated methods.

"Stable transformation" is intended to mean that the nucleotide construct introduced into a plant integrates into the genome of the plant and is capable of being inherited by the progeny thereof. "Transient transformation" is intended to mean that a polynucleotide is introduced into the plant and does not integrate into the genome of the plant or a polypeptide is introduced into a plant.

Transformation protocols as well as protocols for introducing polypeptides or polynucleotide sequences into plants may vary depending on the type of plant or plant cell, i.e., monocot or dicot, targeted for transformation. Suitable methods of introducing polypeptides and polynucleotides into plant cells include microinjection (Crossway, et al., (1986) Biotechniques 4:320-334), electroporation (Riggs, et al., (1986) Proc. Natl. Acad. Sci. USA 83:5602-5606, *Agrobacterium-*mediated transformation (U.S. Patent No. 5,563,055 and U.S. Patent No. 5,981,840), direct gene transfer (Paszkowski, et al., (1984) EMBO J. 3:2717-2722), and ballistic particle acceleration (see, for example, U.S. Patent Nos. 4,945,050; U.S. Patent No. 5,879,918; U.S. Patent No. 5,886,244; and, 5,932,782; Tomes, et al., (1995) in Plant Cell, Tissue, and Organ Culture: Fundamental Methods, ed. Gamborg and Phillips (Springer-Verlag, Berlin); McCabe, et al., (1988) Biotechnology 6:923-926); and Lec1 transformation (WO 00/28058). Also see, Weissinger, et al., (1988) Ann. Rev. Genet. 22:421-477; Sanford, et al., (1987) Particulate Science and Technology 5:27-37 (onion); Christou, et al., (1988) Plant Physiol. 87:671-674 (soybean); McCabe, et al., (1988) Bio/Technology 6:923-926 (soybean); Finer and McMullen (1991) In Vitro Cell Dev. Biol. 27P:175-182 (soybean); Singh, et al., (1998) Theor. Appl. Genet. 96:319-324 (soybean); Datta, et al., (1990) Biotechnology 8:736-740 (rice); Klein, et al., (1988) Proc. Natl. Acad. Sci. USA 85:4305-4309 (maize); Klein, et al., (1988) Biotechnology 6:559-563 (maize); U.S. Patent Nos. 5,240,855; 5,322,783; and, 5,324,646; Klein, et al., (1988) Plant Physiol. 91:440-444 (maize); Fromm, et al., (1990) Biotechnology 8:833-839 (maize); Hooykaas-Van Slogteren, et al., (1984) Nature (London) 311:763-764; U.S. Patent No. 5,736,369 (cereals); Bytebier, et al., (1987) Proc. Natl. Acad. Sci. USA 84:5345-5349 (Liliaceae); De Wet, et al., (1985) in The Experimental Manipulation of Ovule Tissues, ed. Chapman, et al., (Longman, New York), pp. 197-209 (pollen); Kaeppler, et al., (1990) Plant Cell Reports 9:415-418 and Kaeppler, et al., (1992) Theor. Appl. Genet. 84:560-566 (whisker-mediated transformation); D'Halluin, et al., (1992) Plant Cell 4:1495-1505 (electroporation); Li, et al., (1993) Plant Cell Reports 12:250-255 and Christou and Ford (1995) Annals of Botany 75:407-413 (rice); Osjoda, et al., (1996) Nature Biotechnology 14:745-750 (maize via *Agrobacterium tumefaciens*); all of which are herein incorporated by reference.

In specific embodiments, the Dof sequences or variants and fragments thereof can be provided to a plant using a variety of transient transformation methods. Such transient transformation methods include, but are not limited to, the introduction of the Dof protein or variants and fragments thereof directly into the plant or the introduction of the Dof transcript into the plant. Such methods include, for example, microinjection or particle bombardment. See, for example, Crossway, et al., (1986) Mol Gen. Genet. 202:179-185; Nomura, et al., (1986) Plant Sci. 44:53-58; Hepler, et al., (1994) Proc. Natl. Acad. Sci. 91:2176-2180 and Hush, et al., (1994) The Journal of Cell Science 107:775-784, all of which are herein incorporated by reference. Alternatively, the Dof polynucleotide can be transiently transformed into the plant using techniques known in the art. Such techniques include viral vector system and the precipitation of the polynucleotide in a manner that precludes subsequent release of the DNA. Thus, the transcription from the particle-bound DNA can occur, but the frequency with which it is released to become integrated into the genome is greatly reduced. Such methods include the use particles coated with polyethylimine (PEl; Sigma #P3143).

In other embodiments, the polynucleotide of the invention may be introduced into plants by contacting plants with a virus or viral nucleic acids. Generally, such methods involve incorporating a nucleotide construct of the invention within a viral DNA or RNA molecule. It is recognized that the a Dof sequence or a variant or fragment thereof may be initially synthesized as part of a viral polyprotein, which later may be processed by proteolysis *in vivo* or *in vitro* to produce the desired recombinant protein. Further, it is recognized that promoters of the invention also encompass promoters utilized for transcription by viral RNA polymerases. Methods for introducing polynucleotides into plants and expressing a protein encoded therein, involving viral DNA or RNA molecules, are known in the art. See, for example, U.S. Patent Nos. 5,889,191, 5,889,190, 5,866,785, 5,589,367, 5,316,931, and Porta, et al., (1996) Molecular Biotechnology 5:209-221; herein incorporated by reference.

Methods are known in the art for the targeted insertion of a polynucleotide at a specific location in the plant genome. In one embodiment, the insertion of the polynucleotide at a desired genomic location is achieved using a site-specific recombination system. See, for example, WO99/25821, WO99/25854, WO99/25840, WO99/25855, and WO99/25853, all of which are herein incorporated by reference. Briefly, the polynucleotide of the invention can be contained in transfer cassette flanked by two non-recombinogenic recombination sites. The transfer cassette is introduced into a plant having stably incorporated into its genome a target site which is flanked by two non-recombinogenic recombination sites that correspond to the sites of the transfer cassette. An appropriate recombinase is provided and the transfer cassette is integrated at the target site. The polynucleotide of interest is thereby integrated at a specific chromosomal position in the plant genome.

The cells that have been transformed may be grown into plants in accordance with conventional ways. See, for example, McCormick, et al., (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide of the invention, for example, an expression cassette of the invention, stably incorporated into their gnome.

### III. Methods of Use

### A. Methods for Modulating Expression of at Least One Dof Sequence or a Variant or Fragment Therefore in a Plant or Plant Part

A "modulated level" or "modulating level" of a polypeptide in the context of the methods of the present invention refers to any increase or decrease in the expression, concentration, or activity of a gene product, including any relative increment in expression, concentration or activity. Any method or composition that modulates expression of a target gene product, either at the level of transcription or translation, or modulates the activity of the target gene product can be used to achieve modulated expression, concentration, activity of the target gene product. In general, the level is increased or decreased by at least 1%, 5%, 10%, 20%, 30%, 40%, 50%, 60%, 70%, 80%, 90% or greater relative to an appropriate control plant, plant part, or cell. Modulation in the present invention may occur during and/or subsequent to growth of the plant to the desired stage of development. In specific embodiments, the polypeptides of the present invention are modulated in monocots, particularly maize.

The expression level of a polypeptide having a Dof domain or a biologically active variant or fragment thereof may be measured directly, for example, by assaying for the level of the Dof polypeptide in the plant, or indirectly, for example, by measuring the level of the polynucleotide encoding the protein or by measuring the activity of the Dof polypeptide in the plant. Methods for determining the activity of the Dof polypeptide are described elsewhere herein.

In specific embodiments, the polypeptide or the polynucleotide of the invention is introduced into the plant cell. Subsequently, a plant cell having the introduced sequence of the invention is selected using methods known to those of skill in the art such as, but not limited to, Southern blot analysis, DNA sequencing, PCR analysis, or phenotypic analysis. A plant or plant part altered or modified by the foregoing embodiments is grown under plant forming conditions for a time sufficient to modulate the concentration and/or activity of polypeptides of the present invention in the plant. Plant forming conditions are well known in the art and discussed briefly elsewhere herein.

It is also recognized that the level and/or activity of the polypeptide may be modulated by employing a polynucleotide that is not capable of directing, in a transformed plant, the expression of a protein or an RNA. For example, the polynucleotides of the invention may be used to design polynucleotide constructs that can be employed in methods for altering or mutating a genomic nucleotide sequence in an organism. Such polynucleotide constructs include, but are not limited to, RNA:DNA vectors, RNA:DNA mutational vectors, RNA:DNA repair vectors, mixed-duplex oligonucleotides, self-complementary RNA:DNA oligonucleotides, and recombinogenic oligonucleobases. Such nucleotide constructs and methods of use are known in the art. See, U.S. Patent Nos. 5,565,350; 5,731,181; 5,756,325; 5,760,012; 5,795,972; and 5,871,984; all of which are herein incorporated by reference. See also, WO 98/49350, WO 99/07865, WO 99/25821, and Beetham, et al., (1999) Proc. Natl. Acad. Sci. USA 96:8774-8778; herein incorporated by reference.

It is therefore' recognized that methods of the present invention do not depend on the incorporation of the entire polynucleotide into the genome, only that the plant or cell thereof is altered as a result of the introduction of the polynucleotide into a cell. In one embodiment of the invention, the genome may be altered following the introduction of the polynucleotide into a cell. For example, the polynucleotide, or any part thereof, may incorporate into the genome of the plant. Alterations to the genome of the present invention include, but are not limited to, additions, deletions, and substitutions of nucleotides into the genome. While the methods of the present invention do not depend on additions, deletions, and substitutions of any particular number of nucleotides, it is recognized that such additions, deletions, or substitutions comprises at least one nucleotide.

In one embodiment, the activity and/or level of a Dof polypeptide is increased. An increase in the level and/or activity of the Dof polypeptide can be achieved by providing to the plant a Dof polypeptide or a biologically active variant or fragment thereof. As discussed elsewhere herein, many methods are known in the art for providing a polypeptide to a plant including, but not limited to, direct introduction of the Dof polypeptide into the plant or introducing into the plant (transiently or stably) a polynucleotide construct encoding a polypeptide having Dof activity. It is also recognized that the methods of the invention may employ a polynucleotide that is not capable of directing in the transformed plant the expression of a protein or an RNA. Thus, the level and/or activity of a Dof polypeptide may be increased by altering the gene encoding the Dof polypeptide or its promoter. See, e.g., Kmiec, U.S. Patent 5,565,350; Zarling, et al., PCT/US93/03868. Therefore mutagenized plants that carry mutations in Dof genes, where the mutations increase expression of the Dof gene or increase the activity of the encoded Dof polypeptide are provided.

In other embodiments, the activity and/or level of the Dof polypeptide of the invention is reduced or eliminated by introducing into a plant a polynucleotide that inhibits the level or activity of a polypeptide. The polynucleotide may inhibit the expression of Dof directly, by preventing translation of the Dof messenger RNA, or indirectly, by encoding a polypeptide that inhibits the transcription or translation of a Dof gene encoding a Dof protein. Methods for inhibiting or eliminating the expression of a gene in a plant are well known in the art, and any such method may be used in the present invention to inhibit the expression of at least one Dof sequence in a plant. In other embodiments of the invention, the activity of a Dof polypeptide is reduced or eliminated by transforming a plant cell with a sequence encoding a polypeptide that inhibits the activity of the Dof polypeptide. In other embodiments, the activity of a Dof polypeptide may be reduced or eliminated by disrupting the gene encoding the Dof polypeptide. The invention encompasses mutagenized plants that carry mutations in Dof genes, where the mutations reduce expression of the Dof gene or inhibit the Dof activity of the encoded Dof polypeptide.

Reduction of the activity of specific genes (also known as gene silencing or gene suppression) is desirable for several aspects of genetic engineering in plants. Many techniques for gene silencing are well known to one of skill in the art, including, but not limited to, antisense technology (see, e.g., Sheehy, et al., (1988) Proc. Natl. Acad. Sci. USA 85:8805-8809; and U.S. Patent Nos. 5,107,065; 5,453,566; and 5,759,829); cosuppression (e.g., Taylor (1997) Plant Cell 9:1245; Jorgensen (1990) Trends Biotech. 8(12):340-344; Flavell (1994) Proc. Natl. Acad. Sci. USA 91:3490-3496; Finnegan, et al., (1994) Bio/Technology 12:883-888; and Neuhuber, et al., (1994) Mol. Gen. Genet. 244:230-241); RNA interference (Napoli, et al., (1990) Plant Cell 2:279-289; U.S. Patent No. 5,034,323; Sharp (1999) Genes Dev. 13:139-141; Zamore, et al., (2000) Cell 101:25-33; and Montgomery, et al., (1998) Proc. Natl. Acad. Sci. USA 95:15502-15507), virus-induced gene silencing (Burton, et al., (2000) Plant Cell 12:691-705; and Baulcombe (1999) Curr. Op. Plant Bio. 2:109-113); target-RNA-specific ribozymes (Haseloff, et al., (1988) Nature 334:585-591); hairpin structures (Smith, et al., (2000) Nature 407:319-320; WO 99/53050; WO 02/00904; WO 98/53083; Chuang and Meyerowitz (2000) Proc. Natl. Acad. Sci. USA 97:4985-4990; Stoutjesdijk, et al., (2002) Plant Physiol. 129:1723-1731; Waterhouse and Helliwell (2003) Nat. Rev. Genet. 4:29-38; Pandolfini, et al., BMC Biotechnology 3:7, U.S. Patent Publication No. 20030175965; Panstruga, et al., (2003) Mol. Biol. Rep. 30:135-140; Wesley, et al., (2001) Plant J. 27:581-590; Wang and Waterhouse (2001), Curr. Opin. Plant Biol. 5:146-150; U.S. Patent Publication No. 20030180945; and,' WO 02/00904, all of which are herein incorporated by reference); ribozymes (Steinecke, et al., (1992) EMBO J. 11:1525; and Perriman, et al., (1993) Antisense Res. Dev. 3:253); oligonucleotide-mediated targeted modification (e.g., WO 03/076574 and WO 99/25853); Zn-finger targeted molecules (e.g., WO 01/52620; WO 03/048345; and WO 00/42219); transposon tagging (Maes, et al., (1999) Trends Plant Sci. 4:90-96; Dharmapuri and Sonti (1999) FEMS Microbiol. Left. 179:53-59; Meissner, et al., (2000) Plant J. 22:265-274; Phogat, et al., (2000) J. Biosci. 25:57-63; Walbot (2000) Curr. Opin. Plant Biol. 2:103-107; Gai, et al., (2000) Nucleic Acids Res. 28:94-96; Fitzmaurice, et al., (1999) Genetics 153:1919-1928; Bensen, et al., (1995) Plant Cell 7:75-84; Mena, et a/., (1996) Science 274:1537-1540; and U.S. Patent No. 5,962,764); each of which is herein incorporated by reference; and other methods or combinations of the above methods known to those of skill in the art.

It is recognized that with the polynucleotides of the invention, antisense constructions, complementary to at least a portion of the messenger RNA (mRNA) for the Dof sequences can be constructed. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, optimally 80%, more optimally 85% sequence identity to the corresponding antisensed sequences may be used. Furthermore, portions of the antisense nucleotides may be used to disrupt the expression of the target gene. Generally, sequences of at least 50 nucleotides, 100 nucleotides, 200 nucleotides, 300, 400, 450, 500, 550 or greater may be used.

The polynucleotides of the present invention may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a polynucleotide that corresponds to the transcript of the endogenous gene. Typically, such a nucleotide sequence has substantial sequence identity to the sequence of the transcript of the endogenous gene, optimally greater than about 65% sequence identity, more optimally greater than about 85% sequence identity, most optimally greater than about 95% sequence identity. See, U.S. Patent Nos. 5,283,184 and 5,034,323; herein incorporated by reference.

Thus, many methods may be used to reduce or eliminate the activity of a Dof polypeptide or a biologically active variant or fragment thereof. In addition, combinations of methods may be employed to reduce or eliminate the activity of at least one Dof polypeptide. It is further recognized that the level of a single Dof sequence can be modulated to produce the desired phenotype. Alternatively, is may be desirable to modulate (increase and/or decrease) the level of expression of multiple sequences having a Dof domain or a biologically active variant or fragment thereof. To decrease the level of a single Dof sequence (or highly related Dof sequences) suppression constructs can be employed that target the suppression of a specific Dof sequence or a specific subset of Dof sequences. Alternatively, if it is desirable to suppress a wide range of Dof sequences, the suppression constructs could employ sequences that are highly conserved among Dof family members, such as the Dof domain.

As discussed above, a variety of promoters can be employed to modulate the level of the Dof sequence. In one embodiment, the expression of the heterologous polynucleotide which modulates the level of at least one Dof polypeptide can be regulated by a tissue-preferred promoter, particularly, a leaf-preferred promoter (i.e., mesophyll-preferred promoter or a bundle sheath preferred promoter) and/or a seed-preferred promoter (i.e., an endosperm-preferred promoter or an embryo-preferred promoter).

### B. Methods to Modulate Carbon Fixation, Nitrogen Assimilation, Yield and/or Stress Tolerance in a Plant

Nitrogen assimilation is essential to the growth and development of plants, and therefore, large quantities of nitrogen fertilizers are used on plants to maximize crop yields. Such nitrogen fertilizers, however, aside from constituting the single most expense farm input, have negative impacts on the environment. Accordingly, methods and compositions are provided to increase the ability of a plant or plant part to assimilate nitrogen and thereby improve plant yields. Such methods comprise modulating the level of at least one Dof polynucleotide having a Dof domain in a plant or plant part and thereby increasing nitrogen assimilation (increased nitrogen use efficiency) and/or plant yield.

An increase in nitrogen assimilation can be assayed by determining the nitrogen content of the plant or plant part. For example, increasing the level of nitrogen assimilation can comprise an increase in overall nitrogen content of the plant or plant part of about 0.1%, 0.5%, 1%, 3% 5%, 10%, 15%, 20%, 25%, 30%, 35%, 40%, 45%, 50%, 55%, 60%, 65%, 70%, 75%, 80%, 85%, 90%, 95% or greater when compared to a control plant or plant part. Alternatively, the increased level of the nitrogen content can include about a 0.5 fold, 1 fold, 2 fold, 4 fold, 8 fold, 16 fold or 32 fold increase in overall increase in nitrogen level in the plant or a plant part when compared to a control plant or' plant part. Methods to assay for the level of nitrogen are known. See, for example, Yanagisawa, et al., (2004) PNAS 101:7833-7838 and Stitt, et al., (1989) Methods Enzymol. 174:518-552, both of which are herein incorporated by reference in their entirety.

An increase in nitrogen assimilation can also be assayed by determining the level of amino acids in a plant or plant part. "Increasing the level of an amino acid" includes any increase in amino acid level in the plant or plant part. For example, increasing the level of an amino acid can comprise an increase in overall amino acid content of the plant or plant part of about 0.1%, 0.5%, 1%, 3% 5%, 10%, 15%, 20%, 25%, 30%, 35%, 40%, 45%, 50%, 55%, 60%, 65%, 70%, 75%, 80%, 85%, 90%, 95% or greater when compared to a control plant or plant part. Alternatively, the increased level of the amino acid can include about a 0.5 fold, 1 fold, 2 fold, 4 fold, 8 fold, 16 fold or 32 fold increase in overall increase in amino acid level in the plant or a plant part when compared to a control plant or plant part.

It is further recognized that the increase in the level of an amino acid need not be an overall increase in amino acid level, but also includes a change in the level of a single amino acid or a combination of amino acids. In this embodiment, the increase in amino acid level need not be an overall increase in amino acid concentration, but also includes a change in the ratio of various amino acids. For example, an increase in amino acid content could be reflected through an elevated level of glutamine or glutamate, which are good markers for nitrogen utilization. See, for example, Stitt, et al., (1999) Plant Cell Environ. 22:583-621, Matt, et al., (2002) Plant J. 30:663-677, and Foyer, et al., (2003) J. Exp. Bot. 54:585-593, and Yanagisawa, et al., (2004) PNAS 101:7833-7838, all of which are herein incorporated by reference.

An increase in nitrogen assimilation (increase in nitrogen use efficiency) can also be assayed by monitoring the tolerance of the plant to nitrogen stress. Such assays are discussed in further detail elsewhere herein. Briefly, a modulation in nitrogen assimilation can be assayed by determining if the plant or plant part displays better growth under low nitrogen conditions when compared to a control plant or plant part. Such a phenotype could comprise the lack of leaf discoloration under low nitrogen growth conditions. See, for example, Yanagisawa, et al., (2004) Proc. Natl. Acad. Sci 101:7833-7838, herein incorporated by reference.

The methods and compositions further can be used to increase yield in a plant. As used herein, the term "improved yield" means any improvement in the yield of any measured plant product. The improvement in yield can comprise a 0.1%, 0.5%, 1%, 3%, 5%, 10%, 15%, 20%, 30%, 40%, 50%, 60%, 70%, 80%, 90% or greater increase in measured plant product. Alternatively, the increased plant yield can comprise about a 0.5 fold, 1 fold, 2 fold, 4 fold, 8 fold, 16 fold or 32 fold increase in measured plant products. For example, an increase in the bu/acre yield of soybeans or corn derived from a crop having the present treatment as compared with the bu/acre yield from untreated soybeans or corn cultivated under the same conditions would be considered an improved yield.

Methods are also provided to improve stress tolerance of a plant. The methods of the invention comprise modulating the level of a polypeptide having a Dof domain in a plant or plant part and thereby increasing the stress tolerance of a plant.

As used herein, abiotic stress tolerance includes, but is not limited to, increased yield, growth, biomass, health, or other measure that, when compared to an appropriate control plant, indicates tolerance to a stress which includes, but is not limited to, heat stress, salt stress, cold stress (including cold stress during germination), heat stress, water stress (including but not limited to drought stress), and nitrogen stress (including high and low nitrogen).

"Heat tolerance" is defined herein as a measure of the ability of the plant to grow under conditions where heat or warmer temperature would detrimentally affect the growth, vigor, yield, and/or size, of an appropriate control plant. Plants exhibiting an improved heat tolerance grow better under conditions of heat stress than non-heat tolerant plants.

"Cold tolerance" is defined herein as a measure of the ability of a plant to grow under conditions where cold or cooler temperature would detrimentally affect the growth, vigor, yield, and/or size, of an appropriate control plant. Plants exhibiting an improved cold tolerance grow better under conditions of cold stress than non-cold tolerant plants.

"Drought" as defined herein refers to a period of dryness that, especially when prolonged, can cause damage to crops or prevent their successful growth (i.e., decreased vigor, growth, size, root length, and/or and various other physiologic and physical measures). Plants exhibiting an improved drought tolerance grow better under conditions of drought stress than non-drought tolerant plants.

"Nitrogen stress" is defined herein as either an increase or decrease in the presence of nitrogen that can cause damage to crops or prevent their successful growth (i.e., decreased vigor, growth, size, root length, and/or and various other physiologic and physical measures). Plants that exhibit an improved tolerance to nitrogen stress grow better under conditions of low and/or high nitrogen stress than the appropriate control plants from the same species. Methods to assay for improved tolerance to nitrogen stress are known. See, for example, Sepehri, et al., (2003) Journal of Biol. Sciences 3:578-584; Henry, et al., (1992) Int J Plant Sci 153:178-85; Banzinger, et al., (2000) Breeding for Drought and Nitrogen Stress Tolerance in Maize: From Theory to Practice. Mexico, D.F.:CIMMYT; Dhugga and Waines (1989) Crop Science 29:1232; and Sicher, et al., (2005) Pysiologia Plantarum 123:219, each of which is herein incorporated by reference.

Accordingly, various methods to increase nitrogen assimilation, increase yield, and/or increase the stress tolerance of a plant are provided. In one embodiment, increasing nitrogen assimilation and/or increase yield, and/or increasing the stress tolerance of a plant or plant part comprises introducing into the plant or plant part a heterologous polynucleotide; and, expressing the heterologous polynucleotide in the plant or plant part. In this method, the expression of the heterologous polynucleotide modulates the level of at least one Dof polypeptide in the plant or plant part, where the Dof polypeptide comprises a Dof domain having an amino acid sequence set forth in SEQ ID NO: 145 or a variant or fragment of the domain.

In specific embodiments, modulation of the level of the Dof polypeptide comprises an increase in the level of at least one Dof polypeptide. In such methods, the heterologous polynucleotide introduced into the plant encodes a polypeptide having a Dof domain or a biologically active variant or fragment thereof. In specific embodiments, the heterologous polynucleotide comprises the sequence set forth in at least one SEQ ID NO: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152 or 153 and/or a biologically active variant or fragment thereof.

In other embodiments, modulating the level of at least one Dof polypeptide comprises decreasing in the level of at least one Dof polypeptide. In such methods, the heterologous polynucleotide introduced into the plant need not encode a functional Dof polypeptide, but rather the expression of the polynucleotide results in the decreased expression of a Dof polypeptide comprising a Dof domain or a biologically active variant or fragment of the Dof domain. In specific embodiments, the Dof polypeptide having the decreased level is set forth in at least one of SEQ ID NOS: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127, 130, 133, ,135, 138, 141, 144, 154, 155, 156, 157, 158, 159 or 160 or a biologically active variant or fragment thereof.

Exemplary promoters that can be used to modulate the level of a Dof polypeptide are described elsewhere herein. In one embodiment, the expression of the heterologous polynucleotide to modulate the level of at least one Dof polypeptide is regulated by a tissue-preferred promoter, particularly, a vascular-preferred promoter, a leaf-preferred promoter (i.e., mesophyll-preferred promoter or a bundle sheath preferred promoter) and/or a seed-preferred promoter (i.e., an endosperm-preferred promoter or an embryo-preferred promoter).

### EXPERIMENTAL

### Example 1 Sequence Analysis and Expression Data for Maize Dof Sequences

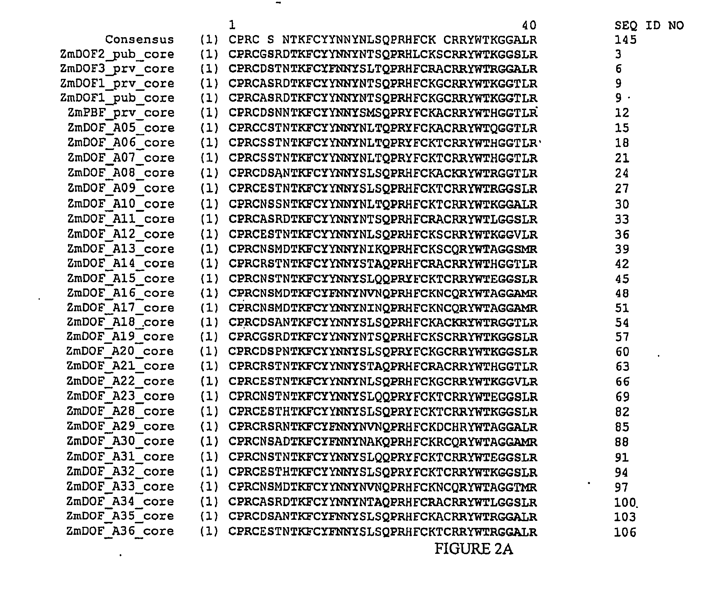

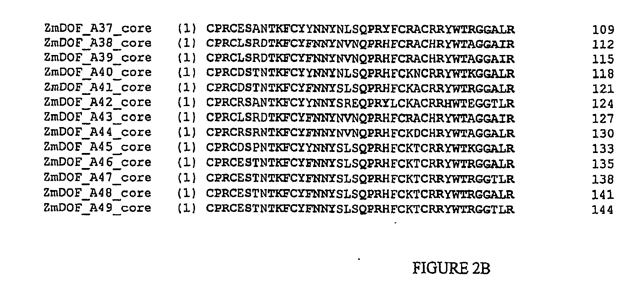

A sequence analysis of the Dof sequences set forth in SEQ ID NOS: 1-144 and 146 was performed. Figure 2 provides a summary of the Dof domain encoded by SEQ ID NOS: 1-144 and 146. The alignment set forth in Figure 2 was generated using the "Needle" program in the publicly available EMBOSS suite of tools. This program uses the Needleman-Wunsch algorithm. For proteins, the GAP default parameters (i.e., a gap penalty of 8) were used. See, also, emboss.sourceforge.net/apps/needle.html.

Table 1 provides a summary of the sequences having the highest sequence identity and similarity to the polypeptides encoded by SEQ ID NOS: 1-144 and 146, and 147-160. Table 2 provides a summary of the overall percent sequence identity shared between the polypeptides encoded by SEQ ID NOS: 1-144 and 146. The alignment data provided in Table 2 was generated using the VNT19.0 AlignX tool (February 4, 2002) which is a component of the Vector NTI Suite 7.1.

Table 3 provides a summary of the expression data of the maize Dof sequences and provides the mean parts per million for the indicated tissue with classic MPSS data.

| **Assigned Gene Name** | **SEQ ID NO** | **Translation Top Swiss-Prof Hit ID** | **Translation Top Swiss-Prot Hit E-value** | **Translation Top Swiss-Prot Hit Description** | **Translation Top Swiss-Prot Hit Best HSP Percent Identity** | **Translation Top Swiss-Prot Hit Best HSP Percent Similarity** |
|---|---|---|---|---|---|---|
| ZmDOF2_pub | 1, 2, 3 | MNBA MAIZE | 6.00E-42 | (P38564) DNA-binding protein MNB1A | 46 | 54 |
| ZmDOF3_prv | 4, 5, 6 | MNBA MAIZE | 3.00E-24 | (P38564) DNA-binding protein MNB1A | 40 | 52 |
| ZmDOF1_prv | 7, 8,9, 147, | MNBA_MAIZE 154 | 1.00E-139 | (P38564) DNA-binding protein MNB1A | 100 | 100 |
| ZmPBF_prv | 10, 11, 12 | DAG2_ARATH | 2.00E-30 | (Q9ZPY0) DOF zinc finger protein DAG2 (Dof affecting germination 2) | 33 | 44 |
| ZmDOF_A05 | 13, 14, 15, 148, 155 | DAG1_ARATH | 8.00E-29 | (Q43385) DOF zinc finger protein DAG1 (Dof affecting germination 1) (Transcription factor BBFa) (AtBBFa) (rolB domain B factor a) | 35 | 47 |
| ZmDOF_A06 | 16, 17, 18 | DAG2_ARATH | 9.00E-27 | (Q9ZPY0) DOF zinc finger protein DAG2 (Dof affecting germination 2) | 78 | 87 |
| ZmDOF_A07 | 19, 20, 21, 149, 156 | DAG2_ARATH | 9.00E-27 | (Q9ZPY0) DOF zinc finger protein DAG2 (Dof affecting germination 2) | 78 | 87 |
| ZmDOF_A08 | 22, 23, 24 | DAG2_ARATH | 6.00E-26 | (Q9ZPY0) DOF zinc finger protein DAG2 (Dof affecting germination 2) | 45 | 54 |
| ZmDOF_A09 | 25, 26, 37, 150, 157 | DAG2_ARATH | 2.00E-28 | (Q9ZPY0) DOF zinc finger protein DAG2 (Dof affecting germination 2) | 55 | 64 |
| ZmDOF_A10 | 28, 29, 30, 151, 158 | DAG1_ARATH | 7.00E-25 | (Q43385) DOF zinc finger protein DAG1 (Dof affecting germination 1) (Transcription factor BBFa) (AtBBFa) (rolB domain B factor a) | 39 | 60 |
| ZmDOF_A11 | 31,32,33 | MNBA_MAIZE | 6.00E-27 | (P38564) DNA-binding protein MNB1A | 59 | 67 |
| ZmDOF_A12 | 34, 35, 36 | MNBA_MAIZE | 1.00E-30 | (P38564) DNA-binding protein MNB1A | 46 | 55 |
| ZmDOF_A13 | 37, 38, 39 | DAG2_ARATH | 7.00E-24 | (Q9ZPY0) DOF zinc finger protein DAG2 (Dof affecting germination 2) | 58 | 75 |
| ZmDOF_A14 | 40, 41, 42, 152,159 | DAG2_ARATH | 5.00E-25 | (Q9ZPY0) DOF zinc finger protein DAG2 (Dof affecting germination 2) | 60 | 75 |
| ZmDOF_A15 | 43, 44, 45 | DAG2_ARATH | 3.00E-48 | (Q9ZPY0) DOF zinc finger protein DAG2 (Dof affecting germination 2) | 37 | 48 |
| ZmDOF_A16 | 46, 47, 48 | DAG1_ARATH | 2.00E-23 | (Q43385) DOF zinc finger protein DAG1 (Dof affecting germination 1) (Transcription factor BBFa) (AtBBFa) (rolB domain B factor a) | 38 | 58 |
| ZmDOF_A17 | 49, 50, 51 | DAG2_ARATH | 5.00E-24 | (Q9ZPY0) DOF zinc finger protein DAG2 (Dof affecting germination 2) | 57 | 78 |
| ZmDOF_A18 | 52, 53, 54 | DAG2_ARATH | 9.00E-27 | (Q9ZPY0) DOF zinc finger protein DAG2 (Dof affecting germination 2) | 64 | 73 |
| ZmDOF_A19 | 55, 56, 57 | MNBA MAIZE | 4.00E-53 | (P38564) DNA-binding protein MNB1A | 45 | 54 |
| ZmDOF_A20 | 58, 59, 60 | DAG1_ARATH | 9.00E-26 | (Q43385) DOF zinc finger protein DAG1 (Dof affecting germination 1) (Transcription factor BBFa) (AtBBFa) (rolB domain B factor a) | 68 | 82 |
| ZmDOF_A21 61, | 62, 63, 153, 160 | DAG2_ARATH | 5.00E-24 | (Q9ZPY0) DOF zinc finger protein DAG2 (Dof affecting germination 2) | 56 | 70 |
| ZmDOF A22 | 64,65,66 | MNBA MAIZE | 6.00E-28 | (P38564) DNA-binding protein MNB1A | 33 | 46 |
| ZmDOF_A23 | 67, 68, 69 | DAG2_ARATH | 7.00E-35 | (Q9ZPY0) DOF zinc finger protein DAG2 (Dof affecting germination 2) | 49 | 60 |
| ZmDOF_A24 | 70, 71, 72 | DAG2_ARATH | 2.00E-04 | (Q9ZPY0) DOF zinc finger protein DAG2 (Dof affecting germination 2) | 68 | 79 |
| ZmDOF_A25 | 73, 74, 75 | - | - | - | - | - |
| ZmDOF_A26 | 76, 77, 78 | FUS_BOVIN | 2.00E-04 | (Q28009) RNA-binding protein FUS (Pigpen protein) | 36 | 43 |
| ZmDOF_A27 | 79, 80 | MNBA_MAIZE | 3.00E-16 | (P38564) DNA-binding protein MNB1A | 73 | 80 |
| ZmDOF_A28 | 81, 82 | DAG1_ARATH | 5.00E-28 | (Q43385) DOF zinc finger protein DAG1 (Dof affecting germination 1) (Transcription factor BBFa) (AtBBFa) (rolB domain B factor a) | 64 | 77 |
| ZmDOF_A29 | 83, 84, 85 | MNBA MAIZE | 3.00E-22 | (P38564) DNA-binding protein MNB1A | 56 | 63 |
| ZmDOF_A30 | 86, 87, 88 | DAG2_ARATH | 4.00E-22 | (Q9ZPY0) DOF zinc finger protein DAG2 (Dof affecting germination 2) | 51 | 72 |
| ZmDOF_A31 | 89, 90, 91 | DAG2_ARATH | 3.00E-42 | (Q9ZPY0) DOF zinc finger protein DAG2 (Dof affecting germination 2) | 34 | 46 |
| ZmDOF_A32 | 92, 93, 94 | DOF56_ARATH | 9.00E-36 | Dof zinc finger protein DOF5.6 (AtDOF5.6) | 41 | 50 |
| ZmDOF_A33 | 95, 96, 97 | MNBA MAIZE | 5.00E-23 | (P38564) DNA-binding protein MNB1A | 39 | 50 |
| ZmDOF_A34 | 98, 99, 100 | MNBA MAIZE | 3.00E-27 | (P38564) DNA-binding protein MNB1A | 40 | 50 |
| ZmDOF_A35 | 101, 102, 103 | MNBA_MAIZE | 1.00E-26 | (P38564) DNA-binding protein MNB1A | 38 | 47 |
| ZmDOF_A36 | 104, 105, 106 | DAG2_ARATH | 8.00E-27 | (Q9ZPY0) DOF zinc finger protein DAG2 (Dof affecting germination 2) | 52 | 69 |
| ZmDOF_A37 | 107, 108, 109 | MNBA_MAIZE | 1.00E-24 | (P38564) DNA-binding protein MNB1A | 53 | 64 |
| ZmDOF_A38 | 110,111,112 | MNBA_MAIZE | 1.00E-22 | (P38564) DNA-binding protein MNB1A | 54 | 59 |
| ZmDOF_A39 | 113, 114, 115 | MNBA MAIZE | 3.00E-23 | (P38564) DNA-binding protein MNB1A | 54 | 59 |
| ZmDOF_A40 | 116, 117, 118 | DAG2_ARATH | 1.00E-28 | (Q9ZPY0) DOF zinc finger protein DAG2 (Dof affecting germination 2) | 65 | 82 |
| ZmDOF_A41 | 119, 120, 121 | MNBA_MAIZE | 8.00E-27 | (P38564) DNA-binding protein MNB1A | 39 | 48 |
| ZmDOF_A42 | 122, 123, 124 | MNBA_MAIZE | 2.00E-21 | (P38564) DNA-binding protein MNB1A | 46 | 61 |
| ZmDOF A43 | 125, 126, 127 | MNBA MAIZE | 2.00E-21 | (P38564) DNA-binding protein MNB1A | 53 | 60 |
| ZmDOF A44 | 128, 129, 130 | MNBA_MAIZE | 8.00E-22 | (P38564) DNA-binding protein MNB1A | 53 | 63 |
| ZmDOF_A45 | 131, 132, 133 | DAG1_ARATH | 5.00E-29 | (Q43385) DOF zinc finger protein DAG1 (Dof affecting germination 1) (Transcription factor BBFa) (AtBBFa) (rolB domain B factor a) | 52 | 68 |
| ZmDOF_A46 | 134,135 | DAG2_ARATH | 3.00E-26 | (Q9ZPY0) DOF zinc finger protein DAG2 (Dof affecting germination 2) | 70 | 85 |
| ZmDOF_A47 | 136, 137, 138 | DAG2_ARATH | 8.00E-26 | (Q9ZPY0) DOF zinc finger protein DAG2 (Dof affecting germination 2) | 53 | 68 |
| ZmDOF_A48 | 139, 140, 141 | DAG2_ARATH | 1.00E-25 | (Q9ZPY0) DOF zinc finger protein DAG2 (Dof affecting germination 2) | 70 | 85 |
| ZmDOF_A49 | 142, 143, 144 | DAG2_ARATH | 8.00E-26 | (Q9ZPY0) DOF zinc finger protein DAG2 (Dof affecting germination 2) | 70 | 85 |

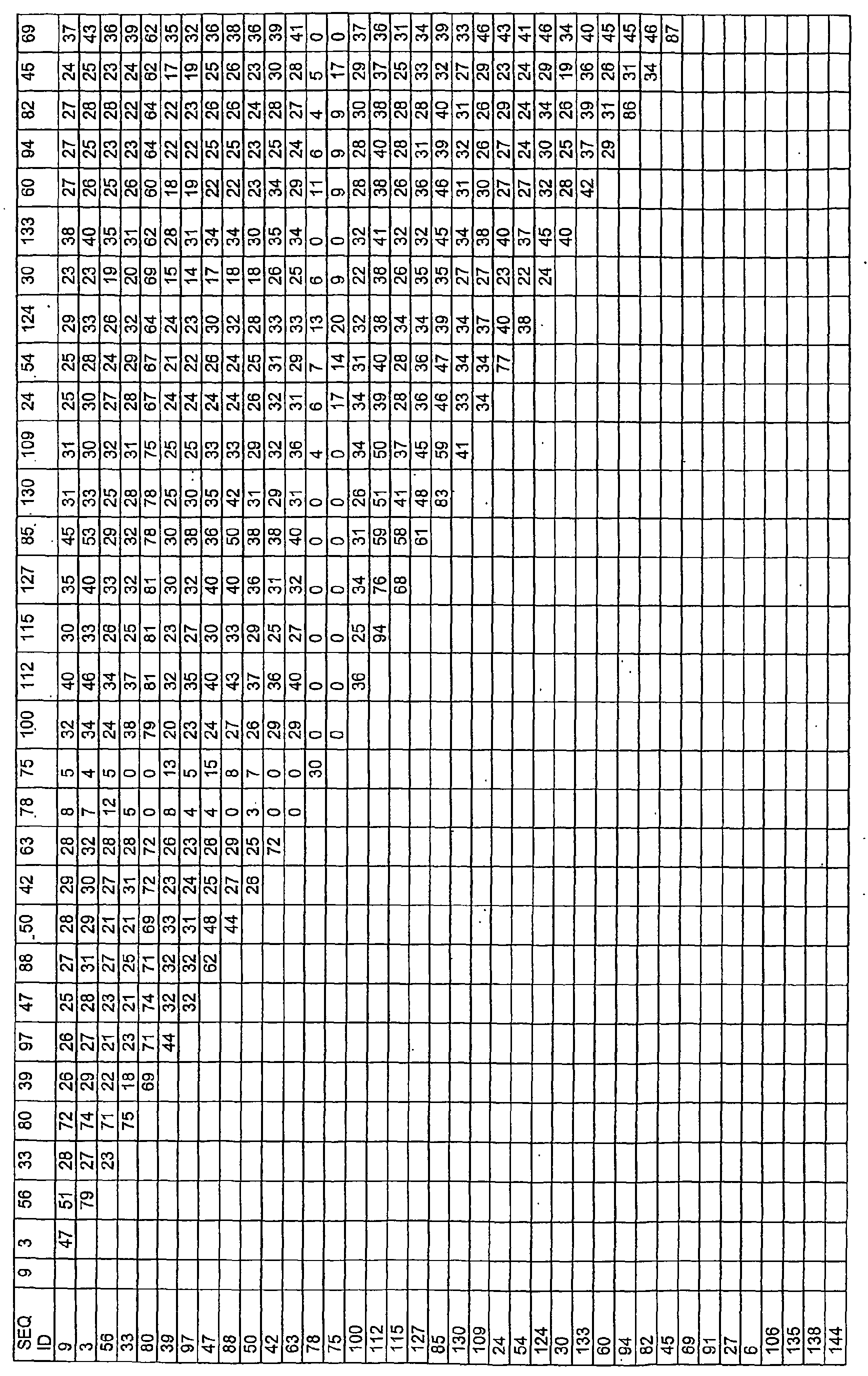

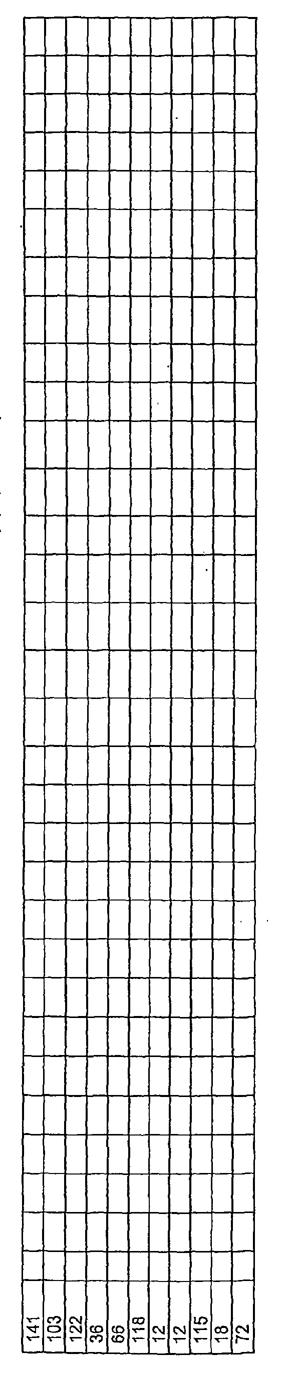

| SEQ ID | 91 | 27 | 6 | 106 | 135 | 138 | 144 | 141 | 103 | 122 | 36 | 66 | 118 | 12 | 12 | 115 | 18 | 72 |
|---|---|---|---|---|---|---|---|---|---|---|---|---|---|---|---|---|---|---|
| 9 | 26 | 25 | 27 | 37 | 44 | 24 | 24 | 25 | 27 | 28 | 29 | 30 | 42 | 24 | 24 | 26 | 26 | 19 |
| 3 | 28 | 28 | 24 | 40 | 48 | 27 | 27 | 28 | 31 | 31 | 34 | 34 | 46 | 23 | 23 | 26 | 26 | 18 |
| 56 | 25 | 24 | 24 | 32 | 38 | 25 | 25 | 23 | 26 | 25 | 30 | 31 | 38 | 20 | 20 | 22 | 22 | 17 |
| 33 | 25 | 23 | 24 | 27 | 29 | 24 | 24 | 22 | 23 | 22 | 26 | 28 | 35 | 22 | 22 | 26 | 24 | 17 |
| 80 | 62 | 66 | 73 | 68 | 68 | 68 | 68 | 68 | 69 | 69 | 67 | 64 | 72 | 67 | 67 | 67 | 62 | 75 |
| 39 | 16 | 19 | 16 | 28 | 30 | 17 | 20 | 17 | 19 | 17 | 22 | 24 | 33 | 20 | 20 | 21 | 20 | 13 |
| 97 | 19 | 21 | 20 | 25 | 29 | 18 | 22 | 17 | 19 | 18 | 21 | 23 | 35 | 22 | 22 | 18 | 18 | 12 |
| 47 | 24 | 21 | 23 | 27 | 29 | 22 | 23 | 21 | 22 | 20 | 21 | 24 | 37 | 21 | 21 | 23 | 24 | 15 |
| 88 | 23 | 24 | 23 | 35 | 40 | 25 | 26 | 26 | 24 | 22 | 26 | 28 | 36 | 22 | 22 | 24 | 26 | 18 |
| 50 | 24 | 21 | 22 | 27 | 29 | 22 | 22 | 20 | 24 | 22 | 26 | 30 | 35 | 20 | 20 | 23 | 20 | 13 |
| 42 | 29 | 31 | 37 | 33 | 36 | 31 | 31 | 28 | 32 | 32 | 31 | 37 | 36 | 28 | 28 | 30 | 31 | 26 |
| 63 | 28 | 27 | 36 | 33 | 39 | 30 | 30 | 28 | 31 | 31 | 29 | 33 | 36 | 25 | 25 | 29 | 31 | 25 |
| 78 | 4 | 7 | 7 | 0 | 0 | 6 | 7 | 3 | 6 | 4 | 2 | 5 | 0 | 21 | 21 | 94 | 70 | 67 |
| 75 | 17 | 13 | 5 | 0 | 0 | 5 | 5 | 0 | 0 | 6 | 4 | 5 | 0 | 0 | 0 | 26 | 26 | 26 |
| 100 | 30 | 26 | 31 | 34 | 35 | 30 | 30 | 27 | 27 | 24 | 31 | 34 | 34 | 24 | 24 | 27 | 26 | 18 |
| 112 | 35 | 44 | 50 | 40 | 39 | 40 | 40 | 40 | 44 | 39 | 39 | 42 | 47 | 34 | 34 | 38 | 39 | 36 |
| 115 | 23 | 30 | 31 | 32 | 37 | 27 | 27 | 26 | 33 | 30 | 27 | 28 | 35 | 22 | 22 | 24 | 26 | 18 |
| 127 | 34 | 32 | 39 | 36 | 40 | 34 | 34 | 35 | 37 | 32 | 36 | 38 | 38 | 28 | 28 | 30 | 30 | 22 |
| 85 | 35 | 30 | 53 | 31 | 32 | 37 | 37 | 31 | 34 | 29 | 41 | 46 | 52 | 34 | 34 | 36 | 34 | 52 |
| 130 | 29 | 28 | 34 | 28 | 32 | 30 | 30 | 29 | 28 | 26 | 31 | 35 | 41 | 25 | 25 | 24 | 24 | 18 |
| 109 | 30 | 27 | 31 | 41 | 53 | 28 | 28 | 28 | 31 | 31 | 30 | 31 | 45 | 30 | 30 | 31 | 27 | 17 |
| 24 | 24 | 26 | 26 | 43 | 53 | 27 | 29 | 26 | 29 | 30 | 26 | 31 | 37 | 26 | 26 | 22 | 25 | 17 |
| 54 | 24 | 25 | 24 | 39 | 45 | 23 | 24 | 22 | 29 | 28 | 26 | 32 | 36 | 25 | 25 | 23 | 24 | 17 |
| 124 | 28 | 27 | 33 | 37 | 38 | 27 | 27 | 24 | 29 | 28 | 27 | 26 | 39 | 26 | 26 | 27 | 26 | 19 |
| 30 | 20 | 18 | 22 | 28 | 31 | 22 | 24 | 20 | 22 | 23 | 21 | 24 | 32 | 18 | 18 | 20 | 19 | 11 |
| 133 | 40 | 42 | 38 | 40 | 44 | 41 | 41 | 38 | 40 | 40 | 40 | 48 | 48 | 33 | 33 | 32 | 33 | 21 |
| 60 | 24 | 22 | 24 | 36 | 41 | 25 | 26 | 26 | 27 | 26 | 25 | 29 | 41 | 25 | 25 | 24 | 26 | 16 |
| 94 | 31 | 26 | 26 | 37 | 43 | 26 | 26 | 24 | 30 | 30 | 28 | 29 | 38 | 25 | 25 | 26 | 27 | 18 |
| 82 | 32 | 29 | 30 | 37 | 44 | 28 | 28 | 27 | 32 | 33 | 29 | 33 | 37 | 26 | 26 | 27 | 29 | 18 |
| 45 | 59 | 26 | 24 | 37 | 44 | 26 | 27 | 26 | 27 | 27 | 28 | 27 | 36 | 22 | 22 | 23 | 21 | 13 |
| 69 | 68 | 40 | 40 | 40 | 44 | 42 | 42 | 43 | 40 | 40 | 44 | 43 | 47 | 39 | 39 | 43 | 39 | 32 |
| 91 | | 25 | 24 | 37 | 42 | 26 | 27 | 26 | 29 | 29 | 29 | 27 | 36 | 23 | 23 | 24 | 22 | 13 |
| 27 | | | 34 | 43 | 47 | 36 | 38 | 33 | 32 | 33 | 27 | 29 | 41 | 22 | 22 | 23 | 26 | 20 |
| 6 | | | | 56 | 65 | 39 | 40 | 36 | 37 | 40 | 23 | 27 | 43 | 20 | 20 | 22 | 23 | 15 |
| 106 | | | | | 75 | 88 | 87 | 74 | 50 | 50 | 42 | 43 | 48 | 32 | 32 | 32 | 34 | 32 |
| 135 | | | | | | 84 | 84 | 96 | 54 | 53 | 46 | 47 | 55 | 37 | 37 | 38 | 39 | 42 |
| 138 | | | | | | | 99 | 83 | 44 | 45 | 28 | 28 | 34 | 22 | 22 | 22 | 23 | 17 |
| 144 | | | | | | | | 84 | 43 | 43 | 28 | 28 | 34 | 22 | 22 | 23 | 25 | 18 |
| 141 | | | | | | | | | 43 | 43 | 26 | 26 | 36 | 21 | 21 | 21 | 22 | 16 |
| 103 | | | | | | | | | | 91 | 26 | 28 | 42 | 25 | 25 | 26 | 25 | 20 |
| 122 | | | | | | | | | | | 27 | 29 | 43 | 26 | 26 | 26 | 24 | 19 |
| 36 | | | | | | | | | | | | 76 | 45 | 22 | 22 | 24 | 25 | 17 |
| 66 | | | | | | | | | | | | | 44 | 24 | 24 | 26 | 27 | 18 |
| 118 | | | | | | | | | | | | | | 37 | 37 | 34 | 35 | 23 |
| 12 | | | | | | | | | | | | | | | 100 | 41 | 43 | 36 |
| 12 | | | | | | | | | | | | | | | | 41 | 43 | 36 |
| 115 | | | | | | | | | | | | | | | | | 64 | 59 |
| 18 | | | | | | | | | | | | | | | | | | 95 |
| 72 | | | | | | | | | | | | | | | | | | |

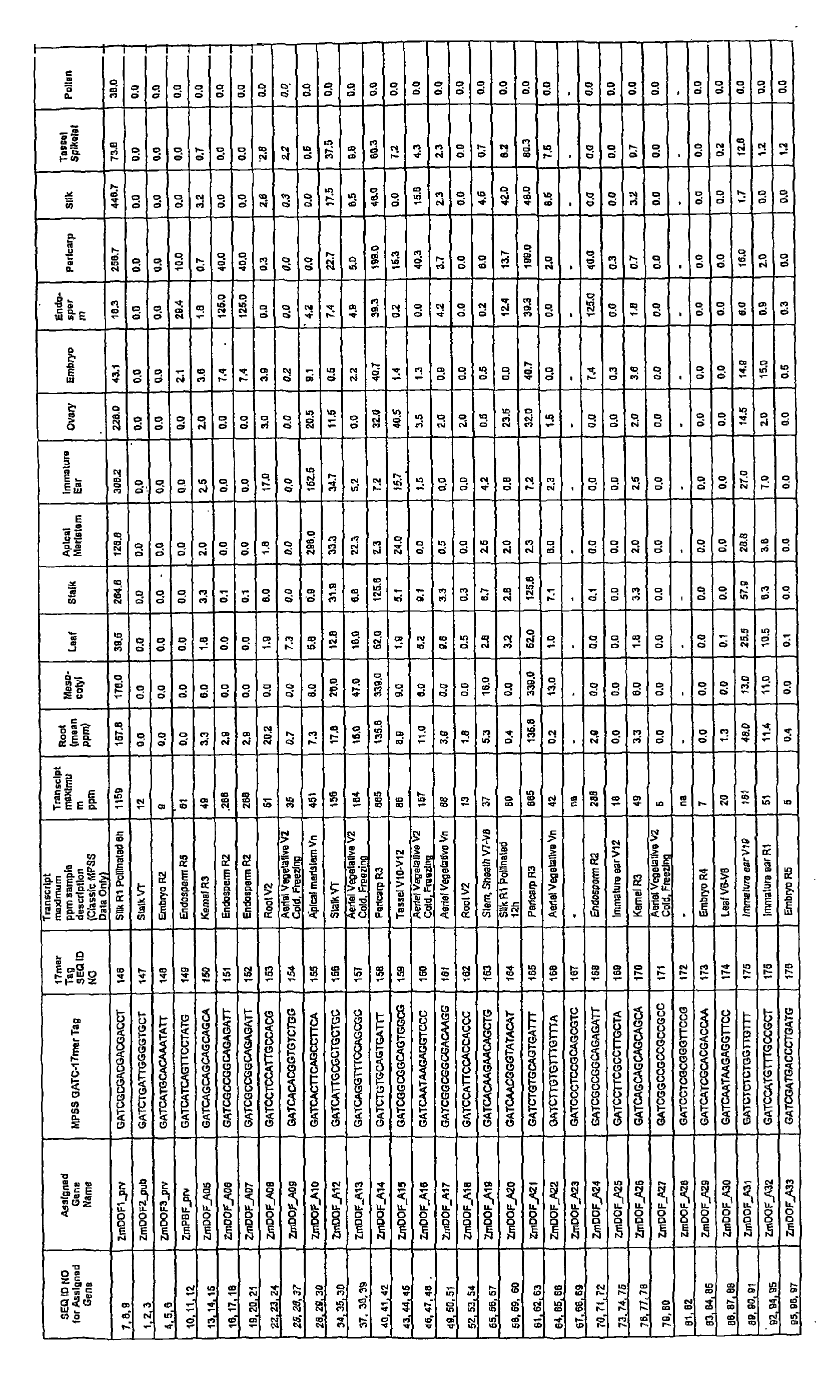

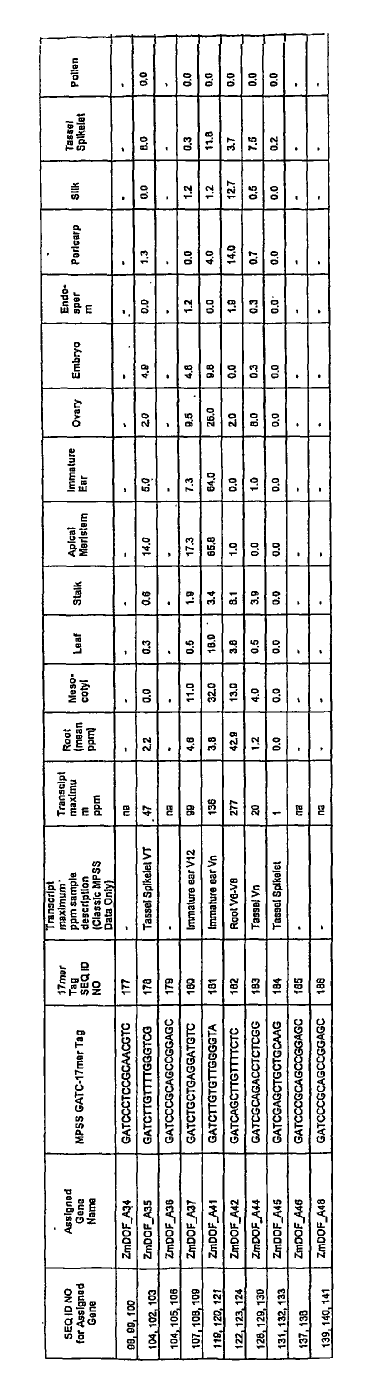

### Example 2. Overexpression of Dof Sequences to Modulate Nitrogen Assimilation in Maize

Immature maize embryos from greenhouse donor plants are bombarded with a plasmid containing a Dof sequence (such as Zm-DOF1, 9, 10, 11, 14, 15, 16, 17, 18, 20, 21, or 22) under the control of the UBI promoter and the selectable marker gene PAT (Wohlleben, et al., (1988) Gene 70:25-37), which confers resistance to the herbicide Bialaphos. Alternatively, the selectable marker gene is provided on a separate plasmid. Transformation is performed as follows. Media recipes follow below.

### Preparation of Target Tissue

The ears are husked and surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5cm target zone in preparation for bombardment.

A plasmid vector comprising the Dof sequence operably linked to a ubiquitin promoter is made. This plasmid DNA plus plasmid DNA containing a PAT selectable marker is precipitated onto 1.1 µm (average diameter) tungsten pellets using a CaCl₂ precipitation procedure as follows: 100 µl prepared tungsten particles in water; 10 µl (1 µg) DNA in Tris EDTA buffer (1 µg total DNA); 100 µl 2.5 M CaCl₂; and, 10 µl 0.1 M spermidine.

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment.

The sample plates are bombarded at level #4 in particle gun (U.S. Patent No. 5,240,855). All samples receive a single shot at 650 PSI, with a total of ten aliquots taken from each tube of prepared particles/DNA.

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity. Plants are monitored and scored for an increase in nitrogen use efficiency, increase yield, or an increase in stress tolerance.

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2,4-D, and 2.88 g/l L-proline (brought to volume with D-I H₂O following adjustment to pH 5.8 with KOH); 2.0 g/l Gelrite (added after bringing to volume with D-I H₂O); and 8.5 mg/l silver nitrate (added after sterilizing the medium and cooling to room temperature). Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H₂O following adjustment to pH 5.8 with KOH); 3.0 g/l Gelrite (added after bringing to volume with D-I H₂O); and 0.85 mg/l silver nitrate and 3.0 mg/l bialaphos(both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H₂O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H₂O after adjusting to pH 5.6); 3.0 g/l Gelrite (added after bringing to volume with D-I H₂O) and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H₂O), 0.1 g/l myo-inositol, and 40.0 g/l sucrose (brought to volume with polished D-I H₂O after adjusting pH to 5.6); and 6 g/l bacto-agar (added after bringing to volume with polished D-I H₂O), sterilized and cooled to 60°C.

### Example 3. Suppression of Multiple Dof sequences in Maize.

Multiple maize Dof sequences can be targeted for suppression using an RNAi construct which is designed to target a nucleotide sequence encoding the Dof domain sequence from, for example, Zm-Dof1. Briefly, to target multiple Dof sequences, the DNA sequence encoding the Dof domain (or a sequence having at least 70%, 80%, 90%, or greater sequence identity to the Dof domain) is employed and used to make inverted repeats in a vector. For example, a constant having Zm-Dof 1 (Dof Domain):: ADH1 intron 1::ATTB2:: Zm-Dof 1(Dof Domain can be employed).

For *Agrobacterium*-mediated transformation of maize with one or more suppression constructs that specifically target at least one Dof sequence, the method of Zhao is employed (U.S. Patent No. 5,981,840, and PCT patent publication WO98/32326; the contents of which are hereby incorporated by reference). Briefly, immature embryos are isolated from maize and the embryos contacted with a suspension of *Agrobacterium,* where the bacteria are capable of transferring the Dof suppression sequence operably linked to a seed-preferred promoter to at least one cell of at least one of the immature embryos (step 1: the infection step). In this step the immature embryos are immersed in an *Agrobacterium* suspension for the initiation of inoculation. The embryos are co-cultured for a time with the *Agrobacterium* (step 2: the co-cultivation step). The immature embryos are cultured on solid medium following the infection step. Following this co-cultivation period an optional "resting" step is contemplated. In this resting step, the embryos are incubated in the presence of at least one antibiotic known to inhibit the growth of *Agrobacterium* without the addition of a selective agent for plant transformants (step 3: resting step). The immature embryos are cultured on solid medium with antibiotic, but without a selecting agent, for elimination of *Agrobacterium* and for a resting phase for the infected cells. Next, inoculated embryos are cultured on medium containing a selective agent and growing transformed callus is recovered (step 4: the selection step). The immature embryos are cultured on solid medium with a selective agent resulting in the selective growth of transformed cells. The callus is then regenerated into plants (step 5: the regeneration step), and calli grown on selective medium are cultured on solid medium to regenerate the plants.

A decrease of Dof sequence expression can be measured directly by assaying for the level of Dof transcripts, or the decrease in expression can be measured by assaying for an increase in nitrogen assimilation, an increase in a stress response or an increase in yield.

### Example 4. Suppression of Individual Target Dof sequences in Maize.

An individual Dof sequence or, alternatively, a subset of Dof sequences, can be targeted for suppression by using an RNAi construct which is designed to target the desired subset of Dof sequences. For example, DOF1, DOF10, and DOF14 can be individually targeted. Briefly, to suppress individual Dof genes the DNA sequence specific to each Dof (i.e., 3' UTR, 5' UTR, or specific regions of the CDS) can be used to make the inverted repeats. For example, the Dof 1 3' UTR can be targeted, a region of the Dof 14 coding sequence or a region of the Dof 10 coding sequence. For example, a construct comprising Zm-Dof 1 (3' UTR):: ADH1 intron 1:: ATTB2:: Zm-Dof 1 (3' UTR) or a construct comprising Zm Dof 14:: ADH1 intron 1:: ATTB2:: Zm Dof 14 or a construct comprising Zm Dof 10:: ADH1 intron 1:: ATTB2:: Zm Dof 10 are constructed.

For *Agrobacterium-*mediated transformation of maize with one or more suppression constructs that specifically target at least one Dof sequence, the method of Zhao is employed (U.S. Patent No. 5,981,840, and PCT patent publication WO98/32326; the contents of which are hereby incorporated by reference). Briefly, immature embryos are isolated from maize and the embryos contacted with a suspension of *Agrobacterium*, where the bacteria are capable of transferring the Dof suppression sequence operably linked to a seed-preferred promoter to at least one cell of at least one of the immature embryos (step 1: the infection step). In this step the immature embryos are immersed in an *Agrobacterium* suspension for the initiation of inoculation. The embryos are co-cultured for a time with the *Agrobacterium* (step 2: the co-cultivation step). The immature embryos are cultured on solid medium following the infection step. Following this co-cultivation period an optional "resting" step is contemplated. In this resting step, the embryos are incubated in the presence of at least one antibiotic known to inhibit the growth of *Agrobacterium* without the addition of a selective agent for plant transformants (step 3: resting step). The immature embryos are cultured on solid medium with antibiotic, but without a selecting agent, for elimination of *Agrobacterium* and for a resting phase for the infected cells. Next, inoculated embryos are cultured on medium containing a selective agent and growing transformed callus is recovered (step 4: the selection step). The immature embryos are cultured on solid medium with a selective agent resulting in the selective growth of transformed cells. The callus is then regenerated into plants (step 5: the regeneration step), and calli grown on selective medium are cultured on solid medium to regenerate the plants.

A decrease of Dof sequence expression can be measured directly by assaying for the level of Dof transcripts, or the decrease in expression can be measured by assaying for an increase in nitrogen assimilation, an increase in a stress response or an increase in yield.

### Example 5. Modulating Nitrogen Assimilation in Soybean

Soybean embryos are bombarded with a plasmid containing the suppression cassette for at least one Dof sequence operably linked to a leaf-preferred promoter as follows. To induce somatic embryos, cotyledons, 3-5 mm in length dissected from surface-sterilized, immature seeds of the soybean cultivar A2872, are cultured in the light or dark at 26°C on an appropriate agar medium for six to ten weeks. Somatic embryos producing secondary embryos are then excised and placed into a suitable liquid medium. After repeated selection for clusters of somatic embryos that multiplied as early, globular-staged embryos, the suspensions are maintained as described below.

Soybean embryogenic suspension cultures can maintained in 35 ml liquid media on a rotary shaker, 150 rpm, at 26°C with florescent lights on a 16:8 hour day/night schedule. Cultures are subcultured every two weeks by inoculating approximately 35 mg of tissue into 35 ml of liquid medium.

Soybean embryogenic suspension cultures may then be transformed by the method of particle gun bombardment (Klein, et al., (1987) Nature (London) 327:70-73, U.S. Patent No. 4,945,050).

A selectable marker gene that can be used to facilitate soybean transformation is a transgene composed of the 35S promoter from Cauliflower Mosaic Virus (Odell, et al., (1985) Nature 313:810-812), the hygromycin phosphotransferase gene from plasmid pJR225 (from *E*. *coli;* Gritz, et al., (1983) Gene 25:179-188), and the 3' region of the nopaline synthase gene from the T-DNA of the Ti plasmid of *Agrobacterium tumefaciens.* The expression cassette comprising the suppression cassette for the Dof sequence operably linked to the leaf-preferred promoter can be isolated as a restriction fragment. This fragment can then be inserted into a unique restriction site of the vector carrying the marker gene.

To 50 µl of a 60 mg/ml 1 µm gold particle suspension is added (in order): 5 µl DNA (1 µg/µl), 20 µl spermidine (0.1 M), and 50 µl CaCl₂ (2.5 M). The particle preparation is then agitated for three minutes, spun in a microfuge for 10 seconds and the superatant removed. The DNA-coated particles are then washed once in 400 µl 70% ethanol and resuspended in 40 µl of anhydrous ethanol. The DNA/particle suspension can be sonicated three times for one second each. Five microliters of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi, and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post-bombardment with fresh media containing 50 mg/ml hygromycin. This selective media can be refreshed weekly. Seven to eight weeks post-bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### Example 6. Manipulation of ZM-DOFs in Maize

The coding regions of several ZM-DOFs were driven by ZM-UBI PRO, a strong constitutive promoter, for overexpression in maize. A terminator sequence (NOS or PlNll) was used downstream of the DOFs coding region. In all the transgenic events 'UBI:MOPAT:PINII' was used as a herbicide resistant selectable marker and in some of these transgenic 'ZM-LTP2PRO:RFP:PINII' was used to sort out the transgenic seeds from the segregating non-transgenic seeds. In some cases other promoter such as ZM-PEPC1 and ZM-GOS2 were also used to drive a mesophyll cell specific and a weak constitutive expression of ZM-DOFs, respectively. All these vectors were transformed in to introEF09B genotype following Agrobacterium-mediated maize transformation protocols. In all the overexpression transgenic events the molecular analysis in terms of tranegne copy number, transgene expression and actin control was performed in T0 events. In each contruct the T0 events were sorted for high, medium and low transgene expression level within that construct (see, Table 4). Single-copy trasnsgene expressing (and/or RFP expressing seeds) events were selected to advance for further experimentations. ZM-DOF1 allele from B73 inbred when driven by ZM-UBI promoter appeared to be lethal in several different transformation experiments. Hence, the published (Yanagisawa and Izui (1992) JBC 288:16028) ZM-DOF1 allele from the maize inbred H84 was cloned by RT-PCR. DOF1 allele from B73 and H84 inbreds are 97% identical at protein level. Phosphorylation sites prediction analysis reveal that serine at 47 position, which is a putative phosphorylation site with 100% probability, is present in B73 but missing in H84 allele of ZM-DOF1. Post translational modification (such as phosphorylation) of transcription factors have been known to modify their activities. Transformation experiments with the ZM-DOF1 allele from H84 inbred are currently in progress. RNAi vectors for Zm-DOF1, 7, 10 and 14 were also generated. Two of the DOF1 RNAi vectors (PHP26339 and 26340) were dropped from the list as the molecular analysis of T0 transgenic events didn't show any significant reduction in endogenous DOF1 mRNA. Single trangene copy and transgene expressing plants from 11 different ZM-DOFs related PHPs are currently in the genetic nursery (GN) to bulk up the seeds for further experiments and test crosses for field evaluation in future. Six PHPs are currently in transformation pipe line and T0 events are expected to be in the green house by end of this year. In addition, the Agrobacteria containing 12 different DOF related PHPs are ready for maize transformation. The details of all DOF-related PHPs and their current status are summarized Table 4.

**TABLE4**

| **EST** | **PHP# or pRG#** | **PROMOTER** | **GENE** |
|---|---|---|---|
| cpls1s.pk012.e5 (partial) | PHP25990 | ZM-UBI | ZM-DOF1-B73 |
| cpls1s.pk012.e5 (partial) | PHP25991 | ZM-PEPC1 | ZM-DOF1-B73 |
| cta1n.pk0008.h10 | PHP25995 | ZM-UBI | ZM-DOF14 |
| p0031.ccmay70rb | PHP25996 | ZM-UBI | ZM-DOF17 |
| csc1c.pk006.n23 | PHP25997 | ZM-UBI | ZM-DOF20 |
| p0095.cwsbj71ra | PHP25998 | ZM-UBI | ZM-DOF16 |
| p0111.cipmh48r | PHP26337 | ZM-UBI | ZM-DOF9 |
| cpf1c.pk006.i22a | PHP26338 | ZM-UBI | ZM-DOF10 |
| cpls1s.pk012.e5 (partial) | PHP26339 | ZM-UBI | ZM-DOF1 RNAi |
| cpls1s.pk012.e5 (partial) | PHP26340 | ZM-UBI | ZM-DOF1 RNAi |
| cie1s.pk002.a5 | PHP27070 | ZM-UBI | ZM-DOF10 RNAi |
| cpf1c.pk006.i22a | PHP26910 | ZM-UBI | ZM-DOF11 |
| cen1.pk0109.b4:fis | PHP27748 | ZM-UBI | ZM-DOF7 |
| cen1.pk0109.b4:fis | PHP27749 | ZM-GOS2 | ZM-DOF7 |
| cco1n.pk082.a18 | PHP28364 | ZM-UBI | ZM-DOF12 |
| cds3f.pk001.j20 | PHP28365 | ZM-UBI | ZM-DOF13 |
| cfp5n.pk066.b23 | PHP28866 | ZM-UBI | ZM-DOF5 (7-2) |
| cfp5n.pk066.b23 | PHP28863 | ZM-GOS2 | ZM-DOF5 (7-2) |
| No EST, by RT-PCR | PHP28864 | ZM-UBI | ZM-DOF1-H84 |
| No EST, by RT-PCR | PHP28865 | ZM-PEPC1 | ZM-DOF1-H84 |
| cbn10.pk0039.e7 | PHP26911 | ZM-UBI | ZM-DOF15 |
| cest1s.pk002.f23 | PHP26924 | ZM-UBI | ZM-DOF21 |
| cepe7.pk0012.h2 | PHP26912 | ZM-UBI | ZM-DOF18 |
| cco1n.pk072.j23 | PHP26913 | ZM-UBI | ZM-DOF22 |
| cta1n.pk0008.h10 | PHP26923 | ZM-UBI | ZM-DOF14 RNAi |
| cen1.pk0109.b4:fis | PHP27750 | ZM-UBI | ZM-DOF7 RNAi |
| cpls1s.pk012.e5 (partial) | pRG974 | 35S-TET-OP | ZM-DOF1-B73 |
| cfp7n.pk005.a14 | pRG1020 | ZM-UBI | ZM-DOF8 |
| cfp3n.pk069.i19. | pRG1029 | ZM-UBI | ZM-DOF28 |
| cfp7n.pk069.o21 | pRG1021 | ZM-UBI | ZM-DOF30 |
| cfp1n.pk071.e24 | pRG1022 | ZM-UBI | ZM-DOF33 |
| cfp2n.pk002.k24 | pRG1023 | ZM-UBI | ZM-DOF34 |

Continued detailed phenotypic analysis (visual, molecular, biochemical/enzymatic, physiological, stress etc.) of these maize transgenic events is ongoing.

### Example 7. Overexpression of ZM-DOFs in Arabidopsis

In addition to overexpressing ZM-DOFs in maize, Arabidopsis transgenic lines overexpressing ZM-DOFs under the control a constitutive ZM-UBI promoter were generated. In some cases a week constitutive (ZM-GOS2) or a mysophill cell specific promoter (ZM-PEPC1) was also used to drive the expression of ZM-DOFs. A terminator sequence (NOS or PINII) was used downstream of the DOFs coding region. In all the transgenic events 'UBI:MOPAT:PINII' was used as a herbicide resistant selectable marker. These overexpression vectors were transformed in to Arabidopsis thaliana ecotype Columbia-0 by Agobacterium mediated 'Floral-Dip' method (Clough and Bent (1998) *Plant Journal* 16:735). T0 seeds were screened for T1 transformants in soil for herbicide resistance. For molecular analysis of the transgenic T1 events, RT-PCRs were conducted to detect the transgene expression, actin control and the presence of genomic DNA in the RNA preparations. In each contruct the T1 events were sorted for high, medium and low transgene expression level within that construct (see attached, Table 5). Transgene expressing events were advanced for further studies. In total Arabidopsis transgenic lines were generated for 26 different overexpression PHPs representing 22 different ZM-DOFs. The status of various ZM-DOFs overexpression experiments in Arabidopsis is summarized in the following table.

**Table5**

| **EST** | **PHP#/pRG#** | **PROMOTER** | **GENE** |
|---|---|---|---|
| RT-PCR | PHP25990 | ZM-UBI | ZM-DOF1-B73 |
| RT-PCR | PHP25991 | ZM-PEPC1 | ZM-DOF1-B73 |
| No EST, by RT-PCR | PHP28864 | ZM-UBI | ZM-DOF1-H84 |
| No EST, by RT-PCR | PHP28865 | ZM-PEPC1 | ZM-DOF1-H84 |
| cfp5n-pk066.b23 | PHP28863 | ZM-GOS2 | ZM-DOF5 |
| cfp5n.pk066.b23 | PHP28866 | ZM-UBI | ZM-DOF5 |
| cen1.pk0109.b4:fis | PHP27748 | ZM-UBI | ZM-DOF7 |
| cen1.pk0109.b4:fis | PHP27749 | ZM-GOS2 | ZM-DOF7 |
| cfp7n.pk005.a14 | pRG1020 | ZM-UBI | ZM-DOF8 |
| p0111.cipmh48r | PHP26337 | ZM-UBI | ZM-DOF9 |
| cpf1c.pk006.i22a | PHP26338 | ZM-UBI | ZM-DOF10 |
| cpf1c.pk006.i22a | PHP26910 | ZM-UBI | ZM-DOF11 |
| cco1n.pk082.a18 | PHP28364 | ZM-UBI | ZM-DOF12 |
| cds3f.pk001.j20 | PHP28365 | ZM-UBI | ZM-DOF13 |
| cta1n.pk0008.h10 | PHP25995 | ZM-UBI | ZM-DOF14 |
| cbn10.pk0039.e7 | PHP26911 | ZM-UBI | ZM-DOF15 |
| p0095.cwsbj71ra | PHP25998 | ZM-UBI | ZM-DOF16 |
| p0031.ccmay70rb | PHP25996 | ZM-UBI | ZM-DOF17 |
| cepe7.pk0012.h2 | PHP26912 | ZM-UBI | ZM-DOF18 |
| csc1c.pk006.n23 | PHP25997 | ZM-UBI | ZM-DOF20 |
| cest1s.pk002.f23 | PHP26924 | ZM-UBI | ZM-DOF21 |
| cco1n.pk072.j23 | PHP26913 | ZM-UBI | ZM-DOF22 |
| cfp3n.pk069.i19 | pRG1029 | ZM-UBI | ZM-DOF28 |
| cfp7n.pk069.o21 | pRG1021 | ZM-UBI | ZM-DOF30 |
| cfp1n.pk071.e24 | pRG1022 | ZM-UBI | ZM-DOF33 |
| cfp2n.pk002.k24 | pRG1023 | ZM-UBI | ZM-DOF34 |

Continued detailed phenotypic analysis (visual, molecular, biochemical/enzymatic, physiological, stress etc.) of these Arabidopsis transgenic lines is ongoing.

### Example 8. UBI PRO::ZM-DOF1-H84 (PHP28865) overexpression In Arabidopsis up-regulates AtPEPC1 and AtPPDK1

In an initial experiment to overexpress in Arabidopsis the ZM-DOF1 allele from B73 maize inbred under the control of ZM-UBI promoter did not yield any transgene expressing events. This observation was consistent with that found in maize transformation experiments. A clone of the published ZM-DOF1 (Yanagisawa and Izui (1992) *JBC* 288:16028) allele from H84 maize inbred by RT-PCR on young leaf RNA was prepared. DOF1 allele from B73 and H84 inbreds are 97% identical at protein level. Over-expression of ZM-DOF1 allele from H84 inbred with ZM-UBI promoter resulted in several transgene expressing events and several of the events showed a significant up-regulation of AtPEPC1 (At3g14940) and AtPPDK1 (At5g08570), which is consistent with the results reported in a published study (Yanagisawa, et al., (2004) PNAS 101:7833). Experiments also generated several transgenic Arabidopsis lines overexpressing ZM-DOF1. alleles from both B73 and H84 inbreds under the control of a mesopyhl cell specific ZM-PEPC1 promoter having no detectable significant difference in AtPEPC1, AtPPDK1 expression levels.

### Example 9 ZM-DOF7 is an Endosperm specific gene

Initial Lynx MPSS expression analysis showed that ZM-DOF7 is expressed only in endosperm libraries. In order to confirm this observation, a saturated RT-PCRs (35 cycles) were conducted on total RNA isolated from various organs of maize inbred B73. Total RNAs isolated from V3 seedlings, immature ear, mature leaf, endosperm (14DAP), young leaf, internode and roots were used to perform RT-PCR experiment with ZM-DOF7 gene specific (P894BC#11843 & P895BC#188944) and AT-Actin2 (P815BC#99547 & P816BC#99548) primers. The RT-PCR data reveals that ZM-DOF7 is expressed in endosperm (14DAP) only among different organs tested.

### Example 10 Sub-cellular localization of ZM-DOF10 and ZM-DOF14

In order to determine the sub-cellular localization, ZM-DOF10 and ZM-DOF14 were tagged with RFP and driven by a strong constitutive ZM-UBI promoter. A vector expressing RFP alone under the control of ZM-UBI promoter was also used as a control. These three constructs were bombarded into the onion epidermal cells for RFP fusion protein localization. The results clearly indicate the ZM-DOF10-RFP and ZM-DOF14-RFP are predominantly localized in nucleus whereas RFP alone is present more or less everywhere (e.g., cytosol). Initial Lynx MPSS expression analysis showed that ZM-DOF10 is expressed in apical meristems and immature ear libraries. To further determine the spatial expression pattern of ZM-DOF10, *in-situ* hybridization experiments were performed on V3 shoot apical meristem (SAM) and immature ear tips and bases. The comparison of the data from hybridization of sense and antisense strands of ZM-DOF10 suggests that DOF10 was expressed in specific cell layers in V3 SAM whereas in immature ear this gene is expressed only in the tip (actively growing cells, meristem).

### Example 11. Variants of Dof Sequences

### A. Variant Nucleotide Sequences of Dof Sequences That Do Not Alter the Encoded Amino Acid Sequence

The Dof nucleotide sequence set forth in SEQ ID NO: 1, 2, 4, 5, 7, 8, 10, 11, 13, 14, 16, 17, 19, 20, 22, 23, 25, 26, 28, 29, 31, 32, 34, 35, 37, 38, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 95, 96, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152, or 153 is used to generate variant nucleotide sequences having the nucleotide sequence of the open reading frame with about 70%, 76%, 81%, 86%, 92% and 97% nucleotide sequence identity when compared to the starting unaltered ORF nucleotide sequence of the appropriate SEQ ID NO. These functional variants are generated using a standard codon table. While the nucleotide sequence of the variant is altered, the amino acid sequence encoded by the open reading frame does not change.

### B. Variant Amino Acid Sequences of a Dof Sequence

Variant amino acid sequences of Dof sequence are generated. In this example, one amino acid is altered. Specifically, the open reading frame set forth in SEQ ID NO: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 11 B, 121, 124, 127, 130, 133, 135, 138, 141, 144, 154, 155, 156, 157, 158, 159 or 160 is reviewed to determined the appropriate amino acid alteration. The selection of the amino acid to change is made by consulting the protein alignment (with the other orthologs and other gene family members from various species). See Figures 1 and Table 1. An amino acid is selected that is deemed not to be under high selection pressure (not highly conserved) and which is rather easily substituted by an amino acid with similar chemical characteristics (i.e., similar functional side-chain). Using the protein alignment set forth in Figure 1, Table 1 and the consensus sequence set forth in SEQ ID NO: 145, an appropriate amino acid can be changed. Once the targeted amino acid is identified, the procedure outlined in Example 6A is followed. Variants having about 70%, 75%, 81%, 86%, 92% and 97% nucleic acid sequence identity to SEQ ID NO: 3, 6, 9, 12, 15, 18, 21, 24, 27, 30, 33, 36, 39, 42, 45, 48, 51, 54, 57, 60, 63, 66, 69, 72, 75, 78, 80, 82, 85, 88, 91, 94, 97, 100, 103, 106, 109, 112, 115, 118, 121, 124, 127., 130, 133, 135, 138, 141, 144, 154, 155, 156, 157, 158, 159 or 160 are generated using this method.

### C. Additional Variant Amino Acid Sequences of Dof Sequences

In this example, artificial protein sequences are created having 82%, 87%, 92% and 97% identity relative to the reference protein sequence. This latter effort requires identifying conserved and variable regions from the alignment set forth in. Figures 1 and 2 and then the judicious application of an amino acid substitutions table. These parts will be discussed in more detail below.

Largely, the determination of which amino acid sequences are altered is made based on the conserved regions among Dof protein or among the other Dof proteins. See Figures 1, 2 and Table 1. Based on the sequence alignment, the various regions of the Dof sequences that can likely be altered are represented in lower case letters, while the conserved regions are represented by capital letters. It is recognized that conservative substitutions can be made in the conserved regions below without altering function. In addition, one of skill will understand that functional variants of the Dof sequence of the invention can have minor non-conserved amino acid alterations in the conserved domain.

Artificial protein sequences are then created that are different from the original in the intervals of 80-85%, 85-90%, 90-95%, and 95-100% identity. Midpoints of these intervals are targeted, with liberal latitude of plus or minus 1%, for example. The amino acids substitutions will be effected by a custom Perl script. The substitution table is provided below in Table 6.

**Table 6. Substitution Table**

| **Amino Acid** | **Strongly Similar and Optimal Substitution** | **Rank of Order to Change** | **Comment** |
|---|---|---|---|
| I | L,V | 1 | 50:50 substitution |
| L | I,V | 2 | 50:50 substitution |
| V | I,L | 3 | 50:50 substitution |
| A | G | 4 | |
| G | A | 5 | |
| D | E | 6 | |
| E | D | 7 | |
| W | Y | 8 | |
| Y | W | 9 | |
| S | T | 10 | |
| T | S | 11 | |
| K | R | 12 | |
| R | K | 13 | |
| N | Q | 14 | |
| Q | N | 15 | |
| F | Y | 16 | |
| M | L | 17 | First methionine cannot change |
| H | | Na | No good substitutes |
| C | | Na | No good substitutes |
| P | | Na | No good substitutes |

First, any conserved amino acids in the protein that should not be changed is identified and "marked off" for insulation from the substitution. The start methionine will of course be added to this list automatically. Next, the changes are made.

H, C, and P are not changed in any circumstance. The changes will occur with isoleucine first, sweeping N-terminal to C-terminal. Then leucine, and so on down the list until the desired target it reached. Interim number substitutions can be made so as not to cause reversal of changes. The list is ordered 1-17, so start with as many isoleucine changes as needed before leucine, and so on down to methionine. Clearly many amino acids will in this manner not need to be changed. L, I and V will involved a 50:50 substitution of the two alternate optimal substitutions.

The variant amino acid sequences are written as output. Perl script is used to calculate the percent identities. Using this procedure, variants of Dof sequences are generating having about 82%, 87%, 92% and 97% amino acid identity to the starting unaltered ORF nucleotide sequence of the corresponding SEQ lD NO.

The article "a" and "an" are used herein to refer to one or more than one (i.e., to at least one) of the grammatical object of the article. By way of example, "an element" means one or more element.

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, certain changes and modifications may be practiced within the scope of the appended claims.

## Claims

1. An isolated polypeptide comprising an amino acid sequence selected from:
(a) the amino acid sequence comprising SEQ ID NO: 39, 97, 3, 6, 9, 12, 15, 18, 21, 24, 27, 30, 33, 36, 42, 45, 48, 51, 54, 57, 60, 63, 66, 69, 72, 75, 78, 80, 82, 85, 88, 91, 94, 100, 103, 106, 109, 112, 115, 118, 121, 124, 127, 130, 133, 135, 138, 141, 144, 154, 155, 156, 157, 158, 159 or 160;
(b) the amino acid sequence comprising at least 90% sequence identity to SEQ ID NO: 39, 97, 3, 6, 9, 12, 15, 18, 21, 24, 27, 30, 33, 36, 42, 45, 48, 51, 54, 57, 60, 63, 66, 69, 72, 75, 78, 80, 82, 85, 88, 91, 94, 100, 103, 106, 109, 112, 115, 118, 121, 124, 127, 130, 133, 135, 138, 141, 144, 154, 155, 156, 167, 158, 159 or 160 wherein said polypeptide has the ability to modulate transcription; and,
(c) the amino acid sequence comprising at least 40 consecutive amino acids of SEQ ID NO: 39, 97, 3, 6, 9, 12, 15, 18, 21, 24, 27, 30, 33, 36, 42, 45, 48, 51, 54, 57, 60, 63, 66, 69, 72, 75, 78, 80, 82, 85, 88, 91, 94, 100, 103, 106, 109, 112, 115, 118, 121, 124, 127, 130, 133, 135, 138, 141, 144, 154, 155, 156, 157, 158, 159 or 160, wherein said polypeptide retains the ability to modulate transcription.

2. An isolated polynucleotide comprising a nucleotide sequence selected from:
(a) the nucleotide sequence comprising SEQ ID NO: 37, 38, 95, 96, 1, 2, 4, 5, 7, 8, 10, 11, 13, 14, 16, 17, 19, 20, 22, 23, 25, 26, 28, 29, 31, 32, 34, 35, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143,146, 147, 148, 149, 150, 151, 152 or 153;
(b) the nucleotide sequence encoding an amino acid sequence comprising SEQ ID NO: 39, 97, 3, 6, 9, 12, 15, 18, 21, 24, 27, 30, 33, 36, 42, 45, 48, 51, 54, 57, 60, 63, 66, 69, 72, 75, 78, 80, 82, 85, 88, 91, 94, 100, 103, 106, 109, 112, 115, 118, 121, 124, 127, 130, 133, 135, 138, 141, 144, 154, 155, 156, 157, 158, 159 or 160;
(c) the nucleotide sequence comprising at least 90% sequence identity to SEQ ID NO: 37, 38, 95, 96, 1, 2, 4, 5, 7, 8, 10, 11, 13, 14, 16, 17, 19, 20, 22, 23, 25, 26, 28, 29, 31, 32, 34, 35, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152 or 153 wherein said polynucleotide encodes a polypeptide having the ability to modulate transcription or the expression of the polynucleotide in a plant decrease the expression of at least one Dof polypeptide;
(d) the nucleotide sequence comprising at least 40 consecutive nucleotides of SEQ ID NO: 37, 38, 95, 96, 1, 2, 4, 5, 7, 8, 10, 11, 13, 14, 16, 17, 19, 20, 22, 23, 25, 26, 28, 29, 31, 32, 34, 35, 40, 41, 43, 44, 46, 47, 49, 50, 52, 53, 55, 56, 58, 59, 61, 62, 64, 65, 67, 68, 70, 71, 73, 74, 76, 77, 79, 81, 83, 84, 86, 87, 89, 90, 92, 93, 98, 99, 101, 102, 104, 105, 107, 108, 110, 111, 113, 114, 116, 117, 119, 120, 122, 123, 125, 126, 128, 129, 131, 132, 134, 136, 137, 139, 140, 142, 143, 146, 147, 148, 149, 150, 151, 152 or 153 or a complement thereof, wherein said polynucleotide encodes a polypeptide having the ability to modulate transcription or the expression of the polynucleotide in a plant decrease the expression of at least one Dof polypeptide;
(e) the nucleotide sequence that hybridizes under stringent conditions to the complement of the nucleotide sequence of a) or b), wherein said stringent conditions comprise hybridization in 50% formamide, 1 M NaCl, 1% SDS at 37°C, and a wash in 0.1X SSC at 60°C to 65°C, wherein said polynucleotide encodes a polypeptide having the ability to modulate transcription or the expression of the polynucleotide in a plant decrease the expression of at least one Dof polypeptide; and,
(f) the nucleotide sequence encoding an amino acid sequence having at least 90% sequence identity to SEQ ID NO: 39, 97, 3, 6, 9, 12, 15, 18, 21, 24, 27, 30, 33, 36, 42, 45, 48, 51, 54, 57, 60, 63, 66, 69, 72, 75, 78, 80, 82, 85, 88, 91, 94, 100, 103, 106, 109, 112, 115, 118, 121, 124, 127, 130, 133, 135, 138, 141, 144. 154, 155, 156, 157, 158, 159 or 160 wherein said polynucleotide encodes a polypeptide having has the ability to modulate transcription or expression of the polynucleotide in a plant decrease the expression of at least one Dof polypeptide.

3. An expression cassette comprising the polynucleotide of claim 2.

4. The expression cassette of claim 3, wherein said polynucleotide is operably linked to a promoter that drives expression in a plant, wherein optionally said promoter is a tissue-preferred promoter, a constitutive promoter, or an inducible promoter, and wherein said tissue-preferred promoter is optionally selected from a leaf-preferred promoter, a mesophyll-preferred promoter, a bundle sheath-preferred promoter, a seed-preferred promoter, an endosperm-preferred promoter, or an embryo-preferred promoter.

5. A plant or plant part comprising a polynucleotide operably linked to a promoter that drives expression in the plant, wherein said polynucleotide comprises the nucleotide sequence of claim 2.

6. A method for modulating the level of a Dof polypeptide in a plant or a plant part comprising introducing into said plant or plant part a heterologous polynucleotide comprising a nucleotide sequence of claim 2 and expressing said heterologous polynucleotide, wherein optionally said polynucleotide is operably linked to a promoter as defined in claim 4, and wherein optionally the level of the Dof polypeptide is
(a) increased; or
(b) decreased.

7. The method of claim 6, wherein the yield of the plant is increased; or the nitrogen use efficiency in said plant or plant part is increased; or the stress response of the plant is improved.

8. A method for increasing nitrogen use efficiency in a plant, increasing yield in a plant or improving the stress response of a plant comprising
a) introducing into said plant a heterologous polynucleotide; and,
b) expressing said polynucleotide in the plant from an operably linked leaf-preferred promoter or a vascular preferred promoter;
wherein expression of said heterologous polynucleotide modulates the level of at least one Dof polypeptide comprising an amino acid sequence set forth in SEQ ID NO: 145 or a biologically active variant or fragment thereof, wherein said biologically active variant comprises at least 80% sequence identity to SEQ ID NO: 145 and said Dof polypeptide is capable of modulating transcription,
wherein optionally said heterologous polynucleotide encodes a Dof polypeptide, and wherein optionally expression of said heterologous polynucleotide decreases the level of at least one Dof polynucleotide.

9. The method of claim 8, wherein said leaf-preferred promoter comprises a bundle sheath-preferred promoter or a mesophyll-preferred promoter.

10. An isolated expression cassette comprising a polynucleotide operably linked to a heterologous leaf-preferred promoter or a vascular preferred promoter,
wherein said polynucleotide is selected from:
a) a polynucleotide encoding a Dof polypeptide comprising an amino acid sequence set forth in SEQ ID NO: 145;
b) a polynucleotide encoding a Dof polypeptide comprising an amino acid sequence having at least 80% sequence identity to SEQ ID NO: 145, said Dof polypeptide is capable of modulating transcription; and,
c) a polynucleotide which when expressed in a plant decrease the expression level of a Dof polypeptide comprising an amino acid sequence set forth in SEQ ID NO: 145; and,
d) a polynucleotide which when expressed in a plant decrease the expression level of a Dof polypeptide comprising an amino acid sequence having at least 80% sequence identity to SEQ ID NO: 145, said Dof polypeptide is capable of modulating transcription.

11. A plant or plant part comprising a heterologous expression cassette of claim 10.

12. The plant or plant part of claim 5 or 11, wherein said plant is:
(a) a monocot, which is optionally maize, wheat, rice, barley, sorghum, or rye; or
(b) a dicot, which is optionally soybean, Brassica, sunflower, cotton, or alfalfa.

13. The method of claim 6, or plant or plant part of claim 11 or 12, wherein said polynucleotide is stably incorporated into the genome of the plant.

14. The plant part of claim 12 or 13, wherein said plant part is a cell.

15. A seed having stably incorporated into its genome the polynucleotide of claim 2 or the expression cassette of claim 10.


SEQUENCE LISTING

<110> Pioneer Hi-Bred International, Inc.

<120> DOF (DNA Binding With One Finger)
  Sequences and Methods of Use

<130> 2039

<150> 60/735,645
<151> 2005-11-10

<160> 160

<170> FastSEQ for Windows Version 4.0

<210> 1
<211> 1037
<212> DNA
<213> Zea mays

<400> 1
ccaagggcta ccccgtgccg gttgccaagg aggagaggcc ggcgccgggc ggcgacccgt 60
gcccgcggtg cgggtcgcgg gacaccaagt tctgctacta caacaactac aacacgtcgc 120
agccgcgcca cttgtgcaag tcgtgccgcc gctactggac caagggcggg tccctccgca 180
acgtccccgt cgggggcggc acccgcaaga gcagcagcag ctcctcatca tcctcggcgg 240
cgacgacgac gacgacgacg acgtcaacct cgcccggcgc cgcgcccaag gccacgaaac 300
ggtcgaagaa ctctaagcgc cgccgcgtcg cgcctgcccc ggaccccgcc gcgcccggca 360
cggacgcctc caccgccgac gtcgcgagca cggcaccgtc cgcagcgacg gtggctgcct 420
cggagaagcc gagcgcgacg gagcacgcag cggcggctgt cgccactgag aagccgccgg 480
ccgcgcctcc ggtgtccgtc ggcgctttcg cggacacgtc cccggcgcca gatgctggga 540
gcggcggcgt tagggagctg ctgccgcacc cgagccgctt cgagtggccg tcgggctgcg 600
acctggggcc gccctactgg ggctggggca ccagcgtgct cgccgacacc gacccggcgc 660
tgttcctcaa tctgccgtga gtgacccgtg acaacgccat ggcgcccatg gttaactagt 720
caaccatcat cacacggtcg acgagatgag aggaacagag cacgcagtgc gtcgggctct 780
tgaagctagg tgctgtagta gtctcctaag cttactgttg tctagaccga cagtctcgcg 840
tacttgtgta gttgtgttga tggtttttaa tcgccgcttt gtactgcaat tctagtaaaa 900
aggctagcat gggatagccg ggctctgcac ggatctgtgc ttgctttgac agtttgatct 960
gattggggtg ctttcttatt ttaaggccat gagaatcgca agaacaggga gcggcgaact 1020
tcgaagaaaa aaaaaaa 1037

<210> 2
<211> 678
<212> DNA
<213> Zea mays

<400> 2
aagggctacc ccgtgccggt tgccaaggag gagaggccgg cgccgggcgg cgacccgtgc 60
ccgcggtgcg ggtcgcggga caccaagttc tgctactaca acaactacaa cacgtcgcag 120
ccgcgccact tgtgcaagtc gtgccgccgc tactggacca agggcgggtc cctccgcaac 180
gtccccgtcg ggggcggcac ccgcaagagc agcagcagct cctcatcatc ctcggcggcg 240
acgacgacga cgacgacgac gtcaacctcg cccggcgccg cgcccaaggc cacgaaacgg 300
tcgaagaact ctaagcgccg ccgcgtcgcg cctgccccgg accccgccgc gcccggcacg 360
gacgcctcca ccgccgacgt cgcgagcacg gcaccgtccg cagcgacggt ggctgcctcg 420
gagaagccga gcgcgacgga gcacgcagcg gcggctgtcg ccactgagaa gccgccggcc 480

gcgcctccgg tgtccgtcgg cgctttcgcg gacacgtccc cggcgccaga tgctgggagc 540
ggcggcgtta gggagctgct gccgcacccg agccgcttcg agtggccgtc gggctgcgac 600
ctggggccgc cctactgggg ctggggcacc agcgtgctcg ccgacaccga cccggcgctg 660
ttcctcaatc tgccgtga 678

<210> 3
<211> 225
<212> PRT
<213> Zea mays

<400> 3
Lys Gly Tyr Pro Val Pro Val Ala Lys Glu Glu Arg Pro Ala Pro Gly
1               5                   10                  15
Gly Asp Pro Cys Pro Arg Cys Gly Ser Arg Asp Thr Lys Phe Cys Tyr
            20                  25                  30
Tyr Asn Asn Tyr Asn Thr Ser Gln Pro Arg His Leu Cys Lys Ser Cys
        35                  40                  45
Arg Arg Tyr Trp Thr Lys Gly Gly Ser Leu Arg Asn Val Pro Val Gly
    50                  55                  60
Gly Gly Thr Arg Lys Ser Ser Ser Ser Ser Ser Ser Ser Ser Ala Ala
65                  70                  75                  80
Thr Thr Thr Thr Thr Thr Thr Ser Thr Ser Pro Gly Ala Ala Pro Lys
                85                  90                  95
Ala Thr Lys Arg Ser Lys Asn Ser Lys Arg Arg Arg Val Ala Pro Ala
            100                 105                 110
Pro Asp Pro Ala Ala Pro Gly Thr Asp Ala Ser Thr Ala Asp Val Ala
        115                 120                 125
Ser Thr Ala Pro Ser Ala Ala Thr Val Ala Ala Ser Glu Lys Pro Ser
    130                 135                 140
Ala Thr Glu His Ala Ala Ala Ala Val Ala Thr Glu Lys Pro Pro Ala
145                 150                 155                 160
Ala Pro Pro Val Ser Val Gly Ala Phe Ala Asp Thr Ser Pro Ala Pro
                165                 170                 175
Asp Ala Gly Ser Gly Gly Val Arg Glu Leu Leu Pro His Pro Ser Arg
            180                 185                 190
Phe Glu Trp Pro Ser Gly Cys Asp Leu Gly Pro Pro Tyr Trp Gly Trp
        195                 200                 205
Gly Thr Ser Val Leu Ala Asp Thr Asp Pro Ala Leu Phe Leu Asn Leu
    210                 215                 220
Pro
225

<210> 4
<211> 1582
<212> DNA
<213> Zea mays

<400> 4
cttgctatcc tgatccaacg tcacactgat actatatact catcgactgc tgctagtata 60
tactagctgc gcagtttgaa cgacgaattg aaatcttcag ctttttattt attattgatt 120
gactcgatcg tatatcttcc tgttccaccc atctcctatc tattgatgct ttattcattt 180
ccttttctca tctttaccct ggttcgattt tgcagcacca gcagcagcag caccaccacc 240
agcagcaagg ccacggccac ctgcagattg ccgtcagcgg cagcggcgac gccaatcatg 300
agctcctcca gcaaccaatc atggggggag cgcttcccga tggaggagga ggaggtggtg 360
gcgggggaca ggttgggggc cccgccaagc ccatgtccat ggcggagcgc gcgcgcctcg 420
cgaggatccc actgccggag ccgggactca agtgcccgcg ctgcgactca accaacacca 480
agttctgcta cttcaacaac tactccctca cgcagccgcg ccacttctgc cgggcctgcc 540
gccgctactg gacgcgtggc ggcgcgctcc gcaacgtgcc ggtcggtggc ggataccgcc 600
gccacgccaa gcgcgccaag cccaagcagc agcagcagca cgcggccgcc gggaccggag 660
ctgccaacgg cgcgatgcag cagcctcccg ctgggtctat ggcgtcgtcg gccgccgcct 720

gcaccgcgac cacgacgatg acgaccaacg cgctggacgc cgggcccggc ggcatgctgc 780
ccatgctgcc gccgctcgtc cgcctagcag acttcgacgc aatgagcctc ggctccagct 840
tctccgggat atcgtccatg gggaagcccg gatccatcgg cgcggcctgc tacccgcact 900
ctgtcggcgg gctggagcag tggagggtgc agcagatgca gagcttcccg ttcttgcatg 960
cgatggacca gggcccgctg gggccacctc tggccatggc gatggcggcg ccaggaggga 1020
tgttccagct aggtctagac accaccagtg ataacagccg tggcggtggc ggcggcggct 1080
gcggcgaaga cgggtcgtct gcgggagagg cgctccatat gatgcaagca gccaccaaga 1140
gggagagcta cccggcacca ccaagagcca tgtacggcga ccaacaccac aatcacctcg 1200
ctgctgctgg tggctacact tcctattcca ccaatgctgc tgcaggtaac catctcttgt 1260
aatggccggc cgatcgatcg atcgagagct caacaattca agtgtcctgc tatagctagc 1320
tactacgacg tcgtgctacg atcgtatcgg tttcggttcg ttcctacaaa tatagcctag 1380
agatagagtg tctctgtctg tgtgatcgat ggtattgtta tgatcatata gaaaagacca 1440
gtgtagcatg catgcacttg ttgcaatgtt tgctttcaag aagaaactgg agggaggaga 1500
ggctcggttt ggatgctgat catgcacaaa tattactagt gtctacagct gctacttcat 1560
tataaaaaaa aaaaaaaaaa aa 1582

<210> 5
<211> 870
<212> DNA
<213> Zea mays

<400> 5
atgtccatgg cggagcgcgc gcgcctcgcg aggatcccac tgccggagcc gggactcaag 60
tgcccgcgct gcgactcaac caacaccaag ttctgctact tcaacaacta ctccctcacg 120
cagccgcgcc acttctgccg ggcctgccgc cgctactgga cgcgtggcgg cgcgctccgc 180
aacgtgccgg tcggtggcgg ataccgccgc cacgccaagc gcgccaagcc caagcagcag 240
cagcagcacg cggccgccgg gaccggagct gccaacggcg cgatgcagca gcctcccgct 300
gggtctatgg cgtcgtcggc cgccgcctgc accgcgacca cgacgatgac gaccaacgcg 360
ctggacgccg ggcccggcgg catgctgccc atgctgccgc cgctcgtccg cctagcagac 420
ttcgacgcaa tgagcctcgg ctccagcttc tccgggatat cgtccatggg gaagcccgga 480
tccatcggcg cggcctgcta cccgcactct gtcggcgggc tggagcagtg gagggtgcag 540
cagatgcaga gcttcccgtt cttgcatgcg atggaccagg gcccgctggg gccacctctg 600
gccatggcga tggcggcgcc aggagggatg ttccagctag gtctagacac caccagtgat 660
aacagccgtg gcggtggcgg cggcggctgc ggcgaagacg ggtcgtctgc gggagaggcg 720
ctccatatga tgcaagcagc caccaagagg gagagctacc cggcaccacc aagagccatg 780
tacggcgacc aacaccacaa tcacctcgct gctgctggtg gctacacttc ctattccacc 840
aatgctgctg caggtaacca tctcttgtaa 870

<210> 6
<211> 289
<212> PRT
<213> Zea mays

<400> 6
Met Ser Met Ala Glu Arg Ala Arg Leu Ala Arg Ile Pro Leu Pro Glu
1               5                   10                  15
Pro Gly Leu Lys Cys Pro Arg Cys Asp Ser Thr Asn Thr Lys Phe Cys
            20                  25                  30
Tyr Phe Asn Asn Tyr Ser Leu Thr Gln Pro Arg His Phe Cys Arg Ala
        35                  40                  45
Cys Arg Arg Tyr Trp Thr Arg Gly Gly Ala Leu Arg Asn Val Pro Val
    50                  55                  60
Gly Gly Gly Tyr Arg Arg His Ala Lys Arg Ala Lys Pro Lys Gln Gln
65                  70                  75                  80
Gln Gln His Ala Ala Ala Gly Thr Gly Ala Ala Asn Gly Ala Met Gln
                85                  90                  95
Gln Pro Pro Ala Gly Ser Met Ala Ser Ser Ala Ala Ala Cys Thr Ala
            100                 105                 110
Thr Thr Thr Met Thr Thr Asn Ala Leu Asp Ala Gly Pro Gly Gly Met
        115                 120                 125

Leu Pro Met Leu Pro Pro Leu Val Arg Leu Ala Asp Phe Asp Ala Met
    130                 135                 140
Ser Leu Gly Ser Ser Phe Ser Gly Ile Ser Ser Met Gly Lys Pro Gly
145                 150                 155                 160
Ser Ile Gly Ala Ala Cys Tyr Pro His Ser Val Gly Gly Leu Glu Gln
                165                 170                 175
Trp Arg Val Gln Gln Met Gln Ser Phe Pro Phe Leu His Ala Met Asp
            180                 185                 190
Gln Gly Pro Leu Gly Pro Pro Leu Ala Met Ala Met Ala Ala Pro Gly
        195                 200                 205
Gly Met Phe Gln Leu Gly Leu Asp Thr Thr Ser Asp Asn Ser Arg Gly
    210                 215                 220
Gly Gly Gly Gly Gly Cys Gly Glu Asp Gly Ser Ser Ala Gly Glu Ala
225                 230                 235                 240
Leu His Met Met Gln Ala Ala Thr Lys Arg Glu Ser Tyr Pro Ala Pro
                245                 250                 255
Pro Arg Ala Met Tyr Gly Asp Gln His His Asn His Leu Ala Ala Ala
            260                 265                 270
Gly Gly Tyr Thr Ser Tyr Ser Thr Asn Ala Ala Ala Gly Asn His Leu
        275                 280                 285
Leu

<210> 7
<211> 1225
<212> DNA
<213> Zea mays

<400> 7
gcccccctcc tttcaactcg ctcgctcgct cgccttccat ctttctccct ctgtcggcag 60
tctgcagcag cccagcgccg tcgcgcatgc aggaggcgtc atcggcggcg gcggcggggg 120
ccgagcccgg ccgtcggcgg gcgcagcatc agttcgccgg cgtggacctc cggcggccca 180
aggggtacgc ggcgccggcg ccggcgccgg cggtgggcga gggggacccg tgcccgcggt 240
gtgcgtcgcg ggacaccaag ttctgctact acaacaacta caacacctcc cagccgcgcc 300
acttctgcaa gggctgccgc cgctactgga ccaagggtgg cacgctgcgc aacgtccccg 360
tcggcggcgg cacccgcaag aagccctcct cctcctcctc gtcgtcgtcc tacgtggccg 420
ccgcggacgc cgacaggcag cccaagaaga agcccgccag caagaagcgc cgcgtcgtgg 480
cgccggcccc ggagctcgcc accgcggccg acccaggcaa gacggcgacc accaccacga 540
cgacgagcga gatcaccacg gagactggcg cgctggagga ctccgactcc ctggcgcacc 600
tgctgctgca gcccgggaca gaggacgcgg aggccgtcgc gctcggcctc ggcctctccg 660
acttcccctc cgccgggaag gcggtgctgg acgacgagga ctcgttcgtg tggcccgccg 720
cgtcgttcga catgggcgcg tgctgggccg gcgcagggtt cgccgacccg gaccccgcct 780
gcatcttcct caacctcccg tgacagccac acacggtcac ggcgccgagg cgtacgtcct 840
cctgctctgc tctgctctgc gcctgatgat gagtgcaagg aaatggagcg ctttgattct 900
tcagctattt gatgcatctg ctaattgatt tggcagtggt ggtaaggtac acgcacgatg 960
agctgactgc gtggttcttg gtttgggcct tagtatatga tgacgatgac gatgatacta 1020
gttgtatgtg tgacgtgaga gacgatcgcg acgacgacct tgaattttag tttctgagat 1080
gaaaaaggtc aactagtggc ctctgtatga ctttagtttt actcatgaac tcatcagatg 1140
gctggctatg gctgtggctg tggctgtggc taactcatcg tcaaaaaaaa aaaaaaaaaa 1200
aaaaaaaaaa aaaaaaaaaa aaaaa                                       1225

<210> 8
<211> 723
<212> DNA
<213> Zea mays

<400> 8
atgcaggagg cgtcatcggc ggcggcgggg gccgagcccg gccgtcgggc ggcgcagcat 60
cagttcgccg gcgtggacct ccggcggccc aaggggtacg cgccgccggc gccggcggtg 120
agcgaggggg acccgtgccc gcggtgtgcg tcgcgggaca ccaagttctg ctactacaac 180

aactacaaca cctcccagcc gcgccacttc tgcaagggct gccgccgcta ctggaccaag 240
ggcggcacgc tgcgcaacgt ccccgtcggc ggcggcaccc gcaagaagcc ctccttgtcg 300
tcgtcgtcgt cgtcgtccta cgcggccgcc gcggacgccg acaggcagcc caagaagaag 360
cccgccagca agaagcgccg cgtcgtggcg ccggccccgg agctcgccgc cgcggccgac 420
ccaggcaaga cggcacccac cacgacgacg acgacgacga cgacgagcga gatcaccacg 480
gagactggcg cgctggagga ctccgactcc ctggcgcacc tgctgctgca gcccgggaca 540
gaggacgcgg aggccgtcgc gctcgggctc ggcctctccg acttcccctc cgccgggaag 600
gcggtgctgg acgacgagga ctcgttcgtg tggcccgccg cgtcgttcga catgggcgcg 660
tgctgggccg gcgcagggtt cgccgacccg gaccccgcat gcatcttcct caacctcccg 720
tga 723

<210> 9
<211> 240
<212> PRT
<213> Zea mays

<400> 9
Met Gln Glu Ala Ser Ser Ala Ala Ala Gly Ala Glu Pro Gly Arg Arg
1               5                   10                  15
Ala Ala Gln His Gln Phe Ala Gly Val Asp Leu Arg Arg Pro Lys Gly
            20                  25                  30
Tyr Ala Pro Pro Ala Pro Ala Val Ser Glu Gly Asp Pro Cys Pro Arg
        35                  40                  45
Cys Ala Ser Arg Asp Thr Lys Phe Cys Tyr Tyr Asn Asn Tyr Asn Thr
    50                  55                  60
Ser Gln Pro Arg His Phe Cys Lys Gly Cys Arg Arg Tyr Trp Thr Lys
65                  70                  75                  80
Gly Gly Thr Leu Arg Asn Val Pro Val Gly Gly Gly Thr Arg Lys Lys
                85                  90                  95
Pro Ser Leu Ser Ser Ser Ser Ser Ser Ser Tyr Ala Ala Ala Ala Asp
            100                 105                 110
Ala Asp Arg Gln Pro Lys Lys Lys Pro Ala Ser Lys Lys Arg Arg Val
        115                 120                 125
Val Ala Pro Ala Pro Glu Leu Ala Ala Ala Ala Asp Pro Gly Lys Thr
    130                 135                 140
Ala Pro Thr Thr Thr Thr Thr Thr Thr Thr Thr Ser Glu Ile Thr Thr
145                 150                 155                 160
Glu Thr Gly Ala Leu Glu Asp Ser Asp Ser Leu Ala His Leu Leu Leu
                165                 170                 175
Gln Pro Gly Thr Glu Asp Ala Glu Ala Val Ala Leu Gly Leu Gly Leu
            180                 185                 190
Ser Asp Phe Pro Ser Ala Gly Lys Ala Val Leu Asp Asp Glu Asp Ser
        195                 200                 205
Phe Val Trp Pro Ala Ala Ser Phe Asp Met Gly Ala Cys Trp Ala Gly
    210                 215                 220
Ala Gly Phe Ala Asp Pro Asp Pro Ala Cys Ile Phe Leu Asn Leu Pro
225                 230                 235                 240

<210> 10
<211> 1490
<212> DNA
<213> Zea mays

<400> 10
cttcttccca gcgacaagag aaaggattag aaaaaggaaa gatccatgga catgatctcc 60
ggcagcactg cagcaacatc aacaccccac aacaaccaac aggcggtgat gttgtcatcc 120
cccattataa aggaggaagc tagggaccca aagcagacac gagccatgcc ccaaataggt 180
ggcagtgggg agcgtaagcc gaggccgcaa ctacctgagg cgctcaagtg cccacgctgc 240
gactccaaca acaccaagtt ttgctactac aacaattata gcatgtcaca accacgctac 300
ttttgcaagg cttgccgccg ctattggaca catggtggta ccctccgcaa tgtccccatt 360

ggtggtgggt gtcgcaagaa caaacatgcc tctagatttg tcttgggctc tcacacctca 420
tcgtcctcat ctgctaccta tgcaccatta tcccctagca ccaacgctag ctctagcaat 480
atgagcatca acaaacatat gatgatggtg cctaacatga cgatgcctac cccaacgaca 540
atgggcttat tccctaatgt gctcccaaca cttatgccga caggtggagg cgggggcttt 600
gacttcacta tggacaacca acatagatca ttgtccttca caccaatgtc tctacctagc 660
caggggccag tgcctatgct ggctgcagga gggagtgagg caacaccgtc tttcctagag 720
atgctgagag gagggatttt tcatggtagt agtagctata acacaagtct cacgatgagt 780
ggtggcaaca atggaatgga caagccattt tcgctgccat catatggtgc aatgtgcaca 840
aatgggttga gtggctcaac cactaatgat gccagacaac tggtggggcc tcagcaggat 900
aacaaggcca tcatgaagag cagtaataac aacaatggtg tatcattgtt gaacctctac 960
tggaacaagc acaacaacaa caacaacaac aacaacaaca acaacaacaa caacaacaac 1020
aagggacaat aaggttagtg tgccagaccg tggaagcgtt gctgctataa ataatgcaat 1080
tgggtagtag tacccagtga aatcaggaga gactagtagc ctagggtgca ttttgattta 1140
tttagttttg gtcaagatga caagtcatca tgaatcaccc tttttattca tttgcatgtt 1200
ttgatgagag ttcatttgca gtaagctata tatcattgga taattaatca tttggttgga 1260
tcatcagttc ctatgggcat tttcttcatt ttttctattc tttaatttat taactaatgt 1320
atagcttttc caacttaact acttaatttg gatagatgaa gcatgttgtg taacatgaat 1380
tatggtagcc agaaccgtat ctatatacta ttggtaataa caatttgttg ttgcatatgt 1440
atcttttgaa tgtgttattc cttttgtttc aactttcaat gagaaaattt 1490

<210> 11
<211> 987
<212> DNA
<213> Zea mays

<400> 11
atggacatga tctccggcag cactgcagca acatcaacac cccacaacaa ccaacaggcg 60
gtgatgttgt catcccccat tataaaggag gaagctaggg acccaaagca gacacgagcc 120
atgccccaaa taggtggcag tggggagcgt aagccgaggc cgcaactacc tgaggcgctc 180
aagtgcccac gctgcgactc caacaacacc aagttttgct actacaacaa ttatagcatg 240
tcacaaccac gctacttttg caaggcttgc cgccgctatt ggacacatgg tggtaccctc 300
cgcaatgtcc ccattggtgg tgggtgtcgc aagaacaaac atgcctctag atttgtcttg 360
ggctctcaca cctcatcgtc ctcatctgct acctatgcac cattatcccc tagcaccaac 420
gctagctcta gcaatatgag catcaacaaa catatgatga tggtgcctaa catgacgatg 480
cctaccccaa cgacaatggg cttattccct aatgtgctcc caacacttat gccgacaggt 540
ggaggcgggg gctttgactt cactatggac aaccaacata gatcattgtc cttcacacca 600
atgtctctac ctagccaggg gccagtgcct atgctggctg caggagggag tgaggcaaca 660
ccgtctttcc tagagatgct gagaggaggg atttttcatg gtagtagtag ctataacaca 720
agtctcacga tgagtggtgg caacaatgga atggacaagc cattttcgct gccatcatat 780
ggtgcaatgt gcacaaatgg gttgagtggc tcaaccacta atgatgccag acaactggtg 840
gggcctcagc aggataacaa ggccatcatg aagagcagta ataacaacaa tggtgtatca 900
ttgttgaacc tctactggaa caagcacaac aacaacaaca acaacaacaa caacaacaac 960
aacaacaaca acaacaaggg acaataa 987

<210> 12
<211> 328
<212> PRT
<213> Zea mays

<400> 12
Met Asp Met Ile Ser Gly Ser Thr Ala Ala Thr Ser Thr Pro His Asn
1               5                   10                  15
Asn Gln Gln Ala Val Met Leu Ser Ser Pro Ile Ile Lys Glu Glu Ala
            20                  25                  30
Arg Asp Pro Lys Gln Thr Arg Ala Met Pro Gln Ile Gly Gly Ser Gly
        35                  40                  45
Glu Arg Lys Pro Arg Pro Gln Leu Pro Glu Ala Leu Lys Cys Pro Arg
    50                  55                  60
Cys Asp Ser Asn Asn Thr Lys Phe Cys Tyr Tyr Asn Asn Tyr Ser Met
65                  70                  75                  80

Ser Gln Pro Arg Tyr Phe Cys Lys Ala Cys Arg Arg Tyr Trp Thr His
                85                  90                  95
Gly Gly Thr Leu Arg Asn Val Pro Ile Gly Gly Gly Cys Arg Lys Asn
            100                 105                 110
Lys His Ala Ser Arg Phe Val Leu Gly Ser His Thr Ser Ser Ser Ser
        115                 120                 125
Ser Ala Thr Tyr Ala Pro Leu Ser Pro Ser Thr Asn Ala Ser Ser Ser
    130                 135                 140
Asn Met Ser Ile Asn Lys His Met Met Met Val Pro Asn Met Thr Met
145                 150                 155                 160
Pro Thr Pro Thr Thr Met Gly Leu Phe Pro Asn Val Leu Pro Thr Leu
                165                 170                 175
Met Pro Thr Gly Gly Gly Gly Gly Phe Asp Phe Thr Met Asp Asn Gln
            180                 185                 190
His Arg Ser Leu Ser Phe Thr Pro Met Ser Leu Pro Ser Gln Gly Pro
        195                 200                 205
Val Pro Met Leu Ala Ala Gly Gly Ser Glu Ala Thr Pro Ser Phe Leu
    210                 215                 220
Glu Met Leu Arg Gly Gly Ile Phe His Gly Ser Ser Ser Tyr Asn Thr
225                 230                 235                 240
Ser Leu Thr Met Ser Gly Gly Asn Asn Gly Met Asp Lys Pro Phe Ser
                245                 250                 255
Leu Pro Ser Tyr Gly Ala Met Cys Thr Asn Gly Leu Ser Gly Ser Thr
            260                 265                 270
Thr Asn Asp Ala Arg Gln Leu Val Gly Pro Gln Gln Asp Asn Lys Ala
        275                 280                 285
Ile Met Lys Ser Ser Asn Asn Asn Asn Gly Val Ser Leu Leu Asn Leu
    290                 295                 300
Tyr Trp Asn Lys His Asn Asn Asn Asn Asn Asn Asn Asn Asn Asn Asn
305                 310                 315                 320
Asn Asn Asn Asn Asn Lys Gly Gln
                325

<210> 13
<211> 1665
<212> DNA
<213> Zea mays

<400> 13
cttttctcta tatacaacta aaggactcat catctctctt cctctccctc gtcttcctgc 60
gtgcacaacc ggatccttcg ccttgctact gttcggcgct tccacaacag ctagcttgct 120
gaaaggaagg agccagcgcc catggacatg acctccaaca ccagcaacag cgctgcagca 180
acatcatcgg ctccccacaa ccaccagccg gtgcaggagg ccatggtgcc atctccaacc 240
ggaaaggagc aaggcaggaa caccaagaaa gcgcggccgg gccggcggca acggggaact 300
aagccgcggc cactacagga cacggcgctc aactgcccgc gctgctgctc caccaacacc 360
aagttttgct actacaacaa ctacaacctg acgcagccgc gctacttctg caaggcatgc 420
cgccgctact ggacacaagg cggcaccctc cgcaacgtcc ccgtcggcgg cggctgccgc 480
aggaacaagc gcgcctccgg ctcctgctcg tcctctgccg ccgccgccgc accgctatgc 540
tccagcgcta ccgccaccgg cgccgagatg gtgaacaccg tcaacacgcg cctgatgctg 600
atgtctaccg ccggtaccac catgtcgacg acgatatcca tgccctcccc ggcgacaggc 660
ttgtttccga gtagcatgct cccgacgaca tttacgccga caggcagtag cggcttccac 720
ctcgccatgg acgaccagca gcagcagggt ttcctgccct tcgcgccaca gtctttgtcc 780
caccaccatc accaggcgct ggagctggct cctgcaggga atgacacgac gccaactttc 840
ctggacatgc tgacaggagg gtatcttgat ggcggtagaa gcatcagcga cagcagctac 900
ggcgctggtc tcgccatgag cggtggtggc agcaatggaa tggacatgcc gtttctgctg 960
cctgggatgg gggccgcctc agcaaccgat cccatgcaga cgcagctgct ggcgggaatg 1020
agaatgaaca atgtagcagg tgggctccag tgggtgtcat cagagcctga caactacacc 1080
aacgacgacg gtgcttatgc agcaggacca gccgctggac tgcagcatca ccagcagcag 1140
caggttgttg gcgatcagca gcagcagcag gagaacaaag cgaatcagcg cagcaaaaac 1200
aacaacggcg gcggcggcgg tgggtcgtcg gtgtacagct tctactggac caacaccagc 1260

aacagcgatg gggcagagtt gtagtgtgcc tctgccagtg ctagagccac aggagacgcg 1320
aaatcgctgc tgcaaactcc ctcgcgacga ggaaggggac gcgattcacc gttaagcact 1380
agctcagttt atagaggata ggatgcatat tcgcgtttga ttaatctgca ttgtggtcgt 1440
gatggcaagt catcatgcac atcgcccttt ttgttcaaat gcatgcgtta gttcgttttc 1500
atcagagttt acttgctgca gtctatcatc gccggcagag attagttagg tgtctatgct 1560
tttatattca tcagtagttc tggataataa tgtcttcgta ttcctcagtt tctttgttaa 1620
gtcgtctaaa actttccatg tgtaaaaaaa aaaaaaaaaa aaaaa 1665

<210> 14
<211> 1143
<212> DNA
<213> Zea mays

<400> 14
atggacatga cctccaacac cagcaacagc gctgcagcaa catcatcggc tccccacaac 60
caccagccgg tgcaggaggc catggtgcca tctccaaccg gaaaggagca aggcaggaac 120
accaagaaag cgcggccggg ccggcggcaa cggggaacta agccgcggcc actacaggac 180
acggcgctca actgcccgcg ctgctgctcc accaacacca agttttgcta ctacaacaac 240
tacaacctga cgcagccgcg ctacttctgc aaggcatgcc gccgctactg gacacaaggc 300
ggcaccctcc gcaacgtccc cgtcggcggc ggctgccgca ggaacaagcg cgcctccggc 360
tcctgctcgt cctctgccgc cgccgccgca ccgctatgct ccagcgctac cgccaccggc 420
gccgagatgg tgaacaccgt caacacgcgc ctgatgctga tgtctaccgc cggtaccacc 480
atgtcgacga cgatatccat gccctccccg gcgacaggct tgtttccgag tagcatgctc 540
ccgacgacat ttacgccgac aggcagtagc ggcttccacc tcgccatgga cgaccagcag 600
cagcagggtt tcctgccctt cgcgccacag tctttgtccc accaccatca ccaggcgctg 660
gagctggctc ctgcagggaa tgacacgacg ccaactttcc tggacatgct gacaggaggg 720
tatcttgatg gcggtagaag catcagcgac agcagctacg gcgctggtct cgccatgagc 780
ggtggtggca gcaatggaat ggacatgccg tttctgctgc ctgggatggg ggccgcctca 840
gcaaccgatc ccatgcagac gcagctgctg gcgggaatga gaatgaacaa tgtagcaggt 900
gggctccagt gggtgtcatc agagcctgac aactacacca acgacgacgg tgcttatgca 960
gcaggaccag ccgctggact gcagcatcac cagcagcagc aggttgttgg cgatcagcag 1020
cagcagcagg agaacaaagc gaatcagcgc agcaaaaaca acaacggcgg cggcggcggt 1080
gggtcgtcgg tgtacagctt ctactggacc aacaccagca acagcgatgg ggcagagttg 1140
tag 1143

<210> 15
<211> 380
<212> PRT
<213> Zea mays

<400> 15
   Met Asp Met Thr Ser Asn Thr Ser Asn Ser Ala Ala Ala Thr Ser Ser
   1 5 10 15
   Ala Pro His Asn His Gln Pro Val Gln Glu Ala Met Val Pro Ser Pro
   20 25 30
   Thr Gly Lys Glu Gln Gly Arg Asn Thr Lys Lys Ala Arg Pro Gly Arg
   35 40 45
   Arg Gln Arg Gly Thr Lys Pro Arg Pro Leu Gln Asp Thr Ala Leu Asn
   50 55 60
   Cys Pro Arg Cys Cys Ser Thr Asn Thr Lys Phe Cys Tyr Tyr Asn Asn
   65 70 75 80
   Tyr Asn Leu Thr Gln Pro Arg Tyr Phe Cys Lys Ala Cys Arg Arg Tyr
   85 90 95
   Trp Thr Gln Gly Gly Thr Leu Arg Asn Val Pro Val Gly Gly Gly Cys
   100 105 110
   Arg Arg Asn Lys Arg Ala Ser Gly Ser Cys Ser Ser Ser Ala Ala Ala
   115 120 125
   Ala Ala Pro Leu Cys Ser Ser Ala Thr Ala Thr Gly Ala Glu Met Val
   130 135 140
   Asn Thr Val Asn Thr Arg Leu Met Leu Met Ser Thr Ala Gly Thr Thr
   145 150 155 160
   Met Ser Thr Thr Ile Ser Met Pro Ser Pro Ala Thr Gly Leu Phe Pro
                   165                 170                 175
   Ser Ser Met Leu Pro Thr Thr Phe Thr Pro Thr Gly Ser Ser Gly Phe
               180                 185                 190
   His Leu Ala Met Asp Asp Gln Gln Gln Gln Gly Phe Leu Pro Phe Ala
           195                 200                 205
   Pro Gln Ser Leu Ser His His His His Gln Ala Leu Glu Leu Ala Pro
       210                 215                 220
   Ala Gly Asn Asp Thr Thr Pro Thr Phe Leu Asp Met Leu Thr Gly Gly
   225                 230                 235                 240
   Tyr Leu Asp Gly Gly Arg Ser Ile Ser Asp Ser Ser Tyr Gly Ala Gly
                   245                 250                 255
   Leu Ala Met Ser Gly Gly Gly Ser Asn Gly Met Asp Met Pro Phe Leu
               260                 265                 270
   Leu Pro Gly Met Gly Ala Ala Ser Ala Thr Asp Pro Met Gln Thr Gln
           275                 280                 285
   Leu Leu Ala Gly Met Arg Met Asn Asn Val Ala Gly Gly Leu Gln Trp
       290                 295                 300
   Val Ser Ser Glu Pro Asp Asn Tyr Thr Asn Asp Asp Gly Ala Tyr Ala
   305                 310                 315                 320
   Ala Gly Pro Ala Ala Gly Leu Gln His His Gln Gln Gln Gln Val Val
                   325                 330                 335
   Gly Asp Gln Gln Gln Gln Gln Glu Asn Lys Ala Asn Gln Arg Ser Lys
               340                 345                 350
   Asn Asn Asn Gly Gly Gly Gly Gly Gly Ser Ser Val Tyr Ser Phe Tyr
           355                 360                 365
   Trp Thr Asn Thr Ser Asn Ser Asp Gly Ala Glu Leu
       370                 375                 380

<210> 16
<211> 1553
<212> DNA
<213> Zea mays

<400> 16
cccgtgtacc gtacacagca ggatcctccg ccttccttgt gttccgcgct tccacaacag 60
tttaccgcaa ggaaggaacc atggacatga actccaacgc caacaacagc actgccgcag 120
cagcatcggc tcccatcaac aaccagcagg aggctgtggt gtcatcccca accagaaagg 180
agcaagccag gaaccccaag aaggcgcggg cggcgccgca gcaggcgggc ggcagcgggg 240
agcctaggcc gcggcctccg ccggacgcgg cgcacagctg cccgcgctgc tcctccacca 300
acacaaagtt ctgctactac aacaactaca acctgacgca gccgcgctac ttctgcaaga 360
cgtgccgccg ctactggaca cacggcggca ccctccgcaa cgtccccgtc ggcggcggct 420
gccgcaggaa caagcgcgcc tccagctcct cgtccccgtt tccgggcccc tccagcaccg 480
ccgccaccag cgccgcgatg gagaagaccg tcagcacgcg gctgatgctg atggcgacca 540
gcaccatggc gatgccctcc ccgacggcag gcctgtttgt tcccgatgac atgtccccgg 600
cattcacgcc gacgacgggc ggtagcggct tcgacgacct cgccggcatg gacgagcagc 660
accagcaggg cttcctgccc ttctcgccgc tgtccctgtc cgaccaggcg ccggagctgg 720
ctcctggagg agggggtgac acgacgccgt ctttcctgga catgctgaca ggagggtatc 780
tcgatggcgg cggctacggc ggcatgagcg gtggcagcga tgcgatggac atgccgttct 840
cgctgcctga gatggggccc ccgacaactg atccaatgcc gtttcagctc cagtggacgt 900
catcagagct tgacaactac atcaacgacg acggtggtta tgcagcagga ccagccgccg 960
gagtgcagca gcagcagcag cagcagcagc agcagattaa tggtggtgat caccagaagc 1020
aggacgagaa caaagaggcg gggaacggca aaggcaacga cgacggcggc ggcgggtcgt 1080
cgtcggtgta cagcttctgg atgaacacca gcggcagcga cggggcagag gggtagtgcg 1140
ccactgccag tgccagccac aggaggaggc gaaagtgctg ctgcaaactc cctcgcatca 1200
tctggtcggt gcaagtgcag cagacatcct tcgtcgacgc aatcaaatca tcaaaaggtg 1260
gcaacaggga gtgaaagggg gaagaagttc accatccagc gtagaggata gggtgcatgt 1320
tcatgtttga tttatctgca ttgtggtcgg tcgtggttgc aagtcatctt gcaccaccct 1380
ttgttttggt tcagtttgca tgcgttcgtt cgtcagagtt ttacttgcag cagtctttgg 1440
atcgccggca gagattagtt aggtatctat gctttgtttt cgtcagttcg ttctggataa 1500

tgtcttaata attcctcagt ttatttcttt gttaactaaa aaaaaaaaaa aaa 1553

<210> 17
<211> 1056
<212> DNA
<213> Zea mays

<400> 17
atggacatga actccaacgc caacaacagc actgccgcag cagcatcggc tcccatcaac 60
aaccagcagg aggctgtggt gtcatcccca accagaaagg agcaagccag gaaccccaag 120
aaggcgcggg cggcgccgca gcaggcgggc ggcagcgggg agcctaggcc gcggcctccg 180
ccggacgcgg cgcacagctg cccgcgctgc tcctccacca acacaaagtt ctgctactac 240
aacaactaca acctgacgca gccgcgctac ttctgcaaga cgtgccgccg ctactggaca 300
cacggcggca ccctccgcaa cgtccccgtc ggcggcggct gccgcaggaa caagcgcgcc 360
tccagctcct cgtccccgtt tccgggcccc tccagcaccg ccgccaccag cgccgcgatg 420
gagaagaccg tcagcacgcg gctgatgctg atggcgacca gcaccatggc gatgccctcc 480
ccgacggcag gcctgtttgt tcccgatgac atgtccccgg cattcacgcc gacgacgggc 540
ggtagcggct tcgacgacct cgccggcatg gacgagcagc accagcaggg cttcctgccc 600
ttctcgccgc tgtccctgtc cgaccaggcg ccggagctgg ctcctggagg aggggggtgac 660
acgacgccgt ctttcctgga catgctgaca ggagggtatc tcgatggcgg cggctacggc 720
ggcatgagcg gtggcagcga tgcgatggac atgccgttct cgctgcctga gatggggccc 780
ccgacaactg atccaatgcc gtttcagctc cagtggacgt catcagagct tgacaactac 840
atcaacgacg acggtggtta tgcagcagga ccagccgccg gagtgcagca gcagcagcag 900
cagcagcagc agcagattaa tggtggtgat caccagaagc aggacgagaa caaagaggcg 960
gggaacggca aaggcaacga cgacggcggc ggcgggtcgt cgtcggtgta cagcttctgg 1020
atgaacacca gcggcagcga cggggcagag ggtag 1056

<210> 18
<211> 351
<212> PRT
<213> Zea mays

<400> 18
Met Asp Met Asn Ser Asn Ala Asn Asn Ser Thr Ala Ala Ala Ala Ser
1               5                   10                  15
Ala Pro Ile Asn Asn Gln Gln Glu Ala Val Val Ser Ser Pro Thr Arg
            20                  25                  30
Lys Glu Gln Ala Arg Asn Pro Lys Lys Ala Arg Ala Ala Pro Gln Gln
        35                  40                  45
Ala Gly Gly Ser Gly Glu Pro Arg Pro Arg Pro Pro Pro Asp Ala Ala
    50                  55                  60
His Ser Cys Pro Arg Cys Ser Ser Thr Asn Thr Lys Phe Cys Tyr Tyr
65                  70                  75                  80
Asn Asn Tyr Asn Leu Thr Gln Pro Arg Tyr Phe Cys Lys Thr Cys Arg
                85                  90                  95
Arg Tyr Trp Thr His Gly Gly Thr Leu Arg Asn Val Pro Val Gly Gly
            100                 105                 110
Gly Cys Arg Arg Asn Lys Arg Ala Ser Ser Ser Ser Ser Pro Phe Pro
        115                 120                 125
Gly Pro Ser Ser Thr Ala Ala Thr Ser Ala Ala Met Glu Lys Thr Val
    130                 135                 140
Ser Thr Arg Leu Met Leu Met Ala Thr Ser Thr Met Ala Met Pro Ser
145                 150                 155                 160
Pro Thr Ala Gly Leu Phe Val Pro Asp Asp Met Ser Pro Ala Phe Thr
                165                 170                 175
Pro Thr Thr Gly Gly Ser Gly Phe Asp Asp Leu Ala Gly Met Asp Glu
            180                 185                 190
Gln His Gln Gln Gly Phe Leu Pro Phe Ser Pro Leu Ser Leu Ser Asp
        195                 200                 205
Gln Ala Pro Glu Leu Ala Pro Gly Gly Gly Gly Asp Thr Thr Pro Ser
    210                 215                 220

Phe Leu Asp Met Leu Thr Gly Gly Tyr Leu Asp Gly Gly Gly Tyr Gly
225                 230                 235                 240
Gly Met Ser Gly Gly Ser Asp Ala Met Asp Met Pro Phe Ser Leu Pro
                245                 250                 255
Glu Met Gly Pro Pro Thr Thr Asp Pro Met Pro Phe Gln Leu Gln Trp
            260                 265                 270
Thr Ser Ser Glu Leu Asp Asn Tyr Ile Asn Asp Asp Gly Gly Tyr Ala
        275                 280                 285
Ala Gly Pro Ala Ala Gly Val Gln Gln Gln Gln Gln Gln Gln Gln Gln
    290                 295                 300
Gln Ile Asn Gly Gly Asp His Gln Lys Gln Asp Glu Asn Lys Glu Ala
305                 310                 315                 320
Gly Asn Gly Lys Gly Asn Asp Asp Gly Gly Gly Gly Ser Ser Ser Val
                325                 330                 335
Tyr Ser Phe Trp Met Asn Thr Ser Gly Ser Asp Gly Ala Glu Gly
            340                 345                 350

<210> 19
<211> 1553
<212> DNA
<213> Zea mays

<400> 19
cccgtgtacc gtacacagca ggatcctccg ccttccttgt gttccgcgct tccacaacag 60
tttaccgcaa ggaaggaacc atggacatga actccaacgc caacaacagc actgccgcag 120
cagcatcggc tcccatcaac aaccagcagg aggctgtggt gtcatcccca accagaaagg 180
agcaagccag gaaccccaag aaggcgcggg cggcgccgca gcaggcgggc ggcagcgggg 240
agcctaggcc gcggcctccg ccggacgcgg cgcacagctg cccgcgctgc tcctccacca 300
acacaaagtt ctgctactac aacaactaca acctgacgca gccgcgctac ttctgcaaga 360
cgtgccgccg ctactggaca cacggcggca ccctccgcaa cgtccccgtc ggcggcggct 420
gccgcaggaa caagcgcgcc tccagctcct cgtccccgtt tccgggcccc tccagcaccg 480
ccgccaccag cgccgcgatg gagaagaccg tcagcacgcg gctgatgctg atggcgacca 540
gcaccatggc gatgccctcc ccgacggcag gcctgtttgt tcccgatgac atgtccccgg 600
cattcacgcc gacgacgggc ggtagcggct tcgacgacct cgccggcatg gacgagcagc 660
accagcaggg cttcctgccc ttctcgccgc tgtccctgtc cgaccaggcg ccggagctgg 720
ctcctggagg agggggtgac acgacgccgt ctttcctgga catgctgaca ggagggtatc 780
tcgatggcgg cggctacggc ggcatgagcg gtggcagcga tgcgatggac atgccgttct 840
cgctgcctga gatggggccc ccgacaactg atccaatgcc gtttcagctc cagtggacgt 900
catcagagct tgacaactac atcaacgacg acggtggtta tgcagcagga ccagccgccg 960
gagtgcagca gcagcagcag cagcagcagc agcagattaa tggtggtgat caccagaagc 1020
aggacgagaa caaagaggcg gggaacggca aaggcaacga cgacggcggc ggcgggtcgt 1080
cgtcggtgta cagcttctgg atgaacacca gcggcagcga cggggcagag gggtagtgcg 1140
ccactgccag tgccagccac aggaggaggc gaaagtgctg ctgcaaactc cctcgcatca 1200
tctggtcggt gcaagtgcag cagacatcct tcgtcgacgc aatcaaatca tcaaaaggtg 1260
gcaacaggga gtgaaagggg gaagaagttc accatccagc gtagaggata gggtgcatgt 1320
tcatgtttga tttatctgca ttgtggtcgg tcgtggttgc aagtcatctt gcaccaccct 1380
ttgttttggt tcagtttgca tgcgttcgtt cgtcagagtt ttacttgcag cagtctttgg 1440
atcgccggca gagattagtt aggtatctat gctttgtttt cgtcagttcg ttctggataa 1500
tgtcttaata attcctcagt ttatttcttt gttaactaaa aaaaaaaaaa aaa 1553

<210> 20
<211> 1056
<212> DNA
<213> Zea mays

<400> 20
atggacatga actccaacgc caacaacagc actgccgcag cagcatcggc tcccatcaac 60
aaccagcagg aggctgtggt gtcatcccca accagaaagg agcaagccag gaaccccaag 120
aaggcgcggg cggcgccgca gcaggcgggc ggcagcgggg agcctaggcc gcggcctccg 180
ccggacgcgg cgcacagctg cccgcgctgc tcctccacca acacaaagtt ctgctactac 240

aacaactaca acctgacgca gccgcgctac ttctgcaaga cgtgccgccg ctactggaca 300
cacggcggca ccctccgcaa cgtccccgtc ggcggcggct gccgcaggaa caagcgcgcc 360
tccagctcct cgtccccgtt tccgggcccc tccagcaccg ccgccaccag cgccgcgatg 420
gagaagaccg tcagcacgcg gctgatgctg atggcgacca gcaccatggc gatgccctcc 480
ccgacggcag gcctgtttgt tcccgatgac atgtccccgg cattcacgcc gacgacgggc 540
ggtagcggct tcgacgacct cgccggcatg gacgagcagc accagcaggg cttcctgccc 600
ttctcgccgc tgtccctgtc cgaccaggcg ccggagctgg ctcctggagg agggggtgac 660
acgacgccgt ctttcctgga catgctgaca ggagggtatc tcgatggcgg cggctacggc 720
ggcatgagcg gtggcagcga tgcgatggac atgccgttct cgctgcctga gatggggccc 780
ccgacaactg atccaatgcc gtttcagctc cagtggacgt catcagagct tgacaactac 840
atcaacgacg acggtggtta tgcagcagga ccagccgccg gagtgcagca gcagcagcag 900
cagcagcagc agcagattaa tggtggtgat caccagaagc aggacgagaa caaagaggcg 960
gggaacggca aaggcaacga cgacggcggc ggcgggtcgt cgtcggtgta cagcttctgg 1020
atgaacacca gcggcagcga cggggcagag ggtag 1056

<210> 21
<211> 351
<212> PRT
<213> Zea mays

<400> 21
Met Asp Met Asn Ser Asn Ala Asn Asn Ser Thr Ala Ala Ala Ala Ser
1               5                   10                  15
Ala Pro Ile Asn Asn Gln Gln Glu Ala Val Val Ser Ser Pro Thr Arg
            20                  25                  30
Lys Glu Gln Ala Arg Asn Pro Lys Lys Ala Arg Ala Ala Pro Gln Gln
        35                  40                  45
Ala Gly Gly Ser Gly Glu Pro Arg Pro Arg Pro Pro Pro Asp Ala Ala
    50                  55                  60
His Ser Cys Pro Arg Cys Ser Ser Thr Asn Thr Lys Phe Cys Tyr Tyr
65                  70                  75                  80
Asn Asn Tyr Asn Leu Thr Gln Pro Arg Tyr Phe Cys Lys Thr Cys Arg
                85                  90                  95
Arg Tyr Trp Thr His Gly Gly Thr Leu Arg Asn Val Pro Val Gly Gly
            100                 105                 110
Gly Cys Arg Arg Asn Lys Arg Ala Ser Ser Ser Ser Ser Pro Phe Pro
        115                 120                 125
Gly Pro Ser Ser Thr Ala Ala Thr Ser Ala Ala Met Glu Lys Thr Val
    130                 135                 140
Ser Thr Arg Leu Met Leu Met Ala Thr Ser Thr Met Ala Met Pro Ser
145                 150                 155                 160
Pro Thr Ala Gly Leu Phe Val Pro Asp Asp Met Ser Pro Ala Phe Thr
                165                 170                 175
Pro Thr Thr Gly Gly Ser Gly Phe Asp Asp Leu Ala Gly Met Asp Glu
            180                 185                 190
Gln His Gln Gln Gly Phe Leu Pro Phe Ser Pro Leu Ser Leu Ser Asp
        195                 200                 205
Gln Ala Pro Glu Leu Ala Pro Gly Gly Gly Gly Asp Thr Thr Pro Ser
    210                 215                 220
Phe Leu Asp Met Leu Thr Gly Gly Tyr Leu Asp Gly Gly Gly Tyr Gly
225                 230                 235                 240
Gly Met Ser Gly Gly Ser Asp Ala Met Asp Met Pro Phe Ser Leu Pro
                245                 250                 255
Glu Met Gly Pro Pro Thr Thr Asp Pro Met Pro Phe Gln Leu Gln Trp
            260                 265                 270
Thr Ser Ser Glu Leu Asp Asn Tyr Ile Asn Asp Asp Gly Gly Tyr Ala
        275                 280                 285
Ala Gly Pro Ala Ala Gly Val Gln Gln Gln Gln Gln Gln Gln Gln Gln
    290                 295                 300
Gln Ile Asn Gly Gly Asp His Gln Lys Gln Asp Glu Asn Lys Glu Ala
305                 310                 315                 320

Gly Asn Gly Lys Gly Asn Asp Asp Gly Gly Gly Gly Ser Ser Ser Val
                325                 330                 335
Tyr Ser Phe Trp Met Asn Thr Ser Gly Ser Asp Gly Ala Glu Gly
            340                 345                 350

<210> 22
<211> 1585
<212> DNA
<213> Zea mays

<400> 22
tttgttttct agcttttgtc agcgaacctt cgccctgaaa accccaccca ctcttcctct 60
ccgatcctcc tagcgcctca cttgcttcat tccattccat tccattccac tacccaatac 120
ccatctcccc gccggccttt cccctcccgc gtgccgatct gcctccggag gctagctcga 180
ccggccgatc tagctcgcgc gccgccgctt tcagctcgat cagctgccgc acgtcgtcca 240
ccgacccgcc gtcctgtgag cgcgatcgac agattactaa gatggctggc gcggggtgtg 300
cgacagccgt gcagcggccg gcggcggcgg gggcgccggc tgctgtggca aggaccgcgg 360
tcggggcagg cggcgcggtc gccgacccgc gcgcggaggc gctgcggtgc ccgcgctgcg 420
actcggccaa caccaagttc tgctactaca acaactactc gctgtcgcag ccgcgccact 480
tctgcaaggc gtgcaagcgc tactggacgc gcgggggcac gctccgcaac gtccccgtcg 540
gcgggggctg ccgcaagaac aagcgctcct ccaggagcgg cggcggcggg aggaatgggt 600
cctcctcctc cttctcctcc tcctcggcgg actcgacggc gctgcccctc ccgccgcctc 660
cgacgacgat ggggtccctg ccgtcgtcgc tggggctgcc cgggggcgcg tcgctcctcc 720
tcgggtccgc gctccctggc ggtgccggtg accaccacca cctcggcctt ttccaggcgg 780
ccatgcagtc ggtggtctcc tccgacgcga ccgcctacga cgagatgcag cagcaacagc 840
agacgcagct ggaccacctg ctgggcctcg gatacggagg aggcgccggc gcgcagatcc 900
agctcaagcc gtggatgcag gaggccgccg gggcaggcgg cgcgcccggg atcatggaca 960
gcttctacgc gccgctgctg tccagctccc tcgtgccggg gctggaggag ctgcacgtca 1020
aggcggaggc cgccggagcc ggggatcacc agcagaagcc gtcatcaagg gaccagcaga 1080
gcgccagctg ggactgggag ctgccgacgc cgtcgtcgtc caacgtcgac gccaacgtcg 1140
tcaccgcgtc tgacgcgctc atggccgccg ccgccgccgc gtccatgaac cccgctgtta 1200
gtgttagctc cacgccctcc acggcaccaa ccgccccctc ctcgttctta tactggggca 1260
acagcggcat tggcggcgct gccgcggcct ggccagacct cgccaactgc ggatcctcca 1320
ttgccacgct cttctagcca agccgcgcct ggctgtcctg ctggcctcgt taacttggtt 1380
tttttgtacc ttaatttctg gttaattttt ttcttgactg aaataacatg taaatttgcg 1440
gggaaaagaa aactgagatg agagagagag aacgctgtga aagaagagag ctagaccatc 1500
tcaccacctc atagggcaga aacttagaca aatcaaacaa atgatccaat ccaatatttc 1560
aagagaattc cttcgccgcg actat 1585

<210> 23
<211> 1056
<212> DNA
<213> Zea mays

<400> 23
atggctggcg cggggtgtgc gacagccgtg cagcggccgg cggcggcggg ggcgccggct 60
gctgtggcaa ggaccgcggt cggggcaggc ggcgcggtcg ccgacccgcg cgcggaggcg 120
ctgcggtgcc cgcgctgcga ctcggccaac accaagttct gctactacaa caactactcg 180
ctgtcgcagc cgcgccactt ctgcaaggcg tgcaagcgct actggacgcg cgggggcacg 240
ctccgcaacg tccccgtcgg cgggggctgc cgcaagaaca agcgctcctc caggagcggc 300
ggcggcggga ggaatgggtc ctcctcctcc ttctcctcct cctcggcgga ctcgacggcg 360
ctgcccctcc cgccgcctcc gacgacgatg gggtccctgc cgtcgtcgct ggggctgccc 420
gggggcgcgt cgctcctcct cgggtccgcg ctccctggcg gtgccggtga ccaccaccac 480
ctcggccttt tccaggcggc catgcagtcg gtggtctcct ccgacgcgac cgcctacgac 540
gagatgcagc agcaacagca gacgcagctg gaccacctgc tgggcctcgg atacggagga 600
ggcgccggcg cgcagatcca gctcaagccg tggatgcagg aggccgccgg ggcaggcggc 660
gcgcccggga tcatggacag cttctacgcg ccgctgctgt ccagctccct cgtgccgggg 720
ctggaggagc tgcacgtcaa ggcggaggcc gccggagccg gggatcacca gcagaagccg 780
tcatcaaggg accagcagag cgccagctgg gactgggagc tgccgacgcc gtcgtcgtcc 840
aacgtcgacg ccaacgtcgt caccgcgtct gacgcgctca tggccgccgc cgccgccgcg 900

tccatgaacc ccgctgttag tgttagctcc acgccctcca cggcaccaac cgccccctcc 960
tcgttcttat actggggcaa cagcggcatt ggcggcgctg ccgcggcctg gccagacctc 1020
gccaactgcg gatcctccat tgccacgctc ttctag 1056

<210> 24
<211> 351
<212> PRT
<213> Zea mays

<400> 24
Met Ala Gly Ala Gly Cys Ala Thr Ala Val Gln Arg Pro Ala Ala Ala
1               5                   10                  15
Gly Ala Pro Ala Ala Val Ala Arg Thr Ala Val Gly Ala Gly Gly Ala
            20                  25                  30
Val Ala Asp Pro Arg Ala Glu Ala Leu Arg Cys Pro Arg Cys Asp Ser
        35                  40                  45
Ala Asn Thr Lys Phe Cys Tyr Tyr Asn Asn Tyr Ser Leu Ser Gln Pro
    50                  55                  60
Arg His Phe Cys Lys Ala Cys Lys Arg Tyr Trp Thr Arg Gly Gly Thr
65                  70                  75                  80
Leu Arg Asn Val Pro Val Gly Gly Gly Cys Arg Lys Asn Lys Arg Ser
                85                  90                  95
Ser Arg Ser Gly Gly Gly Gly Arg Asn Gly Ser Ser Ser Ser Phe Ser
            100                 105                 110
Ser Ser Ser Ala Asp Ser Thr Ala Leu Pro Leu Pro Pro Pro Pro Thr
        115                 120                 125
Thr Met Gly Ser Leu Pro Ser Ser Leu Gly Leu Pro Gly Gly Ala Ser
    130                 135                 140
Leu Leu Leu Gly Ser Ala Leu Pro Gly Gly Ala Gly Asp His His His
145                 150                 155                 160
Leu Gly Leu Phe Gln Ala Ala Met Gln Ser Val Val Ser Ser Asp Ala
                165                 170                 175
Thr Ala Tyr Asp Glu Met Gln Gln Gln Gln Gln Thr Gln Leu Asp His
            180                 185                 190
Leu Leu Gly Leu Gly Tyr Gly Gly Gly Ala Gly Ala Gln Ile Gln Leu
        195                 200                 205
Lys Pro Trp Met Gln Glu Ala Ala Gly Ala Gly Gly Ala Pro Gly Ile
    210                 215                 220
Met Asp Ser Phe Tyr Ala Pro Leu Leu Ser Ser Ser Leu Val Pro Gly
225                 230                 235                 240
Leu Glu Glu Leu His Val Lys Ala Glu Ala Ala Gly Ala Gly Asp His
                245                 250                 255
Gln Gln Lys Pro Ser Ser Arg Asp Gln Gln Ser Ala Ser Trp Asp Trp
            260                 265                 270
Glu Leu Pro Thr Pro Ser Ser Ser Asn Val Asp Ala Asn Val Val Thr
        275                 280                 285
Ala Ser Asp Ala Leu Met Ala Ala Ala Ala Ala Ala Ser Met Asn Pro
    290                 295                 300
Ala Val Ser Val Ser Ser Thr Pro Ser Thr Ala Pro Thr Ala Pro Ser
305                 310                 315                 320
Ser Phe Leu Tyr Trp Gly Asn Ser Gly Ile Gly Gly Ala Ala Ala Ala
                325                 330                 335
Trp Pro Asp Leu Ala Asn Cys Gly Ser Ser Ile Ala Thr Leu Phe
            340                 345                 350

<210> 25
<211> 1638
<212> DNA
<213> Zea mays

<400> 25
attctccacc acctccgagc ttgccagagg actccacctc tttcttctcg catgcttcct 60
tttctttcca aacacaaatg cgcagcaaag aaagtgcaaa tcataaagaa aagagcagaa 120
ttcaaagcag ccggaaagct gactagctag aaggagtgaa gagagagaag tatatctcag 180
caacgcctcc atggtcttcc cttcaccttc agtgcaggtc tacctagatc cacatccacc 240
taattggaat aaccagcagc agcaaggtca gcagccgagg gcgaatgggg gagctgatgc 300
accgttgctg cccctgggtc cggcggcagc tacatcggcg gctccggagc aaggtgggtt 360
gcctagaagc tcatcaatgg ctaatgccgc cgtggctgcg cacgcgaggc ccaactcaat 420
ggcggagcgc gcgcggctgg cgcggatgcc gcatccggag ccagcgctca agtgcccgcg 480
ttgcgaatcc accaacacca agttctgcta ctacaacaac tactccctct cccagccccg 540
ccacttctgc aagacgtgcc gccgctactg gacgcgcggc ggatcccttc gcaacgtccc 600
cgtaggtgga ggctgccgtc gcaacaagcg ttcgtccaag tcctcatcct cctctgctgg 660
gtcttcctcc tcaaagatgt ctccctcagg aaggctactg ggtggtccat cagctacacc 720
gtccaccacc ccaggcacta ccggtgcgat cattactccg ggtctcagct ctttctctca 780
ccacttgccg ttcttgggct ccatgcaccc gtcagggccc aacctaggtc tagctttctc 840
cgccggactg ccgctagtcg gcatgcagca cctggacacg gtggatcagt ttccggtggc 900
aagcggcggc ggcaccacca taggtgcatc tctagagcag tggagagtgc gacagcagca 960
gcagcagttc ccattcatga ctgggggaat actggatctg tcgcagccgc cgacgtacca 1020
attcggtttg gaagctaacc gaggaggcag cggctcagct gcggtggcgt tcaattcagg 1080
acagaccacg acgaccagtg ctaccacggg aaggcaggaa gggtcatcaa agaagatggg 1140
ggatagtaaa ggagaagata tgagcttaca gaagcagtac atggtacctc tacgccacgg 1200
atcaggatca cacggtgtct gggatgggag tgctggtgga actggcagca acggtggtgg 1260
tactggcaat ggcggttcaa gttggccaat gaacatgatt cctggattcc attcttcgtc 1320
cactagcggt tgcaatgaca gtggcttgtc gtagtactct ctagctaggg gctggggcaa 1380
tgtcctatca agaaaaatat tttggagatg catggttgag tctgagcaaa tgctagctat 1440
agctaggacg atggaggaac aggcgatgac caatccacag gcatgttcat catctgctat 1500
tcttcatcgg aattgtagaa gcacactgct acctcatgat ggtcacaggt aggagtttaa 1560
agtaaagctg ctctttgttt aaaccttttt ctgtcatttg gttttgaact taaaggtgtt 1620
ctctgcattg tgtccata 1638

<210> 26
<211> 1158
<212> DNA
<213> Zea mays

<400> 26
atggtcttcc cttcaccttc agtgcaggtc tacctagatc cacatccacc taattggaat 60
aaccagcagc aaggtcagca gccgagggcg aatgggggag ctgatgcacc gttgctgccc 120
gtgggtccgg cggcagctac atcggcggct ccggagcaag gtgggttgcc tagaagctca 180
tcaatggcta atgccgccgt ggctgcgcac gcgaggccca actcaatggc ggagcgcgcg 240
cggctggcgc ggatgccgca tccggagcca gcgctcaagt gcccgcgttg cgaatccacc 300
aacaccaagt tctgctacta caacaactac tccctctccc agccccgcca cttctgcaag 360
acgtgccgcc gctactggac gcgcggcgga tcccttcgca acgtccccgt aggtggaggc 420
tgccgtcgca acaagcgttc gtccaagtcc tcatcctcct ctgctgggtc ttcctcctca 480
aagatgtctc cctcaggaag gctactgggt ggtccatcag ctacaccgtc caccacccca 540
ggcactaccg gtgcgatcat tactccgggt ctcagctctt tctctcacca cttgccgttc 600
ttgggctcca tgcacccgtc agggcccaac ctaggtctag ctttctccgc cggactgccg 660
ctagtcggca tgcagcacct ggacacggtg gatcagtttc cggtggcaag cggcggcggc 720
accaccatag gtgcatctct agagcagtgg agagtgcgac agcagcagca gcagttccca 780
ttcatgactg ggggaatact ggatctgtcg cagccgccga cgtaccaatt cggtttggaa 840
gctaaccgag gaggcagcgg ctcagctgcg gtggcgttca attcaggaca gaccacgacg 900
accagtgcta ccacgggaag gcaggaaggg tcatcaaaga agatggggga tagtaaagga 960
gaagatatga gcttacagaa gcagtacatg gtacctctac gccacggatc aggatcacac 1020
ggtgtctggg atgggagtgc tggtggaact ggcagcaacg gtggtggtac tggcaatggc 1080
ggttcaagtt ggccaatgaa catgattcct ggattccatt cttcgtccac tagcggttgc 1140
aatgacagtg gcttgtag 1158

<210> 27
<211> 385
<212> PRT
<213> Zea mays

<400> 27
Met Val Phe Pro Ser Pro Ser Val Gln Val Tyr Leu Asp Pro His Pro
1               5                   10                  15
Pro Asn Trp Asn Asn Gln Gln Gln Gly Gln Gln Pro Arg Ala Asn Gly
            20                  25                  30
Gly Ala Asp Ala Pro Leu Leu Pro Val Gly Pro Ala Ala Ala Thr Ser
        35                  40                  45
Ala Ala Pro Glu Gln Gly Gly Leu Pro Arg Ser Ser Ser Met Ala Asn
    50                  55                  60
Ala Ala Val Ala Ala His Ala Arg Pro Asn Ser Met Ala Glu Arg Ala
65                  70                  75                  80
Arg Leu Ala Arg Met Pro His Pro Glu Pro Ala Leu Lys Cys Pro Arg
                85                  90                  95
Cys Glu Ser Thr Asn Thr Lys Phe Cys Tyr Tyr Asn Asn Tyr Ser Leu
            100                 105                 110
Ser Gln Pro Arg His Phe Cys Lys Thr Cys Arg Arg Tyr Trp Thr Arg
        115                 120                 125
Gly Gly Ser Leu Arg Asn Val Pro Val Gly Gly Gly Cys Arg Arg Asn
    130                 135                 140
Lys Arg Ser Ser Lys Ser Ser Ser Ser Ser Ala Gly Ser Ser Ser Ser
145                 150                 155                 160
Lys Met Ser Pro Ser Gly Arg Leu Leu Gly Gly Pro Ser Ala Thr Pro
                165                 170                 175
Ser Thr Thr Pro Gly Thr Thr Gly Ala Ile Ile Thr Pro Gly Leu Ser
            180                 185                 190
Ser Phe Ser His His Leu Pro Phe Leu Gly Ser Met His Pro Ser Gly
        195                 200                 205
Pro Asn Leu Gly Leu Ala Phe Ser Ala Gly Leu Pro Leu Val Gly Met
    210                 215                 220
Gln His Leu Asp Thr Val Asp Gln Phe Pro Val Ala Ser Gly Gly Gly
225                 230                 235                 240
Thr Thr Ile Gly Ala Ser Leu Glu Gln Trp Arg Val Arg Gln Gln Gln
                245                 250                 255
Gln Gln Phe Pro Phe Met Thr Gly Gly Ile Leu Asp Leu Ser Gln Pro
            260                 265                 270
Pro Thr Tyr Gln Phe Gly Leu Glu Ala Asn Arg Gly Gly Ser Gly Ser
        275                 280                 285
Ala Ala Val Ala Phe Asn Ser Gly Gln Thr Thr Thr Thr Ser Ala Thr
    290                 295                 300
Thr Gly Arg Gln Glu Gly Ser Ser Lys Lys Met Gly Asp Ser Lys Gly
305                 310                 315                 320
Glu Asp Met Ser Leu Gln Lys Gln Tyr Met Val Pro Leu Arg His Gly
                325                 330                 335
Ser Gly Ser His Gly Val Trp Asp Gly Ser Ala Gly Gly Thr Gly Ser
            340                 345                 350
Asn Gly Gly Gly Thr Gly Asn Gly Gly Ser Ser Trp Pro Met Asn Met
        355                 360                 365
Ile Pro Gly Phe His Ser Ser Ser Thr Ser Gly Cys Asn Asp Ser Gly
    370                 375                 380
Leu
385

<210> 28
<211> 1729
<212> DNA
<213> Zea mays

<400> 28
   gctcctctcc ctgctccgac gtctttataa accgccacac cccggtacca ctaccccacc 60
   ccagccccca tcctctgatt cataccacaa agctagaacc tgaaagcagc cagccacacc 120
   actccgctcc actccacctt tgccatctcc aagaaagaac ccagcctctt ctgcttcaag 180
   aagccttttt tgatcctgag ttatcgccat ccatcaccat gatccaagaa ctgctcggcg 240
   gggccgccat ggaccagctc aagagcgtga atgagtccct gcccctgttg ctgcactcgg 300
   tcatctccaa cccatcgccc acgtcgtcgt cgtcgacgtc gtcgcgctcg tctgcgcagc 360
   agcatcagca gcagcggtcg acgtcggcaa cgtcgtcgcc gcaagcaggg cagcagcagc 420
   agcagcaggg ccaggggcaa ggagcggagc agacgcctct gcggtgcccc aggtgtaact 480
   cctccaacac caagttctgc tactacaaca actacaacct cacccagccg cgccacttct 540
   gcaagacgtg ccgccggtac tggaccaagg gcggcgcgct ccgcaacgtg cccatcggcg 600
   gcggctgccg caagccgcgc cccatgccca cgcccgtcac gaagccggcg gtctcatgca 660
   aggccgtggg cggcgcgcag tcgctgggcc tcggcgtcgg cttgggcatg ggcgccggcc 720
   ccggaccctg ggcgtcctcg cagcaggcgg ccgccgcgca gctcatggcg ctgctcaaca 780
   gcgccaggag cgtgcaggga ggcggcggcg gcaacatgca caggctcctc ggcctcgacg 840
   ccgtggccca cctgcccctc catgtcctgc cgggcgcggg caataatgcc ggtggcacgg 900
   cgccgtcgtt ctggccgcag gccgcgccgc gtgtcatccc cgcaccgccg cacatggact 960
   cccagctcgg catggggccg ctgggccagc acgacgtgct gtcgagcctc gggctaaagc 1020
   tgcccccgcc atcgccgtcg ccggcggcaa gctactacag cgaccagctg cacgcggtgg 1080
   tgagcagcgc cgccggacgc ggacacgagt acgaaacagc cgcctgcgcc acgtcactgc 1140
   cttgcaccac ggcgctgacc tccctcccgc cggccgcgtc gagcgtgtcc gctgcactgg 1200
   ccagcgccgc cacggtcggg ctagacctcc cgctggtctc cctctccgcg cccgagatgc 1260
   agtactgggc cgggccggcg gcgatgtccg tggcgtggcc ggacttgccc acccccaacg 1320
   gcgcgttccc gtgagagaca aacatggacc tcatctgctg tacggatggc gcagctgttt 1380
   acccacccac atgacacact agctagtacg taatcagtag ctcgatcact tcagccttca 1440
   gtgtagcttt atcttgccgt taatttgact gtgttatgag tgagaactcc actctggggg 1500
   aggttattaa tcaatggtgt ttctattact cttgtttttg ttcactagtg ggagcatggg 1560
   agtaactttt cttactgtta ttaatcagag tttttattac tcttgttggt aatatctttt 1620
   tctatttatc ttctctatat ctgcatgctt gtgagctgta tgtagtgtag tagcaatagt 1680
   agaagtgatc cataactttg tatctgttgt taaaaaaaaa aaaaaaaaa 1729

<210> 29
<211> 1392
<212> DNA
<213> Zea mays

<400> 29
   atgatccaag aactgctcgg cggggccgcc atggaccagc tcaagagcgt gaatgagtcc 60
   ctgcccctgt tgctgcactc ggtcatctcc aacccatcgc ccacgtcgtc gtcgtcgacg 120
   tcgtcgtcgc gctcgtctgc gcagcagcat cagcagcagc ggtcgacgtc ggcaacgtcg 180
   tcgccgcaag cagggcagca gcagcagcag cagggccagg ggcaaggagc ggagcagacg 240
   cctctgcggt gccccaggtg taactcctcc aacaccaagt tctgctacta caacaactac 300
   aacctcaccc agccgcgcca cttctgcaag acgtgccgcc ggtactggac caagggcggc 360
   gcgctccgca acgtgcccat cggcggcggc tgccgcaagc cgcgccccat gcccacgccc 420
   gtcacgaagc cggcggtctc atgcaaggcc gtgggcggcg cgcagtcgct gggcctcggc 480
   gtcggcttgg gcatgggcgc cggccccgga ccctgggcgt cctcgcagca ggcggccgcc 540
   gcgcagctca tggcgctgct caacagcgcc aggagcgtgc agggaggcgg cggcggcaac 600
   atgcacaggc tcctcggcct cgacgccgtg gcccacctgc ccctccatgt cctgccgggc 660
   gcgggcaata atgccggtgg cacggcgccg tcgttctggc cgcaggccgc gccgcgtgtc 720
   atccccgcac cgccgcacat ggactcccag ctcggcatgg ggccgctggg ccagcacgac 780
   gtgctgtcga gcctcgggct aaagctgccc ccgccatcgc cgtcgccggc ggcaagctac 840
   tacagcgacc agctgcacgc ggtggtgagc agcgccgccg gacgcggaca cgagtacgaa 900
   acagccgcct gcgccacgtc actgccttgc accacggcgc tgacctccct cccgccggcc 960
   catggggccg ctgggccagc acgacgtgct gtcgagcctc gggctaaagc tgcccccgcc 1020
   atcgccgtcg ccggcggcaa gctactacag cgaccagctg cacgcggtgg tgagcagcgc 1080
   cgccggacgc ggacacgagt acgaaacagc cgcctgcgcc acgtcactgc cttgcaccac 1140
   ggcgctgacc tccctcccgc cggccgcgtc gagcgtgtcc gctgcactgg ccagcgccgc 1200
   cacggtcggg ctagacctcc cgctggtctc cctctccgcg cccgagatgc agtactgggc 1260
   cgggccggcg gcgatgtccg tggcgtggcc ggacttgccc acccccaacg gcgcgttccc 1320
   gtgagagaca aacatggacc tcatctgctg tacggatggc gcagctgttt acccacccac 1380
   atgacacatt ga 1392

<210> 30

<211> 463
<212> PRT
<213> Zea mays

<400> 30
Met Ile Gln Glu Leu Leu Gly Gly Ala Ala Met Asp Gln Leu Lys Ser
1               5                   10                  15
Val Asn Glu Ser Leu Pro Leu Leu Leu His Ser Val Ile Ser Asn Pro
            20                  25                  30
Ser Pro Thr Ser Ser Ser Ser Thr Ser Ser Ser Arg Ser Ser Ala Gln
        35                  40                  45
Gln His Gln Gln Gln Arg Ser Thr Ser Ala Thr Ser Ser Pro Gln Ala
    50                  55                  60
Gly Gln Gln Gln Gln Gln Gln Gly Gln Gly Gln Gly Ala Glu Gln Thr
65                  70                  75                  80
Pro Leu Arg Cys Pro Arg Cys Asn Ser Ser Asn Thr Lys Phe Cys Tyr
                85                  90                  95
Tyr Asn Asn Tyr Asn Leu Thr Gln Pro Arg His Phe Cys Lys Thr Cys
            100                 105                 110
Arg Arg Tyr Trp Thr Lys Gly Gly Ala Leu Arg Asn Val Pro Ile Gly
        115                 120                 125
Gly Gly Cys Arg Lys Pro Arg Pro Met Pro Thr Pro Val Thr Lys Pro
    130                 135                 140
Ala Val Ser Cys Lys Ala Val Gly Gly Ala Gln Ser Leu Gly Leu Gly
145                 150                 155                 160
Val Gly Leu Gly Met Gly Ala Gly Pro Gly Pro Trp Ala Ser Ser Gln
                165                 170                 175
Gln Ala Ala Ala Ala Gln Leu Met Ala Leu Leu Asn Ser Ala Arg Ser
            180                 185                 190
Val Gln Gly Gly Gly Gly Gly Asn Met His Arg Leu Leu Gly Leu Asp
        195                 200                 205
Ala Val Ala His Leu Pro Leu His Val Leu Pro Gly Ala Gly Asn Asn
    210                 215                 220
Ala Gly Gly Thr Ala Pro Ser Phe Trp Pro Gln Ala Ala Pro Arg Val
225                 230                 235                 240
Ile Pro Ala Pro Pro His Met Asp Ser Gln Leu Gly Met Gly Pro Leu
                245                 250                 255
Gly Gln His Asp Val Leu Ser Ser Leu Gly Leu Lys Leu Pro Pro Pro
            260                 265                 270
Ser Pro Ser Pro Ala Ala Ser Tyr Tyr Ser Asp Gln Leu His Ala Val
        275                 280                 285
Val Ser Ser Ala Ala Gly Arg Gly His Glu Tyr Glu Thr Ala Ala Cys
    290                 295                 300
Ala Thr Ser Leu Pro Cys Thr Thr Ala Leu Thr Ser Leu Pro Ala Arg
305                 310                 315                 320
His Gly Ala Ala Gly Pro Ala Arg Arg Ala Val Glu Pro Arg Ala Lys
                325                 330                 335
Ala Ala Pro Ala Ile Ala Val Ala Gly Gly Lys Leu Leu Gln Arg Pro
            340                 345                 350
Ala Ala Arg Gly Gly Glu Gln Arg Arg Arg Thr Arg Thr Arg Val Arg
        355                 360                 365
Asn Ser Arg Leu Arg His Val Thr Ala Leu His His Gly Ala Asp Leu
    370                 375                 380
Pro Pro Ala Gly Arg Val Glu Arg Val Arg Cys Thr Gly Gln Arg Arg
385                 390                 395                 400
His Gly Arg Ala Arg Pro Pro Ala Gly Leu Pro Leu Arg Ala Arg Asp
                405                 410                 415
Ala Val Leu Gly Arg Ala Gly Gly Asp Val Arg Gly Val Ala Gly Leu
            420                 425                 430
Ala His Pro Gln Arg Arg Val Pro Val Arg Asp Lys His Gly Pro His
        435                 440                 445

Leu Leu Tyr Gly Trp Arg Ser Cys Leu Pro Thr His Met Thr His
    450                 455                 460

<210> 31
<211> 1257
<212> DNA
<213> Zea mays

<400> 31
cttcgatctc caatccccag ctagctaact atccaatggc gggccaagtg atggaagctc 60
aggcgtgccg gctgcagcct cccaccatgg cgccgttcgc gccactacct catcagtaca 120
acaacacctg caagcacctc aacacgacga tggccgccac ttccggcaac aacaacagca 180
acggcagcac cgctaccgcg gctggcggtg cggcggccga cggcatggcc gcctacctcc 240
agcacctgca gcaggcgggc gccgaggcgg cggccaagag cggcggcggc ggcggcgcgg 300
cgcgcgggga gcagtgcccg cggtgcgcgt cgcgcgacac caagttctgc tactacaaca 360
actacaacac gtcccagccg cgccacttct gccgcgcctg ccgacgctac tggacgctgg 420
gcgggtccct ccgcaacgtc cccgtcggcg ggtccacgcg caagcgcccg cgcctggcgc 480.
accaccacca gcacgccagg ctcgctcctc cggcgccgca cgtcttcggc ctcacgccga 540
tgatgcctcc tcccttgcaa ccgtcgtcgt cgacatcgtc gtcgcaggga cagggacagg 600
gacagggaca gagcggcggc ctcctcggct cactgttcgc gctcggcgcc gcgggcgcgg 660
ggccgccgct gctcgaaggc cgcggcgcag ggtcgtcgtt cgacttcgac ctcgggctcg 720
gactcccgac gggggcctta cacctgggcg cggccggaca aatgcaaggc ctcgggctca 780
tggggggagg aggtgcctcc gcagccgggt cgtcgtcctt cctctggccc gccgcggggc 840
tgctggtgga caacgacagc gtggacacgt ggaagatgcc aggcgccgct gcgggttcaa 900
tgtggccaga cttctctttt ccggcggcag cggcgccaca aaccgccgga ttgcttcatg 960
gcggaggcca cctgatgtga gacgagacgc cgcgcttcct tcgacgtact accccctagc 1020
gctagcccta gattctagcg catgcagccc ggtgtccggg ccggtttgca ttgcatagca 1080
tacatagtag ttggtctttt tttcagcatt gtattattgg atcatgtgta cgtaattaag 1140
ccgtgcttca gttttccttg actctgttaa gctataaagg gccctccaaa tctgcagttt 1200
tggggcgttt gtttcagaaa atcatattct agaattcgat tctatcacaa aaaaaaa 1257

<210> 32
<211> 945
<212> DNA
<213> Zea mays

<400> 32
atggcgggcc aagtgatgga agctcaggcg tgccggctgc agcctcccac catggcgccg 60
ttcgcgccac tacctcatca gtacaacaac acctgcaagc acctcaacac gacgatggcc 120
gccacttccg gcaacaacaa cagcaacggc agcaccgcta ccgcggctgg cggtgcggcg 180
gccgacggca tggccgccta cctccagcac ctgcagcagg cgggcgccga ggcggcggcc 240
aagagcggcg gcggcggcgg cgcggcgcgc ggggagcagt gcccgcggtg cgcgtcgcgc 300
gacaccaagt tctgctacta caacaactac aacacgtccc agccgcgcca cttctgccgc 360
gcctgccgac gctactggac gctgggcggg tccctccgca acgtccccgt cggcgggtcc 420
acgcgcaagc gcccgcgcct ggcgcaccac caccagcacg ccaggctcgc tcctccggcg 480
ccgcacgtct tcggcctcac gccgatgatg cctcctccct tgcaaccgtc gtcgtcgaca 540
tcgtcgtcgc agggacaggg acagggacag ggacagagcg gcggcctcct cggctcactg 600
ttcgcgctcg gcgccgcggg cgcggggccg ccgctgctcg aaggccgcgg cgcagggtcg 660
tcgttcgact tcgacctcgg gctcggactc ccgacggggg ccttacacct gggcgcggcc 720
ggacaaatgc aaggcctcgg gctcatgggg ggaggaggtg cctccgcagc cgggtcgtcg 780
tccttcctct ggcccgccgc ggggctgctg gtggacaacg acagcgtgga cacgtggaag 840
atgccaggcg ccgctgcggg ttcaatgtgg ccagacttct cttttccggc ggcagcggcg 900
ccacaaaccg ccggattgct tcatggcgga ggccacctga tgtga 945

<210> 33
<211> 314
<212> PRT
<213> Zea mays

<400> 33

Met Ala Gly Gln Val Met Glu Ala Gln Ala Cys Arg Leu Gln Pro Pro
1               5                   10                  15
Thr Met Ala Pro Phe Ala Pro Leu Pro His Gln Tyr Asn Asn Thr Cys
            20                  25                  30
Lys His Leu Asn Thr Thr Met Ala Ala Thr Ser Gly Asn Asn Asn Ser
        35                  40                  45
Asn Gly Ser Thr Ala Thr Ala Ala Gly Gly Ala Ala Ala Asp Gly Met
    50                  55                  60
Ala Ala Tyr Leu Gln His Leu Gln Gln Ala Gly Ala Glu Ala Ala Ala
65                  70                  75                  80
Lys Ser Gly Gly Gly Gly Gly Ala Ala Arg Gly Glu Gln Cys Pro Arg
                85                  90                  95
Cys Ala Ser Arg Asp Thr Lys Phe Cys Tyr Tyr Asn Asn Tyr Asn Thr
            100                 105                 110
Ser Gln Pro Arg His Phe Cys Arg Ala Cys Arg Arg Tyr Trp Thr Leu
        115                 120                 125
Gly Gly Ser Leu Arg Asn Val Pro Val Gly Gly Ser Thr Arg Lys Arg
    130                 135                 140
Pro Arg Leu Ala His His His Gln His Ala Arg Leu Ala Pro Pro Ala
145                 150                 155                 160
Pro His Val Phe Gly Leu Thr Pro Met Met Pro Pro Pro Leu Gln Pro
                165                 170                 175
Ser Ser Ser Thr Ser Ser Ser Gln Gly Gln Gly Gln Gly Gln Gly Gln
            180                 185                 190
Ser Gly Gly Leu Leu Gly Ser Leu Phe Ala Leu Gly Ala Ala Gly Ala
        195                 200                 205
Gly Pro Pro Leu Leu Glu Gly Arg Gly Ala Gly Ser Ser Phe Asp Phe
    210                 215                 220
Asp Leu Gly Leu Gly Leu Pro Thr Gly Ala Leu His Leu Gly Ala Ala
225                 230                 235                 240
Gly Gln Met Gln Gly Leu Gly Leu Met Gly Gly Gly Gly Ala Ser Ala
                245                 250                 255
Ala Gly Ser Ser Ser Phe Leu Trp Pro Ala Ala Gly Leu Leu Val Asp
            260                 265                 270
Asn Asp Ser Val Asp Thr Trp Lys Met Pro Gly Ala Ala Ala Gly Ser
        275                 280                 285
Met Trp Pro Asp Phe Ser Phe Pro Ala Ala Ala Ala Pro Gln Thr Ala
    290                 295                 300
Gly Leu Leu His Gly Gly Gly His Leu Met
305                 310

<210> 34
<211> 1494
<212> DNA
<213> Zea mays

<400> 34
ggccgagcgg gtggagctct agccacgccc acgctcccgc ctccccggca aacacactca 60
cgcccgccga ggccacatcc taacacgttg tagacgccgc caccgcctga cgccatgcag 120
gagttccagt ccatccctgg cctggccggg cggctcttcg gcggggccgc ggcccccggc 180
gacctccggc gcgcgcaggc ggcgcagcat ggccccggcg gagcgcggtg cggcggtgcc 240
tcgcccgcgg cgccggaggc ggtgaagtgc ccgcggtgcg agtctaccaa caccaagttc 300
tgctactaca acaactacaa cctgtcgcag ccgcgccact tctgcaagag ctgccgccgg 360
tactggacca agggcggcgt cctgcgcaac gtccccgtgg gcggcggctg ccgcaaggcg 420
aagcgcgcct cgtcctcggc gtcggcgtcg gcgtcggtgt cctcgcccgc ctcgtcctcg 480
gcgccgtcca cgcccgcgtc ggccgacgcg ggcaagaacc cgcgccgcgc ctcggcgtcc 540
tcgccctcgc cccgctccaa cagcggcagc gcgagcccca cggccgccgc cgcgacgacc 600
ccgaccccga ccgatccgac cacccccgcc acgccgtcgt cgaacggcgt cgccttcacg 660
ggcagccacc actcgtcgaa ccccttctcc acgatcgacg tggcggccgc gccaccggcg 720
ccgatattcg ccgaccaggc ggcggcgctg gcgtccctct tcgcgcctcc ccctccgccg 780

ccgctcccgg tgttcagctt cgcggcggcg cagccgaagg aggaggaagc gcccaccaat 840
tcggagctgc agcaacacct cgccgcacag gcggcgccgt cgtcgtcggt ctccgaggac 900
atcatggcgc cgttcgcatc cctggacgcc gctgggatat tcgagctcgg cgacgccgcg 960
tcagccgcgg cgtactggag cgccgggagc tgctgggcgg acgtccagga cccgagcttg 1020
tacctaccct agcgtacgtg cttgtttagt tacctatcgc gatcgcttga ttaaagccgc 1080
ggttagatct taacgacggc cggccacgag ctccgtctct ctagctatta ctccatgtcc 1140
tccctgcctc tctctctctg tcttgtttat tggtgatttg gcttcacagg ctgcgtgtct 1200
agaaagctaa catctgtgtt ccgtgtctgc attcaccctg cgagtgtggc ggatcattgc 1260
gctgctgcga tagaattaat cgcattatta ttgcttgttt actctttgta atagtaactt 1320
gtatgaatac atgcatgttt gggggggggg ggaagcaacg taacgtaacc acgagtgtcc 1380
gtgtgttatg ttgtgatgcg cggtcaaagc cgatggtcag gtgtatcgcc aggggatgga 1440
tgatggaaca atggtgcgtg tcattttggt taaaaaaaaa aaaaaaaaaa aaaa 1494

<210> 35
<211> 918
<212> DNA
<213> Zea mays

<400> 35
atgcaggagt tccagtccat ccctggcctg gccgggcggc tcttcggcgg ggccgcggcc 60
cccggcgacc tccggcgcgc gcaggcggcg cagcatggcc ccggcggagc gcggtgcggc 120
ggtgcctcgc ccgcggcgcc ggaggcggtg aagtgcccgc ggtgcgagtc taccaacacc 180
aagttctgct actacaacaa ctacaacctg tcgcagccgc gccacttctg caagagctgc 240
cgccggtact ggaccaaggg cggcgtcctg cgcaacgtcc ccgtgggcgg cggctgccgc 300
aaggcgaagc gcgcctcgtc ctcggcgtcg gcgtcggcgt cggtgtcctc gcccgcctcg 360
tcctcggcgc cgtccacgcc cgcgtcggcc gacgcgggca agaacccgcg ccgcgcctcg 420
gcgtcctcgc cctcgccccg ctccaacagc ggcagcgcga gccccacggc cgccgccgcg 480
acgaccccga ccccgaccga tccgaccacc cccgccacgc cgtcgtcgaa cggcgtcgcc 540
ttcacgggca gccaccactc gtcgaacccc ttctccacga tcgacgtggc ggccgcgcca 600
ccggcgccga tattcgccga ccaggcggcg gcgctggcgt ccctcttcgc gcctccccct 660
ccgccgccgc tcccggtgtt cagcttcgcg gcggcgcagc cgaaggagga ggaagcgccc 720
accaattcgg agctgcagca acacctcgcc gcacaggcgg cgccgtcgtc gtcggtctcc 780
gaggacatca tggcgccgtt cgcatccctg gacgccgctg ggatattcga gctcggcgac 840
gccgcgtcag ccgcggcgta ctggagcgcc gggagctgct gggcggacgt ccaggacccg 900
agcttgtacc taccctag 918

<210> 36
<211> 305
<212> PRT
<213> Zea mays

<400> 36
Met Gln Glu Phe Gln Ser Ile Pro Gly Leu Ala Gly Arg Leu Phe Gly
1               5                   10                  15
Gly Ala Ala Ala Pro Gly Asp Leu Arg Arg Ala Gln Ala Ala Gln His
            20                  25                  30
Gly Pro Gly Gly Ala Arg Cys Gly Gly Ala Ser Pro Ala Ala Pro Glu
        35                  40                  45
Ala Val Lys Cys Pro Arg Cys Glu Ser Thr Asn Thr Lys Phe Cys Tyr
    50                  55                  60
Tyr Asn Asn Tyr Asn Leu Ser Gln Pro Arg His Phe Cys Lys Ser Cys
65                  70                  75                  80
Arg Arg Tyr Trp Thr Lys Gly Gly Val Leu Arg Asn Val Pro Val Gly
                85                  90                  95
Gly Gly Cys Arg Lys Ala Lys Arg Ala Ser Ser Ser Ala Ser Ala Ser
            100                 105                 110
Ala Ser Val Ser Ser Pro Ala Ser Ser Ser Ala Pro Ser Thr Pro Ala
        115                 120                 125
Ser Ala Asp Ala Gly Lys Asn Pro Arg Arg Ala Ser Ala Ser Ser Pro
    130                 135                 140

Ser Pro Arg Ser Asn Ser Gly Ser Ala Ser Pro Thr Ala Ala Ala Ala
145                 150                 155                 160
Thr Thr Pro Thr Pro Thr Asp Pro Thr Thr Pro Ala Thr Pro Ser Ser
                165                 170                 175
Asn Gly Val Ala Phe Thr Gly Ser His His Ser Ser Asn Pro Phe Ser
            180                 185                 190
Thr Ile Asp Val Ala Ala Ala Pro Pro Ala Pro Ile Phe Ala Asp Gln
        195                 200                 205
Ala Ala Ala Leu Ala Ser Leu Phe Ala Pro Pro Pro Pro Pro Pro Leu
    210                 215                 220
Pro Val Phe Ser Phe Ala Ala Ala Gln Pro Lys Glu Glu Glu Ala Pro
225                 230                 235                 240
Thr Asn Ser Glu Leu Gln Gln His Leu Ala Ala Gln Ala Ala Pro Ser
                245                 250                 255
Ser Ser Val Ser Glu Asp Ile Met Ala Pro Phe Ala Ser Leu Asp Ala
            260                 265                 270
Ala Gly Ile Phe Glu Leu Gly Asp Ala Ala Ser Ala Ala Ala Tyr Trp
        275                 280                 285
Ser Ala Gly Ser Cys Trp Ala Asp Val Gln Asp Pro Ser Leu Tyr Leu
    290                 295                 300
Pro
305

<210> 37
<211> 2080
<212> DNA
<213> Zea mays

<400> 37
   ctttcaggcg tcgccaactc cacttgtcag atcttgcccg tatggtatgg agctcgccgg 60
   agcagcctcc cctcacccgc cgccggaatc ccacgtggcg ccgccccgcc cacctccgca 120
   gccgccggaa gaggatttat gtgaagatag aggggacatg agtgtcaccg gcgaaaagcc 180
   atgcacacat cgggaatcag atgttggtca gacaaatagt tgtagcctta acaattccag 240
   tgagtgtgag aatcatacac ccagcaacga cgaaatatcg gaaccagagt ccaatttgga 300
   gatggccaag actgatcagg gcggcgatgt gccgagcgga gagaaggtcc tgaagaagcc 360
   agacaagatc ctgccgtgcc ctcgctgcaa cagcatggac acgaagttct gctactacaa 420
   caactacaac atcaagcagc caaggcattt ctgcaagagc tgtcagaggt actggaccgc 480
   aggcggaagc atgagaaaca tccccgttgg tgctgggagg cgcaagagca agagctccag 540
   cgcgagttgc cgcagcgtgt tgattcccgt tccaggcagc agtagtgtag ctaatcctgg 600
   cggagaggct tccctgtttc cgttgtccgt aaaagcaaac caagcagcag ttagcttcgg 660
   gcctgattcc cccctctgca cctccatggc ctccgtgctg aagatcggag gaggaggaga 720
   gcaggttaag agctgcagcc ctgcctcagc agcagcacag cccaggaacg gagaaaccca 780
   gacccagacg tgcgcacctt cttctgctgc tacaacacca tcagatggtc cagggaatgg 840
   attgcagaaa ggagcagaga gcgcacacca aaaccaaaac caaaaccaaa acggaatcat 900
   tgggcacagc aacggagtca ctccagtgca tcctataccg ttcttccccg gaccgccttt 960
   cgtgtacccc tggagtccag catggaacgg cattcccgcc atggcagcgg cggtgtgcgc 1020
   agccccagcg acagccgaag cagcgatttc atcagaacac ggcaccgcga gcagcaccgt 1080
   ccagtggaac gtgccaccag cgatcgtgcc cgtgctgcca ccgggattct gcggcccgat 1140
   cccagtcccg gtaatcccgc cgccttccgt ctggccactg atcactccct ggcccaacgc 1200
   agcatggagc gcgccgtggc tcgggcctag cgctagcgtg ccgccggggt catctcggag 1260
   cagcggcagc agcacgtgct ccgacagcgg ctgcggctcc ggctcccccg tcctgggaaa 1320
   gcactcgagg gagtcgaggc cgcagggcga cgagaaggcg gaacgacggt gcctgtggat 1380
   ccccaagacg ctccggatcg acgaccctgt cgaggccgcc aagagctcga tctggacgac 1440
   gctcgggatc gagcctggcg accggggcat gttcaggccg ttccagtcga agcatggacg 1500
   gcagcaggag ctgcaagcgt ccggcgccgc tcgcgccctg caggccaacc cggcggctct 1560
   gtcgcgctcg cagtcttttc aggagacgac gtgattacta cactacagag cagagcgttg 1620
   ctgaaacctg tgtggcatta ctattcagag acgattgtat ttcaacttca aggggggaaa 1680
   tgagagagag agcgcgagag agagattggc ctgtttggtt cactacctca gttgccacac 1740
   tttgcctaac ttttctgact aaggttagtt attcaattcg aacgactaac attaggcaaa 1800
   gtgtggcata tttagtcata aaccaaacat gccatatatg ttctgtacaa tatgattggc 1860
   aacataggtg ctgctgctgt acaaggcagc ggacaccgtt actgtaactg taaccattgg 1920
   aagagtgcat cctcaaacag tgtgttgttt ccatgtagag cgtgccccca acatgttggg 1980
   ttggaggtga tcaggtttcc agcgcgtgcg tgcttggttg tacaagaata actgtcaata 2040
   gcttcccttc aaaaaaaaaa aaaaaaaaaa aaaaaaaaaa 2080

<210> 38
<211> 1548
<212> DNA
<213> Zea mays

<400> 38
atggagctcg ccggagcagc ctcccctcac ccgccgccgg aatcccacgt ggcgccgccc 60
cgcccacctc cgcagccgcc ggaagaggat ttatgtgaag atagagggga catgagtgtc 120
accggcgaaa agccatgcac acatcgggaa tcagatgttg gtcagacaaa tagttgtagc 180
cttaacaatt ccagtgagtg tgagaatcat acacccagca acgacgaaat atcggaacca 240
gagtccaatt tggagatggc caagactgat cagggcggcg atgtgccgag cggagagaag 300
gtcctgaaga agccagacaa gatcctgccg tgccctcgct gcaacagcat ggacacgaag 360
ttctgctact acaacaacta caacatcaag cagccaaggc atttctgcaa gagctgtcag 420
aggtactgga ccgcaggcgg aagcatgaga aacatccccg ttggtgctgg gaggcgcaag 480
agcaagagct ccagcgcgag ttgccgcagc gtgttgattc ccgttccagg cagcagtagt 540
gtagctaatc ctggcggaga ggcttccctg tttccgttgt ccgtaaaagc aaaccaagca 600
gcagttagct tcgggcctga ttcccccctc tgcacctcca tggcctccgt gctgaagatc 660
ggaggaggag gagagcaggt taagagctgc agccctgcct cagcagcagc acagcccagg 720
aacggagaaa cccagaccca gacgtgcgca ccttcttctg ctgctacaac accatcagat 780
ggtccaggga atggattgca gaaaggagca gagagcgcac accaaaacca aaaccaaaac 840
caaaacggaa tcattgggca cagcaacgga gtcactccag tgcatcctat accgttcttc 900
cccggaccgc ctttcgtgta cccctggagt ccagcatgga acggcattcc cgccatggca 960
gcggcggtgt gcgcagcccc agcgacagcc gaagcagcga tttcatcaga acacggcacc 1020
gcgagcagca ccgtccagtg gaacgtgcca ccagcgatcg tgcccgtgct gccaccggga 1080
ttctgcggcc cgatcccagt cccggtaatc ccgccgcctt ccgtctggcc actgatcact 1140
ccctggccca acgcagcatg gagcgcgccg tggctcgggc ctagcgctag cgtgccgccg 1200
gggtcatctc ggagcagcgg cagcagcacg tgctccgaca gcggctgcgg ctccggctcc 1260
cccgtcctgg gaaagcactc gagggagtcg aggccgcagg gcgacgagaa ggcggaacga 1320
cggtgcctgt ggatccccaa gacgctccgg atcgacgacc ctgtcgaggc cgccaagagc 1380
tcgatctgga cgacgctcgg gatcgagcct ggcgaccggg gcatgttcag gccgttccag 1440
tcgaagcatg gacggcagca ggagctgcaa gcgtccggcg ccgctcgcgc cctgcaggcc 1500
aacccggcgg ctctgtcgcg ctcgcagtct tttcaggaga cgacgtga 1548

<210> 39
<211> 515
<212> PRT
<213> Zea mays

<400> 39
Met Glu Leu Ala Gly Ala Ala Ser Pro His Pro Pro Pro Glu Ser His
1               5                   10                  15
Val Ala Pro Pro Arg Pro Pro Pro Gln Pro Pro Glu Glu Asp Leu Cys
            20                  25                  30
Glu Asp Arg Gly Asp Met Ser Val Thr Gly Glu Lys Pro Cys Thr His
        35                  40                  45
Arg Glu Ser Asp Val Gly Gln Thr Asn Ser Cys Ser Leu Asn Asn Ser
    50                  55                  60
Ser Glu Cys Glu Asn His Thr Pro Ser Asn Asp Glu Ile Ser Glu Pro
65                  70                  75                  80
Glu Ser Asn Leu Glu Met Ala Lys Thr Asp Gln Gly Gly Asp Val Pro
                85                  90                  95
Ser Gly Glu Lys Val Leu Lys Lys Pro Asp Lys Ile Leu Pro Cys Pro
            100                 105                 110
Arg Cys Asn Ser Met Asp Thr Lys Phe Cys Tyr Tyr Asn Asn Tyr Asn
        115                 120                 125

Ile Lys Gln Pro Arg His Phe Cys Lys Ser Cys Gln Arg Tyr Trp Thr
    130                 135                 140
Ala Gly Gly Ser Met Arg Asn Ile Pro Val Gly Ala Gly Arg Arg Lys
145                 150                 155                 160
Ser Lys Ser Ser Ser Ala Ser Cys Arg Ser Val Leu Ile Pro Val Pro
                165                 170                 175
Gly Ser Ser Ser Val Ala Asn Pro Gly Gly Glu Ala Ser Leu Phe Pro
            180                 185                 190
Leu Ser Val Lys Ala Asn Gln Ala Ala Val Ser Phe Gly Pro Asp Ser
        195                 200                 205
Pro Leu Cys Thr Ser Met Ala Ser Val Leu Lys Ile Gly Gly Gly Gly
    210                 215                 220
Glu Gln Val Lys Ser Cys Ser Pro Ala Ser Ala Ala Ala Gln Pro Arg
225                 230                 235                 240
Asn Gly Glu Thr Gln Thr Gln Thr Cys Ala Pro Ser Ser Ala Ala Thr
                245                 250                 255
Thr Pro Ser Asp Gly Pro Gly Asn Gly Leu Gln Lys Gly Ala Glu Ser
            260                 265                 270
Ala His Gln Asn Gln Asn Gln Asn Gln Asn Gly Ile Ile Gly His Ser
        275                 280                 285
Asn Gly Val Thr Pro Val His Pro Ile Pro Phe Phe Pro Gly Pro Pro
    290                 295                 300
Phe Val Tyr Pro Trp Ser Pro Ala Trp Asn Gly Ile Pro Ala Met Ala
305                 310                 315                 320
Ala Ala Val Cys Ala Ala Pro Ala Thr Ala Glu Ala Ala Ile Ser Ser
                325                 330                 335
Glu His Gly Thr Ala Ser Ser Thr Val Gln Trp Asn Val Pro Pro Ala
            340                 345                 350
Ile Val Pro Val Leu Pro Pro Gly Phe Cys Gly Pro Ile Pro Val Pro
        355                 360                 365
Val Ile Pro Pro Pro Ser Val Trp Pro Leu Ile Thr Pro Trp Pro Asn
    370                 375                 380
Ala Ala Trp Ser Ala Pro Trp Leu Gly Pro Ser Ala Ser Val Pro Pro
385                 390                 395                 400
Gly Ser Ser Arg Ser Ser Gly Ser Ser Thr Cys Ser Asp Ser Gly Cys
                405                 410                 415
Gly Ser Gly Ser Pro Val Leu Gly Lys His Ser Arg Glu Ser Arg Pro
            420                 425                 430
Gln Gly Asp Glu Lys Ala Glu Arg Arg Cys Leu Trp Ile Pro Lys Thr
        435                 440                 445
Leu Arg Ile Asp Asp Pro Val Glu Ala Ala Lys Ser Ser Ile Trp Thr
    450                 455                 460
Thr Leu Gly Ile Glu Pro Gly Asp Arg Gly Met Phe Arg Pro Phe Gln
465                 470                 475                 480
Ser Lys His Gly Arg Gln Gln Glu Leu Gln Ala Ser Gly Ala Ala Arg
                485                 490                 495
Ala Leu Gln Ala Asn Pro Ala Ala Leu Ser Arg Ser Gln Ser Phe Gln
            500                 505                 510
Glu Thr Thr
        515


<210> 40
<211> 1195
<212> DNA
<213> Zea mays

<400> 40
ggctagctct agcagccctc cgtatatccc agagagcgag gtggtgacca cgcgcgcgcc 60
cacagggccg ggccagcgct agacactcag ctgattgcac tgagcagtga gcactagcta 120
actctcggcc gaatcgctgg gcgtctatta agccgacgcc aacgacgacg gctcctcggc 180

atgcgaggtc ctgctcgccc gggggatggc gcctgcagct tcgatcctct cggtcaccgc 240
cgtcgccggt tccaagcgtc cggccgcttc cgacgctgag ctcccgctcc tcgacctcga 300
ctcctcctcg ctccaccagc agcaggtcag tagcagcgtg atcaatccta cgtacgtacc 360
gtaccgacga cggcacgcac ggcagttctg cgatgatcga tcactgacgt atatactacg 420
cgtctgcggc tgtgggctgg catggcaggg tgacaaggct gggcgcaagg gccaggacca 480
ggaccaccac cagcagcagc agctggagtg cccgcgctgc cgctccacca acaccaagtt 540
ctgctactac aacaactaca gcacggcgca gccgcgccac ttctgccgcg cgtgccgccg 600
ctactggacg cacggcggca cgctgcgcga cgtcccggtt ggcggggcct cgcgccgcgc 660
cggtgggggc ggcaagcggc gcagggtctc ctccgccgag acctcgtcgt cgtcgccgcc 720
ggtgcctgcg tcgctcgcgg acgcgtgcct gtccgacctc ccgtccgtct tcccgttcct 780
cagcgacggc agcttcttcc cgcagctcga cctcggcgcc gtcgtgcttg caccgccggc 840
cttctcctcc tcgtggcggt cggtggcccc ggacttctac gacgggctcg cgccgtgggg 900
cgacatcgcc ggcctcgacc tcagctggac accaccgggg agcgccagcc ggtcgccgtc 960
ttgagggggc tccggtttta gcagtgcggc ctcatgagcc atcctagcaa gcagtgtgtt 1020
ccgttttgat gagccatcct atcctatcct atccagctag gaaaatttaa gcagctcgat 1080
caaaattcgc ctatgccttc actgttagat cagtgaataa tcttgatctg tagttggagc 1140
caggatgtaa gatctgtgca gtgatttatc atgacaataa aattaagtat gtgtc 1195

<210> 41
<211> 618
<212> DNA
<213> Zea mays

<400> 41
atggcgcctg cagcttcgat cctctcggtc accgccgtcg ccggttccaa gcgtccggcc 60
gcttcagacg ctgagctccc gcttctcggc ctcgactcct cctcgctcca ccagcagcag 120
ggtgacaagg ctgggcgcaa gggccaggac caggaccacc agcagcagct ggagtgcccg 180
cgctgccgct ccaccaacac caagttctgc tactacaaca actacagcac ggcgcagccg 240
cggcacttct gccgcgcgtg ccgccgctac tggacgcacg gcggcacgct gcgcgacgtc 300
ccggttggcg gggcctcgcg ccgcgccggt aggggcggca agcggcgcag ggtctcctcc 360
gccgagacct cctcgtcgtc gtcgccgccg ccgatgcctg cgtcgctcgc ggacgcgtgc 420
ctgtccgacc tcccgtccgt cttcccgttc ctcagcgacg gcagcttctt cccgcagctc 480
gacctcggcg ccgtcgtgct tgcaccgccg gccttctcct cctcgtggcg gtcggtggcc 540
ccggacttct acgacgggct cgcgccgtgg ggcgacatcg ccggcctcga cctcagctgg 600
acaccaccgg ggaactag 618

<210> 42
<211> 205
<212> PRT
<213> Zea mays

<400> 42
Met Ala Pro Ala Ala Ser Ile Leu Ser Val Thr Ala Val Ala Gly Ser
1               5                   10                  15
Lys Arg Pro Ala Ala Ser Asp Ala Glu Leu Pro Leu Leu Gly Leu Asp
            20                  25                  30
Ser Ser Ser Leu His Gln Gln Gln Gly Asp Lys Ala Gly Arg Lys Gly
        35                  40                  45
Gln Asp Gln Asp His Gln Gln Gln Leu Glu Cys Pro Arg Cys Arg Ser
    50                  55                  60
Thr Asn Thr Lys Phe Cys Tyr Tyr Asn Asn Tyr Ser Thr Ala Gln Pro
65                  70                  75                  80
Arg His Phe Cys Arg Ala Cys Arg Arg Tyr Trp Thr His Gly Gly Thr
                85                  90                  95
Leu Arg Asp Val Pro Val Gly Gly Ala Ser Arg Arg Ala Gly Arg Gly
            100                 105                 110
Gly Lys Arg Arg Arg Val Ser Ser Ala Glu Thr Ser Ser Ser Ser Ser
        115                 120                 125
Pro Pro Pro Met Pro Ala Ser Leu Ala Asp Ala Cys Leu Ser Asp Leu
    130                 135                 140

Pro Ser Val Phe Pro Phe Leu Ser Asp Gly Ser Phe Phe Pro Gln Leu
145                 150                 155                 160
Asp Leu Gly Ala Val Val Leu Ala Pro Pro Ala Phe Ser Ser Ser Trp
                165                 170                 175
Arg Ser Val Ala Pro Asp Phe Tyr Asp Gly Leu Ala Pro Trp Gly Asp
            180                 185                 190
Ile Ala Gly Leu Asp Leu Ser Trp Thr Pro Pro Gly Asn
        195                 200                 205

<210> 43
<211> 1584
<212> DNA
<213> Zea mays

<400> 43
tcttttccca tcactgacga cgagaggtgg aggttctggc cggagcagct gagaggtgca 60
taaaaaatat tgtaaaaaaa taatcctcca ctgctagatt ggctattggg caagagagag 120
cggagagggg aagggaagga ggcataattc cactcacact ggccaaccac agggcacagg 180
gtagcattct ctctcttcgt cctcaagaaa tccagcggaa gctcatcttc ttcaccctcc 240
cttctctcca catctccctt cggactacta ctgcatccaa ggagcaaaga attgtggggg 300
gtagcaaagg aattggaggt cttgcatgga tgcagcccac tggcagcagg gcctagggct 360
ggtgaagccc atggaggaga tgctgatggc ggccaacgcg ggcgccgcaa atccgagcca 420
aagctcgaat ccgaacccgc cggcgccggc gccgtcgttg gcacctgggg ggctcctggg 480
cggtggcgcg ccggcgcccc tggcgggcgc gggtagtacc gagcggcgcg cgcggccgca 540
gaaggagaag gcgctcaact gcccgcggtg caactccacc aacaccaaat tctgctacta 600
caacaactac agcctccagc agccacgcta cttctgcaag acgtgccgcc gctactggac 660
ggagggcgga tccctccgca acgtccccgt gggcggcggc tcccgcaaga acaagcgctc 720
ctcatcgtcg gcgtcggcgt ctgcctccac ctccggctcg gtcacgagct cgtccatggc 780
cagcacggcg ggggccgggt ccaagaaccc gaagctggcg cacgagggcg cccacgacct 840
caacctggcg ttcccgcacc acggtggcct gcacgccccc gagttcgcgg cgttcccgag 900
tctggagagc agcaacgtgt gcaacccggg cggcgggatg acgagcaacg gtcggggcgg 960
cggcgcgggg cccgcggtcg gcgcgctctc ggcaatggag ctgttgcgga gctccggctg 1020
ctacatgccg ctgcagatgc cgatgccgat ggcgatgcca ggagattaca cggcggcagg 1080
gttcgcgctc ggagagtacc gcacgccgcc gcctccaccg tcacagagcg tgctcgggtt 1140
ctctctcgac gcgcacgggc cggggtccgg tgctaccgcg gcggggtacg gttccagcgc 1200
ggggttgcag ggcgtgccgg agaacgcggg caggttattg ttccccttcg aagacttgaa 1260
gccgccggtt ggctctgaag gtgggggtgg tgcaaccggt ggcgccagcg atggaaatag 1320
cagccatact cagtttgacc acaacaacaa ggagcaaggc ggcggcggca cgggtgccgg 1380
gcacgacacg ccggggttct ggagcggcat gatcggcggc agtggcgctt cttggtaatg 1440
gagaaacatg cacggccggg gggcggcgcc atgcatggct catgcaaagg ctccctgcga 1500
gcgtgcatgg gcatgcagta gagacaaggg ttcaataaga ggttggcatt ggtggtggtt 1560
gccctgaaaa aaaaaaaaaa aaaa                                        1584

<210> 44
<211> 1113
<212> DNA
<213> Zea mays

<400> 44
atggatgcag cccactggca gcagggccta gggctggtga agcccatgga ggagatgctg 60
atggcggcca acgcgggcgc cgcaaatccg agccaaagct cgaatccgaa cccgccggcg 120
ccggcgccgt cgttggcacc tggggggctc ctgggcggtg gcgcgccggc gcccctggcg 180
ggcgcgggta gtaccgagcg gcgcgcgcgg ccgcagaagg agaaggcgct caactgcccg 240
cggtgcaact ccaccaacac caaattctgc tactacaaca actacagcct ccagcagcca 300
cgctacttct gcaagacgtg ccgccgctac tggacggagg gcggatccct ccgcaacgtc 360
cccgtgggcg gcggctcccg caagaacaag cgctcctcat cgtcggcgtc ggcgtctgcc 420
tccacctccg gctcggtcac gagctcgtcc atggccagca cggcgggggc cgggtccaag 480
aacccgaagc tggcgcacga gggcgcccac gacctcaacc tggcgttccc gcaccacggt 540
ggcctgcacg cccccgagtt cgcggcgttc ccgagtctgg agagcagcaa cgtgtgcaac 600
ccgggcggcg ggatgacgag caacggtcgg ggcggcggcg cggggcccgc ggtcggcgcg 660

ctctcggcaa tggagctgtt gcggagctcc ggctgctaca tgccgctgca gatgccgatg 720
ccgatggcga tgccaggaga ttacacggcg gcagggttcg cgctcggaga gtaccgcacg 780
ccgccgcctc caccgtcaca gagcgtgctc gggttctctc tcgacgcgca cggcccgggg 840
tccggtgcta ccgcggcggg gtacggttcc agcgcggggt tgcagggcgt gccggagaac 900
gcgggcaggt tattgttccc cttcgaagac ttgaagccgc cggttggctc tgaaggtggg 960
ggtggtgcaa ccggtggcgc cagcgatgga aatagcagcc atactcagtt tgaccacaac 1020
aacaaggagc aaggcggcgg cggcacgggt gccgggcacg acacgccggg gttctggagc 1080
ggcatgatcg gcggcagtgg cgcttcttgg taa 1113

<210> 45
<211> 370
<212> PRT
<213> Zea mays

<400> 45
Met Asp Ala Ala His Trp Gln Gln Gly Leu Gly Leu Val Lys Pro Met
1               5                   10                  15
Glu Glu Met Leu Met Ala Ala Asn Ala Gly Ala Ala Asn Pro Ser Gln
            20                  25                  30
Ser Ser Asn Pro Asn Pro Pro Ala Pro Ala Pro Ser Leu Ala Pro Gly
        35                  40                  45
Gly Leu Leu Gly Gly Gly Ala Pro Ala Pro Leu Ala Gly Ala Gly Ser
    50                  55                  60
Thr Glu Arg Arg Ala Arg Pro Gln Lys Glu Lys Ala Leu Asn Cys Pro
65                  70                  75                  80
Arg Cys Asn Ser Thr Asn Thr Lys Phe Cys Tyr Tyr Asn Asn Tyr Ser
                85                  90                  95
Leu Gln Gln Pro Arg Tyr Phe Cys Lys Thr Cys Arg Arg Tyr Trp Thr
            100                 105                 110
Glu Gly Gly Ser Leu Arg Asn Val Pro Val Gly Gly Gly Ser Arg Lys
        115                 120                 125
Asn Lys Arg Ser Ser Ser Ser Ala Ser Ala Ser Ala Ser Thr Ser Gly
    130                 135                 140
Ser Val Thr Ser Ser Ser Met Ala Ser Thr Ala Gly Ala Gly Ser Lys
145                 150                 155                 160
Asn Pro Lys Leu Ala His Glu Gly Ala His Asp Leu Asn Leu Ala Phe
                165                 170                 175
Pro His His Gly Gly Leu His Ala Pro Glu Phe Ala Ala Phe Pro Ser
            180                 185                 190
Leu Glu Ser Ser Asn Val Cys Asn Pro Gly Gly Gly Met Thr Ser Asn
        195                 200                 205
Gly Arg Gly Gly Gly Ala Gly Pro Ala Val Gly Ala Leu Ser Ala Met
    210                 215                 220
Glu Leu Leu Arg Ser Ser Gly Cys Tyr Met Pro Leu Gln Met Pro Met
225                 230                 235                 240
Pro Met Ala Met Pro Gly Asp Tyr Thr Ala Ala Gly Phe Ala Leu Gly
                245                 250                 255
Glu Tyr Arg Thr Pro Pro Pro Pro Pro Ser Gln Ser Val Leu Gly Phe
            260                 265                 270
Ser Leu Asp Ala His Gly Pro Gly Ser Gly Ala Thr Ala Ala Gly Tyr
        275                 280                 285
Gly Ser Ser Ala Gly Leu Gln Gly Val Pro Glu Asn Ala Gly Arg Leu
    290                 295                 300
Leu Phe Pro Phe Glu Asp Leu Lys Pro Pro Val Gly Ser Glu Gly Gly
305                 310                 315                 320
Gly Gly Ala Thr Gly Gly Ala Ser Asp Gly Asn Ser Ser His Thr Gln
                325                 330                 335
Phe Asp His Asn Asn Lys Glu Gln Gly Gly Gly Gly Thr Gly Ala Gly
            340                 345                 350
His Asp Thr Pro Gly Phe Trp Ser Gly Met Ile Gly Gly Ser Gly Ala
        355                 360                 365

Ser Trp
    370

<210> 46
<211> 1913
<212> DNA
<213> Zea mays

<400> 46
catccgttcg tttgtttgcc cttttttgcc ggcctcctcg tctcttccgg ttccggctac 60
aagtgaagtg agctcggccg tctgttgcct agcgtgctgc ttcgttcgtc gttggcttgc 120
agcagcttgc cgaagagaag aaagcagcag gcccggagct ttctgtttgt ttggatccga 180
gtgagaagaa gatggtggcc gagtgccacg gaggaggagg agcagcagca ggaggagggg 240
actttctcat caagctcttc gggatgacca tccccgtgcc ggagtgcggc gacgccaagg 300
atcttcagca gagcaagagc aacaggtgga ccgagcagga tcaggattcc catggcctgg 360
agaccgcgcc cgcgcccgcg cacacggaca cctccgaccc gtccccgcag ccggaggtcg 420
tggacgccga ggaccccacg gagacgcaac agaggccctg caacggcgac ggcgacggcg 480
atgctgccgg ccagagggag aagctgaaga agcccgacaa ggtgctgccg tgcccgcgct 540
gcaacagcat ggacaccaag ttctgctact tcaacaacta caacgtcaac cagccgcgcc 600
acttctgcaa gaactgccag cggtactgga cggccggtgg cgccatgcgc aacgtgcccg 660
tgggggccgg ccgccgcaag aacaagaacg ccgccgcctc gcacttcttc cagagggtcc 720
gggcgaccaa cgccacggtg ctcagcttcg ggggccatgg cggcgcgcct cctgctgccc 780
gcttggacct ggacctcgcc gagcagctga gccaccagct ggccccggtc aggagcgccg 840
gcgacgcggg ccgcccttgc agcgaaggat cgagcagcag ggacggcaac ggcagcaggt 900
cttctgtaga cgaagctgca gcaaacgcag acgacgggtc agtgcagcag cacccaggcc 960
cagcaagcat gaacagcagc ggggcaaccg tgtggccgcc gtacagctgc gccccagctc 1020
cggcggcgta cttcccccag ggcatcgcga ttccgatcta cccggccgca ccggcctact 1080
ggggctgcat ggttcccgga gcttggagcc tgccatggcc ggtgcagcac ccgtcgtcgt 1140
cgtcgtcgcc caccaccacc agtgctcctt cagtcgtctc gtcatccggg gcagctgacg 1200
actcatcatc ccacgcgctg ggcaagcgcc cccgggaccg ggagggtgac gatgggagaa 1260
acggcggcaa cgccaaggtg tgggcgccca agagcatccg gatagacgac gtggacgagg 1320
tggccaggag ctcaatctgg tccctcgtcg ggatcaaagg cgaccagacg aagcagcagg 1380
acgcagcaga cgaccacgcc ggtgggcaca gcaagcagct cgggacggtg ttcgagccca 1440
agcgcggcga ggccaccaag aaggccatga tgacaagctc gccgctcctt cacgcgaacc 1500
ccgttgcgct cacgcgctcc gtggccttcc aggaggggtc ttgattcttc ttcaagtaca 1560
tccatcagca gcagcaggaa tttcctcatg cagcgttcct tctcatctga acttctgaac 1620
tgaacatgag ctgatcagcc tgacagtacg tactgaattt tgtttttata taaatcatca 1680
taggattgta atatctatct atatgctgag tgggaaactt agctagcgta tgtgtaagtg 1740
gacttgtaat ataggcggca cgcaggatgt acatagaatt aattagctat atgggtttga 1800
gaaagaccag gagatcaata agaggtccct gggtgtagat aaataacatt aggggaagga 1860
atgtattgct tcaggtattt cactcttcaa aaaaaaaaaa aaaaaaaaaa aaa 1913

<210> 47
<211> 1353
<212> DNA
<213> Zea mays

<400> 47
atggtggccg agtgccacgg aggaggagga gcagcagcag gaggagggga ctttctcatc 60
aagctcttcg ggatgaccat ccccgtgccg gagtgcggcg acgccaagga tcttcagcag 120
agcaagagca acaggtggac cgagcaggat caggattccc atggcctgga gaccgcgccc 180
gcgcccgcgc acacggacac ctccgacccg tccccgcagc cggaggtcgt ggacgccgag 240
gaccccacgg agacgcaaca gaggccctgc aacggcgacg gcgacggcga tgctgccggc 300
cagagggaga agctgaagaa gcccgacaag gtgctgccgt gcccgcgctg caacagcatg 360
gacaccaagt tctgctactt caacaactac aacgtcaacc agccgcgcca cttctgcaag 420
aactgccagc ggtactggac ggccggtggc gccatgcgca acgtgcccgt gggggccggc 480
cgccgcaaga acaagaacgc cgccgcctcg cacttcttcc agagggtccg ggcgaccaac 540
gccacggtgc tcagcttcgg gggccatggc ggcgcgcctc ctgctgcccg cttggacctg 600
gacctcgccg agcagctgag ccaccagctg gccccggtca ggagcgccgg cgacgcgggc 660
cgcccttgca gcgaaggatc gagcagcagg gacggcaacg gcagcaggtc ttctgtagac 720

gaagctgcag caaacgcaga cgacgggtca gtgcagcagc acccaggccc agcaagcatg 780
aacagcagcg gggcaaccgt gtggccgccg tacagctgcg ccccagctcc ggcggcgtac 840
ttcccccagg gcatcgcgat tccgatctac ccggccgcac cggcctactg gggctgcatg 900
gttcccggag cttggagcct gccatggccg gtgcagcacc cgtcgtcgtc gtcgtcgccc 960
accaccacca gtgctccttc agtcgtctcg tcatccgggg cagctgacga ctcatcatcc 1020
cacgcgctgg gcaagcgccc ccgggaccgg gagggtgacg atgggagaaa cggcggcaac 1080
gccaaggtgt gggcgcccaa gagcatccgg atagacgacg tggacgaggt ggccaggagc 1140
tcaatctggt ccctcgtcgg gatcaaaggc gaccagacga agcagcagga cgcagcagac 1200
gaccacgccg gtgggcacag caagcagctc gggacggtgt tcgagcccaa gcgcggcgag 1260
gccaccaaga aggccatgat gacaagctcg ccgctccttc acgcgaaccc cgttgcgctc 1320
acgcgctccg tggccttcca ggaggggtct tga 1353

<210> 48
<211> 450
<212> PRT
<213> Zea mays

<400> 48
Met Val Ala Glu Cys His Gly Gly Gly Gly Ala Ala Ala Gly Gly Gly
1               5                   10                  15
Asp Phe Leu Ile Lys Leu Phe Gly Met Thr Ile Pro Val Pro Glu Cys
            20                  25                  30
Gly Asp Ala Lys Asp Leu Gln Gln Ser Lys Ser Asn Arg Trp Thr Glu
        35                  40                  45
Gln Asp Gln Asp Ser His Gly Leu Glu Thr Ala Pro Ala Pro Ala His
    50                  55                  60
Thr Asp Thr Ser Asp Pro Ser Pro Gln Pro Glu Val Val Asp Ala Glu
65                  70                  75                  80
Asp Pro Thr Glu Thr Gln Gln Arg Pro Cys Asn Gly Asp Gly Asp Gly
                85                  90                  95
Asp Ala Ala Gly Gln Arg Glu Lys Leu Lys Lys Pro Asp Lys Val Leu
            100                 105                 110
Pro Cys Pro Arg Cys Asn Ser Met Asp Thr Lys Phe Cys Tyr Phe Asn
        115                 120                 125
Asn Tyr Asn Val Asn Gln Pro Arg His Phe Cys Lys Asn Cys Gln Arg
    130                 135                 140
Tyr Trp Thr Ala Gly Gly Ala Met Arg Asn Val Pro Val Gly Ala Gly
145                 150                 155                 160
Arg Arg Lys Asn Lys Asn Ala Ala Ala Ser His Phe Phe Gln Arg Val
                165                 170                 175
Arg Ala Thr Asn Ala Thr Val Leu Ser Phe Gly Gly His Gly Gly Ala
            180                 185                 190
Pro Pro Ala Ala Arg Leu Asp Leu Asp Leu Ala Glu Gln Leu Ser His
        195                 200                 205
Gln Leu Ala Pro Val Arg Ser Ala Gly Asp Ala Gly Arg Pro Cys Ser
    210                 215                 220
Glu Gly Ser Ser Ser Arg Asp Gly Asn Gly Ser Arg Ser Ser Val Asp
225                 230                 235                 240
Glu Ala Ala Ala Asn Ala Asp Asp Gly Ser Val Gln Gln His Pro Gly
                245                 250                 255
Pro Ala Ser Met Asn Ser Ser Gly Ala Thr Val Trp Pro Pro Tyr Ser
            260                 265                 270
Cys Ala Pro Ala Pro Ala Ala Tyr Phe Pro Gln Gly Ile Ala Ile Pro
        275                 280                 285
Ile Tyr Pro Ala Ala Pro Ala Tyr Trp Gly Cys Met Val Pro Gly Ala
    290                 295                 300
Trp Ser Leu Pro Trp Pro Val Gln His Pro Ser Ser Ser Ser Ser Pro
305                 310                 315                 320
Thr Thr Thr Ser Ala Pro Ser Val Val Ser Ser Ser Gly Ala Ala Asp
                325                 330                 335

Asp Ser Ser Ser His Ala Leu Gly Lys Arg Pro Arg Asp Arg Glu Gly
            340                 345                 350
Asp Asp Gly Arg Asn Gly Gly Asn Ala Lys Val Trp Ala Pro Lys Ser
        355                 360                 365
Ile Arg Ile Asp Asp Val Asp Glu Val Ala Arg Ser Ser Ile Trp Ser
    370                 375                 380
Leu Val Gly Ile Lys Gly Asp Gln Thr Lys Gln Gln Asp Ala Ala Asp
385                 390                 395                 400
Asp His Ala Gly Gly His Ser Lys Gln Leu Gly Thr Val Phe Glu Pro
                405                 410                 415
Lys Arg Gly Glu Ala Thr Lys Lys Ala Met Met Thr Ser Ser Pro Leu
            420                 425                 430
Leu His Ala Asn Pro Val Ala Leu Thr Arg Ser Val Ala Phe Gln Glu
        435                 440                 445
Gly Ser
    450

<210> 49
<211> 1781
<212> DNA
<213> Zea mays

<400> 49
cccacgcttc cgtccatcca gactccagag agataaaatc taattcctgg acctggagtg 60
cagaccgatc gccgccgccg ccgcgttgtt tcttgtgtgc gcagctgtgg tgatggtgcg 120
gtaaaagaag gctgctccat cgctccaaca aaaaaaaaag gtcgagatcg cacgcggcag 180
gcgacaagca gtttccaaag agaagggaag cctagaggaa gggagctaga gatgggggag 240
ggcagagcag gagacggcct catcaagctg ttcgggaaga ccatccccgt gccggagacg 300
gccgccgtcg gcgaggctgc caaggacatc caacaaagcg gcggcagcgg cagcggcacg 360
actgatccga aagggcaaga gaacaacgtt caggactcca caggctcgcc tccgcagcag 420
gaggtcgcgg acaccgaaga ctcgtcggca gacaaacagc agggcgaggc gggcaaccca 480
aaggagaagc tcaagaagcc cgacaagatc ctgccgtgcc cgcggtgcaa cagcatggac 540
accaagttct gctactacaa caactacaac atcaaccagc cgcgccactt ctgcaagaac 600
tgccagaggt actggactgc cggcggtgcc atgcgcaacg tgcccgtggg cgcaggccgg 660
cgcaagagca agagcgcgtc ggccacttcc cacttcctcc agagggtcag ggccggtctg 720
cccgtcgacc cgctcgtctg cgcggcagcc aagactaacg gcacggtgct cagcttcggc 780
tctgccatgt ccagcttaga cctcacggag cagatgaaac agctcaagga gaagctcgtc 840
ccgatagcgg gggacgagcg ctcagttggc tctcgcaatc aaggaccttc tgccaaggca 900
gaagacccgg accggaagga aaatgttaca gcagataaat ccgcgagagt tgttcagcat 960
ccatgcatga cgaacggggt ggccatgtgg ccatttagct gcgcgccacc agtaccggcc 1020
tctgcctgtt acggcccagg cagcatcgca atcccgttct acccggcagc tgctgctgct 1080
gctgcctact ggggttgcat ggttccagga gcttggagtg gcgcatggcc gcctcactcc 1140
ggccagtccg agacgggctc atccattacc tccgcctctc cagcagcatc caccaagtcc 1200
aacatctgct tcacgccagg aaagcaccct agagaccgcg acgaggaagg aggcgccaaa 1260
ggaaatggca aggtgtgggt gcccaagacg atccggatcg acgacgtgga cgaggtggcc 1320
aggagctcta tcctgtcgct aatcgggatc ggcggcgaca aggcaggcaa agatggcggc 1380
agaggctgca agctcgcaag ggtttttgag cagaacgaag aggcggcaag gacggcaact 1440
cctcactcgg cagccatcag cggcttgccg ttcttgcagg ggaacccagc tgcgctctcg 1500
cggtcactga ccttccagga ggggtcttga gcctctcgct cgattcacag ctgagaattt 1560
tgtacattag agggttgtat gtaatctaat ctaggtgggg ggtggggctc atagctgcca 1620
gcggacattg aaactataca atagatagat atttgtagat agccaatata tatgggtcgg 1680
gggaggaaaa aaaggataat gagagcaagt ccccttattt gtatatatta taatggattg 1740
gaatatttgg ttaaaaaaaa aaaaaaaaaa aaaaaaaaaa a                     1781

<210> 50
<211> 1299
<212> DNA
<213> Zea mays

<400> 50

atgggggagg gcagagcagg agacggcctc atcaagctgt tcgggaagac catccccgtg 60
ccggagacgg ccgccgtcgg cgaggctgcc aaggacatcc aacaaagcgg cggcagcggc 120
agcggcacga ctgatccgaa agggcaagag aacaacgttc aggactccac aggctcgcct 180
ccgcagcagg aggtcgcgga caccgaagac tcgtcggcag acaaacagca gggcgaggcg 240
ggcaacccaa aggagaagct caagaagccc gacaagatcc tgccgtgccc gcggtgcaac 300
agcatggaca ccaagttctg ctactacaac aactacaaca tcaaccagcc gcgccacttc 360
tgcaagaact gccagaggta ctggactgcc ggcggtgcca tgcgcaacgt gcccgtgggc 420
gcaggccggc gcaagagcaa gagcgcgtcg gccacttccc acttcctcca gagggtcagg 480
gccggtctgc ccgtcgaccc gctcgtctgc gcggcagcca agactaacgg cacggtgctc 540
agcttcggct ctgccatgtc cagcttagac ctcacggagc agatgaaaca gctcaaggag 600
aagctcgtcc cgatagcggg ggacgagcgc tcagttggct ctcgcaatca aggaccttct 660
gccaaggcag aagacccgga ccggaaggaa aatgttacag cagataaatc cgcgagagtt 720
gttcagcatc catgcatgac gaacggggtg gccatgtggc catttagctg cgcgccacca 780
gtaccggcct ctgcctgtta cggcccaggc agcatcgcaa tcccgttcta cccggcagct 840
gctgctgctg ctgcctactg ggggttgcatg gttccaggag cttggagtgg cgcatggccg 900
cctcactccg gccagtccga gacggctcca tccattacct ccgcctctcc agcagcatcc 960
accaagtcca acatctgctt cacgccagga aagcacccta gagaccgcga cgaggaagga 1020
ggcgccaaag gaaatggcaa ggtgtgggtg cccaagacga tccggatcga cgacgtggac 1080
gaggtggcca ggagctctat cctgtcgcta atcgggatcg gcggcgacaa ggcaggcaaa 1140
gatggcggca gaggctgcaa gctcgcaagg gtttttgagc agaacgaaga ggcggcaagg 1200
acggcaactc ctcactcggc agccatcagc ggcttgccgt tcttgcaggg gaacccagct 1260
gcgctctcgc ggtcactgac cttccaggag gggtcttga 1299

<210> 51
<211> 432
<212> PRT
<213> Zea mays

<400> 51
Met Gly Glu Gly Arg Ala Gly Asp Gly Leu Ile Lys Leu Phe Gly Lys
1               5                   10                  15
Thr Ile Pro Val Pro Glu Thr Ala Ala Val Gly Glu Ala Ala Lys Asp
            20                  25                  30
Ile Gln Gln Ser Gly Gly Ser Gly Ser Gly Thr Thr Asp Pro Lys Gly
        35                  40                  45
Gln Glu Asn Asn Val Gln Asp Ser Thr Gly Ser Pro Pro Gln Gln Glu
    50                  55                  60
Val Ala Asp Thr Glu Asp Ser Ser Ala Asp Lys Gln Gln Gly Glu Ala
65                  70                  75                  80
Gly Asn Pro Lys Glu Lys Leu Lys Lys Pro Asp Lys Ile Leu Pro Cys
                85                  90                  95
Pro Arg Cys Asn Ser Met Asp Thr Lys Phe Cys Tyr Tyr Asn Asn Tyr
            100                 105                 110
Asn Ile Asn Gln Pro Arg His Phe Cys Lys Asn Cys Gln Arg Tyr Trp
        115                 120                 125
Thr Ala Gly Gly Ala Met Arg Asn Val Pro Val Gly Ala Gly Arg Arg
    130                 135                 140
Lys Ser Lys Ser Ala Ser Ala Thr Ser His Phe Leu Gln Arg Val Arg
145                 150                 155                 160
Ala Gly Leu Pro Val Asp Pro Leu Val Cys Ala Ala Ala Lys Thr Asn
                165                 170                 175
Gly Thr Val Leu Ser Phe Gly Ser Ala Met Ser Ser Leu Asp Leu Thr
            180                 185                 190
Glu Gln Met Lys Gln Leu Lys Glu Lys Leu Val Pro Ile Ala Gly Asp
        195                 200                 205
Glu Arg Ser Val Gly Ser Arg Asn Gln Gly Pro Ser Ala Lys Ala Glu
    210                 215                 220
Asp Pro Asp Arg Lys Glu Asn Val Thr Ala Asp Lys Ser Ala Arg Val
225                 230                 235                 240
Val Gln His Pro Cys Met Thr Asn Gly Val Ala Met Trp Pro Phe Ser
                245                 250                 255

Cys Ala Pro Pro Val Pro Ala Ser Ala Cys Tyr Gly Pro Gly Ser Ile
            260                 265                 270
Ala Ile Pro Phe Tyr Pro Ala Ala Ala Ala Ala Ala Ala Tyr Trp Gly
        275                 280                 285
Cys Met Val Pro Gly Ala Trp Ser Gly Ala Trp Pro Pro His Ser Gly
    290                 295                 300
Gln Ser Glu Thr Gly Ser Ser Ile Thr Ser Ala Ser Pro Ala Ala Ser
305                 310                 315                 320
Thr Lys Ser Asn Ile Cys Phe Thr Pro Gly Lys His Pro Arg Asp Arg
                325                 330                 335
Asp Glu Glu Gly Gly Ala Lys Gly Asn Gly Lys Val Trp Val Pro Lys
            340                 345                 350
Thr Ile Arg Ile Asp Asp Val Asp Glu Val Ala Arg Ser Ser Ile Leu
        355                 360                 365
Ser Leu Ile Gly Ile Gly Gly Asp Lys Ala Gly Lys Asp Gly Gly Arg
    370                 375                 380
Gly Cys Lys Leu Ala Arg Val Phe Glu Gln Asn Glu Glu Ala Ala Arg
385                 390                 395                 400
Thr Ala Thr Pro His Ser Ala Ala Ile Ser Gly Leu Pro Phe Leu Gln
                405                 410                 415
Gly Asn Pro Ala Ala Leu Ser Arg Ser Leu Thr Phe Gln Glu Gly Ser
            420                 425                 430

<210> 52
<211> 1511
<212> DNA
<213> Zea mays

<400> 52
gcacgccccg gcccctttgt tttgtagctc ttgtcagcga accttccccc tgaaaacccc 60
acccaccctt cctctccgat cctcctagta gcgcctcact tgcttcattc cgatccattc 120
caccacccat ctccctcctc agctcccctc tccgctctcc ctccctcgct cgcgctctcc 180
acgcggatca ccggaggcta gctagcaagc tcgaccggcg cggccgatct agctcgcgct 240
tgttggccgc ttcagcttag cagcggcggc acgtcgtcaa cctgtgctgt gagaaagact 300
agtagagaga gatacgtgta aggatggctg gcgcgggggg tgcgacggct gtgaagcagc 360
cggcggcggg ggcgcccgac gctggggcca gcaggagcgg ggtcggggct ggcgccgccg 420
gggcgccggt cgccgacccg cgcgccgagg cgctgcggtg cccgcgctgc gactcggcca 480
acaccaagtt ctgctactac aacaactact cgctgtcgca gccgcggcac ttctgcaagg 540
cgtgcaagcg ctactggacg cgcgggggca cgctccgcaa cgtccccgtc ggcgggggct 600
gccgcaagaa caagcgctcc aggagcagcg gcgggcccgg cgccggcggg aggaacgggt 660
cctccgctgc tgctgctgct gctgccgccg ccgccgccgt cacgtcctcc tcggcgccgt 720
cgacgctgtc cctcccgctg catacggggt ccctgccgtc gttgtcctcg gcgctggggc 780
tgcccggggg cgcctcgctc gcgtcgctcc tcctcgggac cgctggctct ggcggtgacc 840
acctcggcct cttccaggcc gccatgcagt cggtggtctc ctcggaggcg accgcctacg 900
agatgcagca gcagcagcag cagcagacgc aggtggacca cctgctaggc ctcggctacg 960
gaggcgccgg cgccggcgcc ggcgcgcaga tccacctcaa gccgtggatg cacgaggcac 1020
ccggtgctgg cgcgggcggg atcatggaca gcttctacgc gccgctgctg tccagctccc 1080
tcgtgccggg cctggaggag ctgcacgtca aggcggaggt cgccggtgcc ggggatcacc 1140
agcagaagcc cgcgcccggg gaccagcaga gcgccagctg ggagctgccg acgccgtcgt 1200
cgtccaacgt cgacgccaac gtcatcgcat ctgacgcgct catggccgcc gccgccgcgt 1260
ccatgaaccc cggggttagc tccgccacag cacccacggc gccaaccgtc ccctcctcct 1320
tcatgtactg gggcaacggc ggtattggcg gcgctgccgc ggcgtggcca gacctcacca 1380
actgcggatc ttccattgcc acgttcttct agccgtctgg ctgtcccgca ctcccgctgg 1440
cctcgttaac ttcgtttttt ttaccttaat tttctggtta atttttcttt gactaaaaaa 1500
aaaaaaaaaa a                                                      1511

<210> 53
<211> 1089
<212> DNA
<213> Zea mays

<400> 53
atggctggcg cggggggtgc gacggctgtg aagcagccgg cggcgggggc gcccgacgct 60
ggggccagca ggagcggggt cggggctggc gccgccgggg cgccggtcgc cgacccgcgc 120
gccgaggcgc tgcggtgccc gcgctgcgac tcggccaaca ccaagttctg ctactacaac 180
aactactcgc tgtcgcagcc gcggcacttc tgcaaggcgt gcaagcgcta ctggacgcgc 240
gggggcacgc tccgcaacgt ccccgtcggc gggggctgcc gcaagaacaa gcgctccagg 300
agcagcggcg ggcccggcgc cggcgggagg aacgggtcct ccgctgctgc tgctgctgct 360
gccgccgccg ccgccgtcac gtcctcctcg gcgccgtcga cgctgtccct cccgctgcat 420
acggggtccc tgccgtcgtt gtcctcggcg ctggggctgc ccgggggcgc ctcgctcgcg 480
tcgctcctcc tcgggaccgc tggctctggc ggtgaccacc tcggcctctt ccaggccgcc 540
atgcagtcgg tggtctcctc ggaggcgacc gcctacgaga tgcagcagca gcagcagcag 600
cagacgcagg tggaccacct gctaggcctc ggctacggag gcgccggcgc cggcgccggc 660
gcgcagatcc acctcaagcc gtggatgcac gaggcacccg gtgctggcgc gggcgggatc 720
atggacagct tctacgcgcc gctgctgtcc agctccctcg tgccgggcct ggaggagctg 780
cacgtcaagg cggaggtcgc cggtgccggg gatcaccagc agaagcccgc gcccggggac 840
cagcagagcg ccagctggga gctgccgacg ccgtcgtcgt ccaacgtcga cgccaacgtc 900
atcgcatctg acgcgctcat ggccgccgcc gccgcgtcca tgaaccccgg ggttagctcc 960
gccacagcac ccacggcgcc aaccgtcccc tcctccttca tgtactgggg caacggcggt 1020
attggcggcg ctgccgcggc gtggccagac ctcaccaact gcggatcttc cattgccacg 1080
ttcttctag 1089

<210> 54
<211> 362
<212> PRT
<213> Zea mays

<400> 54
Met Ala Gly Ala Gly Gly Ala Thr Ala Val Lys Gln Pro Ala Ala Gly
1               5                   10                  15
Ala Pro Asp Ala Gly Ala Ser Arg Ser Gly Val Gly Ala Gly Ala Ala
            20                  25                  30
Gly Ala Pro Val Ala Asp Pro Arg Ala Glu Ala Leu Arg Cys Pro Arg
        35                  40                  45
Cys Asp Ser Ala Asn Thr Lys Phe Cys Tyr Tyr Asn Asn Tyr Ser Leu
    50                  55                  60
Ser Gln Pro Arg His Phe Cys Lys Ala Cys Lys Arg Tyr Trp Thr Arg
65                  70                  75                  80
Gly Gly Thr Leu Arg Asn Val Pro Val Gly Gly Gly Cys Arg Lys Asn
                85                  90                  95
Lys Arg Ser Arg Ser Ser Gly Gly Pro Gly Ala Gly Gly Arg Asn Gly
            100                 105                 110
Ser Ser Ala Ala Ala Ala Ala Ala Ala Ala Ala Ala Ala Val Thr Ser
        115                 120                 125
Ser Ser Ala Pro Ser Thr Leu Ser Leu Pro Leu His Thr Gly Ser Leu
    130                 135                 140
Pro Ser Leu Ser Ser Ala Leu Gly Leu Pro Gly Gly Ala Ser Leu Ala
145                 150                 155                 160
Ser Leu Leu Leu Gly Thr Ala Gly Ser Gly Gly Asp His Leu Gly Leu
                165                 170                 175
Phe Gln Ala Ala Met Gln Ser Val Val Ser Ser Glu Ala Thr Ala Tyr
            180                 185                 190
Glu Met Gln Gln Gln Gln Gln Gln Gln Thr Gln Val Asp His Leu Leu
        195                 200                 205
Gly Leu Gly Tyr Gly Gly Ala Gly Ala Gly Ala Gly Ala Gln Ile His
    210                 215                 220
Leu Lys Pro Trp Met His Glu Ala Pro Gly Ala Gly Ala Gly Gly Ile
225                 230                 235                 240
Met Asp Ser Phe Tyr Ala Pro Leu Leu Ser Ser Ser Leu Val Pro Gly
                245                 250                 255

Leu Glu Glu Leu His Val Lys Ala Glu Val Ala Gly Ala Gly Asp His
            260                 265                 270
Gln Gln Lys Pro Ala Pro Gly Asp Gln Gln Ser Ala Ser Trp Glu Leu
        275                 280                 285
Pro Thr Pro Ser Ser Ser Asn Val Asp Ala Asn Val Ile Ala Ser Asp
    290                 295                 300
Ala Leu Met Ala Ala Ala Ala Ala Ser Met Asn Pro Gly Val Ser Ser
305                 310                 315                 320
Ala Thr Ala Pro Thr Ala Pro Thr Val Pro Ser Ser Phe Met Tyr Trp
                325                 330                 335
Gly Asn Gly Gly Ile Gly Gly Ala Ala Ala Ala Trp Pro Asp Leu Thr
            340                 345                 350
Asn Cys Gly Ser Ser Ile Ala Thr Phe Phe
        355                 360

<210> 55
<211> 1444
<212> DNA
<213> Zea mays

<400> 55
gcaccagcgc cgatgcccat aaacttcgag agcgaccacc agctgaaaca ccccgtcatc 60
tcctactgtc tatgccatac ggcggccgcc gcgacgctcc ctaggctccg agtcactccg 120
agctgggaag gaagagcccg cgttgctcac tcgctcgctc gcatgcagct ttcccacacg 180
gcgccacgca tgcaggagcc cgggcgccgg cccgtaccgc cgttcgcggg cgtcgacctc 240
cgccgcccca ggggctaccc cgctccggcg gcgaaggagg cggaggagga gccggcccca 300
gctccggcgc cgggcggcga cccgtgcccg cggtgcgggt cgcgggacac caagttctgc 360
tactacaaca actacaacac gtcgcagccg cgccacttct gcaagtcgtg ccgccgctac 420
tggaccaagg gcgggtccct ccgtaacgtc cccgtcgggg gcggcacccg caagagcggc 480
tcctcgtcct cggcctcggc aacggcaacg ccaacctcga cgggcgccgc acccaagagc 540
ccgaggtcgt ccaagaactc caagcgtcgc cgcgtcggcc ccgccccggc cccggccccg 600
gccccggacc ccgcccctgc caccgccacc gccaccgctg acgtcgcgaa cacggcaccg 660
tcgacggaag cctcagcagc aacgcgtgcc gcctcggaga agccagccgc gacggaaccc 720
gcagcggcaa cgggtgccgc ctcggagaag ccggccgcga cggagctcgc agcagcggtt 780
gtctccacgg agaaaccgcc ggcggccgtc ggcggtttca cggacacgtc tacggcgccc 840
gagaccgggc tcgcagatgt ctgcagcggc ggcgtgaagg agcttgtgcc ggacccgggc 900
cacttcgagt ggccgtcggg ctgcgatctg gggtcctact ggggcaccag cgtgttcgcc 960
gacaccgacc cggcgctgtt cctgaacctg ccgtgagccg tgacaacgcc acggcgtcca 1020
tgctcaacta atcaaccatc acacgaccca cgagatagat gagatgtgaa ccgagcacgc 1080
tgagcgtcaa gctcttgaag gcttgaagct gtgctgtagt ttcctgagct tactagcgat 1140
ctctgcttgt ctctgatctg ataatctctt actagtcgcc gtttgtgctc cgatctagta 1200
acggctaaca tggtcggctg ttacaagtgc atgaacctgt gcttgatctt gagctaaatc 1260
atggtgtttt tgaggctatg atcgaggatc acaagaacag ctgcaaggag aacggcgact 1320
tgtaagtcgt gtcgttgtaa ctcttggttt ggttggaggc atatattctg aagccgtcta 1380
aagcatgccg cgcaaaaaaa aaaaaaaaaa aaaaaaaaaa aaaaaaaaaa aaaaaaaaaa 1440
aaaa                                                              1444

<210> 56
<211> 984
<212> DNA
<213> Zea mays

<400> 56
atgcccataa acttcgagag cgaccaccag ctgaaacacc ccgtcatctc ctactgtcta 60
tgccatacgg cggccgccgc gacgctccct aggctccgag tcactccgag ctgggaagga 120
agagcccgcg ttgctcactc gctcgctcgc atgcagcttt cccacacggc gccacgcatg 180
caggagcccg ggcgccggcc cgtaccgccg ttcgcgggcg tcgacctccg ccgccccagg 240
ggctaccccg ctccggcggc gaaggaggcg gaggaggagc cggccccagc tccggcgccg 300
ggcggcgacc cgtgcccgcg gtgcgggtcg cgggacacca agttctgcta ctacaacaac 360
tacaacacgt cgcagccgcg ccacttctgc aagtcgtgcc gccgctactg gaccaagggc 420

gggtccctcc gtaacgtccc cgtcgggggc ggcacccgca agagcggctc ctcgtcctcg 480
gcctcggcaa cggcaacgcc aacctcgacg ggcgccgcac ccaagagccc gaggtcgtcc 540
aagaactcca agcgtcgccg cgtcggcccc gccccggccc cggccccggc cccggacccc 600
gcccctgcca ccgccaccgc caccgctgac gtcgcgaaca cggcaccgtc gacggaagcc 660
tcagcagcaa cgcgtgccgc ctcggagaag ccagccgcga cggaacccgc agcggcaacg 720
ggtgccgcct cggagaagcc ggccgcgacg gagctcgcag cagcggttgt ctccacggag 780
aaaccgccgg cggccgtcgg cggtttcacg gacacgtcta cggcgcccga gaccgggctc 840
gcagatgtct gcagcggcgg cgtgaaggag cttgtgccgg acccgggcca cttcgagtgg 900
ccgtcgggct gcgatctggg gtcctactgg ggcaccagcg tgttcgccga caccgacccg 960
gcgctgttcc tgaacctgcc gtga 984

<210> 57
<211> 327
<212> PRT
<213> Zea mays

<400> 57
Met Pro Ile Asn Phe Glu Ser Asp His Gln Leu Lys His Pro Val Ile
1               5                   10                  15
Ser Tyr Cys Leu Cys His Thr Ala Ala Ala Ala Thr Leu Pro Arg Leu
            20                  25                  30
Arg Val Thr Pro Ser Trp Glu Gly Arg Ala Arg Val Ala His Ser Leu
        35                  40                  45
Ala Arg Met Gln Leu Ser His Thr Ala Pro Arg Met Gln Glu Pro Gly
    50                  55                  60
Arg Arg Pro Val Pro Pro Phe Ala Gly Val Asp Leu Arg Arg Pro Arg
65                  70                  75                  80
Gly Tyr Pro Ala Pro Ala Ala Lys Glu Ala Glu Glu Glu Pro Ala Pro
                85                  90                  95
Ala Pro Ala Pro Gly Gly Asp Pro Cys Pro Arg Cys Gly Ser Arg Asp
            100                 105                 110
Thr Lys Phe Cys Tyr Tyr Asn Asn Tyr Asn Thr Ser Gln Pro Arg His
        115                 120                 125
Phe Cys Lys Ser Cys Arg Arg Tyr Trp Thr Lys Gly Gly Ser Leu Arg
    130                 135                 140
Asn Val Pro Val Gly Gly Gly Thr Arg Lys Ser Gly Ser Ser Ser Ser
145                 150                 155                 160
Ala Ser Ala Thr Ala Thr Pro Thr Ser Thr Gly Ala Ala Pro Lys Ser
                165                 170                 175
Pro Arg Ser Ser Lys Asn Ser Lys Arg Arg Arg Val Gly Pro Ala Pro
            180                 185                 190
Ala Pro Ala Pro Ala Pro Asp Pro Ala Pro Ala Thr Ala Thr Ala Thr
        195                 200                 205
Ala Asp Val Ala Asn Thr Ala Pro Ser Thr Glu Ala Ser Ala Ala Thr
    210                 215                 220
Arg Ala Ala Ser Glu Lys Pro Ala Ala Thr Glu Pro Ala Ala Ala Thr
225                 230                 235                 240
Gly Ala Ala Ser Glu Lys Pro Ala Ala Thr Glu Leu Ala Ala Ala Val
                245                 250                 255
Val Ser Thr Glu Lys Pro Pro Ala Ala Val Gly Gly Phe Thr Asp Thr
            260                 265                 270
Ser Thr Ala Pro Glu Thr Gly Leu Ala Asp Val Cys Ser Gly Gly Val
        275                 280                 285
Lys Glu Leu Val Pro Asp Pro Gly His Phe Glu Trp Pro Ser Gly Cys
    290                 295                 300
Asp Leu Gly Ser Tyr Trp Gly Thr Ser Val Phe Ala Asp Thr Asp Pro
305                 310                 315                 320
Ala Leu Phe Leu Asn Leu Pro
                325

<210> 58
<211> 1294
<212> DNA
<213> Zea mays

<400> 58
cgaagctctc ctcgccgcac tcgcgcctgc tcgaccgctc caacaagacc ctctcgcgcc 60
accgcagcag cgtcgtcgtc gctacgcgga tgctgtcttc tcaccgcgag agcctgctgc 120
cctacgcgcc ggggcggcgg gcggcggtgc tgctcgacca ccggcggtac cgcccgaacg 180
tcgaggtggc gcccagctgc ccgcgctgcg actcgcccaa caccaagttc tgctactaca 240
acaactacag cctcagccag ccccgctact tctgcaaggg ctgccgccgc tactggacca 300
agggcggctc cctccgcaac gtgcccgtcg gcggcgggtg ccggaagaac cgcagcaggg 360
gcaagccggt ccgagccgtg gccgtcgatt ccgtggcgcc cagcagcggt ggcgcggcgg 420
cgttcacgca ccgggcgtcg gcgtcgtcgt cgtcggcgtc ccccgccacg ctgcggccgg 480
acatgctact ggaaggcatg ctcggcagcc caagcgagct gtgccagctg acggtgacgg 540
acgacgacgc agcggaaaac ccggctgtgg cgcccgatgg acccaggatc gacctggcgt 600
tgctgtacgc caagttcttg aaccaacagc cgccggcgcc cgagcggcgc gccgtcctgc 660
cggaatcgct cgtcgtcgac accttgagcg ggtcgtcgtc gagcgacgtg ggtcccgtcg 720
tcccatcgcg ggatcatcag ccgttcacga cgcaagacga cgggctgggc gagctgtccg 780
cggcggcgag cgcagagccg agcgctcctc cgccacagtc gtcgtcgtgt gcggaggcgc 840
ttattggggc gttcggcatg gaccagcgct gctacgactc tctgggtttg ccaccagacg 900
gcggggatct agtcctaccg tcgacgtggc ctcttcttgg ggccaagtac gagctgttcg 960
atccgctacc tgaggacgcc gtgagccttc aggacggctt cgccggcgac gaggacgtgt 1020
ggagcggcgc cttggcttgt caagggctgg aagcagctct ctgcaggccg taattttttt 1080
taactcgcct cttgattttc accgtgcttg tgattgtttt atgcgctctt ttctttttcc 1140
tggttgtgat cgtgttcagg agtaatttat atagagacca tatatacagc atgtccagat 1200
catgcattat gatggatcaa cgggtataca tgtgagatat agaaagaact taataaaatt 1260
ttgacatttt gtttgacaaa aaaaaaaaaa aaaa 1294

<210> 59
<211> 984
<212> DNA
<213> Zea mays

<400> 59
atgctgtctt ctcaccgcga gagcctgctg ccctacgcgc cggggcggcg ggcggcggtg 60
ctgctcgacc accggcggta ccgcccgaac gtcgaggtgg cgcccagctg cccgcgctgc 120
gactcgccca acaccaagtt ctgctactac aacaactaca gcctcagcca gccccgctac 180
ttctgcaagg gctgccgccg ctactggacc aagggcggct ccctccgcaa cgtgcccgtc 240
ggcggcgggt gccggaagaa ccgcagcagg ggcaagccgg tccgagccgt ggccgtcgat 300
tccgtggcgc ccagcagcgg tggcgcggcg gcgttcacgc accgggcgtc ggcgtcgtcg 360
tcgtcggcgt cccccgccac gctgcggccg gacatgctac tggaaggcat gctcggcagc 420
ccaagcgagc tgtgccagct gacggtgacg gacgacgacg cagcggaaaa cccggctgtg 480
gcgcccgatg gacccaggat cgacctggcg ttgctgtacg ccaagttctt gaaccaacag 540
ccgccggcgc ccgagcggcg cgccgtcctg ccggaatcgc tcgtcgtcga caccttgagc 600
gggtcgtcgt cgagcgacgt gggtcccgtc gtcccatcgc gggatcatca gccgttcacg 660
acgcaagacg acgggctggg cgagctgtcc gcggcggcga gcgcagagcc gagcgctcct 720
ccgccacagt cgtcgtcgtg tgcggaggcg cttattgggg cgttcggcat ggaccagcgc 780
tgctacgact ctctgggttt gccaccagac ggcggggatc tagtcctacc gtcgacgtgg 840
cctcttcttg gggccaagta cgagctgttc gatccgctac ctgaggacgc cgtgagcctt 900
caggacggct tcgccggcga cgaggacgtg tggagcggcg ccttggcttg tcaagggctg 960
gaagcagctc tctgcaggcc gtaa 984

<210> 60
<211> 327
<212> PRT
<213> Zea mays

<400> 60
Met Leu Ser Ser His Arg Glu Ser Leu Leu Pro Tyr Ala Pro Gly Arg
1               5                   10                  15

Arg Ala Ala Val Leu Leu Asp His Arg Arg Tyr Arg Pro Asn Val Glu
            20                  25                  30
Val Ala Pro Ser Cys Pro Arg Cys Asp Ser Pro Asn Thr Lys Phe Cys
        35                  40                  45
Tyr Tyr Asn Asn Tyr Ser Leu Ser Gln Pro Arg Tyr Phe Cys Lys Gly
    50                  55                  60
Cys Arg Arg Tyr Trp Thr Lys Gly Gly Ser Leu Arg Asn Val Pro Val
65                  70                  75                  80
Gly Gly Gly Cys Arg Lys Asn Arg Ser Arg Gly Lys Pro Val Arg Ala
                85                  90                  95
Val Ala Val Asp Ser Val Ala Pro Ser Ser Gly Gly Ala Ala Ala Phe
            100                 105                 110
Thr His Arg Ala Ser Ala Ser Ser Ser Ser Ala Ser Pro Ala Thr Leu
        115                 120                 125
Arg Pro Asp Met Leu Leu Glu Gly Met Leu Gly Ser Pro Ser Glu Leu
    130                 135                 140
Cys Gln Leu Thr Val Thr Asp Asp Asp Ala Ala Glu Asn Pro Ala Val
145                 150                 155                 160
Ala Pro Asp Gly Pro Arg Ile Asp Leu Ala Leu Leu Tyr Ala Lys Phe
                165                 170                 175
Leu Asn Gln Gln Pro Pro Ala Pro Glu Arg Arg Ala Val Leu Pro Glu
            180                 185                 190
Ser Leu Val Val Asp Thr Leu Ser Gly Ser Ser Ser Ser Asp Val Gly
        195                 200                 205
Pro Val Val Pro Ser Arg Asp His Gln Pro Phe Thr Thr Gln Asp Asp
    210                 215                 220
Gly Leu Gly Glu Leu Ser Ala Ala Ala Ser Ala Glu Pro Ser Ala Pro
225                 230                 235                 240
Pro Pro Gln Ser Ser Ser Cys Ala Glu Ala Leu Ile Gly Ala Phe Gly
                245                 250                 255
Met Asp Gln Arg Cys Tyr Asp Ser Leu Gly Leu Pro Pro Asp Gly Gly
            260                 265                 270
Asp Leu Val Leu Pro Ser Thr Trp Pro Leu Leu Gly Ala Lys Tyr Glu
        275                 280                 285
Leu Phe Asp Pro Leu Pro Glu Asp Ala Val Ser Leu Gln Asp Gly Phe
    290                 295                 300
Ala Gly Asp Glu Asp Val Trp Ser Gly Ala Leu Ala Cys Gln Gly Leu
305                 310                 315                 320
Glu Ala Ala Leu Cys Arg Pro
                325

<210> 61
<211> 1079
<212> DNA
<213> Zea mays

<400> 61
ctcggtcgca ttctcttcct ctcctagtcc ttcccatgat cccatcccat atatcccagc 60
caggtcaccc agagagcgag aagctgattg cgccgcgccc tagctagcgc tctcggccgc 120
atcgccggac gtctattaac ccgccgccgc tcgtcctcgg catgcgcggt tctgctcgcc 180
tgcgagatgg cgcctgcagt ttccatcctc tcggccaccg cctccgccaa gcgaaagcgc 240
cccgccactt ccgacgctga tgagctcccg cacgacgact cctccgcgcc ccaccagcag 300
gtgcagggcc agggccagca accgcggcag cagcagcagc ttgagtgccc gcgctgccgc 360
tccaccaaca ccaagttctg ctactacaac aactacagca cggcgcagcc gcgccacttc 420
tgccgcgcgt gccgccgcta ctggacgcac gggggcacgc tgcgcgacgt ccccgtcggc 480
ggcgcctcgc gccgcgccgc cactggcggc ggcggcggca agcggcgcag ggtctccgcc 540
gagccctcat ccccgccgcc gtcggtcgcg gacgcgtgcc tgccgtccgc cttcccgttc 600
ctcagcgacg gcagcttctt cccgcagctc gacctcgtcg gcggcgttgc gcttgcaccc 660
ccggccttct cctcctcgtg gcagtcggtg ctggtcccgg acttgtacga cgggctcgcg 720
ccgtgggacg acggagcaac ggcggccgcg tggggcgaca tcggtggcct cgacctcagc 780

tggacactgc cggggaactg agtgccagcc gccgtcgcgc gtcttgaggg gctccggttt 840
tagcagtgcg gcctcgtcag gttcagttca gttcagttcc tagcatgcag agctctcaca 900
attctcctat gtcgcctcca ctgtttcttc tcgtccttac tgcgaatgaa atggtggggc 960
tgctagttag atcagtcaat cttgatctgt agctggagcc agagtgtaag atctgtgcag 1020
tgatttatcg tgacaataaa attaagttta gtttgtgtca aaaaaaaaaa aaaaaaaaa 1079

<210> 62
<211> 618
<212> DNA
<213> Zea mays

<400> 62
atggcgcctg cagtttccat cctctcggcc accgcctccg ccaagcgaaa gcgccccgcc 60
acttccgacg ctgatgagct cccgcacgac gactcctccg cgccccacca gcaggtgcag 120
ggccagggcc agcaaccgcg gcagcagcag cagcttgagt gcccgcgctg ccgctccacc 180
aacaccaagt tctgctacta caacaactac agcacggcgc agccgcgcca cttctgccgc 240
gcgtgccgcc gctactggac gcacgggggc acgctgcgcg acgtccccgt cggcggcgcc 300
tcgcgccgcg ccgccactgg cggcggcggc ggcaagcggc gcagggtctc cgccgagccc 360
tcatccccgc cgccgtcggt cgcggacgcg tgcctgccgt ccgccttccc gttcctcagc 420
gacggcagct tcttcccgca gctcgacctc gtcggcggcg ttgcgcttgc acccccggcc 480
ttctcctcct cgtggcagtc ggtgctggtc ccggacttgt acgacgggct cgcgccgtgg 540
gacgacggag caacggcggc cgctctagag gatccaagct tacgtacgcg tgcatgcgac 600
gtcatagctc ttctatag 618

<210> 63
<211> 205
<212> PRT
<213> Zea mays

<400> 63
Met Ala Pro Ala Val Ser Ile Leu Ser Ala Thr Ala Ser Ala Lys Arg
1               5                   10                  15
Lys Arg Pro Ala Thr Ser Asp Ala Asp Glu Leu Pro His Asp Asp Ser
            20                  25                  30
Ser Ala Pro His Gln Gln Val Gln Gly Gln Gly Gln Gln Pro Arg Gln
        35                  40                  45
Gln Gln Gln Leu Glu Cys Pro Arg Cys Arg Ser Thr Asn Thr Lys Phe
    50                  55                  60
Cys Tyr Tyr Asn Asn Tyr Ser Thr Ala Gln Pro Arg His Phe Cys Arg
65                  70                  75                  80
Ala Cys Arg Arg Tyr Trp Thr His Gly Gly Thr Leu Arg Asp Val Pro
                85                  90                  95
Val Gly Gly Ala Ser Arg Arg Ala Ala Thr Gly Gly Gly Gly Gly Lys
            100                 105                 110
Arg Arg Arg Val Ser Ala Glu Pro Ser Ser Pro Pro Pro Ser Val Ala
        115                 120                 125
Asp Ala Cys Leu Pro Ser Ala Phe Pro Phe Leu Ser Asp Gly Ser Phe
    130                 135                 140
Phe Pro Gln Leu Asp Leu Val Gly Gly Val Ala Leu Ala Pro Pro Ala
145                 150                 155                 160
Phe Ser Ser Ser Trp Gln Ser Val Leu Val Pro Asp Leu Tyr Asp Gly
                165                 170                 175
Leu Ala Pro Trp Asp Asp Gly Ala Thr Ala Ala Ala Leu Glu Asp Pro
            180                 185                 190
Ser Leu Arg Thr Arg Ala Cys Asp Val Ile Ala Leu Leu
        195                 200                 205

<210> 64
<211> 1210
<212> DNA

<213> Zea mays

<400> 64
gcgagtggag ctcttgcgtc ttgccacttt gccagttgcc acgctccggg ctcctcggca 60
aacacacagc accagctaag ctagctcacg cccgccgagg ccatatcctt cccagcacgc 120
cgtagacgcc gacgcttgac gccatgcagg agttccaatc catcccgggg ctggcggggc 180
ggctgttcgg cggggcagcg gccgccggcc tccggcgcgc gcaggggcag tgcggcggcg 240
ggggtgcctc ggccgcggcg gccgtgaagt gcccgcggtg cgagtccacc aacaccaagt 300
tctgctacta caacaactac aacctgtcgc agccgcgcca cttctgcaag ggctgccggc 360
ggtactggac caagggcggc gtcctgcgca acgtgcccgt gggcgggggc tgccgcaagg 420
ccaagcgctc gtcggcgccg tccacgccca cgcccacgcc cacgtcggcg gtgggcgacg 480
ccaagagccc gcgccgcgcc tcggcgtcct cgccccgcgg ctccatcagc agcggcagcg 540
gcagcgcgag ccccacgccc aacggcttcg ccttctcctc cacggccgac gtggtggcgc 600
cgccgccggc gccgatattc gccgatcagg cggcggcgct ggcgtctctc ttcgcgcctc 660
cgccgccgct cccggcgttc agcttcgtgg cggcgcaggc taaggaggaa acgtcctcca 720
cccccgctgg cttcgcggcg ccgtactcct cagtatccga ggacatggca ccgttcgcgt 780
ccctggacgc cgctgggatg ttcgagatcg gcgaggacgc gtcggccgcg gcggcgtact 840
ggaacgccgg gagctgctgg acggacgtgc cggacccgag catgatgatg tacctgctac 900
cctaggctgt ttgttttagg tacttgtcac gatcgcttga ttaaggttaa gccgtggtta 960
attaagtgct tcttattaaa cgacgacgac cacgagctac gtctccctac tagtatatta 1020
tatgtccacg ctccacccgc cggatgcctt cctctgcagc gtcgatcttg tgtttgttta 1080
ttggtgattt gtggcttcac agaccgcgtg taagaaaagc taacgtctgt gttgcactcg 1140
taagctgatc attgccctag cttgcaattg caacgctgcg atgaaattat tcgcaaaaaa 1200
aaaaaaaaaa 1210

<210> 65
<211> 762
<212> DNA
<213> Zea mays

<400> 65
atgcaggagt tccaatccat cccggggctg gcggggcggc tgttcggcgg ggcagcggcc 60
gccggcctcc ggcgcgcgca ggggcagtgc ggcggcgggg gtgcctcggc cgcggcggcc 120
gtgaagtgcc cgcggtgcga gtccaccaac accaagttct gctactacaa caactacaac 180
ctgtcgcagc cgcgccactt ctgcaagggc tgccggcggt actggaccaa gggcggcgtc 240
ctgcgcaacg tgcccgtggg cgggggctgc cgcaaggcca agcgctcgtc ggcgccgtcc 300
acgcccacgc ccacgcccac gtcggcggtg ggcgacgcca agagcccgcg ccgcgcctcg 360
gcgtcctcgc cccgcggctc catcagcagc ggcagcggca gcgcgagccc cacgcccaac 420
ggcttcgcct tctcctccac ggccgacgtg gtggcgccgc cgccggcgcc gatattcgcc 480
gatcaggcgg cggcgctggc gtctctcttc gcgcctccgc cgccgctccc ggcgttcagc 540
ttcgtggcgg cgcaggctaa ggaggaaacg tcctccaccc ccgctggctt cgcggcgccg 600
tactcctcag tatccgagga catggcaccg ttcgcgtccc tggacgccgc tgggatgttc 660
gagatcggcg aggacgcgtc ggccgcggcg gcgtactgga acgccgggag ctgctggacg 720
gacgtgccgg acccgagcat gatgatgtac ctgctaccct ag 762

<210> 66
<211> 253
<212> PRT
<213> Zea mays

<400> 66
Met Gln Glu Phe Gln Ser Ile Pro Gly Leu Ala Gly Arg Leu Phe Gly
1               5                   10                  15
Gly Ala Ala Ala Ala Gly Leu Arg Arg Ala Gln Gly Gln Cys Gly Gly
            20                  25                  30
Gly Gly Ala Ser Ala Ala Ala Ala Val Lys Cys Pro Arg Cys Glu Ser
        35                  40                  45
Thr Asn Thr Lys Phe Cys Tyr Tyr Asn Asn Tyr Asn Leu Ser Gln Pro
    50                  55                  60
Arg His Phe Cys Lys Gly Cys Arg Arg Tyr Trp Thr Lys Gly Gly Val
65                  70                  75                  80

Leu Arg Asn Val Pro Val Gly Gly Gly Cys Arg Lys Ala Lys Arg Ser
                85                  90                  95
Ser Ala Pro Ser Thr Pro Thr Pro Thr Pro Thr Ser Ala Val Gly Asp
            100                 105                 110
Ala Lys Ser Pro Arg Arg Ala Ser Ala Ser Ser Pro Arg Gly Ser Ile
        115                 120                 125
Ser Ser Gly Ser Gly Ser Ala Ser Pro Thr Pro Asn Gly Phe Ala Phe
    130                 135                 140
Ser Ser Thr Ala Asp Val Val Ala Pro Pro Pro Ala Pro Ile Phe Ala
145                 150                 155                 160
Asp Gln Ala Ala Ala Leu Ala Ser Leu Phe Ala Pro Pro Pro Pro Leu
                165                 170                 175
Pro Ala Phe Ser Phe Val Ala Ala Gln Ala Lys Glu Glu Thr Ser Ser
            180                 185                 190
Thr Pro Ala Gly Phe Ala Ala Pro Tyr Ser Ser Val Ser Glu Asp Met
        195                 200                 205
Ala Pro Phe Ala Ser Leu Asp Ala Ala Gly Met Phe Glu Ile Gly Glu
    210                 215                 220
Asp Ala Ser Ala Ala Ala Ala Tyr Trp Asn Ala Gly Ser Cys Trp Thr
225                 230                 235                 240
Asp Val Pro Asp Pro Ser Met Met Met Tyr Leu Leu Pro
                245                 250

<210> 67
<211> 606
<212> DNA
<213> Zea mays

<400> 67
gcctcttccc tgctgcatct gcacgccgcc cacacatgct atttttgttc actgttattg 60
cttcttgctg tggttgttaa accgacttgg tcctgataat cctccgcgtt ctactgatgt 120
gctgtcgctg caccatgcgc gtgttgtcgt cagagcctag ggctggtgaa gcccatggag 180
gagatgctga tggcggccag cgcgggcgct gcaaatccga gccaaggctc gaatccgaac 240
ccgccgccgg cggcgcccgt aacgggagcg ggtagcaccg agcggcgcgc gcggccgcag 300
aaggagaaga cgctcacctg cccgcggtgc aactccacca acaccaaatt ctgctactac 360
aacaactaca gcctccagca gccacgctac ttctgcaaga cgtgccgccg ctactggacg 420
gagggcggat ccctccgcag cgtccccgtg ggcggcggct cccgcaagaa caagcgctcc 480
tcctcctcct cctcgtcgtc ggcggcggcg tccgcctcca ccttctcctc ggccacgagc 540
tcgtccatgg ccagcacacc gggggcggcg tccaagagtc cggagctggc gcacgacctc 600
aaccta                                                            606

<210> 68
<211> 456
<212> DNA
<213> Zea mays

<400> 68
cagagcctag ggctggtgaa gcccatggag gagatgctga tggcggccag cgcgggcgct 60
gcaaatccga gccaaggctc gaatccgaac ccgccgccgg cggcgcccgt aacgggagcg 120
ggtagcaccg agcggcgcgc gcggccgcag aaggagaaga cgctcacctg cccgcggtgc 180
aactccacca acaccaaatt ctgctactac aacaactaca gcctccagca gccacgctac 240
ttctgcaaga cgtgccgccg ctactggacg gagggcggat ccctccgcag cgtccccgtg 300
ggcggcggct cccgcaagaa caagcgctcc tcctcctcct cctcgtcgtc ggcggcggcg 360
tccgcctcca ccttctcctc ggccacgagc tcgtccatgg ccagcacacc gggggcggcg 420
tccaagagtc cggagctggc gcacgacctc aaccta                            456

<210> 69
<211> 152
<212> PRT
<213> Zea mays

<400> 69
Gln Ser Leu Gly Leu Val Lys Pro Met Glu Glu Met Leu Met Ala Ala
1               5                   10                  15
Ser Ala Gly Ala Ala Asn Pro Ser Gln Gly Ser Asn Pro Asn Pro Pro
            20                  25                  30
Pro Ala Ala Pro Val Thr Gly Ala Gly Ser Thr Glu Arg Arg Ala Arg
        35                  40                  45
Pro Gln Lys Glu Lys Thr Leu Thr Cys Pro Arg Cys Asn Ser Thr Asn
    50                  55                  60
Thr Lys Phe Cys Tyr Tyr Asn Asn Tyr Ser Leu Gln Gln Pro Arg Tyr
65                  70                  75                  80
Phe Cys Lys Thr Cys Arg Arg Tyr Trp Thr Glu Gly Gly Ser Leu Arg
                85                  90                  95
Ser Val Pro Val Gly Gly Gly Ser Arg Lys Asn Lys Arg Ser Ser Ser
            100                 105                 110
Ser Ser Ser Ser Ser Ala Ala Ala Ser Ala Ser Thr Phe Ser Ser Ala
        115                 120                 125
Thr Ser Ser Ser Met Ala Ser Thr Pro Gly Ala Ala Ser Lys Ser Pro
    130                 135                 140
Glu Leu Ala His Asp Leu Asn Leu
145                 150

<210> 70
<211> 1158
<212> DNA
<213> Zea mays

<400> 70
cccccgctac tggacacacg gcgggaccct ccgcatttcc ccagacggcg gcggctgccg 60
caggaacaag cgcgcctcca gctcctcgtc cccgattccg ggcccctcca gcaccgccgc 120
caccagcgcc gcgatggaga agaccgtcag cacgcggctg atgctgatgg cgaccatcac 180
catggcgatg ccctccccga cggcaggcct gtttgttccc gatgacatgt ccccggcatt 240
cacgccgacg acgtttttta tcggcttcga cgacctcgcc ggcatggacg agcagtacca 300
gcagggcttc ctgcccttct cgccgctgtc cctgtccgac caggcgccgg agctggctcc 360
tggaggaggg ggtgacacga cgccgtcttt cctggacatg ctgacaggag ggtatctcga 420
tggcggcggc tacggcggca tgagcggtgg cagcgatgcg atggacatgc cgttctcgct 480
gcctgagatg gggcccccga caactgatcc aatgccgttt cagctccagt ggacgtcatc 540
agagcttgac aactacatca acgatgacgg tggttatgca gcaggaccag ccgccggagt 600
gcagcagcag cagattaatg gtggtgatca ccagaagcag gacgagaaca aagaggcggg 660
gaacggcaaa ggcaacgacg acgacggcgg cgggtcgtcg tcggtgtaca gcttctggat 720
gaacaccagc agcagcgacg gggcacaggg gtagtgcgcc actgccagtg ccagccacag 780
gaggaggcga aagtgctgct gcgaactccc tcgcatcatc atctgctggt cggtgcaagt 840
gcagcagaca tccttcgtcg acgcaatcaa atcatcaaaa ggtggcaaca gggagtgaaa 900
gggagaagaa gttcatccag cgctagttca gtctcaatta gaggataggg tgcatgttca 960
tgtttgattt atctgcattg tggtcggtcg tggttgcaag tcatcttgca ccaccctttt 1020
gttttggttc agtttgcatg cgttcgttcg tcagagtttt acttgcagca gtctttggat 1080
cgccggcaga gattagttag gtatctatgc tttgttttcg tcagttcgtt ctggatatgt 1140
cttaatattc ctcagttt                                                1158

<210> 71
<211> 753
<212> DNA
<213> Zea mays

<400> 71
ccccgctact ggacacacgg cgggaccctc cgcatttccc cagacggcgg cggctgccgc 60
aggaacaagc gcgcctccag ctcctcgtcc ccgattccgg gcccctccag caccgccgcc 120
accagcgccg cgatggagaa gaccgtcagc acgcggctga tgctgatggc gaccatcacc 180
atggcgatgc cctccccgac ggcaggcctg tttgttcccg atgacatgtc cccggcattc 240

acgccgacga cgttttttat cggcttcgac gacctcgccg gcatggacga gcagtaccag 300
cagggcttcc tgcccttctc gccgctgtcc ctgtccgacc aggcgccgga gctggctcct 360
ggaggagggg gtgacacgac gccgtctttc ctggacatgc tgacaggagg gtatctcgat 420
ggcggcggct acggcggcat gagcggtggc agcgatgcga tggacatgcc gttctcgctg 480
cctgagatgg ggcccccgac aactgatcca atgccgtttc agctccagtg gacgtcatca 540
gagcttgaca actacatcaa cgatgacggt ggttatgcag caggaccagc cgccggagtg 600
cagcagcagc agattaatgg tggtgatcac cagaagcagg acgagaacaa agaggcgggg 660
aacggcaaag gcaacgacga cgacggcggc gggtcgtcgt cggtgtacag cttctggatg 720
aacaccagca gcagcgacgg ggcacagggg tag 753

<210> 72
<211> 250
<212> PRT
<213> Zea mays

<400> 72
Pro Arg Tyr Trp Thr His Gly Gly Thr Leu Arg Ile Ser Pro Asp Gly
1               5                   10                  15
Gly Gly Cys Arg Arg Asn Lys Arg Ala Ser Ser Ser Ser Ser Pro Ile
            20                  25                  30
Pro Gly Pro Ser Ser Thr Ala Ala Thr Ser Ala Ala Met Glu Lys Thr
        35                  40                  45
Val Ser Thr Arg Leu Met Leu Met Ala Thr Ile Thr Met Ala Met Pro
    50                  55                  60
Ser Pro Thr Ala Gly Leu Phe Val Pro Asp Asp Met Ser Pro Ala Phe
65                  70                  75                  80
Thr Pro Thr Thr Phe Phe Ile Gly Phe Asp Asp Leu Ala Gly Met Asp
                85                  90                  95
Glu Gln Tyr Gln Gln Gly Phe Leu Pro Phe Ser Pro Leu Ser Leu Ser
            100                 105                 110
Asp Gln Ala Pro Glu Leu Ala Pro Gly Gly Gly Gly Asp Thr Thr Pro
        115                 120                 125
Ser Phe Leu Asp Met Leu Thr Gly Gly Tyr Leu Asp Gly Gly Gly Tyr
    130                 135                 140
Gly Gly Met Ser Gly Gly Ser Asp Ala Met Asp Met Pro Phe Ser Leu
145                 150                 155                 160
Pro Glu Met Gly Pro Pro Thr Thr Asp Pro Met Pro Phe Gln Leu Gln
                165                 170                 175
Trp Thr Ser Ser Glu Leu Asp Asn Tyr Ile Asn Asp Asp Gly Gly Tyr
            180                 185                 190
Ala Ala Gly Pro Ala Ala Gly Val Gln Gln Gln Gln Ile Asn Gly Gly
        195                 200                 205
Asp His Gln Lys Gln Asp Glu Asn Lys Glu Ala Gly Asn Gly Lys Gly
    210                 215                 220
Asn Asp Asp Asp Gly Gly Gly Ser Ser Ser Val Tyr Ser Phe Trp Met
225                 230                 235                 240
Asn Thr Ser Ser Ser Asp Gly Ala Gln Gly
                245                 250

<210> 73
<211> 163
<212> DNA
<213> Zea mays

<400> 73
cctgcgtgca caacaggatc cttcgccttg ctactgttcg gcgcttccac tgcacaacag 60
tattttctac agctaggaag gagccagagc gcccatggac atgacctcca acaccagcaa 120
cagcgctgca gcagcatcat cggctcccca caaccaccag ccg 163

<210> 74

<211> 69
<212> DNA
<213> Zea mays

<400> 74
atggacatga cctccaacac cagcaacagc gctgcagcag catcatcggc tccccacaac 60
caccagccg 69

<210> 75
<211> 23
<212> PRT
<213> Zea mays

<400> 75
Met Asp Met Thr Ser Asn Thr Ser Asn Ser Ala Ala Ala Ala Ser Ser
1               5                   10                  15
Ala Pro His Asn His Gln Pro
            20

<210> 76
<211> 600
<212> DNA
<213> Zea mays

<400> 76
gctccagtgg gtgtcatcag agcctgacaa ctacaccaac gacgacggtg gttatgcagc 60
aggaccagca gctggagtgc agcatcagca gcaggttgtt ggcgatcagc agcagcagca 120
gcaggagaac aaagcgaatc agcgcagcaa aaacaacaac ggcggcggcg gcggtgggtc 180
gtcggtgtac agctactact ggaccaacac cagcaacagc gatggggcag aggggtagtg 240
tgtgcctctg ccagtgccag agccacagga gacgaaatcg ctgctgcaaa ctccctcgcg 300
acaaagaagg ggacgcgatt caccaccgtt aagcactagc tcagtttgta gaggatagga 360
tgcatattcg cgtttgatta atcagcgttg tggtcgtgat ggcaagtcat cacgcacatc 420
gccctttttg ttcaaatgca tgcgttagtt cgttttcatc agagtttact tgctgcagtc 480
tatcatcgcc ggcagagatt agttaggtgt ctatgctttt atattcatca gtagttctgg 540
ataataatgt cttcgtattc ctcagtttct ttgttaagtc gtctaaaact ttccatgtgt 600

<210> 77
<211> 238
<212> DNA
<213> Zea mays

<400> 77
gctccagtgg gtgtcatcag agcctgacaa ctacaccaac gacgacggtg gttatgcagc 60
aggaccagca gctggagtgc agcatcagca gcaggttgtt ggcgatcagc agcagcagca 120
gcaggagaac aaagcgaatc agcgcagcaa aaacaacaac ggcggcggcg gcggtgggtc 180
gtcggtgtac agctactact ggaccaacac cagcaacagc gatggggcag aggggtag 238

<210> 78
<211> 78
<212> PRT
<213> Zea mays

<400> 78
Leu Gln Trp Val Ser Ser Glu Pro Asp Asn Tyr Thr Asn Asp Asp Gly
1               5                   10                  15
Gly Tyr Ala Ala Gly Pro Ala Ala Gly Val Gln His Gln Gln Gln Val
            20                  25                  30
Val Gly Asp Gln Gln Gln Gln Gln Gln Glu Asn Lys Ala Asn Gln Arg
        35                  40                  45

Ser Lys Asn Asn Asn Gly Gly Gly Gly Gly Gly Ser Ser Val Tyr Ser
    50                  55                  60
Tyr Tyr Trp Thr Asn Thr Ser Asn Ser Asp Gly Ala Glu Gly
65                  70                  75

<210> 79
<211> 126
<212> DNA
<213> Zea mays

<400> 79
ggcgagccgc tgccgtgccc scggtgcggc agccgggaga ccaagttctg ctacttcaac 60
aactacaacg tgcggcagcc gcgccacctc tgccgcgcct gccgccgcta ctggacggcc 120
ggcggt                                                            126

<210> 80
<211> 42
<212> PRT
<213> Zea mays

<400> 80
Gly Glu Pro Leu Pro Cys Pro Arg Cys Gly Ser Arg Glu Thr Lys Phe
 1               5                  10                  15
Cys Tyr Phe Asn Asn Tyr Asn Val Arg Gln Pro Arg His Leu Cys Arg
            20                  25                  30
Ala Cys Arg Arg Tyr Trp Thr Ala Gly Gly
        35                  40

<210> 81
<211> 782
<212> DNA
<213> Zea mays

<400> 81
atgcaacaaa cgtggtctcg tgcttttcag ggcatcgtcg tccccgagga gcctgggatg 60
ggcggatcgt cgtcctcacc gtcagcggaa ctgatcgcat gccctaggcc gcccatgcac 120
gccgcggcgg cggatcaccg gcgtctgcgc ccgcagcacg accagccgct caagtgcccc 180
cggtgcgagt cgacgcacac caagttctgc tactacaaca actacagcct ctcgcagccg 240
cgctacttct gcaagacgtg ccgccgctac tggaccaagg gcggctcgct ccgcaacgtc 300
ccggtcggtg ggggatgccg caagaacaag cgagccagcg ccaagaagca tcccgccgcg 360
acgccgatgc agccacggca cgtggcggag acgggcctcc acctgtcctt ctccggcgtg 420
cagctgccgc cgccgccgtc ccccgccgcc gcggccgacc cgctctgcag cctcggcctt 480
ctggactgga agtacgaccc gatcctcgcg ggttccggtg gcgccgccgc tggctcgttg 540
gatggcgcga gctccgaggc gcacttcgct ggggcgggca tgctgggcat cccaggcggc 600
ggcgagtgct acgcgctgag ttcgctccgg tacgcggccg acctgggcga gcacctgcag 660
ctgcaggggc tcccgttcgg gctacgtgct gagcaggaat cgatggaggt gaaggcggcg 720
gcgacggaga ggacgctgtc gctggactgg tacagcgaga caagccgcgc gccggagagc 780
gc                                                                782

<210> 82
<211> 260
<212> PRT
<213> Zea mays

<400> 82
Met Gln Gln Thr Trp Ser Arg Ala Phe Gln Gly Ile Val Val Pro Glu
 1               5                  10                  15
Glu Pro Gly Met Gly Gly Ser Ser Ser Ser Pro Ser Ala Glu Leu Ile
            20                  25                  30

Ala Cys Pro Arg Pro Pro Met His Ala Ala Ala Ala Asp His Arg Arg
        35                  40                  45
Leu Arg Pro Gln His Asp Gln Pro Leu Lys Cys Pro Arg Cys Glu Ser
    50                  55                  60
Thr His Thr Lys Phe Cys Tyr Tyr Asn Asn Tyr Ser Leu Ser Gln Pro
65                  70                  75                  80
Arg Tyr Phe Cys Lys Thr Cys Arg Arg Tyr Trp Thr Lys Gly Gly Ser
                85                  90                  95
Leu Arg Asn Val Pro Val Gly Gly Gly Cys Arg Lys Asn Lys Arg Ala
            100                 105                 110
Ser Ala Lys Lys His Pro Ala Ala Thr Pro Met Gln Pro Arg His Val
        115                 120                 125
Ala Glu Thr Gly Leu His Leu Ser Phe Ser Gly Val Gln Leu Pro Pro
    130                 135                 140
Pro Pro Ser Pro Ala Ala Ala Ala Asp Pro Leu Cys Ser Leu Gly Leu
145                 150                 155                 160
Leu Asp Trp Lys Tyr Asp Pro Ile Leu Ala Gly Ser Gly Gly Ala Ala
                165                 170                 175
Ala Gly Ser Leu Asp Gly Ala Ser Ser Glu Ala His Phe Ala Gly Ala
            180                 185                 190
Gly Met Leu Gly Ile Pro Gly Gly Gly Glu Cys Tyr Ala Leu Ser Ser
        195                 200                 205
Leu Arg Tyr Ala Ala Asp Leu Gly Glu His Leu Gln Leu Gln Gly Leu
    210                 215                 220
Pro Phe Gly Leu Arg Ala Glu Gln Glu Ser Met Glu Val Lys Ala Ala
225                 230                 235                 240
Ala Thr Glu Arg Thr Leu Ser Leu Asp Trp Tyr Ser Glu Thr Ser Arg
                245                 250                 255
Ala Pro Glu Ser
            260

<210> 83
<211> 2451
<212> DNA
<213> Zea mays

<400> 83
cccccgtgcc ttcgcttttc cgcaacagat tgctcccggt ccactggctt ggcgtcccaa 60
ccccacatac acgcgcgcaa cagttgccag tgcatttata aaaaccggga gattctcctc 120
gaaacacaag ctccagcaaa tgcaaatcac acggcaagtg actcgatcat cacacgagtc 180
gaacggagcc gtaacctttt tcattcttcc cccgcaggtg gccgacccgc ctcatcactg 240
ttgctagtat ccgtccgtcc atgcatctgc ggctcccacc tgtcttgaca tcgtgacgga 300
tggatgggcc aagccgaggt cgagggcttt taaagtagca acgaccgacc cccttcgtcc 360
ccgcccccct agctagctct ctccctgtct gtcaccgccg agtctctcgc agtctgcctc 420
tcgctctcgc tacggagtcc ggaccactct ctctctctct ctctctctct ctctctctct 480
ctctctctct ctctctctct ctctctcttt ctagcagtct agtagctgcg actagcactt 540
ccacctactg gtgttggtgt ttgcttgcct tgacgaacga aggagttggt tcgttgcgtt 600
ccaccttcca aatgctgtcc cacgtcgaga tggcccccgg cgggttcaag ctgttcggca 660
aggtcatcac gcagtgcgcc gagagcgccc cgccggcgcc gccggaggct gctgccgcgg 720
cgtcgagggc cacggccttc gcgcgggaga gggacgaccc ggacgagcgc gaccagccga 780
tggtgaagcg ggaggcggcg gcgtccgacc acgacttcgc cgccgtggac aagcaccacc 840
agcactccgg cggcagcggc gggccgggac cgggaccgga cgagagcgac gacagcaaag 900
ggcagcagca gcagcagcac cacccgcgcc cgcggcatca tcagcatcac catcagcagc 960
agcaggacac cgtggaggcg cgcgccgcgg cggccgcgtc ctcggcgccg ccgctgccgt 1020
gcccgcggtg ccggagccgg aacaccaagt tctgctactt caacaactac aacgtcaacc 1080
agccgcgcca cttctgcaag gactgccacc gctactggac ggcgggcggc gcgctgcgca 1140
acgtccccgt gggcgccggc cgacgcaaga accggccgct cggccccgtc gtcgccggag 1200
ccgtgcccgt gcccgtgccc gtgccggcgc accaccacca ccaccacctg caccccgctg 1260
ctgctgctgc tgcggcggcg gcgggcttcg tcctcgggtt ccccggccag cagcaccctt 1320
cgtcgccgac gtcgccgtcg ccgtcgccgg tctacgccga ccggtggcct gtatgtccgg 1380

accggcggtt ctgagttctg gttagggtgg tttgacttgc gtaatttgct ggtgatgagg 1440
cggggaaggc tgccttgtct gtctgtacgt gggtgtgcgt gcgtgagagg ctggttgatg 1500
gaggacgccg gcatggaaat ggcatctgtt tccttctctt ttctctgcct cgatttcttt 1560
gtttcccgct aaattagctc ggtgtagctg ccagtttaac ctcctttgca gacgtgtgta 1620
aaaagcgtac tgtaatgtaa cagcagacca gggacaggtc aaaaactcga ggtcagcgtg 1680
cgtgctcaac tagtgccgac gaaggtgatg cgatcccatg ctgtccagtc cagtccttag 1740
ctcgcatccc gggagtcgtt ggtgatcatc gcacgaccaa ccccgcgttg tacattcctt 1800
cctgggaaaa ggtgacgacg gacggagtgc tgatgctctg cctctgcgcg cagatggagc 1860
tcacggcatt ggcttcgcag cgctagaagc ctgcccactg cccagcacga atgagagaga 1920
gaatgtgtgt gctctgagag tgagagtaga atggaggaaa gacccgtacc atgtcgatgt 1980
cgatgtggtg ttgaaagcca aaaaggcagg cgatgatgcg gttgaccgcg cgcgcaggcg 2040
cagaggacac agcctttgct acatggccac ggtcacattt tccgtgtgtg gttggcccag 2100
cccaacgctt ttcaccaccc ttttggaaag tttcatcacg tcacgatagg gcacctggct 2160
ctggctggcc gccgggcgcc ttttatttta ccggccatca atgcccgccc cccgcccggc 2220
gaaagatcgc gcggccttta ctaaaaaagc gccgggagat aaatattcca accgtcaact 2280
tgtcaggagt ctgggtggga ggaagaggaa cggaaccaag agaagactac tcctactagc 2340
tgctgccgct ttgcgggctt taaattgctc gcaaagttta ctttagtacg cgggagcggc 2400
cgcgagaccc gtctctcggc tctcgcccgc gtcgtcgcgt gtggttggtt g 2451

<210> 84
<211> 570
<212> DNA
<213> Zea mays

<400> 84
atgctgtccc acgtcgagat ggcccccggc gggttcaagc tgttcggcaa ggtcatcacg 60
cagtgcgccg agagcgcccc gccggcgccg ccggaggctg ctgccgcggc gtcgagggcc 120
acggccttcg cgcgggagag ggacgacccg gacgagcgcg accagccgat ggtgaagcgg 180
gaggcggcgg cgtccgacca cgacttcgcc gccgtggaca agcaccacca gcactccggc 240
ggcagcggcg ggccgggacc gggaccggac gagagcgacg acagcaaggg gcagcagcag 300
cagcagcacc acccgcgccc gcggcatcat cagcatcacc atcagcagca gcaggacacc 360
gtggaggcgc gcgccgcggc ggccgcgtcc tcggcgccgc cgctgccgtg cccgcggtgc 420
cggagccgga acaccaagtt ctgctacttc aacaactaca acgtcaacca gccgcgccac 480
ttctgcaagg actgccaccg ctactggacg gcgggcggcg cgctgcgcaa cgtccccgtg 540
ggcgccggcc gacgcaagaa ccggccgctc 570

<210> 85
<211> 190
<212> PRT
<213> Zea mays

<400> 85
Met Leu Ser His Val Glu Met Ala Pro Gly Gly Phe Lys Leu Phe Gly
1               5                   10                  15
Lys Val Ile Thr Gln Cys Ala Glu Ser Ala Pro Pro Ala Pro Pro Glu
            20                  25                  30
Ala Ala Ala Ala Ala Ser Arg Ala Thr Ala Phe Ala Arg Glu Arg Asp
        35                  40                  45
Asp Pro Asp Glu Arg Asp Gln Pro Met Val Lys Arg Glu Ala Ala Ala
    50                  55                  60
Ser Asp His Asp Phe Ala Ala Val Asp Lys His His Gln His Ser Gly
65                  70                  75                  80
Gly Ser Gly Gly Pro Gly Pro Gly Pro Asp Glu Ser Asp Asp Ser Lys
                85                  90                  95
Gly Gln Gln Gln Gln Gln His His Pro Arg Pro Arg His His Gln His
            100                 105                 110
His His Gln Gln Gln Gln Asp Thr Val Glu Ala Arg Ala Ala Ala Ala
        115                 120                 125
Ala Ser Ser Ala Pro Pro Leu Pro Cys Pro Arg Cys Arg Ser Arg Asn
    130                 135                 140

Thr Lys Phe Cys Tyr Phe Asn Asn Tyr Asn Val Asn Gln Pro Arg His
145                 150                 155                 160
Phe Cys Lys Asp Cys His Arg Tyr Trp Thr Ala Gly Gly Ala Leu Arg
                165                 170                 175
Asn Val Pro Val Gly Ala Gly Arg Arg Lys Asn Arg Pro Leu
            180                 185                 190

<210> 86
<211> 1900
<212> DNA
<213> Zea mays

<400> 86
ttttgccggc ctcctcgtct cctccggttc cggctaccta cctgcgggac gtcgacctcg 60
tccgtctgtc gcctagctag ctgcctcggt tttcgcttga agcagcagca gcagcaagcc 120
gggagcttct gtgtttgttt cgatcggagt gagaagaaga tggttgcgga gtgccaagga 180
ggaggagact tcctcatcaa gctcttcggg aagaccatcc ccgtgccgga gccggagccg 240
gagtccggcg acgccaagat cgtggacgcc gatgacgacc ccaagaagca cagcgacggc 300
gacgctgcca ggcagaggga gaagctgagg gagcccgaca aggtgctgcc gtgcccgcgc 360
tgtaacagcg cggacaccaa gttctgctac ttcaacaact acaacgcgaa gcagccgcgc 420
cacttctgca agcgctgcca gcgctactgg accgcgggcg gcgccatgcg caacgtgcca 480
gtgggggccg gccgccgcaa gaacaagaac gccgccgcct cgcactcgca cctcctccat 540
aggacgacga cgactgccaa cggcgcgatg ctcagcttcg cccctcccgg tcctggcctt 600
gcctgcctct gcctggacct cgccgcgcag ttcggccacc tggccccggt caggacgccc 660
gccgggaccc cgtctcgtcc ttgcacttgc agcgaagggt cgaccgacag ggacggcacg 720
tcttccgtcg tagacgaatc tgcagcaagc ggggacggac cagtgcagct gcaccaccac 780
ccagcaagcg ttaacaccgg gtggccgcca tacagcagcc cacctccggc ggcgccgtat 840
ttctcgccgg gcatctcgat tccggtatac cccgccgcgc cggggtactg gggctgcatg 900
gttcccgggg cttggagcct gccatggccg gtgcagccgc agcccccgtc gtcgcggtcg 960
caggggcagg gcctctcgtc gtcgtcgtcg ccgccgccgc ccaccaccac caccatcggt 1020
actccttcag tctcatcatc atccggggcc gctgactttg actcccacgc cctggggctg 1080
ggcctgggca agcacccgcg ggaccgggac ggcgacgacg ggaggaagag gaacggcagt 1140
gcccatggca gcggcaacgc caaggtgtgg gctcccaaga ccatccggat agacgatgtg 1200
gacgaggtgg ccaggagctc catctggtcc ctcgtcgggg tcagaggcga cagggagcag 1260
cagggcgcag ccgcgcaggt gacaagctcg ggacagtgtt cgagcccagg ggcctgcagg 1320
ccaccaagaa ggccatggcg acaagctcgc cgctccttca cgccaacccc gtcgcgctca 1380
cgcgctcggt ggcgttccat gagggctctt gattttgcca tctcaacatg atgctcctaa 1440
tcagactgac agcactgatt tgttttggat aaatcatcag aggacccctc acatgcacgc 1500
tgattcacat acctctttgg ttgcgtagga gaatcagact cctcgtatgc attgaccata 1560
actcgactca tgtttagtac gctgcatctt aaaaactgag cacacataca ctggtgcaac 1620
ctaatgttat ttaggtgcac ctgtagatac ccagaaaact tacgtattcg ggcggccagg 1680
ctcacacgag cctcgcatgc aagcagagat ccaatcacac tggtagctac ttgtggacgg 1740
cggggcggca cgcaatttgg atgggagaaa gaacaggaga tcaataagag gttcctgctc 1800
gtagataaat aaataacatt atctaacttt ttcgttgcgt tctctgtgca agtccttttt 1860
ttcctcgtgc ccttttcact cttttgtttg ggacaaattg                       1900

<210> 87
<211> 1272
<212> DNA
<213> Zea mays

<400> 87
atggttgcgg agtgccaagg aggaggagac ttcctcatca agctcttcgg gaagaccatc 60
cccgtgccgg agccggagcc ggagtccggc gacgccaaga tcgtggacgc cgatgacgac 120
cccaagaagc acagcgacgg cgacgctgcc aggcagaggg agaagctgag ggagcccgac 180
aaggtgctgc cgtgcccgcg ctgtaacagc gcggacacca agttctgcta cttcaacaac 240
tacaacgcga agcagccgcg ccacttctgc aagcgctgcc agcgctactg gaccgcgggc 300
ggcgccatgc gcaacgtgcc agtgggggcc ggccgccgca agaacaagaa cgccgccgcc 360
tcgcactcgc acctcctcca taggacgacg acgactgcca acggcgcgat gctcagcttc 420
gcccctcccg gtcctggcct tgcctgcctc tgcctggacc tcgccgcgca gttcggccac 480

ctggccccgg tcagggacgc cgccgggacc ccgtctcgtc cttgcacttg cagcgaaggg 540
tcgaccgaca gggacggcac gtcttccgtc gtagacgaat ctgcagcaag cggggacgga 600
ccagtgcagc tgcaccacca cccagcaagc gttaacaccg ggtggccgcc atacagcagc 660
ccacctccgg cggcgccgta tttctcgccg ggcatctcga ttccggtata ccccgccgcg 720
ccggggtact ggggctgcat ggttcccggg gcttggagcc tgccatggcc ggtgcagccg 780
cagcccccgt cgtcgcggtc gcaggggcag ggcctctcgt cgtcgtcgtc gccgccgccg 840
cccaccacca ccaccatcgg tactccttca gtctcatcat catccggggc cgctgacttt 900
gactcccacg ccctggggct gggcctgggc aagcacccgc gggaccggga cggcgacgac 960
gggaggaaga ggaacggcag tgcccatggc agcggcaacg ccaaggtgtg ggctcccaag 1020
accatccgga tagacgatgt ggacgaggtg gccaggagct ccatctggtc cctcgtcggg 1080
gtcagaggcg acagggagca gcagggcgca gccgcgcagg tgacaagctc gggacagtgt 1140
tcgagcccag gggcctgcag gccaccaaga aggccatggc gacaagctcg ccgctccttc 1200
acgccaaccc cgtcgcgctc acgcgctcgg tggcgttcca tgagggctct tgattttgcc 1260
atctcaacat ga 1272

<210> 88
<211> 423
<212> PRT
<213> Zea mays

<400> 88
Met Val Ala Glu Cys Gln Gly Gly Gly Asp Phe Leu Ile Lys Leu Phe
1               5                   10                  15
Gly Lys Thr Ile Pro Val Pro Glu Pro Glu Pro Glu Ser Gly Asp Ala
            20                  25                  30
Lys Ile Val Asp Ala Asp Asp Asp Pro Lys Lys His Ser Asp Gly Asp
        35                  40                  45
Ala Ala Arg Gln Arg Glu Lys Leu Arg Glu Pro Asp Lys Val Leu Pro
    50                  55                  60
Cys Pro Arg Cys Asn Ser Ala Asp Thr Lys Phe Cys Tyr Phe Asn Asn
65                  70                  75                  80
Tyr Asn Ala Lys Gln Pro Arg His Phe Cys Lys Arg Cys Gln Arg Tyr
                85                  90                  95
Trp Thr Ala Gly Gly Ala Met Arg Asn Val Pro Val Gly Ala Gly Arg
            100                 105                 110
Arg Lys Asn Lys Asn Ala Ala Ala Ser His Ser His Leu Leu His Arg
        115                 120                 125
Thr Thr Thr Thr Ala Asn Gly Ala Met Leu Ser Phe Ala Pro Pro Gly
    130                 135                 140
Pro Gly Leu Ala Cys Leu Cys Leu Asp Leu Ala Ala Gln Phe Gly His
145                 150                 155                 160
Leu Ala Pro Val Arg Asp Ala Ala Gly Thr Pro Ser Arg Pro Cys Thr
                165                 170                 175
Cys Ser Glu Gly Ser Thr Asp Arg Asp Gly Thr Ser Ser Val Val Asp
            180                 185                 190
Glu Ser Ala Ala Ser Gly Asp Gly Pro Val Gln Leu His His His Pro
        195                 200                 205
Ala Ser Val Asn Thr Gly Trp Pro Pro Tyr Ser Ser Pro Pro Pro Ala
    210                 215                 220
Ala Pro Tyr Phe Ser Pro Gly Ile Ser Ile Pro Val Tyr Pro Ala Ala
225                 230                 235                 240
Pro Gly Tyr Trp Gly Cys Met Val Pro Gly Ala Trp Ser Leu Pro Trp
                245                 250                 255
Pro Val Gln Pro Gln Pro Pro Ser Ser Arg Ser Gln Gly Gln Gly Leu
            260                 265                 270
Ser Ser Ser Ser Ser Pro Pro Pro Pro Thr Thr Thr Thr Ile Gly Thr
        275                 280                 285
Pro Ser Val Ser Ser Ser Ser Gly Ala Ala Asp Phe Asp Ser His Ala
    290                 295                 300
Leu Gly Leu Gly Leu Gly Lys His Pro Arg Asp Arg Asp Gly Asp Asp
305                 310                 315                 320

Gly Arg Lys Arg Asn Gly Ser Ala His Gly Ser Gly Asn Ala Lys Val
                325                 330                 335
Trp Ala Pro Lys Thr Ile Arg Ile Asp Asp Val Asp Glu Val Ala Arg
            340                 345                 350
Ser Ser Ile Trp Ser Leu Val Gly Val Arg Gly Asp Arg Glu Gln Gln
        355                 360                 365
Gly Ala Ala Ala Gln Val Thr Ser Ser Gly Gln Cys Ser Ser Pro Gly
    370                 375                 380
Ala Cys Arg Pro Pro Arg Arg Pro Trp Arg Gln Ala Arg Arg Ser Phe
385                 390                 395                 400
Thr Pro Thr Pro Ser Arg Ser Arg Ala Arg Trp Arg Ser Met Arg Ala
                405                 410                 415
Leu Asp Phe Ala Ile Ser Thr
                420

<210> 89
<211> 1496
<212> DNA
<213> Zea mays

<400> 89
gggctagggc tagggaagcc catggaggag atgctcatgg ctggaaacgc aaatctaaac 60
cagaatccaa acccaccgcc ggctgcgccc tcggctcccg gcgcccagag ggcaggcgct 120
cccgccgcgg ttgcggcagc gccgcctagc gctggcgcga ccggaggagc ggggccggag 180
cggcgggcgc ggccgcagaa ggagaaggcg ctcaactgcc cgcggtgcaa ctccaccaat 240
accaagttct gctattacaa caactatagc ctccagcagc cgcgctactt ctgcaagacg 300
tgccgtcgct actggaccga gggcggctcg ctccgcaacg ttccggtggg cggcggctcc 360
aggaagaaca agcgctcgtc ctcggccgtg tcgtcggcgg ctgcggccgc cgccgcctcc 420
acctccgcgg ctatgtctgg cacggtcccc gtggggctcg cggccaagaa ccccaagttg 480
atgcacgagg gcgcgcacga cctcaacctg gcgttccctc accacaacgg ccgcgctctg 540
cagccaccgg agttcccggc gttcccgagc ctggagagca gcagcgtgtg caaccccgga 600
gctgccatgc tgggcaacgg cgccgccggc aggggcatgg gcgcgctctc gggtttggag 660
ctgctgagga gcacgggctg ctacgtccca ctgcagcact ttcagctagg gatgcccgcg 720
gagtacgcag ccgcgggatt ctcgctgggc gagttccgcg ttccgccgcc gccgcagtct 780
cagagtgtgt tcgggttctc cctggacacg cacggcacgg ggggagttgg cggcgccggg 840
ggttacagtg ccgggctgca ggagagcgcg gctggcagga tgctcttccc cttcgaggac 900
ttgaagccgg cagtgagcgc tgctggcggc ggcgcgagca acggagctga tcatcatcac 960
tacgagcaca gcaaagatca agcagcaggc gacggcggcg gagccagcgg cgtcaccggc 1020
ggccacgagg ctccagcagg attctggagc aatagcatga tcgggaatgg cagcagcaat 1080
ggtggcggag gctcttggta aatggtaagg cggcgcgcca tgcatggctc atgcacgcgc 1140
cgttgtcagc atgcggagat aggggggtag agacaaggat cggagttaat aagagggtgg 1200
aggtgattag tgttcacata tggcgtttga tctctctggt tgtttgcctt ctctttttat 1260
tttcggttct tggtgttcgt gtgtgtcaat cgacttgggg gctaacacag caggatatac 1320
agattgttaa ggcggtgaac cgattattta cagcttctct tagccaatcc atctctcgtt 1380
ttctgattct gtaatccaag gaacaagctg tcaatcacgt tgtgcattgc atgcgcatta 1440
tgtagagtat gtattaattg agcagctcta gctatgttag tgttacaagt taaatc 1496

<210> 90
<211> 1101
<212> DNA
<213> Zea mays

<400> 90
gggctagggc tagggaagcc catggaggag atgctcatgg ctggaaacgc aaatctaaac 60
cagaatccaa acccaccgcc ggctgcgccc tcggctcccg gcgcccagag ggcaggcgct 120
cccgccgcgg ttgcggcagc gccgcctagc gctggcgcga ccggaggagc ggggccggag 180
cggcgggcgc ggccgcagaa ggagaaggcg ctcaactgcc cgcggtgcaa ctccaccaat 240
accaagttct gctattacaa caactatagc ctccagcagc cgcgctactt ctgcaagacg 300
tgccgtcgct actggaccga gggcggctcg ctccgcaacg ttccggtggg cggcggctcc 360
aggaagaaca agcgctcgtc ctcggccgtg tcgtcggcgg ctgcggccgc cgccgcctcc 420

acctccgcgg ctatgtctgg cacggtcccc gtggggctcg cggccaagaa ccccaagttg 480
atgcacgagg gcgcgcacga cctcaacctg gcgttccctc accacaacgg ccgcgctctg 540
cagccaccgg agttcccggc gttcccgagc ctggagagca gcagcgtgtg caaccccgga 600
gctgccatgc tgggcaacgg cgccgccggc aggggcatgg gcgcgctctc gggtttggag 660
ctgctgagga gcacgggctg ctacgtccca ctgcagcact ttcagctagg gatgcccgcg 720
gagtacgcag ccgcgggatt ctcgctgggc gagttccgcg ttccgccgcc gccgcagtct 780
cagagtgtgt tcgggttctc cctggacacg cacggcacgg ggggagttgg cggcgccggg 840
ggttacagtg ccgggctgca ggagagcgcg gctggcagga tgctcttccc cttcgaggac 900
ttgaagccgg cagtgagcgc tgctggcggc ggcgcgagca acggagctga tcatcatcac 960
tacgagcaca gcaaagatca agcagcaggc gacggcggcg gagccagcgg cgtcaccggc 1020
ggccacgagg ctccagcagg attctggagc aatagcatga tcgggaatgg cagcagcaat 1080
ggtggcggag gctcttggta a 1101

<210> 91
<211> 366
<212> PRT
<213> Zea mays

<400> 91
   Gly Leu Gly Leu Gly Lys Pro Met Glu Glu Met Leu Met Ala Gly Asn
   1 5 10 15
   Ala Asn Leu Asn Gln Asn Pro Asn Pro Pro Pro Ala Ala Pro Ser Ala
   20 25 30
   Pro Gly Ala Gln Arg Ala Gly Ala Pro Ala Ala Val Ala Ala Ala Pro
   35 40 45
   Pro Ser Ala Gly Ala Thr Gly Gly Ala Gly Pro Glu Arg Arg Ala Arg
   50 55 60
   Pro Gln Lys Glu Lys Ala Leu Asn Cys Pro Arg Cys Asn Ser Thr Asn
   65 70 75 80
   Thr Lys Phe Cys Tyr Tyr Asn Asn Tyr Ser Leu Gln Gln Pro Arg Tyr
   85 90 95
   Phe Cys Lys Thr Cys Arg Arg Tyr Trp Thr Glu Gly Gly Ser Leu Arg
   100 105 110
   Asn Val Pro Val Gly Gly Gly Ser Arg Lys Asn Lys Arg Ser Ser Ser
   115 120 125
   Ala Val Ser Ser Ala Ala Ala Ala Ala Ala Ala Ser Thr Ser Ala Ala
   130 135 140
   Met Ser Gly Thr Val Pro Val Gly Leu Ala Ala Lys Asn Pro Lys Leu
   145 150 155 160
   Met His Glu Gly Ala His Asp Leu Asn Leu Ala Phe Pro His His Asn
   165 170 175
   Gly Arg Ala Leu Gln Pro Pro Glu Phe Pro Ala Phe Pro Ser Leu Glu
   180 185 190
   Ser Ser Ser Val Cys Asn Pro Gly Ala Ala Met Leu Gly Asn Gly Ala
   195 200 205
   Ala Gly Arg Gly Met Gly Ala Leu Ser Gly Leu Glu Leu Leu Arg Ser
   210 215 220
   Thr Gly Cys Tyr Val Pro Leu Gln His Phe Gln Leu Gly Met Pro Ala
   225 230 235 240
   Glu Tyr Ala Ala Ala Gly Phe Ser Leu Gly Glu Phe Arg Val Pro Pro
   245 250 255
   Pro Pro Gln Ser Gln Ser Val Phe Gly Phe Ser Leu Asp Thr His Gly
   260 265 270
   Thr Gly Gly Val Gly Gly Ala Gly Gly Tyr Ser Ala Gly Leu Gln Glu
   275 280 285
   Ser Ala Ala Gly Arg Met Leu Phe Pro Phe Glu Asp Leu Lys Pro Ala
   290 295 300
   Val Ser Ala Ala Gly Gly Gly Ala Ser Asn Gly Ala Asp His His His
   305 310 315 320
   Tyr Glu His Ser Lys Asp Gln Ala Ala Gly Asp Gly Gly Gly Ala Ser
   325 330 335
   Gly Val Thr Gly Gly His Glu Ala Pro Ala Gly Phe Trp Ser Asn Ser
   340 345 350
   Met Ile Gly Asn Gly Ser Ser Asn Gly Gly Gly Gly Ser Trp
   355 360 365

<210> 92
<211> 2028
<212> DNA
<213> Zea mays

<400> 92
taatttaatt ttagtaacta aacaagagca tacaatatat ctatgtgtgg gaggtgttct 60
tatgctgtta tttttgcaag ctgataccag atgctaccta gggttataac agcacgatga 120
gaacattctt cagacctagc tagggttaca catgatgacc ggcatctaag gacacaagaa 180
tttatgtgtc ctcaggttct atctactagt acgagatcat aggaaatcca tgcacatatg 240
ggacattaat gctttaagaa atcaagacaa gtacaccttg catgtctctc atgatatcaa 300
aggttcgtcg ttgtaacttt aacatgtgtt caaaagcgca actacgcgta gaggagatca 360
tgcatggggc cggtctacaa ctgatccatg tgattccaaa agctttggat aacataggcg 420
gatcaagctg atcagagatc taaaatctga tctaattgat gcctccatca tgaaatatca 480
taatagcctt tggagctttg catgcagctc gtactttagc ctactgtact gtatatgcat 540
gctgaccatg tggtcctttc ccgcattccg aggcttccat tctgtcacag ataatgattt 600
gcgatcacac tgccataacg tggtcttgtg cttttcaggg catcgttccc gaggagcctg 660
ggatgggatc gtcgtcaccg tcagcggagc tgatcgcgtg ccctaggcca atgcacgcca 720
cggctgccgc ggcggaccgg cgtctgcgcc cgcagcacga ccagccactc aagtgcccgc 780
gatgcgagtc cacgcacacc aagttctgct actacaacaa ctacagtctc tcccagccgc 840
gctacttctg caagacgtgc cgccgctact ggaccaaagg cggatcgctc cgcaacgtcc 900
cggttggtgg gggatgccgc aagaacaagc gcgccagcgc caagaaacca cccgcgccac 960
cgatgcagcc gccgcacatg tcggagacgg gcctccacct gtccttctcc ggcgtgcagc 1020
agctgccgcc ggccgatccg ctttgcagcc tcggcctttt ggactggaag tatgacccga 1080
tcctcacagg ttccggtggc gtcgctggct cgttggatgg cgccagctcc gagacgcact 1140
tcgcagggac gggcatgctg ggcatcccag gtggcagcgg gggcggcgag tgccacgcgc 1200
tgagcgcgct ccggtacgcg gccggcctgg gcgagaacct gcaggtgcag ggtctcccgt 1260
ttggggcgcg cgctgagcac gacgcgatgg agatgaagcc tgcggcgacg gacaggctgc 1320
tctcgctgga ctggtacagc gagacaagcc gcgcgccaga gagcgccatc agctcgttgg 1380
gcgcgctggg cctgtggagc ggcatgctcg gcggtgcgca ccagcaccat ggctcgtcgg 1440
cggccatcta agaccctagc aaaacgtgcc tccatgcttg cgttgcattg caccagtttg 1500
ctaatggcgt taacaagtat gagtgactac gtagaaatgc ttgttttttt acctgttttt 1560
atcttctgtt tcgtttcgtc gtttgttttt tggacgatcc atgtttgccg ctcgtcattt 1620
cagatgatgt ctctgttgtt gtaccgcttc aaagttcaaa cacttgctgt ggttcaaatt 1680
acagtttgct ggtgattgat gagcccaggt tttatacgta tggaaatatg gattatgtat 1740
ggaactatgt atctcttctc tactcctata aagtatatta attacctaga ctccactgtt 1800
gtctctttta tatataaaaa agctcttata catctttcta atctaactaa accgttagat 1860
cgtaactagt cggttgaccg tatgttacga cagctcacaa taatacccac ataaattatc 1920
tacaaaaaaa gatctcaata ttttttattt attgtctctg ctcttatcat aaagtcataa 1980
tgtgttttat gaacgcactg gtacaatagg caacaaatca gagacttc 2028

<210> 93
<211> 858
<212> DNA
<213> Zea mays

<400> 93
atgatttgcg atcacactgc cataacgtgg tcttgtgctt ttcagggcat cgttcccgag 60
gagcctggga tgggatcgtc gtcaccgtca gcggagctga tcgcgtgccc taggccaatg 120
cacgccacgg ctgccgcggc ggaccggcgt ctgcgcccgc agcacgacca gccactcaag 180
tgcccgcgat gcgagtccac gcacaccaag ttctgctact acaacaacta cagtctctcc 240
cagccgcgct acttctgcaa gacgtgccgc cgctactgga ccaaaggcgg atcgctccgc 300
aacgtcccgg ttggtggggg atgccgcaag aacaagcgcg ccagcgccaa gaaaccaccc 360
gcgccaccga tgcagccgcc gcacatgtcg gagacgggcc tccacctgtc cttctccggc 420
gtgcagcagc tgccgccggc cgatccgctt tgcagcctcg gccttttgga ctggaagtat 480

gacccgatcc tcacaggttc cggtggcgtc gctggctcgt tggatggcgc cagctccgag 540
acgcacttcg cagggacggg catgctgggc atcccaggtg gcagcggggg cggcgagtgc 600
cacgcgctga gcgcgctccg gtacgcggcc ggcctgggcg agaacctgca ggtgcagggt 660
ctcccgtttg gggcgcgcgc tgagcacgac gcgatggaga tgaagcctgc ggcgacggac 720
aggctgctct cgctggactg gtacagcgag acaagccgcg cgccagagag cgccatcagc 780
tcgttgggcg cgctgggcct gtggagcggc atgctcggcg gtgcgcacca gcaccatggc 840
tcgtcggcgg ccatctaa 858

<210> 94
<211> 285
<212> PRT
<213> Zea mays

<400> 94
Met Ile Cys Asp His Thr Ala Ile Thr Trp Ser Cys Ala Phe Gln Gly
1               5                   10                  15
Ile Val Pro Glu Glu Pro Gly Met Gly Ser Ser Ser Pro Ser Ala Glu
            20                  25                  30
Leu Ile Ala Cys Pro Arg Pro Met His Ala Thr Ala Ala Ala Ala Asp
        35                  40                  45
Arg Arg Leu Arg Pro Gln His Asp Gln Pro Leu Lys Cys Pro Arg Cys
    50                  55                  60
Glu Ser Thr His Thr Lys Phe Cys Tyr Tyr Asn Asn Tyr Ser Leu Ser
65                  70                  75                  80
Gln Pro Arg Tyr Phe Cys Lys Thr Cys Arg Arg Tyr Trp Thr Lys Gly
                85                  90                  95
Gly Ser Leu Arg Asn Val Pro Val Gly Gly Gly Cys Arg Lys Asn Lys
            100                 105                 110
Arg Ala Ser Ala Lys Lys Pro Pro Ala Pro Pro Met Gln Pro Pro His
        115                 120                 125
Met Ser Glu Thr Gly Leu His Leu Ser Phe Ser Gly Val Gln Gln Leu
    130                 135                 140
Pro Pro Ala Asp Pro Leu Cys Ser Leu Gly Leu Leu Asp Trp Lys Tyr
145                 150                 155                 160
Asp Pro Ile Leu Thr Gly Ser Gly Gly Val Ala Gly Ser Leu Asp Gly
                165                 170                 175
Ala Ser Ser Glu Thr His Phe Ala Gly Thr Gly Met Leu Gly Ile Pro
            180                 185                 190
Gly Gly Ser Gly Gly Gly Glu Cys His Ala Leu Ser Ala Leu Arg Tyr
        195                 200                 205
Ala Ala Gly Leu Gly Glu Asn Leu Gln Val Gln Gly Leu Pro Phe Gly
    210                 215                 220
Ala Arg Ala Glu His Asp Ala Met Glu Met Lys Pro Ala Ala Thr Asp
225                 230                 235                 240
Arg Leu Leu Ser Leu Asp Trp Tyr Ser Glu Thr Ser Arg Ala Pro Glu
                245                 250                 255
Ser Ala Ile Ser Ser Leu Gly Ala Leu Gly Leu Trp Ser Gly Met Leu
            260                 265                 270
Gly Gly Ala His Gln His His Gly Ser Ser Ala Ala Ile
        275                 280                 285

<210> 95
<211> 1988
<212> DNA
<213> Zea mays

<400> 95
taaagaaacc aaggttgaca cgccacaaca ggagaagggt aatgaaatgg aggttgatgc 60
gccacaaaag gaacatgacg acgaaatgaa aattgacgca caacaagagg aaaaagatga 120
acaaatggaa gccgtcgcat caccaacgca tggatatata gaaccagcca atttacctcc 180

ctcggagtcg gagcacaaga aaggagacga gggaccgatg gacagtgccg aagataaagc 240
agcatcagat gcaaaggggc agaacgagaa gacatcaaat gaggaatcag gccaggacag 300
ggcacttaat aagaagccag ataagatcct accttgccct cggtgcaaca gcatggatac 360
aaagttctgc tattacaaca actacaacgt taatcaacca aggcacttct gcaagaactg 420
ccagcggtac tggactgcag ggggcaccat gaggaacgta cctgttggcg ccgggcggcg 480
caagagtaag aacgcgtcat tgcactaccg tcaactactg atggcccctg actgtatgct 540
ggggcctaga gtggacatat caaagccagt gctccctgaa ggtcttgcat catctccacc 600
tgccccgaca cagccagcca gtagaaatgg aacggtccta aaatttgggc atgaggtacc 660
attttgcgag tcaatggtgt cggcgctgaa cattgatgag cagaacggca acagccctgg 720
aggaccaaca gcaagaggtg aaaacaggga agataataat aaccctggat ccggcacacc 780
accatacaac ggtgtgcctg aaaccatggc ccccgtcgtc ggcaagaacg gggcaccagt 840
tcattgtaac ggggttgccc cagtgcctca gtattacctt ggaacccctt tcatgtaccc 900
ttggaatgta ggatggagca acgtgcctgt gatggtgcca ggtaaaagca tgcccgaacc 960
tgctcctgct ccagagagct gcagtactag ctcagctgta tggatgaacc ctcccatgat 1020
gccaggctca agacctcctc ctagtccagc gtttccatac cctctcgtgc cacccgggct 1080
ctggggctgt ttttccggat ggccagccac ggcttggaac gtaccatgga ccagaaccaa 1140
cgtctgcgtg tcgccgccgc cgccgccgcc gctgtcgccg tcgccatcaa gcaacagcag 1200
cagctgctcg ggcaacgggt ctcctactct gggcaagcat tccagggaca ccaatccgct 1260
gagagaggag aaaagagaga agtcgctgtg ggttcccaag acgctccgga tcgatgaccc 1320
tgatgacgcc gccaagagtt cgatatgggc caccctcggc atcaagcccg gagaccctgg 1380
cactttcatc aagcctttcc agtccaaggt cgagagcaag ggccagaggt cggacgctgc 1440
tcaggtcttg caggcgaacc cggcggcgat gtcgcgctca cagtcgttcc aggagagctc 1500
ttgacttcat acgcaaagtc tgccttatat atagttttgg tactgtaatc cttacatgct 1560
ggtagagttt tgtggcatgc gaaaagtgac ttcgtacctg aagcctatct tattgtttgt 1620
ttcggtactg tattccttac tacatgctag tagagctgtg cggtatgtgg aaaggacaac 1680
agagagcttc ttgtatagcc aattagccac ctagtggaac ttgcaaagag cttccatgag 1740
aactctcgag atacttatgt attgtaaata cttgacatac tggtgttgta tatacttgac 1800
ttactggtgt tgcaaaggaa cttaaaaagg agggactaaa aatttaggtg ctgtttgaaa 1860
tgtaatgcaa atggtaatgg tttacacttg attactggcg ataataagtt tgaatatatt 1920
ggtatcaatt tttagtgtgt tatctgatta cgattgaact aaaataaaca ttatttaacg 1980
ttatcagt 1988

<210> 96
<211> 1458
<212> DNA
<213> Zea mays

<400> 96
atggaggttg atgcgccaca aaaggaacat gacgacgaaa tgaaaattga cgcacaacaa 60
gaggaaaaag atgaacaaat ggaagccgtc gcatcaccaa cgcatggata tatagaacca 120
gccaatttac ctccctcgga gtcggagcac aagaaaggag acgagggacc gatggacagt 180
gccgaagata aagcagcatc agatgcaaag gggcagaacg agaagacatc aaatgaggaa 240
tcaggccagg acagggcact taataagaag ccagataaga tcctaccttg ccctcggtgc 300
aacagcatgg atacaaagtt ctgctattac aacaactaca acgttaatca accaaggcac 360
ttctgcaaga actgccagcg gtactggact gcagggggca ccatgaggaa cgtacctgtt 420
ggcgccgggc ggcgcaagag taagaacgcg tcattgcact accgtcaact actgatggcc 480
cctgactgta tgctggggcc tagagtggac atatcaaagc cagtgctccc tgaaggtctt 540
gcatcatctc cacctgcccc gacacagcca gccagtagaa atggaacggt cctaaaattt 600
gggcatgagg taccattttg cgagtcaatg gtgtcggcgc tgaacattga tgagcagaac 660
ggcaacagcc ctggaggacc aacagcaaga ggtgaaaaca gggaagataa taataaccct 720
ggatccggca caccaccata caacggtgtg cctgaaacca tggcccccgt cgtcggcaag 780
aacggggcac cagttcattg taacggggtt gccccagtgc ctcagtatta ccttggaacc 840
cctttcatgt acccttggaa tgtaggatgg agcaacgtgc ctgtgatggt gccaggtaaa 900
agcatgcccg aacctgctcc tgctccagag agctgcagta ctagctcagc tgtatggatg 960
aaccctccca tgatgccagg ctcaagacct cctcctagtc cagcgtttcc ataccctctc 1020
gtgccacccg ggctctgggg ctgtttttcc ggatggccag ccacggcttg gaacgtacca 1080
tggaccagaa ccaacgtctg cgtgtcgccg ccgccgccgc cgccgctgtc gccgtcgcca 1140
tcaagcaaca gcagcagctg ctcgggcaac gggtctccta ctctgggcaa gcattccagg 1200
gacaccaatc cgctgagaga ggagaaaaga gagaagtcgc tgtgggttcc caagacgctc 1260
cggatcgatg accctgatga cgccgccaag agttcgatat gggccaccct cggcatcaag 1320
cccggagacc ctggcacttt catcaagcct ttccagtcca aggtcgagag caagggccag 1380

aggtcggacg ctgctcaggt cttgcaggcg aacccggcgg cgatgtcgcg ctcacagtcg 1440
ttccaggaga gctcttga 1458

<210> 97
<211> 485
<212> PRT
<213> Zea mays

<400> 97
Met Glu Val Asp Ala Pro Gln Lys Glu His Asp Asp Glu Met Lys Ile
1               5                   10                  15
Asp Ala Gln Gln Glu Glu Lys Asp Glu Gln Met Glu Ala Val Ala Ser
            20                  25                  30
Pro Thr His Gly Tyr Ile Glu Pro Ala Asn Leu Pro Pro Ser Glu Ser
        35                  40                  45
Glu His Lys Lys Gly Asp Glu Gly Pro Met Asp Ser Ala Glu Asp Lys
    50                  55                  60
Ala Ala Ser Asp Ala Lys Gly Gln Asn Glu Lys Thr Ser Asn Glu Glu
65                  70                  75                  80
Ser Gly Gln Asp Arg Ala Leu Asn Lys Lys Pro Asp Lys Ile Leu Pro
                85                  90                  95
Cys Pro Arg Cys Asn Ser Met Asp Thr Lys Phe Cys Tyr Tyr Asn Asn
            100                 105                 110
Tyr Asn Val Asn Gln Pro Arg His Phe Cys Lys Asn Cys Gln Arg Tyr
        115                 120                 125
Trp Thr Ala Gly Gly Thr Met Arg Asn Val Pro Val Gly Ala Gly Arg
    130                 135                 140
Arg Lys Ser Lys Asn Ala Ser Leu His Tyr Arg Gln Leu Leu Met Ala
145                 150                 155                 160
Pro Asp Cys Met Leu Gly Pro Arg Val Asp Ile Ser Lys Pro Val Leu
                165                 170                 175
Pro Glu Gly Leu Ala Ser Ser Pro Pro Ala Pro Thr Gln Pro Ala Ser
            180                 185                 190
Arg Asn Gly Thr Val Leu Lys Phe Gly His Glu Val Pro Phe Cys Glu
        195                 200                 205
Ser Met Val Ser Ala Leu Asn Ile Asp Glu Gln Asn Gly Asn Ser Pro
    210                 215                 220
Gly Gly Pro Thr Ala Arg Gly Glu Asn Arg Glu Asp Asn Asn Asn Pro
225                 230                 235                 240
Gly Ser Gly Thr Pro Pro Tyr Asn Gly Val Pro Glu Thr Met Ala Pro
                245                 250                 255
Val Val Gly Lys Asn Gly Ala Pro Val His Cys Asn Gly Val Ala Pro
            260                 265                 270
Val Pro Gln Tyr Tyr Leu Gly Thr Pro Phe Met Tyr Pro Trp Asn Val
        275                 280                 285
Gly Trp Ser Asn Val Pro Val Met Val Pro Gly Lys Ser Met Pro Glu
    290                 295                 300
Pro Ala Pro Ala Pro Glu Ser Cys Ser Thr Ser Ser Ala Val Trp Met
305                 310                 315                 320
Asn Pro Pro Met Met Pro Gly Ser Arg Pro Pro Pro Ser Pro Ala Phe
                325                 330                 335
Pro Tyr Pro Leu Val Pro Pro Gly Leu Trp Gly Cys Phe Ser Gly Trp
            340                 345                 350
Pro Ala Thr Ala Trp Asn Val Pro Trp Thr Arg Thr Asn Val Cys Val
        355                 360                 365
Ser Pro Pro Pro Pro Pro Pro Leu Ser Pro Ser Pro Ser Ser Asn Ser
    370                 375                 380
Ser Ser Cys Ser Gly Asn Gly Ser Pro Thr Leu Gly Lys His Ser Arg
385                 390                 395                 400
Asp Thr Asn Pro Leu Arg Glu Glu Lys Arg Glu Lys Ser Leu Trp Val
                405                 410                 415

Pro Lys Thr Leu Arg Ile Asp Asp Pro Asp Asp Ala Ala Lys Ser Ser
            420                 425                 430
Ile Trp Ala Thr Leu Gly Ile Lys Pro Gly Asp Pro Gly Thr Phe Ile
        435                 440                 445
Lys Pro Phe Gln Ser Lys Val Glu Ser Lys Gly Gln Arg Ser Asp Ala
    450                 455                 460
Ala Gln Val Leu Gln Ala Asn Pro Ala Ala Met Ser Arg Ser Gln Ser
465                 470                 475                 480
Phe Gln Glu Ser Ser
                485

<210> 98
<211> 1788
<212> DNA
<213> Zea mays

<400> 98
atagggagta tgccggagaa gtggagatag tattagtctg ttgctgattt acagttcgtc 60
gagctctctt tgccgagtgt cacactcggc aaagccttcg tcgaatgttt tctaggcttt 120
gctgagacac tcgacaaaga ggacgattcc ggtagtgata gatggagtgg gtaaaatgag 180
ctaaaattac tttaggggtt atatggacgt catctacagg ttgagaggag taatttagag 240
tttttctcaa taacaaaata ctaatattta agataggaga aaggaccgta ttgttatata 300
tggtttgggt gttggactta tagagtgaag ttgaaaataa tgagtgatat agagaatgag 360
atagggagta tgccggagaa gtggagatag tattagtctg ttgctgattt ggcttagctt 420
tacagccaaa tttattaatt ttgctttgag caaataagac gaacgttttg ttttacaatc 480
ctagcacagg cgttataggt tctgtttgca acccaagcat tatttcacct tcaggattta 540
ttcgtttgga tccctcccgt agtattttgc atgggatttg atccccacga tacctctaaa 600
ttccgtcgta aacaggccgt cagagttcag acacatggtc catatgcatt gtttgttgat 660
ggtagcatgc aaagagcaag aatcaggaat ttgctccatc agtcctctgc ctgcttcgtc 720
tgcaagagaa agctacgtaa cctttgtggc tgtgggcctg tgccaccccc cgctgctgct 780
tcctgctgtc gtcctcgttc gtccctacct agctgcttac ctctctcctg catgctgcgt 840
tgctgccatc acctccgtgc catccgcggc gcgacccggt ccgagcagtc cctctctctc 900
tctctgctct gctccgctcg acaagcactg tgactctgtg agcgacacta ctgcacaccg 960
ccaccgtggc cggccggcgt ccgctgcgct atatgtatac aagcagctag caccaagcag 1020
cacggctctc caactcacag tgccacaccg gcaggcaagg catcgtcgac cgacggcgcg 1080
ccgcagcagc agcttctagc tccaaaccga accaacacgt gctatccacc atggaggcgc 1140
cgctgcacca gttccccttc cccgtgccgc cgccgccgca gccgcaggac gcgctgctgc 1200
agcaggcggt ggcggcgcgc gcgctgatga tggccgggaa aagggcgccg ctgcaggcta 1260
ccgcggcggc gggacgggag cagtgcccgc ggtgcgcgtc gcgggacacc aagttctgct 1320
actacaacaa ctacaacacg gcgcagccgc ggcacttctg ccgcgcgtgc cgccgctact 1380
ggacgctggg gggatccctc cgcaacgtcc ccgtgggcgg gtccacgcgc aagcgccagc 1440
gcccggcgcg gcccacccgc gccatggccg ccgccgccgc cacggcggct acgcaaacgg 1500
caacaacgac cgcctgcagc ccgttcgccg tctctccagc aacaatgctg atacagggcg 1560
cggcaggcgg cggcctcctc gtcagctcgc tgctgctggg ctcggcgtcc gcgtcaccgc 1620
tgctcgcgct cggcacagcg ccgctgctgg aaggccggct tggcttcgac ctcggcctcg 1680
gggacgcagc agcgctagca gccgccgacg ggactcctgg cgacttggct caccaccacc 1740
agccgctgac gctgggcgcc gggccgctcc cgtggcccgc agcgacga 1788

<210> 99
<211> 775
<212> DNA
<213> Zea mays

<400> 99
caagcagcac ggctctccaa ctcacagtgc cacaccggca ggcaaggcat cgtcgaccga 60
cggcgcgccg cagcagcagc ttctagctcc aaaccgaacc aacacgtgct atccaccatg 120
gaggcgccgc tgcaccagtt ccccttcccc gtgccgccgc cgccgcagcc gcaggacgcg 180
ctgctgcagc aggcggtggc ggcgcgcgcg ctgatgatgg ccgggaaaag ggcgccgctg 240
caggctaccg cggcggcggg acgggagcag tgcccgcggt gcgcgtcgcg ggacaccaag 300
ttctgctact acaacaacta caacacggcg cagccgcggc acttctgccg cgcgtgccgc 360

cgctactgga cgctgggggg atccctccgc aacgtccccg tgggcgggtc cacgcgcaag 420
cgccagcgcc cggcgcggcc cacccgcgcc atggccgccg ccgccgccac ggcggctacg 480
caaacggcaa caacgaccgc ctgcagcccg ttcgccgtct ctccagcaac aatgctgata 540
cagggcgcgg caggcggcgg cctcctcgtc agctcgctgc tgctgggctc ggcgtccgcg 600
tcaccgctgc tcgcgctcgg cacagcgccg ctgctggaag gccggcttgg cttcgacctc 660
ggcctcgggg acgcagcagc gctagcagcc gccgacggga ctcctggcga cttggctcac 720
caccaccagc cgctgacgct gggcgccggg ccgctcccgt ggcccgcagc gacga 775

<210> 100
<211> 258
<212> PRT
<213> Zea mays

<400> 100
   Gln Ala Ala Arg Leu Ser Asn Ser Gln Cys His Thr Gly Arg Gln Gly
   1               5                   10                  15
   Ile Val Asp Arg Arg Arg Ala Ala Ala Ala Ala Ser Ser Ser Lys Pro
               20                  25                  30
   Asn Gln His Val Leu Ser Thr Met Glu Ala Pro Leu His Gln Phe Pro
           35                  40                  45
   Phe Pro Val Pro Pro Pro Pro Gln Pro Gln Asp Ala Leu Leu Gln Gln
       50                  55                  60
   Ala Val Ala Ala Arg Ala Leu Met Met Ala Gly Lys Arg Ala Pro Leu
   65                  70                  75                  80
   Gln Ala Thr Ala Ala Ala Gly Arg Glu Gln Cys Pro Arg Cys Ala Ser
                   85                  90                  95
   Arg Asp Thr Lys Phe Cys Tyr Tyr Asn Asn Tyr Asn Thr Ala Gln Pro
               100                 105                 110
   Arg His Phe Cys Arg Ala Cys Arg Arg Tyr Trp Thr Leu Gly Gly Ser
           115                 120                 125
   Leu Arg Asn Val Pro Val Gly Gly Ser Thr Arg Lys Arg Gln Arg Pro
       130                 135                 140
   Ala Arg Pro Thr Arg Ala Met Ala Ala Ala Ala Ala Thr Ala Ala Thr
   145                 150                 155                 160
   Gln Thr Ala Thr Thr Thr Ala Cys Ser Pro Phe Ala Val Ser Pro Ala
                   165                 170                 175
   Thr Met Leu Ile Gln Gly Ala Ala Gly Gly Gly Leu Leu Val Ser Ser
               180                 185                 190
   Leu Leu Leu Gly Ser Ala Ser Ala Ser Pro Leu Leu Ala Leu Gly Thr
           195                 200                 205
   Ala Pro Leu Leu Glu Gly Arg Leu Gly Phe Asp Leu Gly Leu Gly Asp
       210                 215                 220
   Ala Ala Ala Leu Ala Ala Ala Asp Gly Thr Pro Gly Asp Leu Ala His
   225                 230                 235                 240
   His His Gln Pro Leu Thr Leu Gly Ala Gly Pro Leu Pro Trp Pro Ala
                   245                 250                 255
   Ala Thr

<210> 101
<211> 1292
<212> DNA
<213> Zea mays

<400> 101
cagcagcaac cactccagtg tctcctcgac ggcggtgctg gcagcgacca ccaccacctg 60
atgcctccgc cgtcctgcct ggcgccgctg cgcggaggtc ctgctgacac ggcggcgagc 120
gctccagcgg gaggcggctc gtccacctcg gtgccgacga cggcgggagc aggggcaggg 180
gcgggggcga cgcagcctcg ccccgtcgtg tcgatggcgg agcgcgccag gctcgtgcgc 240
gtgccgctcc cggagcccgg cacgctccgc tgcccgcgct gcgactccgc caacaccaag 300

ttctgctact ttaacaacta ctcgctctcg cagccgcgcc acttctgcaa ggcgtgccgc 360
cgctactgga cccgcggcgg cgcgctccgc aacgtgcccg tcggcggcgg gtgcaggcgc 420
aacaccaagc gctccagcaa gaagtcctcc cgcggtggcg gcgcgggcgc cacggcggcc 480
acctcctcgt cctccaccac ctccacctcc accacggcca ccaccaccac gacgagcgcg 540
gccatggcgg cggccgaggc catcgccagc atgcaggcgc agctgcccca cctcggcctc 600
ccgcccgcag cggccgccgc cgcgctcgag gcctcgctgg agggctacca ccaccactac 660
ctcccgctcc agatgcagcc gcagttcctg cagcaggctg gcctgcacgg ctaccatttc 720
gccgacgacg gcagcggcgt cctcgcagac gggttcccga ggggcgtcgt tgcctcgggc 780
cttctcgcgc agctcgccgc ggtgaagatg gaggagcaca gcagcaacgg cggaggtgcc 840
gtcacggcgc atcaacaaga gcagtcgtac tcgtactggc ccggcagcac cggcggtggc 900
agtgggtggc cggcggagtt cttgtcgggg ttcagctcgt cgtcgtcggg gaatgtgttg 960
tgagttgcat gtggccgtcc aggtccagct ggggataaat atgcatacat gcattctgac 1020
gaagctgaac tagctcactc tcctcagtta gtttatgtgg tttggaactt aattggagtc 1080
gtcttcttca attagggctg gtgatcgatc ttgttttggg tcgctgcatg tttttttcct 1140
tacgagtttt aagctgcagg ttaatgtcgt gtagttggta gtttttgcat gtagtgggag 1200
gtgcatgtgt taactcacgg atgtgatacg tgtcaggtta cttaactata ctatgtttaa 1260
ttacatgaga acttggggca tagttgtact ag 1292

<210> 102
<211> 960
<212> DNA
<213> Zea mays

<400> 102
cagcagcaac cactccagtg tctcctcgac ggcggtgctg gcagcgacca ccaccacctg 60
atgcctccgc cgtcctgcct ggcgccgctg cgcggaggtc ctgctgacac ggcggcgagc 120
gctccagcgg gaggcggctc gtccacctcg gtgccgacga cggcgggagc aggggcaggg 180
gcgggggcga cgcagcctcg ccccgtcgtg tcgatggcgg agcgcgccag gctcgtgcgc 240
gtgccgctcc cggagcccgg cacgctccgc tgcccgcgct gcgactccgc caacaccaag 300
ttctgctact ttaacaacta ctcgctctcg cagccgcgcc acttctgcaa ggcgtgccgc 360
cgctactgga cccgcggcgg cgcgctccgc aacgtgcccg tcggcggcgg gtgcaggcgc 420
aacaccaagc gctccagcaa gaagtcctcc cgcggtggcg gcgcgggcgc cacggcggcc 480
acctcctcgt cctccaccac ctccacctcc accacggcca ccaccaccac gacgagcgcg 540
gccatggcgg cggccgaggc catcgccagc atgcaggcgc agctgcccca cctcggcctc 600
ccgcccgcag cggccgccgc cgcgctcgag gcctcgctgg agggctacca ccaccactac 660
ctcccgctcc agatgcagcc gcagttcctg cagcaggctg gcctgcacgg ctaccatttc 720
gccgacgacg gcagcggcgt cctcgcagac gggttcccga ggggcgtcgt tgcctcgggc 780
cttctcgcgc agctcgccgc ggtgaagatg gaggagcaca gcagcaacgg cggaggtgcc 840
gtcacggcgc atcaacaaga gcagtcgtac tcgtactggc ccggcagcac cggcggtggc 900
agtgggtggc cggcggagtt cttgtcgggg ttcagctcgt cgtcgtcggg gaatgtgttg 960

<210> 103
<211> 320
<212> PRT
<213> Zea mays

<400> 103
Gln Gln Gln Pro Leu Gln Cys Leu Leu Asp Gly Gly Ala Gly Ser Asp
1               5                   10                  15
His His His Leu Met Pro Pro Pro Ser Cys Leu Ala Pro Leu Arg Gly
            20                  25                  30
Gly Pro Ala Asp Thr Ala Ala Ser Ala Pro Ala Gly Gly Gly Ser Ser
        35                  40                  45
Thr Ser Val Pro Thr Thr Ala Gly Ala Gly Ala Gly Ala Gly Ala Thr
    50                  55                  60
Gln Pro Arg Pro Val Val Ser Met Ala Glu Arg Ala Arg Leu Val Arg
65                  70                  75                  80
Val Pro Leu Pro Glu Pro Gly Thr Leu Arg Cys Pro Arg Cys Asp Ser
                85                  90                  95

Ala Asn Thr Lys Phe Cys Tyr Phe Asn Asn Tyr Ser Leu Ser Gln Pro
            100                 105                 110
Arg His Phe Cys Lys Ala Cys Arg Arg Tyr Trp Thr Arg Gly Gly Ala
        115                 120                 125
Leu Arg Asn Val Pro Val Gly Gly Gly Cys Arg Arg Asn Thr Lys Arg
    130                 135                 140
Ser Ser Lys Lys Ser Ser Arg Gly Gly Gly Ala Gly Ala Thr Ala Ala
145                 150                 155                 160
Thr Ser Ser Ser Ser Thr Thr Ser Thr Ser Thr Thr Ala Thr Thr Thr
                165                 170                 175
Thr Thr Ser Ala Ala Met Ala Ala Ala Glu Ala Ile Ala Ser Met Gln
            180                 185                 190
Ala Gln Leu Pro His Leu Gly Leu Pro Pro Ala Ala Ala Ala Ala Ala
        195                 200                 205
Leu Glu Ala Ser Leu Glu Gly Tyr His His His Tyr Leu Pro Leu Gln
    210                 215                 220
Met Gln Pro Gln Phe Leu Gln Gln Ala Gly Leu His Gly Tyr His Phe
225                 230                 235                 240
Ala Asp Asp Gly Ser Gly Val Leu Ala Asp Gly Phe Pro Arg Gly Val
                245                 250                 255
Val Ala Ser Gly Leu Leu Ala Gln Leu Ala Ala Val Lys Met Glu Glu
            260                 265                 270
His Ser Ser Asn Gly Gly Gly Ala Val Thr Ala His Gln Gln Glu Gln
        275                 280                 285
Ser Tyr Ser Tyr Trp Pro Gly Ser Thr Gly Gly Gly Ser Gly Trp Pro
    290                 295                 300
Ala Glu Phe Leu Ser Gly Phe Ser Ser Ser Ser Ser Gly Asn Val Leu
305                 310                 315                 320

<210> 104
<211> 809
<212> DNA
<213> Zea mays

<400> 104
cattttgtct cctcctcgtt ggccaccata gaggtgggga gggaatggag aggaaatatt 60
catatgctct tttgcgaaat ctctcttctt tgcttgaata taccatgcca tctcactttt 120
ggtttcattc gctgatatca atttcacctc gcgtgccttt cttctcttta attgttgtgt 180
gatttggaaa tgacgctcct ttcttcagca acaacaagct caccatggcc accttaagtg 240
gcaccggagg aggtggaggc ggcaacgtcg acgcgcatgc gcaccatcat cagctgccgt 300
cgatgccacc tcctgctcgt gttggggctc tcatggctcc tcgtcctaac atggcggctg 360
tggtcgccgc gagcggcggc agcacgagcg acggtagtgg gccaactggc ggtggttccg 420
ttatccggtc gggctccatg accgagcggg ctaggctcgc caagatcccg cagccggagc 480
cggggctcaa gtgcccgcgt tgcgagtcca ccaacaccaa gttttgttac tttaacaact 540
actcgctctc ccagccgcgc cacttctgca agacgtgtcg tcgctactgg actcgtggcg 600
gcgcgcttcg gaacatccct gtcggtggcg ggtgtcgccg caacaagcgc accaagtcgt 660
ccaactcgtc gtcggccacc tccgcttcaa gagcgagcgg gacatcgtcg tccacctcgt 720
ccaccgcgac cggcggcaac agcgccgcca gcatcaagct gcctcaccag ggcacggagg 780
ggcagctgcc tttcctagcc tcgtgcacc 809

<210> 105
<211> 585
<212> DNA
<213> Zea mays

<400> 105
atggccacct taagtggcac cggaggaggt ggaggcggca acgtcgacgc gcatgcgcac 60
catcatcagc tgccgtcgat gccacctcct gctcgtgttg gggctctcat ggctcctcgt 120
cctaacatgg cggctgtggt cgccgcgagc ggcggcagca cgagcgacgg tagtgggcca 180
actggcggtg gttccgttat ccggtcgggc tccatgaccg agcgggctag gctcgccaag 240

atcccgcagc cggagccggg gctcaagtgc ccgcgttgcg agtccaccaa caccaagttt 300
tgttacttta acaactactc gctctcccag ccgcgccact tctgcaagac gtgtcgtcgc 360
tactggactc gtggcggcgc gcttcggaac atccctgtcg gtggcgggtg tcgccgcaac 420
aagcgcacca agtcgtccaa ctcgtcgtcg gccacctccg cttcaagagc gagcggaaca 480
tcgtcgtcca cctcgtccac cgcgaccggc ggcaacagcg ccgccagcat caagctgcct 540
caccaggggc acggagggca gctgcctttc ctagcctcgt gcacc 585

<210> 106
<211> 195
<212> PRT
<213> Zea mays

<400> 106
Met Ala Thr Leu Ser Gly Thr Gly Gly Gly Gly Gly Gly Asn Val Asp
1               5                   10                  15
Ala His Ala His His His Gln Leu Pro Ser Met Pro Pro Pro Ala Arg
            20                  25                  30
Val Gly Ala Leu Met Ala Pro Arg Pro Asn Met Ala Ala Val Val Ala
        35                  40                  45
Ala Ser Gly Gly Ser Thr Ser Asp Gly Ser Gly Pro Thr Gly Gly Gly
    50                  55                  60
Ser Val Ile Arg Ser Gly Ser Met Thr Glu Arg Ala Arg Leu Ala Lys
65                  70                  75                  80
Ile Pro Gln Pro Glu Pro Gly Leu Lys Cys Pro Arg Cys Glu Ser Thr
                85                  90                  95
Asn Thr Lys Phe Cys Tyr Phe Asn Asn Tyr Ser Leu Ser Gln Pro Arg
            100                 105                 110
His Phe Cys Lys Thr Cys Arg Arg Tyr Trp Thr Arg Gly Gly Ala Leu
        115                 120                 125
Arg Asn Ile Pro Val Gly Gly Gly Cys Arg Arg Asn Lys Arg Thr Lys
    130                 135                 140
Ser Ser Asn Ser Ser Ser Ala Thr Ser Ala Ser Arg Ala Ser Gly Thr
145                 150                 155                 160
Ser Ser Ser Thr Ser Ser Thr Ala Thr Gly Gly Asn Ser Ala Ala Ser
                165                 170                 175
Ile Lys Leu Pro His Gln Gly His Gly Gly Gln Leu Pro Phe Leu Ala
            180                 185                 190
Ser Cys Thr
        195

<210> 107
<211> 1005
<212> DNA
<213> Zea mays

<400> 107
ggcggtaccg gcacgggagg cgggcgggag ccggagggcc tgccgtgccc gcgctgcgag 60
tccgccaaca ccaagttctg ctactacaac aactacaacc tgtcccagcc gcgctacttc 120
tgccgggcct gccgccgcta ctggacgcgc gggggagcgc tccgcaacgt ccccgtcggc 180
ggcggcacgc gcaaggccac gcccgccggc cgccgcaagc gcgccggccc ggcgcccgca 240
accgcggcgc cgcctgcgcc tccgcctccg agcgcgctcc acggctcgct gctgcgcccg 300
tacggtgggc tgtccttcgc cgcgccggcc ctggcgtcgc cgctcgccgc cgtggacccg 360
gaccgccggc tgctggacct cggcggcagc ttcacgtcgc tgatcgcgcc aggggccgac 420
gtcggcgtcc acttctccgc cgagttcctc gtgggcggac tcgcgccggc ggccctgcct 480
cgcgcgaccg cctctgtccc tgctctgccg ccaccgccac cgcagcagca tcagcagccg 540
acgacggtgt cccaggcgtt gccggaaggc ctgttctgga gcatggggtg gccggacctg 600
tccatctaag agctccatgc ctccgccggt gtcccaggcg cgctcggtgc atcgtccgtc 660
accggtgccc ggctaatgac tgctgcgtgc gggtgcatca tcgctaatct agctttttct 720
ctttctctct tttagctgtt tgaattaaca gtagactgta gtgctgcatc caggtgatct 780
tttgaggggg gcaagttgtt ttagtcatca tgaaactttt ccagtcagga aggaactgtg 840

agagagtgat ctgatctgct gaggatgtca tgaaaaactc tgcatgctcg gttgccttgc 900
ctgcatgagg acctgtgctg cagtgcgtga gcaattgagc ttgaccaacc gataccatca 960
gcagattgtt ttttgggggg tcgaaaacgt tgagctgatt ggtgc 1005

<210> 108
<211> 609
<212> DNA
<213> Zea mays

<400> 108
ggcggtaccg gcacgggagg cgggcgggag ccggagggcc tgccgtgccc gcgctgcgag 60
tccgccaaca ccaagttctg ctactacaac aactacaacc tgtcccagcc gcgctacttc 120
tgccgggcct gccgccgcta ctggacgcgc gggggagcgc tccgcaacgt ccccgtcggc 180
ggcggcacgc gcaaggccac gcccgccggc cgccgcaagc gcgccggccc ggcgcccgca 240
accgcggcgc cgcctgcgcc tccgcctccg agcgcgctcc acggctcgct gctgcgcccg 300
tacggtgggc tgtccttcgc cgcgccggcc ctggcgtcgc cgctcgccgc cgtggacccg 360
gaccgccggc tgctggacct cggcggcagc ttcacgtcgc tgatcgcgcc aggggccgac 420
gtcggcgtcc acttctccgc cgagttcctc gtgggcggac tcgcgccggc ggccctgcct 480
cgcgcgaccg cctctgtccc tgctctgccg ccaccgccac cgcagcagca tcagcagccg 540
acgacggtgt cccaggcgtt gccggaaggc ctgttctgga gcatggggtg gccggacctg 600
tccatctaa 609

<210> 109
<211> 202
<212> PRT
<213> Zea mays

<400> 109
Gly Gly Thr Gly Thr Gly Gly Gly Arg Glu Pro Glu Gly Leu Pro Cys
1               5                   10                  15
Pro Arg Cys Glu Ser Ala Asn Thr Lys Phe Cys Tyr Tyr Asn Asn Tyr
            20                  25                  30
Asn Leu Ser Gln Pro Arg Tyr Phe Cys Arg Ala Cys Arg Arg Tyr Trp
        35                  40                  45
Thr Arg Gly Gly Ala Leu Arg Asn Val Pro Val Gly Gly Gly Thr Arg
    50                  55                  60
Lys Ala Thr Pro Ala Gly Arg Arg Lys Arg Ala Gly Pro Ala Pro Ala
65                  70                  75                  80
Thr Ala Ala Pro Pro Ala Pro Pro Pro Pro Ser Ala Leu His Gly Ser
                85                  90                  95
Leu Leu Arg Pro Tyr Gly Gly Leu Ser Phe Ala Ala Pro Ala Leu Ala
            100                 105                 110
Ser Pro Leu Ala Ala Val Asp Pro Asp Arg Arg Leu Leu Asp Leu Gly
        115                 120                 125
Gly Ser Phe Thr Ser Leu Ile Ala Pro Gly Ala Asp Val Gly Val His
    130                 135                 140
Phe Ser Ala Glu Phe Leu Val Gly Gly Leu Ala Pro Ala Ala Leu Pro
145                 150                 155                 160
Arg Ala Thr Ala Ser Val Pro Ala Leu Pro Pro Pro Pro Pro Gln Gln
                165                 170                 175
His Gln Gln Pro Thr Thr Val Ser Gln Ala Leu Pro Glu Gly Leu Phe
            180                 185                 190
Trp Ser Met Gly Trp Pro Asp Leu Ser Ile
        195                 200

<210> 110
<211> 498
<212> DNA
<213> Zea mays

<400> 110
cttcagtaac caccgaacca tccacgagcc accgccgctg gcctcgcgca acatgctgcc 60
gcctcacgtc gagatgccga ccaccagggc ggcgggcatc aagctgttcg gcaaggtcat 120
caccacgcat tatcagcagc agcagcagcc gccgcagccc gtgcgccacg cgggcgccgc 180
gccggcgtcg tcggggcgcg ggagcggcgg cggccccggc cccgacttgc tggaggaggt 240
ggcgcgggcg cgcgcggcgg cggccgaggc gcggctgccg tgcccgcgct gcttgagccg 300
ggacaccaag ttctgctact tcaacaacta caacgtgaac cagccgcgcc acttctgccg 360
ggcctgccac cgctactgga cggccggcgg cgccatccgc aacgtgccgg tcggctccgg 420
ccgccgcaag aaccgcccgg tgctgctmcc gccgccgccg ccgccgccgc acgctaccac 480
cgccactact accggcag 498

<210> 111
<211> 446
<212> DNA
<213> Zea mays

<400> 111
atgctgccgc ctcacgtcga gatgccgacc accagggcgg cgggcatcaa gctgttcggc 60
aaggtcatca ccacgcatta tcagcagcag cagcagccgc cgcagcccgt gcgccacgcg 120
ggcgccgcgc cggcgtcgtc ggggcgcggg agcggcggcg gccccggccc cgacttgctg 180
gaggaggtgg cgcgggcgcg cgcggcggcg gccgaggcgc ggctgccgtg cccgcgctgc 240
ttgagccggg acaccaagtt ctgctacttc aacaactaca acgtgaacca gccgcgccac 300
ttctgccggg cctgccaccg ctactggacg gccggcggcg ccatccgcaa cgtgccggtc 360
ggctccggcc gccgcaagaa ccgcccggtg ctgctmccgc cgccgccgcc gccgccgcac 420
gctaccaccg ccactactac cggcag 446

<210> 112
<211> 148
<212> PRT
<213> Zea mays

<400> 112
Met Leu Pro Pro His Val Glu Met Pro Thr Thr Arg Ala Ala Gly Ile
1               5                   10                  15
Lys Leu Phe Gly Lys Val Ile Thr Thr His Tyr Gln Gln Gln Gln Gln
            20                  25                  30
Pro Pro Gln Pro Val Arg His Ala Gly Ala Ala Pro Ala Ser Ser Gly
        35                  40                  45
Arg Gly Ser Gly Gly Gly Pro Gly Pro Asp Leu Leu Glu Glu Val Ala
    50                  55                  60
Arg Ala Arg Ala Ala Ala Ala Glu Ala Arg Leu Pro Cys Pro Arg Cys
65                  70                  75                  80
Leu Ser Arg Asp Thr Lys Phe Cys Tyr Phe Asn Asn Tyr Asn Val Asn
                85                  90                  95
Gln Pro Arg His Phe Cys Arg Ala Cys His Arg Tyr Trp Thr Ala Gly
            100                 105                 110
Gly Ala Ile Arg Asn Val Pro Val Gly Ser Gly Arg Arg Lys Asn Arg
        115                 120                 125
Pro Val Leu Leu Pro Pro Pro Pro Pro Pro Pro His Ala Thr Thr Ala
    130                 135                 140
Thr Thr Thr Gly
145

<210> 113
<211> 761
<212> DNA
<213> Zea mays

<400> 113
tgaatgcctg ataccgatag gcgatagcta gtacgtgata gctccttcag aaaccatcga 60

accatccacg agccaccgcc gctggcctcg cgcaacatgc tgccgcctca cgtcgagatg 120
ccgaccacca gggcggcggg catcaagctg ttcggcaagg tcatcaccac gcattatcag 180
cagcagcagc agccgcagcc cgtgcgccac gcgggcgccg cgccggcgtc gtcggggcgc 240
gggagcggcg gcggcggcgg cggcggcccc gacttgctgg aggaggtggc tcgggcgcgc 300
gcggcggcgg ccgaggcgcg gctgccgtgc ccgcgctgct tgagccggga caccaagttc 360
tgctacttca acaactacaa cgtgaaccag ccgcgccact tctgccgggc ctgccaccgc 420
tactggacgg ccggcggcgc catccgcaac gtgccggtcg gctccggccg ccgcaagaac 480
cgcccggtgc tgctccgccg ccgccgcacg ctgccaccgc cactactacc ggcagtagtg 540
ctactgtcag cgccgacgat agtaacgacc accgccgctc ggcctcgggg tctcctccag 600
tgtttacctc tgcgttcacc gcgccctact acctcggctc gcccgcccag ttcgccacgt 660
cgccgccggc ttacgccgct gccgcgcccc cggggacgac ggggcagtgc ttgtggctgg 720
tggcggcgac cacaaccacg gatcgtctgc tgcaacggac g 761

<210> 114
<211> 665
<212> DNA
<213> Zea mays

<400> 114
atgctgccgc ctcacgtcga gatgccgacc accagggcgg cgggcatcaa gctgttcggc 60
aaggtcatca ccacgcatta tcagcagcag cagcagccgc agcccgtgcg ccacgcgggc 120
gccgcgccgg cgtcgtcggg gcgcgggagc ggcggcggcg gcggcggcgg ccccgacttg 180
ctggaggagg tggctcgggc gcgcgcggcg gcggccgagg cgcggctgcc gtgcccgcgc 240
tgcttgagcc gggacaccaa gttctgctac ttcaacaact acaacgtgaa ccagccgcgc 300
cacttctgcc gggcctgcca ccgctactgg acggccggcg gcgccatccg caacgtgccg 360
gtcggctccg gccgccgcaa gaaccgcccg gtgctgctcc gccgccgccg cacgctgcca 420
ccgccactac taccggcagt agtgctactg tcagcgccga cgatagtaac gaccaccgcc 480
gctcggcctc ggggtctcct ccagtgttta cctctgcgtt caccgcgccc tactacctcg 540
gctcgcccgc ccagttcgcc acgtcgccgc cggcttacgc cgctgccgcg cccccgggga 600
cgacggggca gtgcttgtgg ctggtggcgg cgaccacaac cacggatcgt ctgctgcaac 660
ggacg 665

<210> 115
<211> 221
<212> PRT
<213> Zea mays

<400> 115
Met Leu Pro Pro His Val Glu Met Pro Thr Thr Arg Ala Ala Gly Ile
1               5                   10                  15
Lys Leu Phe Gly Lys Val Ile Thr Thr His Tyr Gln Gln Gln Gln Gln
            20                  25                  30
Pro Gln Pro Val Arg His Ala Gly Ala Ala Pro Ala Ser Ser Gly Arg
        35                  40                  45
Gly Ser Gly Gly Gly Gly Gly Gly Gly Pro Asp Leu Leu Glu Glu Val
    50                  55                  60
Ala Arg Ala Arg Ala Ala Ala Ala Glu Ala Arg Leu Pro Cys Pro Arg
65                  70                  75                  80
Cys Leu Ser Arg Asp Thr Lys Phe Cys Tyr Phe Asn Asn Tyr Asn Val
                85                  90                  95
Asn Gln Pro Arg His Phe Cys Arg Ala Cys His Arg Tyr Trp Thr Ala
            100                 105                 110
Gly Gly Ala Ile Arg Asn Val Pro Val Gly Ser Gly Arg Arg Lys Asn
        115                 120                 125
Arg Pro Val Leu Leu Arg Arg Arg Arg Thr Leu Pro Pro Pro Leu Leu
    130                 135                 140
Pro Ala Val Val Leu Leu Ser Ala Pro Thr Ile Val Thr Thr Thr Ala
145                 150                 155                 160
Ala Arg Pro Arg Gly Leu Leu Gln Cys Leu Pro Leu Arg Ser Pro Arg
                165                 170                 175

Pro Thr Thr Ser Ala Arg Pro Pro Ser Ser Pro Arg Arg Arg Arg Leu
            180                 185                 190
Thr Pro Leu Pro Arg Pro Arg Gly Arg Arg Gly Ser Ala Cys Gly Trp
        195                 200                 205
Trp Arg Arg Pro Gln Pro Arg Ile Val Cys Cys Asn Gly
    210                 215                 220

<210> 116
<211> 607
<212> DNA
<213> Zea mays

<400> 116
tcaaagttta accaaccacc cttttcgcca cttcacaaac tttatcagct tttcaattcc 60
ttgaagaaga agaagaagaa agcctcaaaa ttcaacccat attctccata gaaacgaaac 120
tttatcttca aattcaatca agcagcgagg gcttgaggtg tggaggagta tttaaaatgc 180
aagacccaat gggctttcac caaatgaaag cgccggcttt tcaagagcaa gagcagcagc 240
agctgaaatg cccccgctgt gactcaacca acaccaaatt ctgttactac aacaactata 300
acttgtctca gccccgccat ttctgcaaga actgccgccg ttactggact aaaggcggcg 360
ccctccgtaa catacccgtc ggtggcggca cccgtaaggg caccaaacgc tcctcctcct 420
cctccaccaa caaccctaag cgccagccca acccctctcc agaccccacc ccaaaccaaa 480
aaatccctga tccctctccg ccgccgccga catcatcatc atcgtcgatg tttccccagc 540
agattgtttt gagctcgggg gctcagaatt cggacttgga tatcgactcg acccggatgt 600
atctgtt                                                           607

<210> 117
<211> 431
<212> DNA
<213> Zea mays

<400> 117
atgcaagacc caatgggctt tcaccaaatg aaagcgccgg cttttcaaga gcaagagcag 60
cagcagctga aatgcccccg ctgtgactca accaacacca aattctgtta ctacaacaac 120
tataacttgt ctcagccccg ccatttctgc aagaactgcc gccgttactg gactaaaggc 180
ggcgccctcc gtaacatacc cgtcggtggc ggcacccgta agggcaccaa acgctcctcc 240
tcctcctcca ccaacaaccc taagcgccag cccaacccct ctccagaccc caccccaaac 300
caaaaaatcc ctgatccctc tccgccgccg ccgacatcat catcatcgtc gatgtttccc 360
cagcagattg ttttgagctc gggggctcag aattcggact tggatatcga ctcgacccgg 420
atgtatctgt t                                                      431

<210> 118
<211> 143
<212> PRT
<213> Zea mays

<400> 118
Met Gln Asp Pro Met Gly Phe His Gln Met Lys Ala Pro Ala Phe Gln
1               5                   10                  15
Glu Gln Glu Gln Gln Gln Leu Lys Cys Pro Arg Cys Asp Ser Thr Asn
            20                  25                  30
Thr Lys Phe Cys Tyr Tyr Asn Asn Tyr Asn Leu Ser Gln Pro Arg His
        35                  40                  45
Phe Cys Lys Asn Cys Arg Arg Tyr Trp Thr Lys Gly Gly Ala Leu Arg
    50                  55                  60
Asn Ile Pro Val Gly Gly Gly Thr Arg Lys Gly Thr Lys Arg Ser Ser
65                  70                  75                  80
Ser Ser Ser Thr Asn Asn Pro Lys Arg Gln Pro Asn Pro Ser Pro Asp
                85                  90                  95
Pro Thr Pro Asn Gln Lys Ile Pro Asp Pro Ser Pro Pro Pro Pro Thr
            100                 105                 110

Ser Ser Ser Ser Ser Met Phe Pro Gln Gln Ile Val Leu Ser Ser Gly
        115                 120                 125
Ala Gln Asn Ser Asp Leu Asp Ile Asp Ser Thr Arg Met Tyr Leu
    130                 135                 140

<210> 119
<211> 1583
<212> DNA
<213> Zea mays

<400> 119
agtttcgtca tgcacaagca tgacgaactg gtttctctct ccgtttccta attattattt 60
tagagtgaat ccggcctttt tccaatacga acttcaaatc tctggttgat tgatcgctgt 120
gattcgcaag tttgtttact acctatgctc atgccctggt tgagcataga cacaaaggaa 180
gggcatcatt ataggcatgt tcttgaatat accgtgcttt gaattccagc ctttattaat 240
tgattcgtta tacatacaga tgcagatgca gatgcagcag cagtcaccgc tccagtgtct 300
cctcggcagc ggcggtggca gcgaccacca ccacctcatg cctcctccgt ccggcctggc 360
gccgctgccg gggggtcctg ctgacacagc ggcgagcggt ccggcgggag gcggctcgtc 420
cacctcggcc tcagtgcaag ccgcggcggg agcgggggca ggggcgcagc ctcgccccgt 480
ggtgtcgatg gcggagcgcg cccggctcgc gcgcgtgccg ctgccggagc ccggcacgct 540
ccgatgcccg cgctgtgact cgaccaacac caagttctgc tacttcaata actactcgct 600
gtcgcagccg cgccacttct gcaaggcgtg ccgccgctac tggacccgtg gcggcgcgct 660
ccgcaacgtg cccgtcggcg gcgggtgcag gcgcaacacc aagcgctcca gcaagaagtc 720
gtcccgcggc ggcggcgcgg gcgccacggc ggcaacctcc tcgtcctcga ccacctccac 780
ctccaccacg gccactacca ccaccaccac gagcgcggcc atggcggcgg ccgaggccat 840
cgccggcatg caggcgcagc tgccccacct cggcctcccg cccgcagcgg ccgccgcggc 900
gcttgaggcc tcgctggagg gctaccacca ctacctcccg ctccagatgc agccgcagtt 960
cctgcagcag gctggcctgc acggctacca tttcgccgac gacggcaccg gcgtcctcgc 1020
agctgacggg ttcccgaggg gcgtcgtcgc ctcggggctg ctcgcgcagc tcgcggcggt 1080
gaagatggag gagcacggca gcaacggcgg aggtgccatc gcggcgcatc atgagcagca 1140
gtcctactgg cccggcagca ccggcggtgg cggtgggtgg ccggtggagt tcttgtcggg 1200
gttcagctca tcctcgtcgg ggaatgtgtt gtgagttgca tgtggccgcg tccaggtcca 1260
cctgggcatg catgatgggg ggcatgcatg catggatgct gatgaagctg aactagctca 1320
ccatcctcaa ttagttctag ggtttatgtg gtgtttggaa cttaattggc gtcgtcttct 1380
tcaattaggc tggtgatgat cttgtgttgg ggtaaggttt ttacatgttt tttttcttat 1440
gagttttaag ctggttaatg tcgtgcagtt ggtagttttg catgtagtgg gaagtgcatg 1500
tgttgactca tggaggttat atgtgtcagg ttacttaact atactatgtt taattatatg 1560
agaacttggt gggcatagtt gta 1583

<210> 120
<211> 1038
<212> DNA
<213> Zea mays

<400> 120
atgttcttga atataccgtg ctttgaattc cagcctttat taattgattc gttatacata 60
cagatgcaga tgcagatgca gcagcagtca ccgctccagt gtctcctcgg cagcggcggt 120
ggcagcgacc accaccacct catgcctcct ccgtccggcc tggcgccgct gccggggggt 180
cctgctgaca cagcggcgag cggtccggcg ggaggcggct cgtccacctc ggcctcagtg 240
caagccgcgg cgggagcggg ggcaggggcg cagcctcgcc ccgtggtgtc gatggcggag 300
cgcgcccggc tcgcgcgcgt gccgctgccg gagcccggca cgctccgatg cccgcgctgt 360
gactcgacca acaccaagtt ctgctacttc aataactact cgctgtcgca gccgcgccac 420
ttctgcaagg cgtgccgccg ctactggacc cgtggcggcg cgctccgcaa cgtgcccgtc 480
ggcggcgggt gcaggcgcaa caccaagcgc tccagcaaga agtcgtcccg cggcggcggc 540
gcgggcgcca cggcggcaac ctcctcgtcc tcgaccacct ccacctccac cacggccact 600
accaccacca ccacgagcgc ggccatggcg gcggccgagg ccatcgccgg catgcaggcg 660
cagctgcccc acctcggcct cccgcccgca gcggccgccg cggcgcttga ggcctcgctg 720
gagggctacc accactacct cccgctccag atgcagccgc agttcctgca gcaggctggc 780
ctgcacggct accatttcgc cgacgacggc accggcgtcc tcgcagctga cgggttcccg 840
aggggcgtcg tcgcctcggg gctgctcgcg cagctcgcgg cggtgaagat ggaggagcac 900

ggcagcaacg gcggaggtgc catcgcggcg catcatgagc agcagtccta ctggcccggc 960
agcaccggcg gtggcggtgg gtggccggtg gagttcttgt cggggttcag ctcatcctcg 1020
tcggggaatg tgttgtga 1038

<210> 121
<211> 345
<212> PRT
<213> Zea mays

<400> 121
Met Phe Leu Asn Ile Pro Cys Phe Glu Phe Gln Pro Leu Leu Ile Asp
1               5                   10                  15
Ser Leu Tyr Ile Gln Met Gln Met Gln Met Gln Gln Gln Ser Pro Leu
            20                  25                  30
Gln Cys Leu Leu Gly Ser Gly Gly Gly Ser Asp His His His Leu Met
        35                  40                  45
Pro Pro Pro Ser Gly Leu Ala Pro Leu Pro Gly Gly Pro Ala Asp Thr
    50                  55                  60
Ala Ala Ser Gly Pro Ala Gly Gly Gly Ser Ser Thr Ser Ala Ser Val
65                  70                  75                  80
Gln Ala Ala Ala Gly Ala Gly Ala Gly Ala Gln Pro Arg Pro Val Val
                85                  90                  95
Ser Met Ala Glu Arg Ala Arg Leu Ala Arg Val Pro Leu Pro Glu Pro
            100                 105                 110
Gly Thr Leu Arg Cys Pro Arg Cys Asp Ser Thr Asn Thr Lys Phe Cys
        115                 120                 125
Tyr Phe Asn Asn Tyr Ser Leu Ser Gln Pro Arg His Phe Cys Lys Ala
    130                 135                 140
Cys Arg Arg Tyr Trp Thr Arg Gly Gly Ala Leu Arg Asn Val Pro Val
145                 150                 155                 160
Gly Gly Gly Cys Arg Arg Asn Thr Lys Arg Ser Ser Lys Lys Ser Ser
                165                 170                 175
Arg Gly Gly Gly Ala Gly Ala Thr Ala Ala Thr Ser Ser Ser Ser Thr
            180                 185                 190
Thr Ser Thr Ser Thr Thr Ala Thr Thr Thr Thr Thr Thr Ser Ala Ala
        195                 200                 205
Met Ala Ala Ala Glu Ala Ile Ala Gly Met Gln Ala Gln Leu Pro His
    210                 215                 220
Leu Gly Leu Pro Pro Ala Ala Ala Ala Ala Ala Leu Glu Ala Ser Leu
225                 230                 235                 240
Glu Gly Tyr His His Tyr Leu Pro Leu Gln Met Gln Pro Gln Phe Leu
                245                 250                 255
Gln Gln Ala Gly Leu His Gly Tyr His Phe Ala Asp Asp Gly Thr Gly
            260                 265                 270
Val Leu Ala Ala Asp Gly Phe Pro Arg Gly Val Val Ala Ser Gly Leu
        275                 280                 285
Leu Ala Gln Leu Ala Ala Val Lys Met Glu Glu His Gly Ser Asn Gly
    290                 295                 300
Gly Gly Ala Ile Ala Ala His His Glu Gln Gln Ser Tyr Trp Pro Gly
305                 310                 315                 320
Ser Thr Gly Gly Gly Gly Gly Trp Pro Val Glu Phe Leu Ser Gly Phe
                325                 330                 335
Ser Ser Ser Ser Ser Gly Asn Val Leu
            340                 345

<210> 122
<211> 1491
<212> DNA
<213> Zea mays

<400> 122
agcggggggg cgcatcacag gtccggcacc gacatgcgca gagcgtcacc gtggcactcc 60
cactcccacc gcagcaagcg cgcgccgttc gacgccgccc tctccgccgc caacaacaag 120
ctgccctggc cgtcgccgcg gcgcccggcg aaccatgccg tcctcctttc tgacctcttc 180
ttcctcttcc gcttcgtcgc acctctctta cctgatccct gctagggcgg cgccgccgcc 240
tccctttgcc atggtacgta cgtaccttaa agagtaccac aaacgtcctg atccgtcgat 300
cgtgtagcgt actctctgat acatctcttc cacagttcca ctcagacatg catgcatgca 360
catgcgatga ctagctagcg gtgtgttcct gttcctggca gggacaggga tacggtgcca 420
ccagtggcgg cggcgtggcg aacgccacga gtgccgcggc ggcgcctccg cctccgaggc 480
agggagccag caggaacgcg ggcgcggggc acccgccgct gccgcgcccg ccgccgcggc 540
agtgcccgcg ctgccggtcc gccaacacca agttctgcta ctacaacaac tacagccgcg 600
agcagccacg gtacctctgc aaggcgtgcc gccgccactg gaccgagggc ggcacgctcc 660
gcgacgtgcc cgtcggcggc ggccgcaaga acaggcgcgg cgccaagggc ggcgccgctg 720
cgaaagggtc tgcctcggcc gcggccgcgg ctccggcgca gcagggaggg aacgtcctcg 780
gcgccgacac gttcccaggg gacttgctgc ggcagctggt gcagttccag ccggacgcgg 840
ccgtgggcgg gggcggctac gccatcgacc tgagcgcgtg gcaccaaatg gtcgctgcca 900
cggcgccgcc gccgccgccg ccgggaactg gcggcgatgt cagcagtctc ggtctcggag 960
cggcgggggc gggagcggga gcggaggcca actgcgtcgc gttgcagtac tggagcgaag 1020
acggcatgcc cggtcttgat ggggcctgct aagtgcgtac tactactaga gctttgttat 1080
tactattacg gtattaccac aggagtatgt tccagcctac ttatatatgt gtgcgtgtac 1140
tgcgtgcgaa acatacatac tagttagtat atatgatcag cttgttttct ctacttcctt 1200
gtggcgcgta ctatgtattc ttagctaaat aaaattgtgc gcgtgctaat aaactacgct 1260
gctccaacca tcctaatgcc taaggtgtgg ttcacccttg tccgtttaga gttctggacg 1320
actggacctt gcaaaaattg tgaccatatt tttatttcat gctagcttgc tacccttctg 1380
tcctaaaatt aaacgacttc ttgagtcgtc ggaagtcaaa gtatttaaat cttaactaaa 1440
tttatagaaa acagcattaa tatttatgac acaaaattaa tatcgatatc a 1491

<210> 123
<211> 678
<212> DNA
<213> Zea mays

<400> 123
ctagcggtgt gttcctgttc ctggcaggga cagggatacg gtgccaccag tggcggcggc 60
gtggcgaacg ccacgagtgc cgcggcggcg cctccgcctc cgaggcaggg agccagcagg 120
aacgcgggcg cggggcaccc gccgctgccg cgcccgccgc cgcggcagtg cccgcgctgc 180
cggtccgcca acaccaagtt ctgctactac aacaactaca gccgcgagca gccacggtac 240
ctctgcaagg cgtgccgccg ccactggacc gagggcggca cgctccgcga cgtgcccgtc 300
ggcggcggcc gcaagaacag gcgcggcgcc aagggcggcg ccgctgcgaa agggtctgcc 360
tcggccgcgg ccgcggctcc ggcgcagcag ggagggaacg tcctcggcgc cgacacgttc 420
ccaggggact tgctgcggca gctggtgcag ttccagccgg acgcggccgt gggcgggggc 480
ggctacgcca tcgacctgag cgcgtggcac caaatggtcg ctgccacggc gccgccgccg 540
ccgccgccgg gaactggcgg cgatgtcagc agtctcggtc tcggagcggc gggggcggga 600
gcgggagcgg aggccaactg cgtcgcgttg cagtactgga gcgaagacgg catgcccggt 660
cttgatgggg cctgctaa 678

<210> 124
<211> 225
<212> PRT
<213> Zea mays

<400> 124
Leu Ala Val Cys Ser Cys Ser Trp Gln Gly Gln Gly Tyr Gly Ala Thr
1               5                   10                  15
Ser Gly Gly Gly Val Ala Asn Ala Thr Ser Ala Ala Ala Ala Pro Pro
            20                  25                  30
Pro Pro Arg Gln Gly Ala Ser Arg Asn Ala Gly Ala Gly His Pro Pro
        35                  40                  45
Leu Pro Arg Pro Pro Pro Arg Gln Cys Pro Arg Cys Arg Ser Ala Asn
    50                  55                  60

Thr Lys Phe Cys Tyr Tyr Asn Asn Tyr Ser Arg Glu Gln Pro Arg Tyr
65                  70                  75                  80
Leu Cys Lys Ala Cys Arg Arg His Trp Thr Glu Gly Gly Thr Leu Arg
                85                  90                  95
Asp Val Pro Val Gly Gly Gly Arg Lys Asn Arg Arg Gly Ala Lys Gly
            100                 105                 110
Gly Ala Ala Ala Lys Gly Ser Ala Ser Ala Ala Ala Ala Ala Pro Ala
        115                 120                 125
Gln Gln Gly Gly Asn Val Leu Gly Ala Asp Thr Phe Pro Gly Asp Leu
    130                 135                 140
Leu Arg Gln Leu Val Gln Phe Gln Pro Asp Ala Ala Val Gly Gly Gly
145                 150                 155                 160
Gly Tyr Ala Ile Asp Leu Ser Ala Trp His Gln Met Val Ala Ala Thr
                165                 170                 175
Ala Pro Pro Pro Pro Pro Pro Gly Thr Gly Gly Asp Val Ser Ser Leu
            180                 185                 190
Gly Leu Gly Ala Ala Gly Ala Gly Ala Gly Ala Glu Ala Asn Cys Val
        195                 200                 205
Ala Leu Gln Tyr Trp Ser Glu Asp Gly Met Pro Gly Leu Asp Gly Ala
    210                 215                 220
Cys
225

<210> 125
<211> 947
<212> DNA
<213> Zea mays

<400> 125
cagcgagtgc tagctttgtt ctgattctta gtaaccaagc catgcatatg cctcctctta 60
ccgttctgct aagctaatac atattgaatc gtctgcaaag taactgcatg caagagaaat 120
gtttgaagga cctcgtcggt aaatttatct gctcatgtaa attaaatgaa catgttacat 180
ttcttttgga atgtttcttt agctagtttg taaaaaaatt cactgaaatc ttgcagacaa 240
ccaaccaagg catggcgtat gtgttgtgct gaaacgtcga ccggtaccgc ggcccaaaag 300
gcaccagcac cacaggcatg ctagctatat atatactaca agggcaagtc tactggatgc 360
ctaatagtac gtgacagctc cttcagaaac atcgaaaacc accgaaccag tcaccaccac 420
gcctcggcct cgcgcaacat gctgcctcat gtcgagatgc cggtaccggg cagggcggcg 480
gccgcgtgca ccggcgcggc gggcatcaag ctgttcggca aggtcatcac cacgccgccg 540
caccacgcgg gcgccgcgcc tccgaggctg cagcaggcgg cagcggcgtc ggggcgcggg 600
agcgccgacc tactggagga ggtggcgcgg gcgcgcgcgg cggcggccga ggtgcggctg 660
ccgtgcccgc gctgcctgag ccgggacacc aagttctgct acttcaacaa ctacaacgtc 720
aaccagccgc gccacttctg ccgggcctgc caccgctact ggacggccgg cggcgccatc 780
cgcaacgtgc ccatcggctc cggccgccgc arraaccgcc cggtgctgct cccaccgcgg 840
cgccgcacgc tgtcaccgcc accgataaca gcsgcggtcg ggcgcggggt ctcctccggt 900
gttcggctct gtgttcaccg cgccctacca gcagctcggc gcgccca 947

<210> 126
<211> 509
<212> DNA
<213> Zea mays

<400> 126
atgctgcctc atgtcgagat gccggtaccg ggcagggcgg cggccgcgtg caccggcgcg 60
gcgggcatca agctgttcgg caaggtcatc accacgccgc cgcaccacgc gggcgccgcg 120
cctccgaggc tgcagcaggc ggcagcggcg tcggggcgcg ggagcgccga cctactggag 180
gaggtggcgc gggcgcgcgc ggcggcggcc gaggtgcggc tgccgtgccc gcgctgcctg 240
agccgggaca ccaagttctg ctacttcaac aactacaacg tcaaccagcc gcgccacttc 300
tgccgggcct gccaccgcta ctggacggcc ggcggcgcca tccgcaacgt gcccatcggc 360
tccggccgcc gcarraaccg cccggtgctg ctcccaccgc ggcgccgcac gctgtcaccg 420
ccaccgataa cagcsgcggt cgggcgcggg gtctcctccg gtgttcggct ctgtgttcac 480

cgcgccctac cagcagctcg gcgcgccca                                    509

<210> 127
<211> 169
<212> PRT
<213> Zea mays

<220>
<221> VARIANT
<222> 125
<223> Xaa = Any Amino Acid

<221> VARIANT
<222> 125
<223> Xaa = Any Amino Acid

<400> 127
Met Leu Pro His Val Glu Met Pro Val Pro Gly Arg Ala Ala Ala Ala
 1               5                  10                  15
Cys Thr Gly Ala Ala Gly Ile Lys Leu Phe Gly Lys Val Ile Thr Thr
            20                  25                  30
Pro Pro His His Ala Gly Ala Ala Pro Pro Arg Leu Gln Gln Ala Ala
        35                  40                  45
Ala Ala Ser Gly Arg Gly Ser Ala Asp Leu Leu Glu Glu Val Ala Arg
    50                  55                  60
Ala Arg Ala Ala Ala Ala Glu Val Arg Leu Pro Cys Pro Arg Cys Leu
65                  70                  75                  80
Ser Arg Asp Thr Lys Phe Cys Tyr Phe Asn Asn Tyr Asn Val Asn Gln
                85                  90                  95
Pro Arg His Phe Cys Arg Ala Cys His Arg Tyr Trp Thr Ala Gly Gly
            100                 105                 110
Ala Ile Arg Asn Val Pro Ile Gly Ser Gly Arg Arg Xaa Asn Arg Pro
        115                 120                 125
Val Leu Leu Pro Pro Arg Arg Arg Thr Leu Ser Pro Pro Pro Ile Thr
    130                 135                 140
Ala Ala Val Gly Arg Gly Val Ser Ser Gly Val Arg Leu Cys Val His
145                 150                 155                 160
Arg Ala Leu Pro Ala Ala Arg Arg Ala
                165

<210> 128
<211> 1065
<212> DNA
<213> Zea mays

<400> 128
cttcctttct actactggtg tttcctcgct ttgacggagt tggttggttc cagtctccaa 60
atggtgtccc acgtcgagat gggcctcgct gccggcgggt tcaagctctt cggcaaggtc 120
atcacgcagt gcgccgagag cgctccgccg gcctccttgg tcgcgcggga gagggacgat 180
ccggacgagc gcgaccagcc ggtggtgaag cgggaggcag cggcggcgtc ggacagcgac 240
tcggccgtcg tactcgtaga caagcagcgg cgctccggcg ggccggccga gagcgaggac 300
agcaaggggc agcatcaccc gcgcccgcgg cagccgcagc agcaggacac cgcggaggcc 360
cgcgccgcgg cgtcggcgcc gccgctgccg tgcccgcggt gccggagccg caacaccaag 420
ttctgctact tcaacaacta caacgtcaac cagccgcgcc acttctgcaa ggactgccac 480
cgctactgga ccgcgggcgg cgcgctccgc aacgtccccg tcggcgccgg ccgccgcaag 540
aaccggcccc tcggcgccgc acccatcccc ttggccgtgt ccgtgccggc ccagcacctg 600
cagcacccgc agcaccccgc tgcggcggcg ggccacggcc tcggcctccc cggccagcac 660
ccttcctgcc cgacgtcgcc gtccccggcc tacgccgggc ggtggccggt ctgcccggac 720
cgccggttct gagctgacgt gacgatccga tcgatggatc gatcgatcgc agacctctcg 780
ggagtcggat gtagcgccag gttggttttg acctgcgtaa ttagcgggcg gggtggggga 840

cggaacggaa ggctgccttg tttgtactgt acgtgcttga ggcccaaaag ggggtatgga 900
ttattgatgg aggacgacat ggaaatgtct tatgttttct tctctgtcta gcgtgtacta 960
gtagctcgtc tgtttccttc ttttgttgtt ccctaaccct gcactactag tactagttat 1020
aagctaccag atgtgtgtaa aaagtgtggt actactaata atgta 1065

<210> 129
<211> 732
<212> DNA
<213> Zea mays

<400> 129
cttcctttct actactggtg tttcctcgct ttgacggagt tggttggttc cagtctccaa 60
atggtgtccc acgtcgagat gggcctcgct gccggcgggt tcaagctctt cggcaaggtc 120
atcacgcagt gcgccgagag cgctccgccg gcctccttgg tcgcgcggga gagggacgat 180
ccggacgagc gcgaccagcc ggtggtgaag cgggaggcag cggcggcgtc ggacagcgac 240
tcggccgtcg tactcgtaga caagcagcgg cgctccggcg ggccggccga gagcgaggac 300
agcaaggggc agcatcaccc gcgcccgcgg cagccgcagc agcaggacac cgcggaggcc 360
cgcgccgcgg cgtcggcgcc gccgctgccg tgcccgcggt gccggagccg caacaccaag 420
ttctgctact tcaacaacta caacgtcaac cagccgcgcc acttctgcaa ggactgccac 480
cgctactgga ccgcgggcgg cgcgctccgc aacgtccccg tcggcgccgg ccgccgcaag 540
aaccggcccc tcggcgccgc acccatcccc ttggccgtgt ccgtgccggc ccagcacctg 600
cagcacccgc agcaccccgc tgcggcggcg ggccacggcc tcggcctccc cggccagcac 660
ccttcctgcc cgacgtcgcc gtccccggcc tacgccgggc ggtggccggt ctgcccggac 720
cgccggttct ga 732

<210> 130
<211> 243
<212> PRT
<213> Zea mays

<400> 130
Leu Pro Phe Tyr Tyr Trp Cys Phe Leu Ala Leu Thr Glu Leu Val Gly
1               5                   10                  15
Ser Ser Leu Gln Met Val Ser His Val Glu Met Gly Leu Ala Ala Gly
            20                  25                  30
Gly Phe Lys Leu Phe Gly Lys Val Ile Thr Gln Cys Ala Glu Ser Ala
        35                  40                  45
Pro Pro Ala Ser Leu Val Ala Arg Glu Arg Asp Asp Pro Asp Glu Arg
    50                  55                  60
Asp Gln Pro Val Val Lys Arg Glu Ala Ala Ala Ala Ser Asp Ser Asp
65                  70                  75                  80
Ser Ala Val Val Leu Val Asp Lys Gln Arg Arg Ser Gly Gly Pro Ala
                85                  90                  95
Glu Ser Glu Asp Ser Lys Gly Gln His His Pro Arg Pro Arg Gln Pro
            100                 105                 110
Gln Gln Gln Asp Thr Ala Glu Ala Arg Ala Ala Ala Ser Ala Pro Pro
        115                 120                 125
Leu Pro Cys Pro Arg Cys Arg Ser Arg Asn Thr Lys Phe Cys Tyr Phe
    130                 135                 140
Asn Asn Tyr Asn Val Asn Gln Pro Arg His Phe Cys Lys Asp Cys His
145                 150                 155                 160
Arg Tyr Trp Thr Ala Gly Gly Ala Leu Arg Asn Val Pro Val Gly Ala
                165                 170                 175
Gly Arg Arg Lys Asn Arg Pro Leu Gly Ala Ala Pro Ile Pro Leu Ala
            180                 185                 190
Val Ser Val Pro Ala Gln His Leu Gln His Pro Gln His Pro Ala Ala
        195                 200                 205
Ala Ala Gly His Gly Leu Gly Leu Pro Gly Gln His Pro Ser Cys Pro
    210                 215                 220
Thr Ser Pro Ser Pro Ala Tyr Ala Gly Arg Trp Pro Val Cys Pro Asp
225                 230                 235                 240

Arg Arg Phe

<210> 131
<211> 786
<212> DNA
<213> Zea mays

<400> 131
gaatgtgatg ccccgcacac atgagatgcc attcaataat aggaaccccc gggcgtcttc 60
cctcctcgcc aggaaaccct cctcaccgtt gcaatatttg cttggcatac ttccaatcct 120
tccttatata cgtgaacgca cccacctaat cacctgatac ctagctgcct gctcgtggca 180
ccagcaccac cgatcgccaa gctagctagg acggccgacc gatcgatcga gctgctgcaa 240
gggcggccgc cggcgactct cgatcgctag gcgctagctg cccgccggcg ccggtacgta 300
tatatggcga cgggggacga cgcggtcggg acacggaagg gcggcgccgg caccggcggt 360
gcagctggcg gcgggggcac gcctaccacg cagacgcaac agcagcagca gcagacgccc 420
ccgccgcccg agcagggggt gagctgcccg cgctgcgact ccccgaacac caagttctgc 480
tactacaaca actacagcct gtcgcagccg cgccacttct gcaagacgtg ccgcaggtac 540
tggaccaagg gcggcgcgct ccgcaacgtg cccgtaggcg ggggctgccg caagaacaag 600
cgctcgcgct ccgcggcggc cgcgcgcctc tcgctcaacc tgccgctgga ggccgccgcc 660
gaccagcagg cggcgaggct gggcttcctt ggcgccgccg ctcatccggt gttgtcgtcg 720
tcgtcgatcg gcggcggcgg ccccgcggcc gactaccacc aaccgcaggt gggcgccgcc 780
gtcggg 786

<210> 132
<211> 483
<212> DNA
<213> Zea mays

<400> 132
atggcgacgg gggacgacgc ggtcgggaca cggaagggcg gcgccggcac cggcggtgca 60
gctggcggcg ggggcacgcc taccacgcag acgcaacagc agcagcagca gacgcccccg 120
ccgcccgagc agggggtgag ctgcccgcgc tgcgactccc cgaacaccaa gttctgctac 180
tacaacaact acagcctgtc gcagccgcgc cacttctgca agacgtgccg caggtactgg 240
accaagggcg gcgcgctccg caacgtgccc gtaggcgggg gctgccgcaa gaacaagcgc 300
tcgcgctccg cggcggccgc gcgcctctcg ctcaacctgc cgctggaggc cgccgccgac 360
cagcaggcgg cgaggctggg cttccttggc gccgccgctc atccggtgtt gtcgtcgtcg 420
tcgatcggcg gcggcggccc cgcggccgac taccaccaac cgcaggtggg cgccgccgtc 480
ggg 483

<210> 133
<211> 161
<212> PRT
<213> Zea mays

<400> 133
Met Ala Thr Gly Asp Asp Ala Val Gly Thr Arg Lys Gly Gly Ala Gly
1               5                   10                  15
Thr Gly Gly Ala Ala Gly Gly Gly Gly Thr Pro Thr Thr Gln Thr Gln
            20                  25                  30
Gln Gln Gln Gln Gln Thr Pro Pro Pro Pro Glu Gln Gly Val Ser Cys
        35                  40                  45
Pro Arg Cys Asp Ser Pro Asn Thr Lys Phe Cys Tyr Tyr Asn Asn Tyr
    50                  55                  60
Ser Leu Ser Gln Pro Arg His Phe Cys Lys Thr Cys Arg Arg Tyr Trp
65                  70                  75                  80
Thr Lys Gly Gly Ala Leu Arg Asn Val Pro Val Gly Gly Gly Cys Arg
                85                  90                  95
Lys Asn Lys Arg Ser Arg Ser Ala Ala Ala Ala Arg Leu Ser Leu Asn
            100                 105                 110

Leu Pro Leu Glu Ala Ala Ala Asp Gln Gln Ala Ala Arg Leu Gly Phe
        115                 120                 125
Leu Gly Ala Ala Ala His Pro Val Leu Ser Ser Ser Ser Ile Gly Gly
    130                 135                 140
Gly Gly Pro Ala Ala Asp Tyr His Gln Pro Gln Val Gly Ala Ala Val
145                 150                 155                 160
Gly

<210> 134
<211> 534
<212> DNA
<213> Zea mays

<400> 134
cagcaacagc acgctcaaca tggccagcag cttccaagtg gcggcaacgg cggcggcggc 60
ggcggcatgg acacgcatgc gcatcatcat catcatcagc tgccgccagt gccacctcct 120
cctagcgggg ctctcatggc tcctcgccct gacatgtcgg ctatggtcgt ccctgcgagc 180
ggtggtggtg gcgggccgac tagcggcggc accgctatcc gtccgggctc catgaccgag 240
cgggccaagc tcgccaagat cccgcagccg gagccggggc tcaagtgccc gcgctgcgag 300
tccaccaaca ccaagttctg ctacttcaac aactactcgc tctcccagcc gcgccacttc 360
tgcaagacgt gccgccggta ctggacgcgc ggcggcgcgc tccggaacgt ccccgtgggc 420
ggcgggtgcc gccgtaacaa gcgcaccaag tcgtccaagt ccaactcgtc atcggccgcc 480
tctgcttcag gcggcgcggg cgggacgtcg tcgtccacct cgtccaccgg acgg       534

<210> 135
<211> 178
<212> PRT
<213> Zea mays

<400> 135
Gln Gln Gln His Ala Gln His Gly Gln Gln Leu Pro Ser Gly Gly Asn
 1               5                  10                  15
Gly Gly Gly Gly Gly Gly Met Asp Thr His Ala His His His His His
            20                  25                  30
Gln Leu Pro Pro Val Pro Pro Pro Pro Ser Gly Ala Leu Met Ala Pro
        35                  40                  45
Arg Pro Asp Met Ser Ala Met Val Val Pro Ala Ser Gly Gly Gly Gly
    50                  55                  60
Gly Pro Thr Ser Gly Gly Thr Ala Ile Arg Pro Gly Ser Met Thr Glu
65                  70                  75                  80
Arg Ala Lys Leu Ala Lys Ile Pro Gln Pro Glu Pro Gly Leu Lys Cys
                85                  90                  95
Pro Arg Cys Glu Ser Thr Asn Thr Lys Phe Cys Tyr Phe Asn Asn Tyr
            100                 105                 110
Ser Leu Ser Gln Pro Arg His Phe Cys Lys Thr Cys Arg Arg Tyr Trp
        115                 120                 125
Thr Arg Gly Gly Ala Leu Arg Asn Val Pro Val Gly Gly Gly Cys Arg
    130                 135                 140
Arg Asn Lys Arg Thr Lys Ser Ser Lys Ser Asn Ser Ser Ser Ala Ala
145                 150                 155                 160
Ser Ala Ser Gly Gly Ala Gly Gly Thr Ser Ser Ser Thr Ser Ser Thr
                165                 170                 175
Gly Arg

<210> 136
<211> 1574
<212> DNA

<213> Zea mays

<400> 136
ccatagaggt ggggagggaa tggagaggaa atatccctag gctcttttgt gaaatctctc 60
ctctttgctt gaatatacca agtcgtctct cttttggttt cattcgctga gatatcaatt 120
tcaccttgcg cgcctttctt ccctttaatt gttgtgtgat tgtgtgtttg atttgtaaat 180
gacgttgctt tcttcagcaa ctgcaagctc accatggcca gcttccaagt gacgccggag 240
gaggtggagg cggtggcgtc gacgtgcatg cgcaccatca tcagctaccg ccgatgccac 300
cttctgctcc tggtggggct ctcatggctc ctcgccgtga catggcggct gtggtcgccg 360
cgagcggtgg cagcacgagc ggcggcggtg ggccgactgg cggtggttcc cctatccgtc 420
cgggctccat gaccgagcgg gctaggctcg ccaagatccc gcagccagag ccggggctca 480
agtgcccgcg ttgcgagtcc accaacacca agttttgcta cttcaacaac tactcgctct 540
cccagccgcg ccacttctgc aagacgtgtc gccggtactg gacgcgcggc ggcacgctcc 600
ggaacgtccc cgttggcggc gggtgccgcc gcaacaagcg caccaagtcg tccaagtcca 660
actcgtcgtc agccgcctcc gcttccagcg cgggtgggac gtcgtcgtcc acctcgtcca 720
ccgcgaccgg tggcagcggc agcgccgcca gcatcatgcc gcctcaccag gggcacgggg 780
gacagccgca gttcctagcc tcgttgcacc accctctcgt cggcggggac cactacagca 840
ccggcgcgtc aagattaggg ttccccgggc tgagctcgct ggaccccatg gactaccacc 900
agttcggcgc cggtgccgga ggtgccatcg ggctggagca gtggcgccta ccgcagatac 960
aacagttccc attcctaagc agccgccccg acgccgtgca accgccaatg tctggcattt 1020
acccgttcga agtggaaggc cacggcgggg aaggttccgg cttcgctggt cacatgcttg 1080
gtgaccccaa ggtgtccggc tcagccggac tgatcacgca gctggcgtca gtgaagatgg 1140
aggacaaccc aacgtccgca gcgatggcaa gcagctcacc gagggagttc cttggtctcc 1200
ctaggaacct ccaattatgg ggcgctagcg gcaacggcgg tgcaagtggc aacaatggag 1260
gaggcaccgg caacgctgcc ggtggcggcg acactattcc tccgggtagc agctgggtag 1320
acctgtcagg attcaactcg tcttcgtccg ggaacgtgct gtgacgtgac gcgccccctg 1380
aatctgatct gcatgataag ctggtacagt tgctgcagat gaatggtagc tctatctatt 1440
tatgcaagca ctgaagtttc attttagtca ttgcttatga gaaagttcca cagaaaagga 1500
gttgtggtgc aagattatag agggggggck gsrkggrkgk atmtatrygm tagctgatac 1560
aacgacctgt tgca 1574

<210> 137
<211> 1071
<212> DNA
<213> Zea mays

<400> 137
atgccacctt ctgctcctgg tggggctctc atggctcctc gccgtgacat ggcggctgtg 60
gtcgccgcga gcggtggcag cacgagcggc ggcggtgggc cgactggcgg tggttcccct 120
atccgtccgg gctccatgac cgagcgggct aggctcgcca agatcccgca gccagagccg 180
gggctcaagt gcccgcgttg cgagtccacc aacaccaagt tttgctactt caacaactac 240
tcgctctccc agccgcgcca cttctgcaag acgtgtcgcc ggtactggac gcgcggcggc 300
acgctccgga acgtccccgt tggcggcggg tgccgccgca acaagcgcac caagtcgtcc 360
aagtccaact cgtcgtcagc cgcctccgct tccagcgcgg gtgggacgtc gtcgtccacc 420
tcgtccaccg cgaccggtgg cagcggcagc gccgccagca tcatgccgcc tcaccagggg 480
cacgggggac agccgcagtt cctagcctcg ttgcaccacc ctctcgtcgg cggggaccac 540
tacagcaccg gcgcgtcaag attagggttc cccgggctga gctcgctgga ccccatggac 600
taccaccagt tcggcgccgg tgccggaggt gccatcgggc tggagcagtg gcgcctaccg 660
cagatacaac agttcccatt cctaagcagc cgccccgacg ccgtgcaacc gccaatgtct 720
ggcatttacc cgttcgaagt ggaaggccac ggcggggaag gttccggctt cgctggtcac 780
atgcttggtg accccaaggt gtccggctca gccggactga tcacgcagct ggcgtcagtg 840
aagatggagg acaacccaac gtccgcagcg atggcaagca gctcaccgag ggagttcctt 900
ggtctcccta ggaacctcca attatggggc gctagcggca acggcggtgc aagtggcaac 960
aatggaggag gcaccggcaa cgctgccggt ggcggcgaca ctattcctcc gggtagcagc 1020
tgggtagacc tgtcaggatt caactcgtct tcgtccggga acgtgctgtg a 1071

<210> 138
<211> 356
<212> PRT
<213> Zea mays

<400> 138
Met Pro Pro Ser Ala Pro Gly Gly Ala Leu Met Ala Pro Arg Arg Asp
1               5                   10                  15
Met Ala Ala Val Val Ala Ala Ser Gly Gly Ser Thr Ser Gly Gly Gly
            20                  25                  30
Gly Pro Thr Gly Gly Gly Ser Pro Ile Arg Pro Gly Ser Met Thr Glu
        35                  40                  45
Arg Ala Arg Leu Ala Lys Ile Pro Gln Pro Glu Pro Gly Leu Lys Cys
    50                  55                  60
Pro Arg Cys Glu Ser Thr Asn Thr Lys Phe Cys Tyr Phe Asn Asn Tyr
65                  70                  75                  80
Ser Leu Ser Gln Pro Arg His Phe Cys Lys Thr Cys Arg Arg Tyr Trp
                85                  90                  95
Thr Arg Gly Gly Thr Leu Arg Asn Val Pro Val Gly Gly Gly Cys Arg
            100                 105                 110
Arg Asn Lys Arg Thr Lys Ser Ser Lys Ser Asn Ser Ser Ser Ala Ala
        115                 120                 125
Ser Ala Ser Ser Ala Gly Gly Thr Ser Ser Ser Thr Ser Ser Thr Ala
    130                 135                 140
Thr Gly Gly Ser Gly Ser Ala Ala Ser Ile Met Pro Pro His Gln Gly
145                 150                 155                 160
His Gly Gly Gln Pro Gln Phe Leu Ala Ser Leu His His Pro Leu Val
                165                 170                 175
Gly Gly Asp His Tyr Ser Thr Gly Ala Ser Arg Leu Gly Phe Pro Gly
            180                 185                 190
Leu Ser Ser Leu Asp Pro Met Asp Tyr His Gln Phe Gly Ala Gly Ala
        195                 200                 205
Gly Gly Ala Ile Gly Leu Glu Gln Trp Arg Leu Pro Gln Ile Gln Gln
    210                 215                 220
Phe Pro Phe Leu Ser Ser Arg Pro Asp Ala Val Gln Pro Pro Met Ser
225                 230                 235                 240
Gly Ile Tyr Pro Phe Glu Val Glu Gly His Gly Gly Glu Gly Ser Gly
                245                 250                 255
Phe Ala Gly His Met Leu Gly Asp Pro Lys Val Ser Gly Ser Ala Gly
            260                 265                 270
Leu Ile Thr Gln Leu Ala Ser Val Lys Met Glu Asp Asn Pro Thr Ser
        275                 280                 285
Ala Ala Met Ala Ser Ser Ser Pro Arg Glu Phe Leu Gly Leu Pro Arg
    290                 295                 300
Asn Leu Gln Leu Trp Gly Ala Ser Gly Asn Gly Gly Ala Ser Gly Asn
305                 310                 315                 320
Asn Gly Gly Gly Thr Gly Asn Ala Ala Gly Gly Gly Asp Thr Ile Pro
                325                 330                 335
Pro Gly Ser Ser Trp Val Asp Leu Ser Gly Phe Asn Ser Ser Ser Ser
            340                 345                 350
Gly Asn Val Leu
        355

<210> 139
<211> 1382
<212> DNA
<213> Zea mays

<220>
<221> misc_feature
<222> 182, 183, 184
<223> n = A,T,C or G

<221> misc_feature
<222> 182, 183, 184

<223> n = A,T,C or G

<400> 139
cagcaacagc acgctcaaca tggccagcag cttccaagtg gcggcaacgg cggcggcggc 60
ggcggcatgg acacgcatgc gcatcatcat catcatcagc tgccgccggt gccacctcct 120
cctagcgggg ctctcatggc tcctcgccct gacatgtcgg ctatggtccc tgctgcgagc 180
gnnngtggtg gcgggccgac tagcggcggc accgctatcc gtccgggctc catgaccgag 240
cgggccaagc tcgccaagat cccgcagccg gagccggggc tcaagtgccc gcgctgcgag 300
tccaccaaca ccaagttctg ctacttcaac aactactcgc tctcccagcc gcgccacttc 360
tgcaagacgt gccgccggta ctggacgcgc ggcggcgcgc tccggaacgt ccccgtgggc 420
ggcgggtgcc gccgtaacaa gcgcaccaag tcgtccaagt ccaactcgtc atcggccgcc 480
gcctctgctt caggcggcgc gggcgggacg tcgtcgtcca cctcgtccac cgctaccggc 540
ggcagcagca gcgcgggcgc catcatgccg tctcatcagg ggcagctgca gccgttcctg 600
gcctcgttgc accaccctct tgccggcggc ggcggcggcg gggatcacta cagcaccggc 660
gcgtcaaggt tagggttccc aggactgagc tcgctggacc ccatggacta ccaccagttc 720
ggcgcgggcg cgggcgccgg cgccagcagc gctgccatcg ggctagagca gtggcgtctg 780
ccgcatatac agcagttccc cttcctaagc ggccgccccg accccgtgca accaacaatg 840
tctagcattt acccgttcga cttggaaggg cacggcgggg acgctcccgg cttccccggc 900
gggcacatgc taggtgcctc caaggtgccc ggctcagccg gactaatcac gcagctggca 960
tcggttaaga tggaggacaa cccagcgtct gcggcgatgg cgagcagctc gccgagggag 1020
ttccttagcc tccctgggaa cctccaattc tggggcggcg gcggcaacaa caacggcggt 1080
gcaagtggca acaatggagg aggcgctggc aacggcggtg gtggtggcgg cggtggcgct 1140
gtcgctccgg gcagcagctg ggtggacctg tcaggattca actcgtcttc gtctgggaac 1200
gtcctgtgac atgccctctg gtctgcatgc tagctgcatt aaaaaggtag ccctattatt 1260
tatgcaaagc cttgaatctt cattttagtc atggcttctg agaaagttca acagaaaaaa 1320
ggagttgtgg ttgcaagatt ataggggcta tatatatctg atagctatcg tctgcggtga 1380
cg 1382

<210> 140
<211> 1206
<212> DNA
<213> Zea mays

<400> 140
cagcaacagc acgctcaaca tggccagcag cttccaagtg gcggcaacgg cggcggcggc 60
ggcggcatgg acacgcatgc gcatcatcat catcatcagc tgccgccggt gccacctcct 120
cctagcgggg ctctcatggc tcctcgccct gacatgtcgg ctatggtccc tgctgcgagc 180
ggtggtggcg ggccgactag cggcggcacc gctatccgtc cgggctccat gaccgagcgg 240
gccaagctcg ccaagatccc gcagccggag ccggggctca agtgcccgcg ctgcgagtcc 300
accaacacca agttctgcta cttcaacaac tactcgctct cccagccgcg ccacttctgc 360
aagacgtgcc gccggtactg gacgcgcggc ggcgcgctcc ggaacgtccc cgtgggcggc 420
gggtgccgcc gtaacaagcg caccaagtcg tccaagtcca actcgtcatc ggccgccgcc 480
tctgcttcag gcggcgcggg cgggacgtcg tcgtccacct cgtccaccgc taccggcggc 540
agcagcagcg cgggcgccat catgccgtct catcaggggc agctgcagcc gttcctggcc 600
tcgttgcacc accctcttgc cggcggcggc ggcggcgggg atcactacag caccggcgcg 660
tcaaggttag ggttcccagg actgagctcg ctggacccca tggactacca ccagttcggc 720
gcgggcgcgg gcgccggcgc cagcagcgct gccatcgggc tagagcagtg gcgtctgccg 780
catatacagc agttcccctt cctaagcggc cgccccgacc ccgtgcaacc aacaatgtct 840
agcatttacc cgttcgactt ggaagggcac ggcggggacg ctcccggctt ccccggcggg 900
cacatgctag gtgcctccaa ggtgcccggc tcagccggac taatcacgca gctggcatcg 960
gttaagatgg aggacaaccc agcgtctgcg gcgatggcga gcagctcgcc gagggagttc 1020
cttagcctcc ctgggaacct ccaattctgg ggcggcggcg gcaacaacaa cggcggtgca 1080
agtggcaaca atggaggagg cgctggcaac ggcggtggtg gtggcggcgg tggcgctgtc 1140
gctccgggca gcagctgggt ggacctgtca ggattcaact cgtcttcgtc tgggaacgtc 1200
ctgtga 1206

<210> 141
<211> 402
<212> PRT
<213> Zea mays

<220>
<221> VARIANT
<222> 61, 62
<223> Xaa = Any Amino Acid

<221> VARIANT
<222> 61, 62
<223> Xaa = Any Amino Acid

<400> 141
Gln Gln Gln His Ala Gln His Gly Gln Gln Leu Pro Ser Gly Gly Asn
1               5                   10                  15
Gly Gly Gly Gly Gly Gly Met Asp Thr His Ala His His His His His
            20                  25                  30
Gln Leu Pro Pro Val Pro Pro Pro Pro Ser Gly Ala Leu Met Ala Pro
        35                  40                  45
Arg Pro Asp Met Ser Ala Met Val Pro Ala Ala Ser Xaa Xaa Gly Gly
    50                  55                  60
Gly Pro Thr Ser Gly Gly Thr Ala Ile Arg Pro Gly Ser Met Thr Glu
65                  70                  75                  80
Arg Ala Lys Leu Ala Lys Ile Pro Gln Pro Glu Pro Gly Leu Lys Cys
                85                  90                  95
Pro Arg Cys Glu Ser Thr Asn Thr Lys Phe Cys Tyr Phe Asn Asn Tyr
            100                 105                 110
Ser Leu Ser Gln Pro Arg His Phe Cys Lys Thr Cys Arg Arg Tyr Trp
        115                 120                 125
Thr Arg Gly Gly Ala Leu Arg Asn Val Pro Val Gly Gly Gly Cys Arg
    130                 135                 140
Arg Asn Lys Arg Thr Lys Ser Ser Lys Ser Asn Ser Ser Ser Ala Ala
145                 150                 155                 160
Ala Ser Ala Ser Gly Gly Ala Gly Gly Thr Ser Ser Ser Thr Ser Ser
                165                 170                 175
Thr Ala Thr Gly Gly Ser Ser Ser Ala Gly Ala Ile Met Pro Ser His
            180                 185                 190
Gln Gly Gln Leu Gln Pro Phe Leu Ala Ser Leu His His Pro Leu Ala
        195                 200                 205
Gly Gly Gly Gly Gly Gly Asp His Tyr Ser Thr Gly Ala Ser Arg Leu
    210                 215                 220
Gly Phe Pro Gly Leu Ser Ser Leu Asp Pro Met Asp Tyr His Gln Phe
225                 230                 235                 240
Gly Ala Gly Ala Gly Ala Gly Ala Ser Ser Ala Ala Ile Gly Leu Glu
                245                 250                 255
Gln Trp Arg Leu Pro His Ile Gln Gln Phe Pro Phe Leu Ser Gly Arg
            260                 265                 270
Pro Asp Pro Val Gln Pro Thr Met Ser Ser Ile Tyr Pro Phe Asp Leu
        275                 280                 285
Glu Gly His Gly Gly Asp Ala Pro Gly Phe Pro Gly Gly His Met Leu
    290                 295                 300
Gly Ala Ser Lys Val Pro Gly Ser Ala Gly Leu Ile Thr Gln Leu Ala
305                 310                 315                 320
Ser Val Lys Met Glu Asp Asn Pro Ala Ser Ala Ala Met Ala Ser Ser
                325                 330                 335
Ser Pro Arg Glu Phe Leu Ser Leu Pro Gly Asn Leu Gln Phe Trp Gly
            340                 345                 350
Gly Gly Gly Asn Asn Asn Gly Gly Ala Ser Gly Asn Asn Gly Gly Gly
        355                 360                 365
Ala Gly Asn Gly Gly Gly Gly Gly Gly Gly Gly Ala Val Ala Pro Gly
    370                 375                 380
Ser Ser Trp Val Asp Leu Ser Gly Phe Asn Ser Ser Ser Ser Gly Asn
385                 390                 395                 400
Val Leu

<210> 142
<211> 1075
<212> DNA
<213> Zea mays

<400> 142
tgccaccttc tgctcctggt ggggctctca tggctcctcg ccgtgacatg gcggctgtgg 60
tcgccgcgag cggtggcagc acgagcggcg gcggtgggcc gactggcggt ggtttcccta 120
tccgtccggg ctccatgacc gagcgggcta ggctcgccaa gatcccgcag ccagagccgg 180
ggctcaagtg cccgcgttgc gagtccacca acaccaagtt ttgctacttc aacaactact 240
cgctctccca gccgcgccac ttctgcaaga cgtgtcgccg gtactggacg cgcggcggca 300
cgctccggaa cgtccccgtt ggcggcgggt gccgccgcaa caagcgcacc aagtcgtcca 360
agtccaactc gtcgtcagcc gcctccgctt ccagcgcggg tgggacgtcg tcgtccacct 420
cgtccaccgc gaccggtggc agcggcagcg ccgccagcat catgccgcct caccaggggc 480
acgggggaca gccgcagttc ctagcctcgt tgcaccaccc tctcgtcggc ggggaccact 540
acagcaccgg cgcgtcaaga ttagggttcc ccgggctgag ctcgctggac cccatggact 600
accaccagtt cggcgccggt gccggaggtg ccatcgggct ggagcagtgg cgcctaccgc 660
agatacaaca gttcccattc ctaagcagcc gccccgacgc cgtgcaaccg ccaatgtctg 720
gcatttaccc gttcgaagtg gaaggccacg gcggggaagg ttccggcttc gctggtcaca 780
tgcttggtga ccccaaggtg tccggctcag ccggactgat cacgcagctg gcgtcagtga 840
agatggagga caacccaacg tccgcagcga tggcaagcag ctcaccgagg gagttccttg 900
ggtctcccta ggaacctccc aattatgggg cgctagcggc aacggcggtg caagtggcaa 960
caatggagga ggcaccggca acgctgccgg tggcggcgac actattcctc cgggkrstca 1020
agctggrtwg ayctgwcags akwsmacwmg kcttcgtccg ggaacgtgct ggacg 1075

<210> 143
<211> 909
<212> DNA
<213> Zea mays

<400> 143
ccaccttctg ctcctggtgg ggctctcatg gctcctcgcc gtgacatggc ggctgtggtc 60
gccgcgagcg gtggcagcac gagcggcggc ggtgggccga ctggcggtgg tttccctatc 120
cgtccgggct ccatgaccga gcgggctagg ctcgccaaga tcccgcagcc agagccgggg 180
ctcaagtgcc cgcgttgcga gtccaccaac accaagtttt gctacttcaa caactactcg 240
ctctcccagc cgcgccactt ctgcaagacg tgtcgccggt actggacgcg cggcggcacg 300
ctccggaacg tccccgttgg cggcgggtgc cgccgcaaca agcgcaccaa gtcgtccaag 360
tccaactcgt cgtcagccgc ctccgcttcc agcgcgggtg ggacgtcgtc gtccacctcg 420
tccaccgcga ccggtggcag cggcagcgcc gccagcatca tgccgcctca ccaggggcac 480
gggggacagc cgcagttcct agcctcgttg caccaccctc tcgtcggcgg ggaccactac 540
agcaccggcg cgtcaagatt agggttcccc gggctgagct cgctggaccc catggactac 600
caccagttcg gcgccggtgc cggaggtgcc atcgggctgg agcagtggcg cctaccgcag 660
atacaacagt tcccattcct aagcagccgc cccgacgccg tgcaaccgcc aatgtctggc 720
atttacccgt tcgaagtgga aggccacggc ggggaaggtt ccggcttcgc tggtcacatg 780
cttggtgacc ccaaggtgtc cggctcagcc ggactgatca cgcagctggc gtcagtgaag 840
atggaggaca acccaacgtc cgcagcgatg gcaagcagct caccgaggga gttccttggg 900
tctccctag 909

<210> 144
<211> 302
<212> PRT
<213> Zea mays

<400> 144
Pro Pro Ser Ala Pro Gly Gly Ala Leu Met Ala Pro Arg Arg Asp Met
1               5                   10                  15
Ala Ala Val Val Ala Ala Ser Gly Gly Ser Thr Ser Gly Gly Gly Gly
            20                  25                  30

Pro Thr Gly Gly Gly Phe Pro Ile Arg Pro Gly Ser Met Thr Glu Arg
        35                  40                  45
Ala Arg Leu Ala Lys Ile Pro Gln Pro Glu Pro Gly Leu Lys Cys Pro
    50                  55                  60
Arg Cys Glu Ser Thr Asn Thr Lys Phe Cys Tyr Phe Asn Asn Tyr Ser
65                  70                  75                  80
Leu Ser Gln Pro Arg His Phe Cys Lys Thr Cys Arg Arg Tyr Trp Thr
                85                  90                  95
Arg Gly Gly Thr Leu Arg Asn Val Pro Val Gly Gly Gly Cys Arg Arg
            100                 105                 110
Asn Lys Arg Thr Lys Ser Ser Lys Ser Asn Ser Ser Ser Ala Ala Ser
        115                 120                 125
Ala Ser Ser Ala Gly Gly Thr Ser Ser Ser Thr Ser Ser Thr Ala Thr
    130                 135                 140
Gly Gly Ser Gly Ser Ala Ala Ser Ile Met Pro Pro His Gln Gly His
145                 150                 155                 160
Gly Gly Gln Pro Gln Phe Leu Ala Ser Leu His His Pro Leu Val Gly
                165                 170                 175
Gly Asp His Tyr Ser Thr Gly Ala Ser Arg Leu Gly Phe Pro Gly Leu
            180                 185                 190
Ser Ser Leu Asp Pro Met Asp Tyr His Gln Phe Gly Ala Gly Ala Gly
        195                 200                 205
Gly Ala Ile Gly Leu Glu Gln Trp Arg Leu Pro Gln Ile Gln Gln Phe
    210                 215                 220
Pro Phe Leu Ser Ser Arg Pro Asp Ala Val Gln Pro Pro Met Ser Gly
225                 230                 235                 240
Ile Tyr Pro Phe Glu Val Glu Gly His Gly Gly Glu Gly Ser Gly Phe
                245                 250                 255
Ala Gly His Met Leu Gly Asp Pro Lys Val Ser Gly Ser Ala Gly Leu
            260                 265                 270
Ile Thr Gln Leu Ala Ser Val Lys Met Glu Asp Asn Pro Thr Ser Ala
        275                 280                 285
Ala Met Ala Ser Ser Ser Pro Arg Glu Phe Leu Gly Ser Pro
    290                 295                 300

<210> 145
<211> 40
<212> PRT
<213> Artificial Sequence

<220>
<223> DOF domain consensus sequence

<221> VARIANT
<222> 5, 7, 19, 20, 28, 35
<223> Xaa = Any Amino Acid

<221> VARIANT
<222> 5, 7, 19, 20, 28, 35
<223> Xaa = Any Amino Acid

<400> 145
Cys Pro Arg Cys Xaa Ser Xaa Asp Thr Lys Phe Cys Tyr Phe Asn Asn
 1               5                  10                  15
Tyr Asn Xaa Xaa Gln Pro Arg His Phe Cys Lys Xaa Cys Arg Arg Tyr
            20                  25                  30
Trp Thr Xaa Gly Gly Thr Leu Arg
        35                  40

<210> 146
<211> 792
<212> DNA
<213> Zea mays

<220>
<221> misc_feature
<222> 377
<223> n = A,T,C or G

<221> misc_feature
<222> 377
<223> n = A,T,C or G

<400> 146
tcttyctcct cctcctcctc ctccgccccc gccgcagcct ctgcccctgc agcaggcggc 60
cggggccggg ggcaccsgcg gcgagccgct gccgtgcccs cggtgcggca gccgggagac 120
caagttctgc tacttcaaca actacaacgt gcggcagccg cgccacctct gccgcgcctg 180
ccgccgctac tggacggccg gcggttgcst ccgccgcgtg gcctccgcgt ccccgggccg 240
ccgcaggccg cgcccgaacg ccccgatcgg ccgccgccgc cgccgcgccg tccgcctccg 300
catccgccag ggaggtgggc ggggagcgtt gactcgtcgg cggtcgtagg ggcggcgggt 360
ggcscggkgt tgtccgnchm ctttcgttcg cacggggccg ggggcttcct tcccgcgtga 420
cgccgtgacg tgatgccgcg cgcccgggcc ccaagcctca gtgacgggca cgggccggcc 480
ggttcgcggt ggggaccccg cgcctcgtcg cccttttcac acctactggt cctctccttt 540
tggtttgtta gattctgttt tctatatacc attttaggat tcttttggtg gccaaaaaag 600
atgctgccgg cctgctgcga gcgagagttc agtttggtag gccagtcaaa aagtggtagt 660
cgtactagag tagtagtagt agtagactgg tagtgctgct gctgaatgct gatgcaaaat 720
tcggcagcgg tgaattggtg cctggggtcg gtcaggtcag ttggcagatt tcagccaagc 780
catggtttgt tg 792

<210> 147
<211> 1070
<212> DNA
<213> Zea mays

<400> 147
tccctctgtc ggcagtctgc agcaggccag cgccgtcgcg catgcaggag gcgtcatcgg 60
cggcggcggg ggccgagccc ggccgtcggg cggcgcagca tcagttcgcc ggcgtggacc 120
tccggcggcc caaggggtac gcgccgccgg cgccggcggt gagcgagggg gacccgtgcc 180
cgcggtgtgc gtcgcgggac accaagttct gctactacaa caactacaac acctcccagc 240
cgcgccactt ctgcaagggc tgccgccgct actggaccaa gggcggcacg ctgcgcaacg 300
tccccgtcgg cggcggcacc cgcaagaagc cctccttgtc gtcgtcgtcg tcgtcgtcct 360
acgcggccgc cgcggacgcc gacaggcagc ccaagaagaa gcccgccagc aagaagcgcc 420
gcgtcgtggc gccggccccg gagctcgccg ccgcggccga cccaggcaag acggcaccca 480
ccacgacgac gacgacgacg acgacgagcg agatcaccac ggagactggc gcgctggagg 540
actccgactc cctggcgcac ctgctgctgc agcccgggac agaggacgcg gaggccgtcg 600
cgctcgggct cggcctctcc gacttcccct ccgccgggaa ggcggtgctg gacgacgagg 660
actcgttcgt gtggcccgcc gcgtcgttcg acatgggcgc gtgctgggcc ggcgcagggt 720
tcgccgaccc ggaccccgca tgcatcttcc tcaacctccc gtgacagcca cagacggtca 780
cggcgccgag gcgtacgtcc tcctgctttg ctctgctctg cgcctgatga tgagtgcaag 840
gaaatggagc gctttgattc ttctgctatt ggttgcatct gctaattgat ttagcagtgg 900
tggtaaggta cacgcacgac gagctgactg cgtggttctt ggcttgggcc ttagtatatg 960
atgatgatga tgatactagt tgtatgtgtg acgtgagaga cgatcgcgac gacgaccttg 1020
aattttagtt tctgagatga gaaaggtcaa ctagtggcct ctgtatgact 1070

<210> 148
<211> 1538
<212> DNA
<213> Zea mays

<400> 148

cctccgcctt ccttgtgttc cgcgcttcca caacagttta ccgcaaggaa ggaaccatgg 60
acatgaactc caacgccaac aacagcactg ccgcagcagc atcggctccc atcaacaacc 120
agcaggaggc tgtggtgtca tccccaacca gaaaggagca agccaggaac cccaagaagg 180
cgcgggcggc gccgcagcag gcgggcggca gcggggagcc taggccgcgg cctccgccgg 240
acgcggcgca cagctgcccg cgctgctcct ccaccaacac aaagttctgc tactacaaca 300
actacaacct gacgcagccg cgctacttct gcaagacgtg ccgccgctac tggacacacg 360
gcggcaccct ccgcaacgtc cccgtcggcg gcggctgccg caggaacaag cgcgcctcca 420
gctcctcgtc cccgtttccg ggcccctcca gcaccgccgc caccagcgcc gcgatggaga 480
agaccgtcag cacgcggctg atgctgatgg cgaccagcac catggcgatg ccctccccga 540
cggcaggcct gtttgttccc gatgacatgt ccccggcatt cacgccgacg acgggcggta 600
gcggcttcga cgacctcgcc ggcatggacg agcagcacca gcagggcttc ctgcccttct 660
cgccgctgtc cctgtccgac caggcgccgg agctggctcc tggaggaggg ggtgacacga 720
cgccgtcttt cctggacatg ctgacaggag ggtatctcga tggcggcggc tacggcggca 780
tgagcggtgg cagcgatgcg atggacatgc cgttctcgct gcctgagatg gggcccccga 840
caactgatcc aatgccgttt cagctccagt ggacgtcatc agagcttgac aactacatca 900
acgacgacgg tggttatgca gcaggaccag ccgccggagt gcagcagcag cagcagcagc 960
agcagcagca gattaatggt ggtgatcacc agaagcagga cgagaacaaa gaggcgggga 1020
acggcaaagg caacgacgac ggcggcggcg ggtcgtcgtc ggtgtacagc ttctggatga 1080
acaccagcgg cagcgacggg gcagaggggt agtgcgccac tgccagtgcc agccacagga 1140
ggaggcgaaa gtgctgctgc aaactccctc gcatcatctg gtcggtgcaa gtgcagcaga 1200
catccttcgt cgacgcaatc aaatcatcaa aaggtggcaa cagggagtga aggggggaag 1260
aagttcccca tccagcgtag aggatagggt gcatgttcat gtttgattta tctgcattgt 1320
ggtcggtcgt ggttgcaagt catcttgccc ccccctttgt tttggttcag tttgcatgcg 1380
ttcgttcgtc agagttttac ttgcagcagt ctttggatcg ccggcagaga ttagttaggt 1440
atttatgctt tgttttcgtc agttcgttct ggataatgtc ttaataattc ctcagtttat 1500
ttctttgtta aaaaaaaaa aaaaaaaaa aaaaaaa 1538

<210> 149
<211> 1443
<212> DNA
<213> Zea mays

<400> 149
atgcacgagg cagcatcggc tcccatcaac aaccagcagg aggctgtggt gtcatcccca 60
accagaaagg agcaagccag gaaccccaag aaggcgcggg cggcgccgca gcaggcgggc 120
ggcagcgggg agcctaggcc gcggcctccg ccggacgcgg cgcacagctg cccgcgctgc 180
tcctccacca acacaaagtt ctgctactac aacaactaca acctgacgca gccgcgctac 240
ttctgcaaga cgtgccgccg ctactggaca cacggcggca ccctccgcaa cgtccccgtc 300
ggcggcggct gccgcaggaa caagcgcgcc tccagctcct cgtccccgtt tccgggcccc 360
tccagcaccg ccgccaccag cgccgcgatg gagaagaccg tcagcacgcg gctgatgctg 420
atggcgacca gcaccatggc gatgccctcc ccgacggcag gcctgtttgt tcccgatgac 480
atgtccccgg cattcacgcc gacgacgggc ggtagcggct tcgacgacct cgccggcatg 540
gacgagcagc accagcaggg cttcctgccc ttctcgccgc tgtccctgtc cgaccaggcg 600
ccggagctgg ctcctggagg agggggtgac acgacgccgt ctttcctgga catgctgaca 660
ggagggtatc tcgatggcgg cggctacggc ggcatgagcg gtggcagcga tgcgatggac 720
atgccgttct cgctgcctga gatgggcccc ccgacaactg atccaatgcc gtttcagctc 780
cagtggacgt catcagagct tgacaactac atcaacgacg acggtggtta tgcagcagga 840
ccagccgccg gagtgcagca gcagcagcag cagcagcagc agcagattaa tggtggtgat 900
caccagaagc aggacgagaa caaagaggcg gggaacggca aaggcaacga cgacggcggc 960
ggcgggtcgt cgtcggtgta cagcttctgg atgaacacca gcggcagcga cggggcagag 1020
gggtagtgcg ccactgccag tgccagccac aggaggaggc gaaagtgctg ctgcaaactc 1080
cctcgcatca tctggtcggt gcaagtgcag cagacatcct tcgtcgacgc aatcaaatca 1140
tcaaaggtgg caacagggga gtgaaagggg gaagaagttc accatccagc gtagaggata 1200
gggtgcatgt tcatgtttga tttatctgca ttgtggtcgg tcgtggttgc aagtcatctt 1260
gcaccaccct ttgttttggt tcagtttgca tgcgttcgtt cgtcagagtt ttacttgcag 1320
cagtctttgg atcgccggca gagattagtt aggtatctat gctttgtttt cgtcagttcg 1380
ttctggataa tgtcttaata attcctcagt ttatttcttt gttaactaaa aaaaaaaaaa 1440
aaa 1443

<210> 150
<211> 1158

<212> DNA
<213> Zea mays

<400> 150
atggtcttcc cttcaccttc agtgcaggtc tacctagatc cacatccacc taattggaat 60
aaccagcagc aaggtcagca gccgagggcg aatgggggag ctgatgcacc gttgctgccc 120
gtgggtccgg cggcagctac atcggcggct ccggagcaag gtgggttgcc tagaagctca 180
tcaatggcta atgccgccgt ggctgcgcac gcgaggccca actcaatggc ggagcgcgcg 240
cggctggcgc ggatgccgca tccggagcca gcgctcaagt gcccgcgttg cgaatccacc 300
aacaccaagt tctgctacta caacaactac tccctctccc agccccgcca cttctgcaag 360
acgtgccgcc gctactggac gcgcggcgga tcccttcgca acgtccccgt aggtggaggc 420
tgccgtcgca acaagcgttc gtccaagtcc tcatcctcct ctgctgggtc ttcctcctca 480
aagatgtctc cctcaggaag gctactgggt ggtccatcag ctacaccgtc caccacccca 540
ggcactaccg gtgcgatcat tactccgggt ctcagctctt tctctcacca cttgccgttc 600
ttgggctcca tgcacccgtc agggcccaac ctaggtctag ctttctccgc cggactgccg 660
ctagtcggca tgcagcacct ggacacggtg gatcagtttc cggtggcaag cggcggcggc 720
accaccatag gtgcatctct agagcagtgg agagtgcgac agcagcagca gcagttccca 780
ttcatgactg ggggaatact ggatctgtcg cagccgccga cgtaccaatt cggtttggaa 840
gctaaccgag gaggcagcgg ctcagctgcg gtggcgttca attcaggaca gaccacgacg 900
accagtgcta ccacgggaag gcaggaaggg tcatcaaaga agatggggga tagtaaagga 960
gaagatatga gcttacagaa gcagtacatg gtacctctac gccacggatc aggatcacac 1020
ggtgtctggg atgggagtgc tggtggaact ggcagcaacg gtggtggtac tggcaatggc 1080
ggttcaagtt ggccaatgaa catgattcct ggattccatt cttcgtccac tagcggttgc 1140
aatgacagtg gcttgtag 1158

<210> 151
<211> 1392
<212> DNA
<213> Zea mays

<400> 151
atgatccaag aactgctcgg cggggccgcc atggaccagc tcaagagcgt gaatgagtcc 60
ctgcccctgt tgctgcactc ggtcatctcc aacccatcgc ccacgtcgtc gtcgtcgacg 120
tcgtcgtcgc gctcgtctgc gcagcagcat cagcagcagc ggtcgacgtc ggcaacgtcg 180
tcgccgcaag cagggcagca gcagcagcag cagggccagg ggcaaggagc ggagcagacg 240
cctctgcggt gccccaggtg taactcctcc aacaccaagt tctgctacta caacaactac 300
aacctcaccc agccgcgcca cttctgcaag acgtgccgcc ggtactggac caagggcggc 360
gcgctccgca acgtgcccat cggcggcggc tgccgcaagc cgcgccccat gcccacgccc 420
gtcacgaagc cggcggtctc atgcaaggcc gtgggcggcg cgcagtcgct gggcctcggc 480
gtcggcttgg gcatgggcgc cggccccgga ccctgggcgt cctcgcagca ggcggccgcc 540
gcgcagctca tggcgctgct caacagcgcc aggagcgtgc agggaggcgg cggcggcaac 600
atgcacaggc tcctcggcct cgacgccgtg gcccacctgc ccctccatgt cctgccgggc 660
gcgggcaata atgccggtgg cacggcgccg tcgttctggc cgcaggccgc gccgcgtgtc 720
atccccgcac cgccgcacat ggactcccag ctcggcatgg ggccgctggg ccagcacgac 780
gtgctgtcga gcctcgggct aaagctgccc ccgccatcgc cgtcgccggc ggcaagctac 840
tacagcgacc agctgcacgc ggtggtgagc agcgccgccg gacgcggaca cgagtacgaa 900
acagccgcct gcgccacgtc actgccttgc accacggcgc tgacctccct cccagctcgg 960
catggggccg ctgggccagc acgacgtgct gtcgagcctc gggctaaagc tgcccccgcc 1020
atcgccgtcg ccggcggcaa gctactacag cgaccagctg cacgcggtgg tgagcagcgc 1080
cgccggacgc ggacacgagt acgaaacagc cgcctgcgcc acgtcactgc cttgcaccac 1140
ggcgctgacc tccctcccgc cggccgcgtc gagcgtgtcc gctgcactgg ccagcgccgc 1200
cacggtcggg ctagacctcc cgctggtctc cctctccgcg cccgagatgc agtactgggc 1260
cgggccggcg gcgatgtccg tggcgtggcc ggacttgccc acccccaacg gcgcgttccc 1320
gtgagagaca aacatggacc tcatctgctg tacggatggc gcagctgttt acccacccac 1380
atgacacatt ga 1392

<210> 152
<211> 618
<212> DNA
<213> Zea mays

<400> 152
atggcgcctg cagcttcgat cctctcggtc accgccgtcg ccggttccaa gcgtccggcc 60
gcttcagacg ctgagctccc gcttctcggc ctcgactcct cctcgctcca ccagcagcag 120
ggtgacaagg ctgggcgcaa gggccaggac caggaccacc agcagcagct ggagtgcccg 180
cgctgccgct ccaccaacac caagttctgc tactacaaca actacagcac ggcgcagccg 240
cggcacttct gccgcgcgtg ccgccgctac tggacgcacg gcggcacgct gcgcgacgtc 300
ccggttggcg gggcctcgcg ccgcgccggt aggggcggca agcggcgcag ggtctcctcc 360
gccgagacct cctcgtcgtc gtcgccgccg ccgatgcctg cgtcgctcgc ggacgcgtgc 420
ctgtccgacc tcccgtccgt cttcccgttc ctcagcgacg gcagcttctt cccgcagctc 480
gacctcggcg ccgtcgtgct tgcaccgccg gccttctcct cctcgtggcg gtcggtggcc 540
ccggacttct acgacgggct cgcgccgtgg ggcgacatcg ccggcctcga cctcagctgg 600
acaccaccgg ggaactag 618

<210> 153
<211> 618
<212> DNA
<213> Zea mays

<400> 153
atggcgcctg cagtttccat cctctcggcc accgcctccg ccaagcgaaa gcgccccgcc 60
acttccgacg ctgatgagct cccgcacgac gactcctccg cgccccacca gcaggtgcag 120
ggccagggcc agcaaccgcg gcagcagcag cagcttgagt gcccgcgctg ccgctccacc 180
aacaccaagt tctgctacta caacaactac agcacggcgc agccgcgcca cttctgccgc 240
gcgtgccgcc gctactggac gcacgggggc acgctgcgcg acgtccccgt cggcggcgcc 300
tcgcgccgcg ccgccactgg cggcggcggc ggcaagcggc gcagggtctc cgccgagccc 360
tcatccccgc cgccgtcggt cgcggacgcg tgcctgccgt ccgccttccc gttcctcagc 420
gacggcagct tcttcccgca gctcgacctc gtcggcggcg ttgcgcttgc acccccggcc 480
ttctcctcct cgtggcagtc ggtgctggtc ccggacttgt acgacgggct cgcgccgtgg 540
gacgacggag caacggcggc cgctctagag gatccaagct tacgtacgcg tgcatgcgac 600
gtcatagctc ttctatag 618

<210> 154
<211> 240
<212> PRT
<213> Zea mays

<400> 154
Met Gln Glu Ala Ser Ser Ala Ala Ala Gly Ala Glu Pro Gly Arg Arg
1               5                   10                  15
Ala Ala Gln His Gln Phe Ala Gly Val Asp Leu Arg Arg Pro Lys Gly
            20                  25                  30
Tyr Ala Pro Pro Ala Pro Ala Val Ser Glu Gly Asp Pro Cys Pro Arg
        35                  40                  45
Cys Ala Ser Arg Asp Thr Lys Phe Cys Tyr Tyr Asn Asn Tyr Asn Thr
    50                  55                  60
Ser Gln Pro Arg His Phe Cys Lys Gly Cys Arg Arg Tyr Trp Thr Lys
65                  70                  75                  80
Gly Gly Thr Leu Arg Asn Val Pro Val Gly Gly Gly Thr Arg Lys Lys
                85                  90                  95
Pro Ser Leu Ser Ser Ser Ser Ser Ser Ser Tyr Ala Ala Ala Ala Asp
            100                 105                 110
Ala Asp Arg Gln Pro Lys Lys Lys Pro Ala Ser Lys Lys Arg Arg Val
        115                 120                 125
Val Ala Pro Ala Pro Glu Leu Ala Ala Ala Ala Asp Pro Gly Lys Thr
    130                 135                 140
Ala Pro Thr Thr Thr Thr Thr Thr Thr Thr Thr Ser Glu Ile Thr Thr
145                 150                 155                 160
Glu Thr Gly Ala Leu Glu Asp Ser Asp Ser Leu Ala His Leu Leu Leu
                165                 170                 175
Gln Pro Gly Thr Glu Asp Ala Glu Ala Val Ala Leu Gly Leu Gly Leu
            180                 185                 190

Ser Asp Phe Pro Ser Ala Gly Lys Ala Val Leu Asp Asp Glu Asp Ser
        195                 200                 205
Phe Val Trp Pro Ala Ala Ser Phe Asp Met Gly Ala Cys Trp Ala Gly
    210                 215                 220
Ala Gly Phe Ala Asp Pro Asp Pro Ala Cys Ile Phe Leu Asn Leu Pro
225                 230                 235                 240

<210> 155
<211> 351
<212> PRT
<213> Zea mays

<400> 155
Met Asp Met Asn Ser Asn Ala Asn Asn Ser Thr Ala Ala Ala Ala Ser
1               5                   10                  15
Ala Pro Ile Asn Asn Gln Gln Glu Ala Val Val Ser Ser Pro Thr Arg
            20                  25                  30
Lys Glu Gln Ala Arg Asn Pro Lys Lys Ala Arg Ala Ala Pro Gln Gln
        35                  40                  45
Ala Gly Gly Ser Gly Glu Pro Arg Pro Arg Pro Pro Pro Asp Ala Ala
    50                  55                  60
His Ser Cys Pro Arg Cys Ser Ser Thr Asn Thr Lys Phe Cys Tyr Tyr
65                  70                  75                  80
Asn Asn Tyr Asn Leu Thr Gln Pro Arg Tyr Phe Cys Lys Thr Cys Arg
                85                  90                  95
Arg Tyr Trp Thr His Gly Gly Thr Leu Arg Asn Val Pro Val Gly Gly
            100                 105                 110
Gly Cys Arg Arg Asn Lys Arg Ala Ser Ser Ser Ser Ser Pro Phe Pro
        115                 120                 125
Gly Pro Ser Ser Thr Ala Ala Thr Ser Ala Ala Met Glu Lys Thr Val
    130                 135                 140
Ser Thr Arg Leu Met Leu Met Ala Thr Ser Thr Met Ala Met Pro Ser
145                 150                 155                 160
Pro Thr Ala Gly Leu Phe Val Pro Asp Asp Met Ser Pro Ala Phe Thr
                165                 170                 175
Pro Thr Thr Gly Gly Ser Gly Phe Asp Asp Leu Ala Gly Met Asp Glu
            180                 185                 190
Gln His Gln Gln Gly Phe Leu Pro Phe Ser Pro Leu Ser Leu Ser Asp
        195                 200                 205
Gln Ala Pro Glu Leu Ala Pro Gly Gly Gly Gly Asp Thr Thr Pro Ser
    210                 215                 220
Phe Leu Asp Met Leu Thr Gly Gly Tyr Leu Asp Gly Gly Gly Tyr Gly
225                 230                 235                 240
Gly Met Ser Gly Gly Ser Asp Ala Met Asp Met Pro Phe Ser Leu Pro
                245                 250                 255
Glu Met Gly Pro Pro Thr Thr Asp Pro Met Pro Phe Gln Leu Gln Trp
            260                 265                 270
Thr Ser Ser Glu Leu Asp Asn Tyr Ile Asn Asp Asp Gly Gly Tyr Ala
        275                 280                 285
Ala Gly Pro Ala Ala Gly Val Gln Gln Gln Gln Gln Gln Gln Gln Gln
    290                 295                 300
Gln Ile Asn Gly Gly Asp His Gln Lys Gln Asp Glu Asn Lys Glu Ala
305                 310                 315                 320
Gly Asn Gly Lys Gly Asn Asp Asp Gly Gly Gly Gly Ser Ser Ser Val
                325                 330                 335
Tyr Ser Phe Trp Met Asn Thr Ser Gly Ser Asp Gly Ala Glu Gly
            340                 345                 350

<210> 156

<211> 341
<212> PRT
<213> Zea mays

<400> 156
Met His Glu Ala Ala Ser Ala Pro Ile Asn Asn Gln Gln Glu Ala Val
1               5                   10                  15
Val Ser Ser Pro Thr Arg Lys Glu Gln Ala Arg Asn Pro Lys Lys Ala
            20                  25                  30
Arg Ala Ala Pro Gln Gln Ala Gly Gly Ser Gly Glu Pro Arg Pro Arg
        35                  40                  45
Pro Pro Pro Asp Ala Ala His Ser Cys Pro Arg Cys Ser Ser Thr Asn
    50                  55                  60
Thr Lys Phe Cys Tyr Tyr Asn Asn Tyr Asn Leu Thr Gln Pro Arg Tyr
65                  70                  75                  80
Phe Cys Lys Thr Cys Arg Arg Tyr Trp Thr His Gly Gly Thr Leu Arg
                85                  90                  95
Asn Val Pro Val Gly Gly Gly Cys Arg Arg Asn Lys Arg Ala Ser Ser
            100                 105                 110
Ser Ser Ser Pro Phe Pro Gly Pro Ser Ser Thr Ala Ala Thr Ser Ala
        115                 120                 125
Ala Met Glu Lys Thr Val Ser Thr Arg Leu Met Leu Met Ala Thr Ser
    130                 135                 140
Thr Met Ala Met Pro Ser Pro Thr Ala Gly Leu Phe Val Pro Asp Asp
145                 150                 155                 160
Met Ser Pro Ala Phe Thr Pro Thr Thr Gly Gly Ser Gly Phe Asp Asp
                165                 170                 175
Leu Ala Gly Met Asp Glu Gln His Gln Gln Gly Phe Leu Pro Phe Ser
            180                 185                 190
Pro Leu Ser Leu Ser Asp Gln Ala Pro Glu Leu Ala Pro Gly Gly Gly
        195                 200                 205
Gly Asp Thr Thr Pro Ser Phe Leu Asp Met Leu Thr Gly Gly Tyr Leu
    210                 215                 220
Asp Gly Gly Gly Tyr Gly Gly Met Ser Gly Gly Ser Asp Ala Met Asp
225                 230                 235                 240
Met Pro Phe Ser Leu Pro Glu Met Gly Pro Pro Thr Thr Asp Pro Met
                245                 250                 255
Pro Phe Gln Leu Gln Trp Thr Ser Ser Glu Leu Asp Asn Tyr Ile Asn
            260                 265                 270
Asp Asp Gly Gly Tyr Ala Ala Gly Pro Ala Ala Gly Val Gln Gln Gln
        275                 280                 285
Gln Gln Gln Gln Gln Gln Gln Ile Asn Gly Gly Asp His Gln Lys Gln
    290                 295                 300
Asp Glu Asn Lys Glu Ala Gly Asn Gly Lys Gly Asn Asp Asp Gly Gly
305                 310                 315                 320
Gly Gly Ser Ser Ser Val Tyr Ser Phe Trp Met Asn Thr Ser Gly Ser
                325                 330                 335
Asp Gly Ala Glu Gly
            340


<210> 157
<211> 385
<212> PRT
<213> Zea mays

<400> 157
Met Val Phe Pro Ser Pro Ser Val Gln Val Tyr Leu Asp Pro His Pro
1               5                   10                  15
Pro Asn Trp Asn Asn Gln Gln Gln Gly Gln Gln Pro Arg Ala Asn Gly
            20                  25                  30

Gly Ala Asp Ala Pro Leu Leu Pro Val Gly Pro Ala Ala Ala Thr Ser
        35                  40                  45
Ala Ala Pro Glu Gln Gly Gly Leu Pro Arg Ser Ser Ser Met Ala Asn
    50                  55                  60
Ala Ala Val Ala Ala His Ala Arg Pro Asn Ser Met Ala Glu Arg Ala
65                  70                  75                  80
Arg Leu Ala Arg Met Pro His Pro Glu Pro Ala Leu Lys Cys Pro Arg
                85                  90                  95
Cys Glu Ser Thr Asn Thr Lys Phe Cys Tyr Tyr Asn Asn Tyr Ser Leu
            100                 105                 110
Ser Gln Pro Arg His Phe Cys Lys Thr Cys Arg Arg Tyr Trp Thr Arg
        115                 120                 125
Gly Gly Ser Leu Arg Asn Val Pro Val Gly Gly Gly Cys Arg Arg Asn
    130                 135                 140
Lys Arg Ser Ser Lys Ser Ser Ser Ser Ser Ala Gly Ser Ser Ser Ser
145                 150                 155                 160
Lys Met Ser Pro Ser Gly Arg Leu Leu Gly Gly Pro Ser Ala Thr Pro
                165                 170                 175
Ser Thr Thr Pro Gly Thr Thr Gly Ala Ile Ile Thr Pro Gly Leu Ser
            180                 185                 190
Ser Phe Ser His His Leu Pro Phe Leu Gly Ser Met His Pro Ser Gly
        195                 200                 205
Pro Asn Leu Gly Leu Ala Phe Ser Ala Gly Leu Pro Leu Val Gly Met
    210                 215                 220
Gln His Leu Asp Thr Val Asp Gln Phe Pro Val Ala Ser Gly Gly Gly
225                 230                 235                 240
Thr Thr Ile Gly Ala Ser Leu Glu Gln Trp Arg Val Arg Gln Gln Gln
                245                 250                 255
Gln Gln Phe Pro Phe Met Thr Gly Gly Ile Leu Asp Leu Ser Gln Pro
            260                 265                 270
Pro Thr Tyr Gln Phe Gly Leu Glu Ala Asn Arg Gly Gly Ser Gly Ser
        275                 280                 285
Ala Ala Val Ala Phe Asn Ser Gly Gln Thr Thr Thr Thr Ser Ala Thr
    290                 295                 300
Thr Gly Arg Gln Glu Gly Ser Ser Lys Lys Met Gly Asp Ser Lys Gly
305                 310                 315                 320
Glu Asp Met Ser Leu Gln Lys Gln Tyr Met Val Pro Leu Arg His Gly
                325                 330                 335
Ser Gly Ser His Gly Val Trp Asp Gly Ser Ala Gly Gly Thr Gly Ser
            340                 345                 350
Asn Gly Gly Gly Thr Gly Asn Gly Gly Ser Ser Trp Pro Met Asn Met
        355                 360                 365
Ile Pro Gly Phe His Ser Ser Ser Thr Ser Gly Cys Asn Asp Ser Gly
    370                 375                 380
Leu
385

<210> 158
<211> 463
<212> PRT
<213> Zea mays

<400> 158
Met Ile Gln Glu Leu Leu Gly Gly Ala Ala Met Asp Gln Leu Lys Ser
1               5                   10                  15
Val Asn Glu Ser Leu Pro Leu Leu Leu His Ser Val Ile Ser Asn Pro
            20                  25                  30
Ser Pro Thr Ser Ser Ser Ser Thr Ser Ser Ser Arg Ser Ser Ala Gln
        35                  40                  45

Gln His Gln Gln Gln Arg Ser Thr Ser Ala Thr Ser Ser Pro Gln Ala
    50                  55                  60
Gly Gln Gln Gln Gln Gln Gln Gly Gln Gly Gln Gly Ala Glu Gln Thr
65                  70                  75                  80
Pro Leu Arg Cys Pro Arg Cys Asn Ser Ser Asn Thr Lys Phe Cys Tyr
                85                  90                  95
Tyr Asn Asn Tyr Asn Leu Thr Gln Pro Arg His Phe Cys Lys Thr Cys
            100                 105                 110
Arg Arg Tyr Trp Thr Lys Gly Gly Ala Leu Arg Asn Val Pro Ile Gly
        115                 120                 125
Gly Gly Cys Arg Lys Pro Arg Pro Met Pro Thr Pro Val Thr Lys Pro
    130                 135                 140
Ala Val Ser Cys Lys Ala Val Gly Gly Ala Gln Ser Leu Gly Leu Gly
145                 150                 155                 160
Val Gly Leu Gly Met Gly Ala Gly Pro Gly Pro Trp Ala Ser Ser Gln
                165                 170                 175
Gln Ala Ala Ala Ala Gln Leu Met Ala Leu Leu Asn Ser Ala Arg Ser
            180                 185                 190
Val Gln Gly Gly Gly Gly Gly Asn Met His Arg Leu Leu Gly Leu Asp
        195                 200                 205
Ala Val Ala His Leu Pro Leu His Val Leu Pro Gly Ala Gly Asn Asn
    210                 215                 220
Ala Gly Gly Thr Ala Pro Ser Phe Trp Pro Gln Ala Ala Pro Arg Val
225                 230                 235                 240
Ile Pro Ala Pro Pro His Met Asp Ser Gln Leu Gly Met Gly Pro Leu
                245                 250                 255
Gly Gln His Asp Val Leu Ser Ser Leu Gly Leu Lys Leu Pro Pro Pro
            260                 265                 270
Ser Pro Ser Pro Ala Ala Ser Tyr Tyr Ser Asp Gln Leu His Ala Val
        275                 280                 285
Val Ser Ser Ala Ala Gly Arg Gly His Glu Tyr Glu Thr Ala Ala Cys
    290                 295                 300
Ala Thr Ser Leu Pro Cys Thr Thr Ala Leu Thr Ser Leu Pro Ala Arg
305                 310                 315                 320
His Gly Ala Ala Gly Pro Ala Arg Arg Ala Val Glu Pro Arg Ala Lys
                325                 330                 335
Ala Ala Pro Ala Ile Ala Val Ala Gly Gly Lys Leu Leu Gln Arg Pro
            340                 345                 350
Ala Ala Arg Gly Gly Glu Gln Arg Arg Arg Thr Arg Thr Arg Val Arg
        355                 360                 365
Asn Ser Arg Leu Arg His Val Thr Ala Leu His His Gly Ala Asp Leu
    370                 375                 380
Pro Pro Ala Gly Arg Val Glu Arg Val Arg Cys Thr Gly Gln Arg Arg
385                 390                 395                 400
His Gly Arg Ala Arg Pro Pro Ala Gly Leu Pro Leu Arg Ala Arg Asp
                405                 410                 415
Ala Val Leu Gly Arg Ala Gly Gly Asp Val Arg Gly Val Ala Gly Leu
            420                 425                 430
Ala His Pro Gln Arg Arg Val Pro Val Arg Asp Lys His Gly Pro His
        435                 440                 445
Leu Leu Tyr Gly Trp Arg Ser Cys Leu Pro Thr His Met Thr His
    450                 455                 460

<210> 159
<211> 205
<212> PRT
<213> Zea mays

<400> 159
Met Ala Pro Ala Ala Ser Ile Leu Ser Val Thr Ala Val Ala Gly Ser
1               5                   10                  15
Lys Arg Pro Ala Ala Ser Asp Ala Glu Leu Pro Leu Leu Gly Leu Asp
            20                  25                  30
Ser Ser Ser Leu His Gln Gln Gln Gly Asp Lys Ala Gly Arg Lys Gly
        35                  40                  45
Gln Asp Gln Asp His Gln Gln Gln Leu Glu Cys Pro Arg Cys Arg Ser
    50                  55                  60
Thr Asn Thr Lys Phe Cys Tyr Tyr Asn Asn Tyr Ser Thr Ala Gln Pro
65                  70                  75                  80
Arg His Phe Cys Arg Ala Cys Arg Arg Tyr Trp Thr His Gly Gly Thr
                85                  90                  95
Leu Arg Asp Val Pro Val Gly Gly Ala Ser Arg Arg Ala Gly Arg Gly
            100                 105                 110
Gly Lys Arg Arg Arg Val Ser Ser Ala Glu Thr Ser Ser Ser Ser Ser
        115                 120                 125
Pro Pro Pro Met Pro Ala Ser Leu Ala Asp Ala Cys Leu Ser Asp Leu
    130                 135                 140
Pro Ser Val Phe Pro Phe Leu Ser Asp Gly Ser Phe Phe Pro Gln Leu
145                 150                 155                 160
Asp Leu Gly Ala Val Val Leu Ala Pro Pro Ala Phe Ser Ser Ser Trp
                165                 170                 175
Arg Ser Val Ala Pro Asp Phe Tyr Asp Gly Leu Ala Pro Trp Gly Asp
            180                 185                 190
Ile Ala Gly Leu Asp Leu Ser Trp Thr Pro Pro Gly Asn
        195                 200                 205

<210> 160
<211> 205
<212> PRT
<213> Zea mays

<400> 160
Met Ala Pro Ala Val Ser Ile Leu Ser Ala Thr Ala Ser Ala Lys Arg
1               5                   10                  15
Lys Arg Pro Ala Thr Ser Asp Ala Asp Glu Leu Pro His Asp Asp Ser
            20                  25                  30
Ser Ala Pro His Gln Gln Val Gln Gly Gln Gly Gln Gln Pro Arg Gln
        35                  40                  45
Gln Gln Gln Leu Glu Cys Pro Arg Cys Arg Ser Thr Asn Thr Lys Phe
    50                  55                  60
Cys Tyr Tyr Asn Asn Tyr Ser Thr Ala Gln Pro Arg His Phe Cys Arg
65                  70                  75                  80
Ala Cys Arg Arg Tyr Trp Thr His Gly Gly Thr Leu Arg Asp Val Pro
                85                  90                  95
Val Gly Gly Ala Ser Arg Arg Ala Ala Thr Gly Gly Gly Gly Gly Lys
            100                 105                 110
Arg Arg Arg Val Ser Ala Glu Pro Ser Ser Pro Pro Pro Ser Val Ala
        115                 120                 125
Asp Ala Cys Leu Pro Ser Ala Phe Pro Phe Leu Ser Asp Gly Ser Phe
    130                 135                 140
Phe Pro Gln Leu Asp Leu Val Gly Gly Val Ala Leu Ala Pro Pro Ala
145                 150                 155                 160
Phe Ser Ser Ser Trp Gln Ser Val Leu Val Pro Asp Leu Tyr Asp Gly
                165                 170                 175
Leu Ala Pro Trp Asp Asp Gly Ala Thr Ala Ala Ala Leu Glu Asp Pro
            180                 185                 190
Ser Leu Arg Thr Arg Ala Cys Asp Val Ile Ala Leu Leu
        195                 200                 205